# EUROPEAN PATENT SPECIFICATION

(11) **EP 3 262 189 B1**
(45) Date of publication and mention of the grant of the patent: **08.12.2021**
(21) Application number: 16710551.9
(22) Date of filing: 26.02.2016
(51) Int. Cl.: C12Q 1/6844, C12Q 1/6869, C12Q 1/6806

(54) **METHODS FOR BARCODING NUCLEIC ACIDS FOR SEQUENCING**
VERFAHREN ZUR STRICHCODIERUNG VON NUKLEINSÄUREN ZUR SEQUENZIERUNG
PROCÉDÉS POUR LE MARQUAGE D'ACIDES NUCLÉIQUES AU MOYEN DE CODES À BARRES EN VUE DU SÉQUENÇAGE

(30) Priority: 27.02.2015 US 201562126397 P; 05.03.2015 US 201562128849 P; 13.05.2015 US 201562160976 P; 10.06.2015 US 201562173899 P; 17.07.2015 US 201562194075 P
(43) Date of publication of application: 03.01.2018
(73) Proprietor: Becton, Dickinson and Company, Franklin Lakes, NJ 07417 (US)
(72) Inventor: FU, Glenn, Menlo Park, CA 94025 (US); WILHELMY, Julie, Menlo Park, CA 94025 (US); SIMBIRSKY, Maria, Menlo Park, CA 94025 (US); SHUM, Eleen, Menlo Park, CA 94025 (US)
(74) Representative: Carpmaels & Ransford LLP
(86) International application number: PCT/US2016/019971
(87) International publication number: WO 2016/138500

(56) References cited:
- WO-A1-2013/188831
- WO-A1-2014/144495
- WO-A1-2015/031691
- WO-A1-2015/103339
- WO-A2-2013/173394
- WO-A2-2014/015084
- C. B. JABARA ET AL: "Accurate sampling and deep sequencing of the HIV-1 protease gene using a Primer ID", PROCEEDINGS OF THE NATIONAL ACADEMY OF SCIENCES, vol. 108, no. 50, 30 November 2011 (2011-11-30), pages 20166-20171, XP055161674, ISSN: 0027-8424, DOI: 10.1073/pnas.1110064108

## Description

### FIELD

Embodiments in the following relate to embodiments of the disclosure which are not necessarily embodiments of the invention. Embodiments provided herein relate to methods for labeling a nucleic acid in a sample with a stochastic barcode. Some embodiments relate to methods for characterizing a sample by identifying the TCR alpha chain or beta chain of a T cell.

### BACKGROUND

Methods and compositions for labeling nucleic acid molecules for amplification or sequencing have been developed. Stochastic counting on nucleic acid targets is an important quantification method. However, a drawback to previous work has been that regents designed for particular targets would be expensive to design for use in stochastic labeling. Herein described are methods and compositions for labeling targets for stochastic counting.

WO 2013/188831 A1 describes uniquely tagged rearranged adaptive immune receptor genes in a complex gene set. WO 2014/144495 A1 describes single cell bar-coding for antibody discovery. WO 2014/015084 A2 describes system and methods for detecting genetic variation. WO 2015/031691 A1 describes massively parallel single cell analysis. WO 2015/103339 A1 describes analysis of nucleic acids associated with single cells using nucleic acid barcodes.

### SUMMARY

The invention provides a method of labeling a target nucleic acid in a sample, comprising: hybridizing a target with a first oligonucleotide comprising a universal adaptor sequence and a target-specific sequence; extending said first oligonucleotide to generate a first nucleotide sequence; hybridizing said first nucleotide sequence with a second oligonucleotide; extending said second oligonucleotide to generate a second nucleotide sequence; hybridizing said second nucleotide sequence with a third oligonucleotide comprising a binding site for a first amplification primer and the universal adaptor sequence, wherein the third oligonucleotide comprises a stochastic barcode, wherein the stochastic barcode comprises a molecular label; extending said third oligonucleotide to generate a third nucleotide sequence; and amplifying said third nucleotide sequence using a first amplification primer to generate a plurality of a fourth nucleotide sequences, wherein each of the plurality of the fourth nucleotide sequences comprises said target and the stochastic barcode, wherein the target is an mRNA molecule; and wherein the molecular label comprises a nucleic acid sequence that provides a counter for the specific occurrence of the target nucleic acid.

The invention also provides a method of labeling a target in a sample for quantification of said target, comprising: hybridizing said target with a first oligonucleotide comprising a first universal adaptor sequence and a target-specific sequence; extending said first oligonucleotide to generate a first nucleotide sequence; hybridizing said first nucleotide sequence with a second oligonucleotide comprising a second universal adaptor sequence and a second target-specific sequence; extending said second oligonucleotide to generate a second nucleotide sequence; hybridizing said second nucleotide sequence with a third oligonucleotide comprising a binding site for a first amplification primer and the first universal adaptor sequence; extending said third oligonucleotide to generate a third nucleotide sequence; hybridizing said third nucleotide sequence with a fourth oligonucleotide comprising a binding site for a second amplification primer and the second universal adaptor sequence, wherein one or both of the third oligonucleotide and the fourth oligonucleotide comprises part of a stochastic barcode, wherein the stochastic barcode comprises a molecular label; extending said fourth oligonucleotide to generate a fourth nucleotide sequence; and amplifying said fourth nucleotide sequence using a first amplification primer and a second amplification primer to generate a plurality of a fifth nucleotide sequences, wherein each of the plurality of the fifth nucleotide sequences comprises said target and the stochastic barcode, wherein the target is an mRNA molecule; and wherein the molecular label comprises a nucleic acid sequence that provides a counter for the specific occurrence of the target nucleic acid.

The invention also provides a method of labeling a target in a sample for quantification of said target, comprising: hybridizing said target with a first oligonucleotide comprising a target-specific sequence and a binding site for a first amplification primer; extending said first oligonucleotide to generate a first nucleotide sequence; hybridizing said first nucleotide sequence with a second oligonucleotide comprising a universal adaptor sequence and a second target-specific sequence; extending said second oligonucleotide to generate a second nucleotide sequence; hybridizing said second nucleotide sequence with a third oligonucleotide comprising a binding site for a second amplification primer and a sequence that specifically binds the universal adaptor sequence, wherein the third oligonucleotide comprises a stochastic barcode, wherein the stochastic barcode comprises a molecular label; extending said third oligonucleotide to generate a third nucleotide sequence; and amplifying said third nucleotide sequence using a first amplification primer and a second amplification primer to generate a plurality of a fourth nucleotide sequences, wherein each of the plurality of the fourth nucleotide sequences comprises said target and the stochastic barcode, wherein the target is an mRNA molecule, and wherein the molecular label comprises a nucleic acid sequence that provides a counter for the specific occurrence of the target nucleic acid.

The invention also provides a method of labeling a target in a sample for quantification of said target, comprising: hybridizing said target with a first oligonucleotide comprising a target-specific sequence and a first amplification primer sequence in the presence of a second oligonucleotide comprising a universal adaptor sequence; extending said first oligonucleotide to generate a first nucleotide sequence comprising a compliment of the universal adaptor sequence; hybridizing said first nucleotide sequence with a third oligonucleotide comprising the universal adaptor sequence, a stochastic barcode, wherein the stochastic barcode comprises a molecular label, and a binding site for a second amplification primer; extending said third oligonucleotide to generate a second nucleotide sequence; and amplifying said second nucleotide sequence using a first amplification primer and a second amplification primer to generate a plurality of a third nucleotide sequences, wherein each of the plurality of the third nucleotide sequences comprises said target and the stochastic barcode, wherein the target is an mRNA molecule; and wherein the molecular label comprises a nucleic acid sequence that provides a counter for the specific occurrence of the target nucleic acid.

The invention also provides a method of labeling a target in a sample for quantification of said target, comprising: hybridizing said target with a first oligonucleotide comprising a universal adaptor sequence and a target-specific sequence; extending said first oligonucleotide to generate a first nucleotide sequence; hybridizing said first nucleotide sequence with a second oligonucleotide comprising a second target-specific sequence and a third oligonucleotide comprising a stochastic barcode, wherein the stochastic barcode comprises a molecular label, and a sequence that specifically binds the universal adaptor sequence; amplifying said first nucleotide sequence to generate a plurality of a second nucleotide sequences, wherein each of the plurality of the second nucleotide sequences comprises said target and said stochastic barcode, wherein the target is an mRNA molecule; and wherein the molecular label comprises a nucleic acid sequence that provides a counter for the specific occurrence of the target nucleic acid.

In one aspect the disclosure provides methods of labeling a nucleic acid in a sample, comprising: hybridizing a target with a first oligonucleotide comprising a universal adaptor sequence and a target-specific sequence; extending said first oligonucleotide to generate a first nucleotide sequence; hybridizing said first nucleotide sequence with a second oligonucleotide; extending said second oligonucleotide to generate a second nucleotide sequence; hybridizing said second nucleotide sequence with a third oligonucleotide comprising a binding site for a first amplification primer and the universal adaptor sequence, wherein one or both of the first oligonucleotide and the third oligonucleotide comprises part of a stochastic barcode; extending said third oligonucleotide to generate a third nucleotide sequence; and amplifying said third nucleotide sequence using a first amplification primer to generate a plurality of a fourth nucleotide sequences, wherein each of the plurality of the fourth nucleotide sequences comprises said target and the stochastic barcode. In some embodiments, the sample is a single cell. In some embodiments, the target is an mRNA molecule. In some embodiments, the mRNA molecule encodes a T cell receptor (TCR) alpha chain or beta chain. In some embodiments, the target-specific sequence specifically binds to the C region of the TCR alpha chain or beta chain. In some embodiments, the methods comprise amplifying said third nucleotide sequence using a second amplification primer. In some embodiments, the second amplification primer specifically binds to a region selected from the group consisting of: a V region of a TCR alpha chain or beta chain, the junction of a V and D region of a TCR alpha chain or beta chain, or any combination thereof. In some embodiments, the second oligonucleotide specifically binds to a region selected from the group consisting of: a V region of a TCR alpha chain or beta chain, the junction of a V and D region of a TCR alpha chain or beta chain, or any combination thereof. In some embodiments, the second oligonucleotide comprises a binding site for a second amplification primer. In some embodiments, the methods comprise amplifying said third nucleotide sequence using the second amplification primer. In some embodiments, the first oligonucleotide comprises the stochastic barcode. In some embodiments, the third oligonucleotide comprises the stochastic barcode. In some embodiments, each of the first oligonucleotide and the third oligonucleotide comprises part of the stochastic barcode. In some embodiments, the stochastic barcode comprises a molecular label. In some embodiments, the stochastic barcode comprises a universal label, a dimension label, a cellular label, or a combination thereof. In some embodiments, the first oligonucleotide or the third oligonucleotide is attached to a solid support. In some embodiments, the sample comprises a plurality of the target. In some embodiments, each of the plurality of the target is labeled with a distinct stochastic barcode. In some embodiments, the methods comprise counting the number of the plurality of the target by counting the distinct stochastic barcodes associated with the target.

In one aspect the disclosure provides methods of labeling a target in a sample for quantification of said target, comprising: hybridizing said target with a first oligonucleotide comprising a first universal adaptor sequence and a target-specific sequence; extending said first oligonucleotide to generate a first nucleotide sequence; hybridizing said first nucleotide sequence with a second oligonucleotide comprising a second universal adaptor sequence and a second target-specific sequence; extending said second oligonucleotide to generate a second nucleotide sequence; hybridizing said second nucleotide sequence with a third oligonucleotide comprising a binding site for a first amplification primer and the first universal adaptor sequence; extending said third oligonucleotide to generate a third nucleotide sequence; hybridizing said third nucleotide sequence with a fourth oligonucleotide comprising a binding site for a second amplification primer and the second universal adaptor sequence, wherein one or both of the third oligonucleotide and the fourth oligonucleotide comprises part of a stochastic barcode; extending said fourth oligonucleotide to generate a fourth nucleotide sequence; and amplifying said fourth nucleotide sequence using a first amplification primer and a second amplification primer to generate a plurality of a fifth nucleotide sequences, wherein each of the plurality of the fifth nucleotide sequences comprises said target and the stochastic barcode. In some embodiments, the sample is a single cell. In some embodiments, the target is an mRNA molecule. In some embodiments, the mRNA molecule encodes a T cell receptor (TCR) alpha chain or beta chain. In some embodiments, the target-specific sequence specifically binds to the C region of the TCR alpha chain or beta chain. In some embodiments, the second target-specific sequence specifically binds to a region selected from the group consisting of: a V region of a TCR alpha chain or beta chain, the junction of a V and D region of a TCR alpha chain or beta chain, or any combination thereof. In some embodiments, the third oligonucleotide comprises the stochastic barcode. In some embodiments, the fourth oligonucleotide comprises the stochastic barcode. In some embodiments, each of the third oligonucleotide and the fourth oligonucleotide comprises part of the stochastic barcode. In some embodiments, the stochastic barcode comprises a molecular label. In some embodiments, the stochastic barcode comprises a universal label, a dimension label, a cellular label, or a combination thereof.

In one aspect the disclosure provides methods of labeling a target in a sample for quantification of said target, comprising: hybridizing said target with a first oligonucleotide comprising a target-specific sequence and a binding site for a first amplification primer; extending said first oligonucleotide to generate a first nucleotide sequence; hybridizing said first nucleotide sequence with a second oligonucleotide comprising a universal adaptor sequence and a second target-specific sequence; extending said second oligonucleotide to generate a second nucleotide sequence; hybridizing said second nucleotide sequence with a third oligonucleotide comprising a binding site for a second amplification primer and a sequence that specifically binds the universal adaptor sequence, wherein one or both of the second oligonucleotide and the third oligonucleotide comprises part of a stochastic barcode; extending said third oligonucleotide to generate a third nucleotide sequence; and amplifying said third nucleotide sequence using a first amplification primer and a second amplification primer to generate a plurality of a fourth nucleotide sequences, wherein each of the plurality of the fourth nucleotide sequences comprises said target and the stochastic barcode. In some embodiments, the sample is a single cell. In some embodiments, the target is an mRNA molecule. In some embodiments, the mRNA molecule encodes a T cell receptor (TCR) alpha chain or beta chain. In some embodiments, the target-specific sequence specifically binds to the C region of the TCR alpha chain or beta chain. In some embodiments, the second target-specific sequence specifically binds to a region selected from the group consisting of: a V region of a TCR alpha chain or beta chain, the junction of a V and D region of a TCR alpha chain or beta chain, or any combination thereof. In some embodiments, the third oligonucleotide comprises the stochastic barcode. In some embodiments, the stochastic barcode comprises a molecular label. In some embodiments, the stochastic barcode comprises a universal label, a dimension label, a cellular label, or a combination thereof.

In one aspect the disclosure provides methods of labeling a target in a sample for quantification of said target, comprising: hybridizing said target with a first oligonucleotide comprising a target-specific sequence and a first amplification primer sequence in the presence of a second oligonucleotide comprising a universal adaptor sequence; extending said first oligonucleotide to generate a first nucleotide sequence comprising a compliment of the universal adaptor sequence; hybridizing said first nucleotide sequence with a third oligonucleotide comprising the universal adaptor sequence, a stochastic barcode, and a binding site for a second amplification primer; extending said third oligonucleotide to generate a second nucleotide sequence; and amplifying said second nucleotide sequence using a first amplification primer and a second amplification primer to generate a plurality of a third nucleotide sequences, wherein each of the plurality of the third nucleotide sequences comprises said target and the stochastic barcode. In some embodiments, the methods comprise extending the first oligonucleotide to generate a first nucleotide sequence comprising a compliment of the universal adaptor sequence by template switching. In some embodiments, the stochastic barcode comprises a molecular label. In some embodiments, the stochastic barcode comprises a universal label, a dimension label, a cellular label, or a combination thereof.

In one aspect the disclosure provides methods of labeling a target in a sample for quantification of said target, comprising: hybridizing said target with a first oligonucleotide comprising a universal adaptor sequence and a target-specific sequence; extending said first oligonucleotide to generate a first nucleotide sequence; hybridizing said first nucleotide sequence with a second oligonucleotide comprising a second target-specific sequence and a third oligonucleotide comprising a stochastic barcode and a sequence that specifically binds the universal adaptor sequence; amplifying said first nucleotide sequence to generate a plurality of a second nucleotide sequences, wherein each of the plurality of the second nucleotide sequences comprises said target and said stochastic barcode. In some embodiments, the sample is a single cell. In some embodiments, the target is an mRNA molecule. In some embodiments, the mRNA molecule encodes a T cell receptor (TCR) alpha chain or beta chain. In some embodiments, the target-specific sequence specifically binds to the C region of the TCR alpha chain or beta chain. In some embodiments, the methods comprise amplifying said third nucleotide sequence using a second amplification primer. In some embodiments, the second amplification primer specifically binds to a region selected from the group consisting of: a V region of a TCR alpha chain or beta chain, the junction of a V and D region of a TCR alpha chain or beta chain, or any combination thereof. In some embodiments, the second oligonucleotide specifically binds to a region selected from the group consisting of: a V region of a TCR alpha chain or beta chain, the junction of a V and D region of a TCR alpha chain or beta chain, or any combination thereof. In some embodiments, the second oligonucleotide comprises a binding site for a second amplification primer. In some embodiments, the methods comprise amplifying said third nucleotide sequence using the second amplification primer. In some embodiments, the first oligonucleotide comprises the stochastic barcode. In some embodiments, the third oligonucleotide comprises the stochastic barcode. In some embodiments, each of the first oligonucleotide and the third oligonucleotide comprises part of the stochastic barcode. In some embodiments, the stochastic barcode comprises a molecular label. In some embodiments, the stochastic barcode comprises a universal label, a dimension label, a cellular label, or a combination thereof. In some embodiments, the first oligonucleotide or the third oligonucleotide is attached to a solid support. In some embodiments, the sample comprises a plurality of the target. In some embodiments, each of the plurality of the target is labeled with a distinct stochastic barcode. In some embodiments, the methods comprise counting the number of the plurality of the target by counting the distinct stochastic barcodes associated with the target.

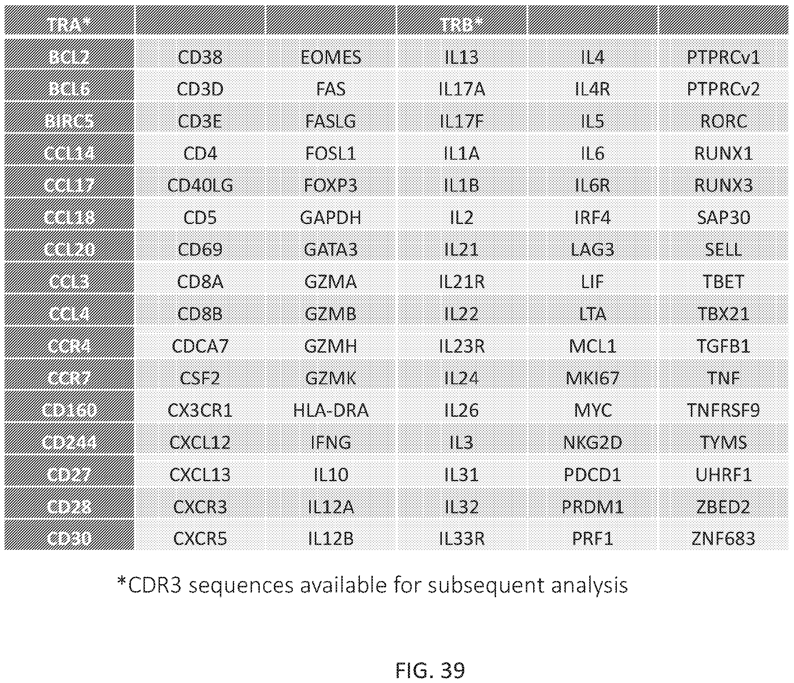

In one aspect the disclosure provides methods of characterizing a sample, comprising: hybridizing a first target in the sample with a first oligonucleotide comprising a first target-specific sequence and a first stochastic barcode, wherein the first target is an mRNA encoding a TCR alpha chain or beta chain; hybridizing a second target in the sample with a second oligonucleotide comprising a second target-specific sequence and a second stochastic barcode; extending the first oligonucleotide and the second oligonucleotide to incorporate the first stochastic barcode into the first target and the second stochastic barcode into the second target; and identifying the sequences of the first target and the second target. In some embodiments, the sample is a single cell. In some embodiments, the first target-specific sequence specifically binds to the C region of the TCR alpha chain or beta chain. In some embodiments, the extending comprises amplifying the first target with a forward primer. In some embodiments, the forward primer specifically binds to a region selected from the group consisting of: a V region of a TCR alpha chain or beta chain, the junction of a V and D region of a TCR alpha chain or beta chain, or any combination thereof. In some embodiments, the second target is selected from the group consisting of the genes listed in Figure 39. In some embodiments, the first stochastic barcode or the second stochastic barcode comprises one or more of a molecular label, a universal label, a dimension label, and a cellular label. In some embodiments, the first stochastic barcode and the second stochastic barcode comprises the same cellular label. In some embodiments, the sample comprises a plurality of the first target or the second target. In some embodiments, each of the plurality of the first target or the second target is labeled with a distinct stochastic barcode. In some embodiments, the methods comprise counting the number of the plurality of the first target or the second target by counting the distinct stochastic barcodes associated with the first target or the second target.

In one aspect the disclosure provides methods of labeling a target in a sample for quantification of said target, comprising: hybridizing said target with a first oligonucleotide comprising a universal adaptor sequence and a target-specific sequence; extending said first oligonucleotide to generate a first nucleotide sequence; hybridizing said first nucleotide sequence with a second oligonucleotide comprising a second target-specific sequence; extending said second oligonucleotide to generate a complementary strand of the first nucleotide sequence and a double-stranded nucleotide sequence; ligating a double-stranded adaptor oligonucleotide comprising a binding site for a first amplification primer to said double-stranded nucleotide sequence to form a ligated nucleotide sequence; amplifying said ligated nucleotide sequence using a first amplification primer and a second amplification primer comprising a stochastic barcode to generate a plurality of a second nucleotide sequence, wherein each of the plurality of the second nucleotide sequence comprises said target and said stochastic barcode. In some embodiments, the sample is a single cell. In some embodiments, the target is an mRNA molecule. In some embodiments, the mRNA molecule encodes a T cell receptor (TCR) alpha chain or beta chain. In some embodiments, the target-specific sequence specifically binds to the C region of the TCR alpha chain or beta chain. In some embodiments, the methods comprise amplifying said third nucleotide sequence using a second amplification primer. In some embodiments, the second amplification primer specifically binds to a region selected from the group consisting of: a V region of a TCR alpha chain or beta chain, the junction of a V and D region of a TCR alpha chain or beta chain, or any combination thereof. In some embodiments, the second oligonucleotide specifically binds to a region selected from the group consisting of: a V region of a TCR alpha chain or beta chain, the junction of a V and D region of a TCR alpha chain or beta chain, or any combination thereof. In some embodiments, the second oligonucleotide comprises a binding site for a second amplification primer. In some embodiments, the methods comprise amplifying said third nucleotide sequence using the second amplification primer. In some embodiments, the first oligonucleotide comprises the stochastic barcode. In some embodiments, the third oligonucleotide comprises the stochastic barcode. In some embodiments, each of the first oligonucleotide and the third oligonucleotide comprises part of the stochastic barcode. In some embodiments, the stochastic barcode comprises a molecular label. In some embodiments, the stochastic barcode comprises a universal label, a dimension label, a cellular label, or a combination thereof. In some embodiments, the first oligonucleotide or the third oligonucleotide is attached to a solid support. In some embodiments, the sample comprises a plurality of the target. In some embodiments, each of the plurality of the target is labeled with a distinct stochastic barcode. In some embodiments, the methods comprise counting the number of the plurality of the target by counting the distinct stochastic barcodes associated with the target.

In one aspect the disclosure provides kits comprising: a first oligonucleotide comprising a universal adaptor sequence and a target-specific sequence; and a second oligonucleotide comprising a stochastic barcode and the universal adaptor sequence or the complement thereof. In some embodiments, the first oligonucleotide or the second oligonucleotide is attached to a solid support. In some embodiments, the target-specific sequence specifically binds to the C region of a TCR alpha chain or beta chain. In some embodiments, the kits comprise a forward primer, wherein the forward primer specifically binds to a region selected from the group consisting of: a V region of a TCR alpha chain or beta chain, the junction of a V and D region of a TCR alpha chain or beta chain, or any combination thereof. In some embodiments, the stochastic barcode comprises one or more of a molecular label, a universal label, a dimension label, and a cellular label.

In one aspect the disclosure provides for a method comprising: contacting one or more receptor target mRNAs and cell-specific target mRNA in a cell with a reverse transcription primer comprising a stochastic barcode; reverse transcribing the one or more receptor target mRNAs and the cell-specific target mRNA to generate receptor target cDNA and cell-specific target cDNA; determining the sequences of the receptor target cDNA and the cell-specific target cDNA, and their corresponding stochastic barcodes; counting the number of the receptor target cDNA and the cell-specific target, thereby characterizing the cell and receptor. In some embodiments, the receptor target mRNAs are selected from the group consisting of one or more TCR alpha chains, one or more TCR beta chains, or any combination thereof. In some embodiments, the cell-specific target mRNA is a gene indicative of a type of immune cell. In some embodiments, the cell-specific target mRNA is selected from a target mRNA listed in Figure 39. In some embodiments, the reverse transcribing comprises primer extension of the stochastic barcode along the receptor target mRNA and the cell-specific target mRNA. In some embodiments, reverse transcription primer binds to a C region of a TCR alpha or beta chain and one of the two or more reverse transcription primers binds to a cell-specific target. In some embodiments, the method further comprises amplifying the cDNA thereby generating cDNA amplicons prior to the determining. In some embodiments, the amplifying comprises amplifying with a first set of forward primers. In some embodiments, the first set of forward primers comprises primers that bind to one or more alpha and beta chains of the receptor. In some embodiments, the first set of forward primers comprises primers that bind to T cell specific targets. In some embodiments, the first set of forward primers comprises primers that bind to a region selected from the group consisting of: a V region of a TCR alpha or beta chain and the junction of a V and D region of a TCR alpha or beta chain, or any combination thereof. In some embodiments, the method further comprises amplifying the cDNA amplicons with a second set of forward primers. In some embodiments, the second set of forward primers bind to a region of the receptor that is 3' of the binding site of the first set of forward primers. In some embodiments, the determining comprises determining the sequence amplicons of the receptor target cDNA and the cell-specific target cDNA. In some embodiments, the characterizing comprises determining which allele of which chain of the receptor is functionally active. In some embodiments, the characterizing comprises determining which allele of which chain of the receptor is more highly expressed compared to other alleles of the receptor. In some embodiments, the characterizing comprises determining the phenotype of the cell. In some embodiments, the characterizing comprises determining the functionality of the cell. In some embodiments, the cell is an immune cell. In some embodiments, the cell is a T cell.

In one aspect the disclosure provides for a kit comprising: a plate, wherein each well of the plate comprises two or more different reverse transcription primers that bind to different sequences, wherein the two or more reverse transcription primers comprise a stochastic barcode; a plurality of gene-specific forward primers; and instructions for use. In some embodiments, the two or more reverse transcription primers is three reverse transcription primers. In some embodiments, the two or more reverse transcription primers binds to a target selected from the group consisting of: one or more T cell marker gene, one or more TCR alpha chain, one or more TCR beta chain, or any combination thereof. In some embodiments, the two or more reverse transcription primers bind to a C region of a TCR alpha or beta chain. In some embodiments, one of the two or more reverse transcription primers binds to a C region of a TCR alpha or beta chain and one of the two or more reverse transcription primers binds to a cell-specific target. In some embodiments, the gene-specific primers bind to T cell specific targets. In some embodiments, the gene-specific primers bind to a region selected from the group consisting of: a V region of a TCR alpha or beta chain and the junction of a V and D region of a TCR alpha or beta chain, or any combination thereof. In some embodiments, the kit further comprises sequencing primers. In some embodiments, the kit further comprises a second plurality of gene-specific primers that are nested primers of the plurality of gene-specific primers. In some embodiments, the kit further comprises software. In some embodiments, the software is used for counting the number of molecules of a target that is reverse transcribed by the one or more stochastic barcodes. In some embodiments, the software is used for identifying the sequences of a target that is reverse transcribed by the one or more stochastic barcodes. In some embodiments, the stochastic barcode comprises a gene-specific region, a molecular label, a sample label, or any combination thereof.

In one aspect the disclosure provides for a method of estimating the number of one or more targets in a sample using a universal adaptor primer comprising: contacting the one or more targets with an oligonucleotide comprising a universal adaptor primer; hybridizing the oligonucleotide with a stochastic barcode; amplifying the one or more targets, thereby incorporating the oligonucleotide and the stochastic barcode into a sequence of the one or more targets; and estimating the number of the one or more targets using the stochastic barcode. In some embodiments, the oligonucleotide further comprises a gene-specific region. In some embodiments, the stochastic barcode comprises a sequence adapted to hybridize to the universal primer adaptor. In some embodiments, the contacting comprises hybridizing the oligonucleotide with the one or more targets. In some embodiments, the contacting comprises extending the oligonucleotide to incorporate the sequence of the one or more targets, thereby generating a first transcript. In some embodiments, the extending is performed with primer extension. In some embodiments, the extending is performed with reverse transcription. In some embodiments, in the contacting further comprises a second extension. In some embodiments, the second extension comprises hybridizing a reverse gene-specific primer to the first transcript. In some embodiments, the second extension comprises hybridizing to the first transcript a primer selected from group consisting of a universal primer, an oligo dT primer, and a random hexamer. In some embodiments, the hybridizing comprises hybridizing the universal primer adaptor sequence to the sequence adapted to hybridize to the universal primer adaptor sequence. In some embodiments, the hybridizing further comprises extending the stochastic barcode to incorporate the sequence of the first transcript. In some embodiments, the amplifying is performed with a universal primer and a gene-specific primer. In some embodiments, the amplifying is performed with a universal primer and a second universal primer. In some embodiments, the second universal primer further comprises a gene-specific region. In some embodiments, the amplifying is performed once. In some embodiments, the amplifying is performed twice. In some embodiments, the stochastic barcode comprises a molecular label, a cellular label, a sample label, and a universal label, or any combination thereof. In some embodiments, the molecular label is 3' of any other label. In some embodiments, the molecular label is 5' of any other label. In some embodiments, the universal primer adaptor sequence is from 5-30 nucleotides in length. In some embodiments, the estimating further comprises determining the sequence of a molecular label of the gene-specific stochastic barcode. In some embodiments, the method further comprises counting occurrences of distinct sequences of the molecular label. In some embodiments, the counting is used to estimate the number of one or more targets. In some embodiments, the determining comprises sequencing the gene-specific stochastic barcode. In some embodiments, the sequencing comprises sequencing with read lengths of at least 100 bases. In some embodiments, the sequencing comprises sequencing with read lengths of at least 500 bases. In some embodiments, the stochastic barcode comprises a molecular label, a cellular label, a sample label, a spatial label, a dimension label, and a universal label, or any combination thereof. In some embodiments, molecular labels on different stochastic barcodes are different. In some embodiments, the different stochastic barcodes comprise the same sample label, cellular label, or both sample label and cellular label. In some embodiments, the sample comprises a single cell. In some embodiments, the sample comprises a plurality of cells. In some embodiments, the plurality of cells comprises a one or more different cell types. In some embodiments, the one or more cell types are selected from the group consisting of: brain cells, heart cells, cancer cells, circulating tumor cells, organ cells, epithelial cells, metastatic cells, benign cells, primary cells, and circulatory cells, or any combination thereof. In some embodiments, the sample comprises a solid tissue. In some embodiments, the sample is obtained from a subject. In some embodiments, the subject is a subject selected from the group consisting of: a human, a mammal, a dog, a rat, a mouse, a fish, a fly, a worm, a plant, a fungi, a bacterium, a virus, a vertebrate, and an invertebrate. In some embodiments, the one or more targets are ribonucleic acid molecules. In some embodiments, the ribonucleic acid molecules are selected from the group consisting of: mRNA, microRNA, mRNA degradation products, and ribonucleic acids comprising a poly-A tail, or any combination thereof. In some embodiments, the targets are deoxyribonucleic acid molecules.

In one aspect the disclosure provides for a method for estimating the number of one or more targets in a sample using a universal primer adaptor and a stochastic barcode comprising: contacting a first oligonucleotide comprising a sequence complementary to a universal primer adaptor sequence and a gene-specific sequence to a second oligonucleotide comprising a sequence encoding the stochastic barcode and sequence encoding the universal primer adaptor sequence; incorporating the gene-specific sequence into the stochastic barcode sequence, thereby generating a gene-specific stochastic barcode; associating the gene-specific stochastic barcode to the one or more targets; and estimating the number of the one or more targets using the gene-specific stochastic barcode. In some embodiments, the contacting comprises hybridizing the universal primer adaptor sequence with the sequence complementary to the universal primer adaptor sequence. In some embodiments, the universal primer adaptor sequence is from 5-30 nucleotides in length. In some embodiments, the gene-specific sequence is from 5-30 nucleotides in length. In some embodiments, the incorporating comprises primer extension of the first oligonucleotide. In some embodiments, the incorporating comprises primer extension of the second oligonucleotide. In some embodiments, the incorporating comprises reverse transcription of the first oligonucleotide. In some embodiments, the incorporating comprises reverse transcription of the second oligonucleotide. In some embodiments, the associating comprises hybridizing the gene-specific stochastic barcode to the one or more targets. In some embodiments, the associating is performed such that each of the one or more targets associates with a unique gene-specific stochastic barcode. In some embodiments, the associating is performed such that at least 95% of the one or more targets associates with a unique gene-specific stochastic barcode. In some embodiments, the estimating comprises generating a target-barcode molecule. In some embodiments, the target-barcode molecule comprises the sequence of a gene-specific stochastic barcode to which it is associated. In some embodiments, the estimating further comprises determining the sequence of a molecular label of the gene-specific stochastic barcode. In some embodiments, the method further comprises counting occurrences of distinct sequences of the molecular label. In some embodiments, the counting is used to estimate the number of one or more targets. In some embodiments, the determining comprises sequencing the gene-specific stochastic barcode. In some embodiments, the sequencing comprises sequencing with read lengths of at least 100 bases. In some embodiments, the sequencing comprises sequencing with read lengths of at least 500 bases. In some embodiments, a first oligonucleotide is attached to a solid support. In some embodiments, the solid supports comprise a bead. In some embodiments, the bead is selected from the group consisting of: silica gel bead, controlled pore glass bead, magnetic bead, Dynabead, Sephadex/Sepharose beads, cellulose beads, and polystyrene beads, or any combination thereof. In some embodiments, the bead comprises a magnetic bead. In some embodiments, the solid support is semi-solid. In some embodiments, the solid support comprises a polymer, a matrix, or a hydrogel. In some embodiments, the solid support comprises a needle array device. In some embodiments, the solid support comprises an antibody. In some embodiments, the solid support comprise a plurality of the stochastic barcodes. In some embodiments, each stochastic barcode of the plurality of stochastic barcodes comprises a molecular label. In some embodiments, molecular labels on different solid supports differ by at least one nucleotide. In some embodiments, the stochastic barcode further comprises a universal label, a sample, label, and a cellular label, or any combination thereof. In some embodiments, the universal label, the sample label, and the cellular label are the same for all stochastic barcodes on the solid support. In some embodiments, the sample comprises a single cell. In some embodiments, the sample comprises a plurality of cells. In some embodiments, the plurality of cells comprises a one or more different cell types. In some embodiments, the one or more cell types are selected from the group consisting of: brain cells, heart cells, cancer cells, circulating tumor cells, organ cells, epithelial cells, metastatic cells, benign cells, primary cells, and circulatory cells, or any combination thereof. In some embodiments, the sample comprises a solid tissue. In some embodiments, the sample is obtained from a subject. In some embodiments, the subject is a subject selected from the group consisting of: a human, a mammal, a dog, a rat, a mouse, a fish, a fly, a worm, a plant, a fungi, a bacterium, a virus, a vertebrate, and an invertebrate. In some embodiments, the one or more targets are ribonucleic acid molecules. In some embodiments, the ribonucleic acid molecules are selected from the group consisting of: mRNA, microRNA, mRNA degradation products, and ribonucleic acids comprising a poly-A tail, or any combination thereof. In some embodiments, the targets are deoxyribonucleic acid molecules.

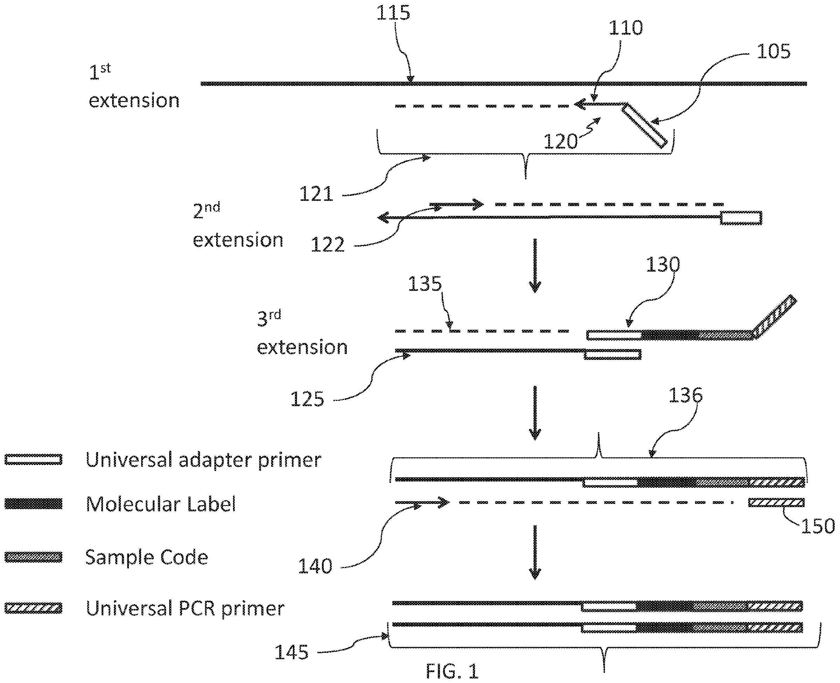

In one aspect, the disclosure provides for a method for estimating the number of a target in a sample using a universal primer adaptor and a stochastic barcode as depicted in one or more of figures 1-42.

### BRIEF DESCRIPTION OF THE DRAWINGS

The novel features of the invention are set forth with particularity in the appended claims. A better understanding of the features and advantages of the methods will be obtained by reference to the following detailed description that sets forth illustrative embodiments of the disclosure, and the accompanying drawings of which:
**Figure 1** depicts an exemplary embodiment of a method for adding a stochastic barcode to a target using a universal adaptor primer. In a first extension step, an oligonucleotide **120** comprising a target-specific sequence **110** and a universal adaptor primer **105** is hybridized to a target **115** to generate a first nucleotide sequence **121.** In a second extension step, a second target-specific primer **122** is used to synthesize a complementary strand **125** of the first nucleotide sequence **121.** In a third extension step, a stochastic barcode comprising a binding partner for the universal adaptor primer **130** can be contacted to the complementary strand **125** through the universal adaptor primer **105** to synthesize a third nucleotide sequence **136.** In an amplification step, a third target-specific primer **140** and a universal PCR primer **150** may be used to amplify the third nucleotide sequence **136** to generate a stochastically barcoded target **145.**
**Figure 2** depicts an exemplary embodiment of a method for adding a stochastic barcode to a target using a universal adaptor primer. The embodiment is similar to that depicted in **Figure 1**, except that a second universal PCR primer is introduced in the second extension step.
**Figure 3** depicts an exemplary embodiment of a method for adding a stochastic barcode to a target using a universal adaptor primer. The embodiment is similar to that depicted in **Figure 2**, except that the order of the molecular label and the sample code is switched in the stochastic barcode.
**Figure 4** depicts an exemplary embodiment of a method for adding a stochastic barcode to a target using a universal adaptor primer. The embodiment is similar to that depicted in **Figure 2****,** except that parts of a stochastic barcode are introduced in both the first extension step and the third extension step.
**Figure 5** depicts an exemplary embodiment of a method for adding a stochastic barcode to a target using a universal adaptor primer. The embodiment is similar to that depicted in **Figure 4****,** except that both the molecular label and the sample code are introduced in both the first extension step.
**Figure 6** depicts an exemplary embodiment of a method for adding a stochastic barcode to a target using a universal adaptor primer. The embodiment is similar to that depicted in **Figure 1**, except that parts of a stochastic barcode are introduced in both the first extension step and the third extension step.
**Figure 7** depicts an exemplary embodiment of a method for adding a stochastic barcode to a target using a universal adaptor primer. The embodiment is similar to that depicted in **Figure 6****,** except that both the molecular label and the sample code are introduced in both the first extension step.
**Figure 8** depicts an exemplary embodiment of a method for adding a stochastic barcode to a target using a universal adaptor primer. The embodiment is similar to that depicted in **Figure 2****,** except that a second universal adaptor primer is introduced in the second extension step, and a second universal PCR primer is introduced in a fourth extension step, and parts of the stochastic barcode are introduced in both the third and fourth extension steps.
**Figure 9** depicts an exemplary embodiment of a method for adding a stochastic barcode to a target using a universal adaptor primer. The embodiment is similar to that depicted in **Figure 8****,** except that the sample code is introduced in the third extension step and the molecular label is introduced in the fourth extension step.
**Figure 10** depicts an exemplary embodiment of a method for adding a stochastic barcode to a target mRNA, wherein the first extension step is a reverse transcription step using a first oligonucleotide comprising a second universal PCR primer and a poly(dT), and the second extension step may use a second oligonucleotide comprising a target-specific sequence and a universal adaptor primer, and the third extension step may use a third oligonucleotide comprising a stochastic barcode and a binding partner for the universal adaptor primer.
**Figure 11** depicts an exemplary embodiment of a method for adding a stochastic barcode to a target mRNA using a universal adaptor primer. The embodiment is similar to that depicted in **Figure 10****,** except that the order of the molecular label and the sample code is switched in the stochastic barcode.
**Figure 12** depicts an exemplary embodiment of a method for adding a stochastic barcode to a target mRNA using a universal adaptor primer. The embodiment is similar to that depicted in **Figure 10****,** except that the universal adaptor primer is introduced in the first extension step using template switching.
**Figure 13** depicts an exemplary embodiment of a method for adding a stochastic barcode to a target mRNA using a universal adaptor primer. The embodiment is similar to that depicted in **Figure 12**, except that the order of the molecular label and the sample code is switched in the stochastic barcode.
**Figure 14** depicts an exemplary embodiment of a method for adding a stochastic barcode to a target using a universal adaptor primer. The embodiment is similar to that depicted in **Figure 1**, wherein a universal adaptor primer can be immobilized on a bead for generating a gene-specific stochastic barcode.
**Figure 15** depicts an exemplary embodiment of a method for adding a stochastic barcode to a target using a universal adaptor primer. The embodiment is similar to that depicted in **Figure 14**, wherein a stochastic barcode comprising a universal adaptor primer can be immobilized on a bead for generating a gene-specific stochastic barcode.
**Figures 16** depicts an exemplary embodiment of how a solid support can comprise a sequence that can hybridize with a universal adaptor primer of the disclosure, which can be part of a stochastic barcode.
**Figures 17** show an exemplary embodiment of how a solid support can comprise a sequence that can hybridize with a universal adaptor primer similar to that depicted in **Figure 16****,** except that the order of the molecular label and the sample code is switched in the stochastic barcode.
**Figures 18** depicts an exemplary embodiment of how a solid support can comprise a sequence that can hybridize with a universal adaptor primer. The embodiment is similar to that depicted in **Figure 16****,** except that a second universal PCR primer is ligated to the target.
**Figures 19** show an exemplary embodiment of how a solid support can comprise a sequence that can hybridize with a universal adaptor primer similar to that depicted in **Figure 18****,** except that the order of the molecular label and the sample code is switched in the stochastic barcode.
**Figures 20** depicts an exemplary embodiment of template switching depicted in **Figure 12** can be performed using a universal adaptor primer attached to a solid support.
**Figures 21** depicts an exemplary embodiment of template switching similar to that depicted in **Figure 20****,** except that the stochastic barcode is introduced during the template switching step.
**Figure 22** depicts an exemplary stochastic barcode.
**Figure 23** illustrates an exemplary process for stochastic labeling of mRNA molecules in single cells.
**Figure 24** illustrates a computer system, which includes a non-transitory computer-readable medium that can be used in connection with embodiments of the present disclosure.
**Figure 25** illustrates an exemplary embodiment of a first example architecture of a computer system that can be used in connection with embodiments of the present disclosure.
**Figure 26** illustrates an exemplary diagram showing a network with a plurality of computer systems for use in the methods of the disclosure.
**Figure 27** illustrates an exemplary a block diagram of a multiprocessor computer system using a shared virtual address memory space in accordance with the methods of the disclosure.
**Figures 28A, 28B,** and **28C** depict an exemplary cartridge for use in the methods of the disclosure.
**Figure 29** illustrates an exemplary embodiment of a method to reduce primer dimers during amplification.
**Figure 30** illustrates an exemplary embodiment of a method to reduce primer dimers during amplification, wherein the amplification primer comprises a universal sequence (e.g., compatible with Illumina sequencers).
**Figure 31** illustrates an exemplary embodiment of the adaptor ligation method of the disclosure.
**Figure 32** shows the mapping and demultiplexing rates using the methods of the disclosure.
**Figure 33** shows the distribution of read counts across amplicons. Genes with single amplicons are labelled in light/dark grey. Multiple amplicons within a gene are colored the same color and are grouped together.
**Figure 34** shows an exemplary embodiment of the computational method of SNP identification in the methods of the disclosure.
**Figure 35** illustrates allele specific expression for select genes. The numbers in the table represent the number of reads containing the indicated base at the SNP position. Values highlighted in dark grey have very low counts while values highlighted in light grey have high counts.
**Figure 36** depicts a schematic for detection of IgG mRNA with gene-specific primers. IgG heavy chain mRNA is depicted in Step 1 with the VDJC structure. Library preparation method of IgG light chain mRNA is similar but without the short D region segment. C-Rev: C region-specific reverse sequence; marker-rev: gene-specific marker reverse primer; MI-SI-Universal: primer sequence that includes molecular and sample index barcodes along with a constant primer sequence; ILMN R1: Illumina Read 1 primer sequence; ILMN R2: Illumina Read 2 primer sequence.
**Figure 37** shows a schematic of adaptor-ligation approach for library preparation. IgG heavy chain mRNA is depicted in Step 1 with the VDJC structure. Library preparation method of IgG light chain mRNA is similar but without the short D region segment. C-Rev: C region-specific reverse primer sequence; marker-rev: gene specific marker reverse sequence; MI-SI-Universal: primer sequence that includes molecular and sample index barcodes along with a constant primer sequence; ILMN R1: Illumina Read 1 primer sequence; ILMN R2: Illumina Read 2 primer sequence.
**Figure 38** shows T Cell Receptor (TCR) and gene marker mRNAs are barcoded with molecular labels (MI) and sample labels (SI) during reverse transcription using either a TCR C-region reverse sequence or poly(A) tail for universal mRNA capture. 2 multiplex PCRs are performed to enrich targets using gene-specific primers and to attach sequencing adapters. This library preparation strategy can amplify the complementary determining region 3 (CDR3) of both TCRα and TCRβ, which is a 'hypervariable' section that spans different rearranged TCR segments.
**Figure 39** shows exemplary genes that can be used in the TCR gene panel.
**Figure 40** shows the TCRα and TCRβ repertoire identified in a T cell RNA sample.
**Figure 41** shows results from principle component analysis of single activated T cells.
**Figure 42** shows an exemplary protocol for one-step RT-PCR for labeling TCR mRNA with a stochastic barcode.

### DETAILED DESCRIPTION

The disclosure provides for methods for estimating the number of targets in a sample using a gene-specific stochastic barcode. The disclosure provides for methods for generating a gene-specific stochastic barcode. The disclosure provides for methods for performing stochastic barcoding in a gene-specific way. For example gene-specific stochastic barcoding can be generated by an exemplary embodiment illustrated in **Figure 1****.** An oligonucleotide comprising a universal primer adaptor **105** and a gene-specific target binding region **110** can be contacted to a target **115.** A first extension reaction can be performed by extending **120** the oligonucleotide to incorporate the sequence of the target **115,** thereby generating a first transcript **121.** A second extension reaction can be performed wherein a reverse gene-specific primer **122** can be used to generate a complementary strand **125** that incorporates the oligonucleotide comprising the gene-specific primer **110** and the universal primer adaptor **105.** A stochastic barcode comprising a universal primer adaptor **130** (or a sequence that can hybridize to a universal primer adaptor) can be contacted to the transcript **125** through the universal primer adaptor sequence **105.** A third extension can be performed wherein a transcript **136** is generated that comprises the target sequence, the universal adaptor primer sequence **105** and the stochastic barcode sequence. After the third extension, excess stochastic barcodes **130** may be removed (e.g., by size exclusion, electrophoresis). An amplification reaction may be performed with a gene specific primer **140** and a universal adaptor primer **150** that can hybridize to the universal label (e.g., universal PCR primer sequence). The amplification can produce a stochastically barcoded target **145,** which can be used for identifying the distinct number of targets in sample. There can be a 1:1 ratio of stochastic barcode association with a target. In this way, the stochastic barcode can be used to count distinct occurrences of the target species.

In some instances, after the third extension, excess stochastic barcodes **130** may not be removed. The excess stochastic barcodes **130** may not be incorporated into the stochastic barcoding reaction (e.g., the third extension) by altering the temperature at which the reaction is performed. The reaction can be performed at a temperature in which one set of oligonucleotides may hybridize to a transcript and another may not. For example, the universal primer and the gene-specific primer **122** may have a Tm of 70°C. The universal adaptor primer sequences may have a Tm of 50°C. If the stochastic barcoding reaction is performed at 70°C, it can prevent excess stochastic barcodes 130 from annealing to targets. The universal adaptor primers can have a Tm of at least 30, 40, 50, 60, 70, or 80°C or more. The universal adaptor primers can have a Tm of at most 30, 40, 50, 60, 70, or 80C or more. The universal primer and gene-specific primer (e.g., extension primers) can have a Tm of at least 30, 40, 50, 60, 70, or 80°C or more. The universal primer and gene-specific primer (e.g., extension primers) can have a Tm of at most 30, 40, 50, 60, 70, or 80°C or more. The Tm of the universal adaptor primers can be at least 10, 20, 30,40, 50, 60, 70, 80, 90, 10, 110, 120, 130, 140, 150, 160, 170, 180, 190, or 200% or more greater than the Tm of the extension primers. The Tm of the universal adaptor primers can be at most 10, 20, 30,40, 50, 60, 70, 80, 90, 10, 110, 120, 130, 140, 150, 160, 170, 180, 190, or 200% or more greater than the Tm of the extension primers.

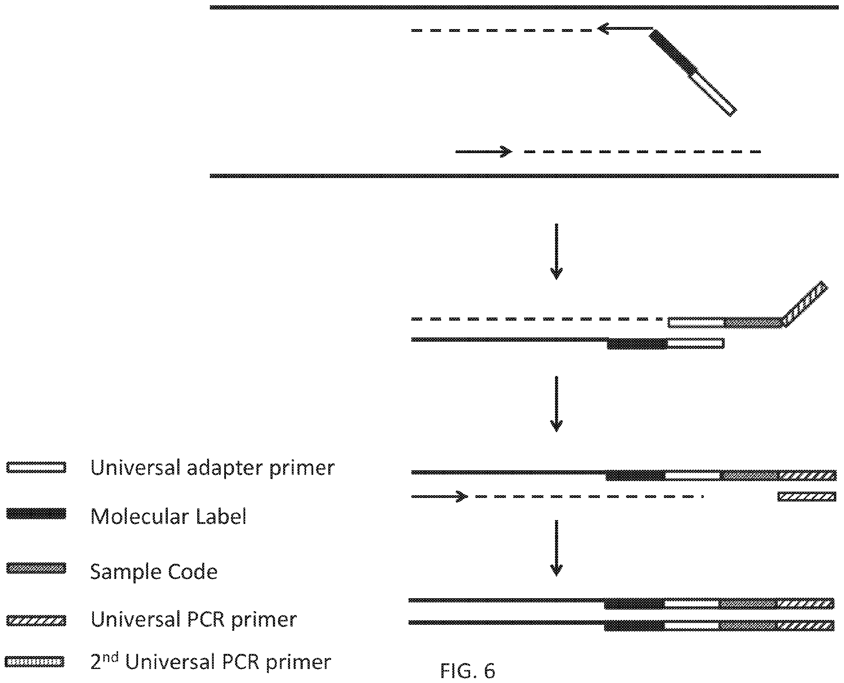

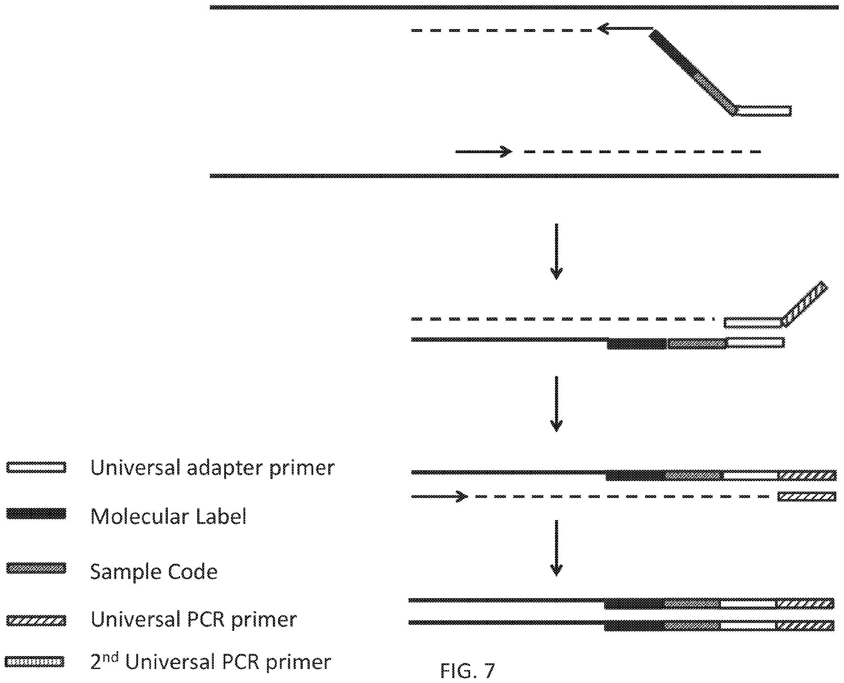

The methods of the disclosure can provide for a way to generating gene-specific stochastic barcodes. For example, the method of generating a gene-specific stochastic barcode of the disclosure can be similar to as described and illustrated in **Figure 1****.** The labels of the stochastic barcode (e.g., sample label (or sample code, which can be used herein is interchangeable with the term "sample label") can be in any order. In some embodiments, all or part of the stochastic barcode can be included in the oligonucleotide **120** comprising the gene-specific primer **110** and the universal primer adaptor **105,** for example, 3' to the universal adaptor primer **105**. **Figures 6** **and** **7** illustrate the method of **Figure 1** but wherein the molecular label (**Figure 6**) or both the molecular label and the sample label (**Figure 7**) are included in the oligonucleotide **120** comprising the gene-specific primer **110** and the universal primer adaptor **105.**

In some embodiments, the disclosure provides for methods for gene-specific amplification for multiple amplicons of the same gene. For example, at least 1, 2, 3, 4, 5, 6, 7, 8, 9 or 10 or more amplicons on one gene may be amplified (e.g., in parallel) using the gene-specific methods of the disclosure. At most 1, 2, 3, 4, 5, 6, 7, 8, 9 or 10 or more amplicons on one gene may be amplified (e.g., in parallel) using the gene-specific methods of the disclosure. In some instances, up to 100 amplicons on one gene may be amplified. The amplicons may be chosen for specific variant calling such as SNP calling, determining of allele-specific expression.

The methods of the disclosure can be used for identifying VDJ regions of antibodies. VDJ recombination, also known as somatic recombination, is a mechanism of genetic recombination in the early stages of immunoglobulin (Ig) and T cell receptor (TCR) production of the immune system. VDJ recombination can nearly randomly combine Variable (V), Diverse (D) and Joining (J) gene segments. Because of its randomness in choosing different genes, is able to diversely encode proteins to match antigens from bacteria, viruses, parasites, dysfunctional cells such as tumor cells and pollens.

The VDJ region can comprise a large 3 Mb locus comprising variable (V) genes, diversity (D) genes and joining (J) genes. These are the segments that can participate in VDJ recombination. There can be constant genes which may not undergo VDJ recombination. The first event in the VDJ recombination of this locus can be that one of the D genes rearranges to one of the J genes. Following this, one of the V genes can be appended to this DJ rearrangement to form the functional VDJ earranged gene that then codes for the variable segment of the heavy chain protein. Both of these steps can be catalyzed by recombinase enzymes, which can delete out the intervening DNA.

This recombination process takes place in a stepwise fashion in progenitor B cells to produce the diversity required for the antibody repertoire. Each B cell may only produce one antibody. This specificity can be achieved by allelic exclusion such that functional rearrangement of one allele signals to prevent further recombination of the second allele.

### Definitions

Unless otherwise defined, all technical terms used herein have the same meaning as commonly understood by one of ordinary skill in the art in the field to which this disclosure belongs. As used in this specification and the appended claims, the singular forms "a," "an," and "the" include plural references unless the context clearly dictates otherwise. Any reference to "or" herein is intended to encompass "and/or" unless otherwise stated.

As used herein the term "associated" or "associated with" can mean that two or more species are identifiable as being co-located at a point in time. An association can mean that two or more species are or were within a similar container. An association can be an informatics association, where for example digital information regarding two or more species is stored and can be used to determine that one or more of the species were co-located at a point in time. An association can also be a physical association. In some instances two or more associated species are "tethered", "attached", or "immobilized" to one another or to a common solid or semisolid surface. An association may refer to covalent or non-covalent means for attaching labels to solid or semi-solid supports such as beads. An association may be a covalent bond between a target and a label.

As used herein, the term "digital counting" can refer to a method for estimating a number of target molecules in a sample. Digital counting can include the step of determining a number of unique labels that have been associated with targets in a sample. This stochastic methodology transforms the problem of counting molecules from one of locating and identifying identical molecules to a series of yes/no digital questions regarding detection of a set of predefined labels.

As used herein, the term "label" or "labels" can refer to nucleic acid codes associated with a target within a sample. A label can be, for example, a nucleic acid label. A label can be an entirely or partially amplifiable label. A label can be entirely or partially sequencable label. A label can be a portion of a native nucleic acid that is identifiable as distinct. A label can be a known sequence. A label can comprise a junction of nucleic acid sequences, for example a junction of a native and non-native sequence. As used herein, the term "label" can be used interchangeably with the terms, "index", "tag," or "label-tag." Labels can convey information. For example, in various embodiments, labels can be used to determine an identity of a sample, a source of a sample, an identity of a cell, and/or a target.

As used herein, the term "non-depleting reservoirs" can refer to a pool of stochastic barcodes made up of many different labels. A non-depleting reservoir can comprise large numbers of different stochastic barcodes such that when the non-depleting reservoir is associated with a pool of targets each target is likely to be associated with a unique stochastic barcode. The uniqueness of each labeled target molecule can be determined by the statistics of random choice, and depends on the number of copies of identical target molecules in the collection compared to the diversity of labels. The size of the resulting set of labeled target molecules can be determined by the stochastic nature of the barcoding process, and analysis of the number of stochastic barcodes detected then allows calculation of the number of target molecules present in the original collection or sample. When the ratio of the number of copies of a target molecule present to the number of unique stochastic barcodes is low, the labeled target molecules are highly unique (i.e. there is a very low probability that more than one target molecule will have been labeled with a given label).

As used herein, the term "sample" can refer to a composition comprising targets. Suitable samples for analysis by the disclosed methods, devices, and systems include cells, tissues, organs, or organisms.

As used herein, the term "sampling device" or "device" can refer to a device which may take a section of a sample and/or place the section on a substrate. A sample device can refer to, for example, a fluorescence activated cell sorting (FACS) machine, a cell sorter machine, a biopsy needle, a biopsy device, a tissue sectioning device, a microfluidic device, a blade grid, and/or a microtome.

As used herein, the term "solid support" can refer to discrete solid or semi-solid surfaces to which a plurality of stochastic barcodes may be attached. A solid support may encompass any type of solid, porous, or hollow sphere, ball, bearing, cylinder, or other similar configuration composed of plastic, ceramic, metal, or polymeric material (e.g., hydrogel) onto which a nucleic acid may be immobilized (e.g., covalently or non-covalently). A solid support may comprise a discrete particle that may be spherical (e.g., microspheres) or have a non-spherical or irregular shape, such as cubic, cuboid, pyramidal, cylindrical, conical, oblong, or disc-shaped, and the like. A plurality of solid supports spaced in an array may not comprise a substrate. A solid support may be used interchangeably with the term "bead."

A solid support can refer to a "substrate." A substrate can be a type of solid support. A substrate can refer to a continuous solid or semi-solid surface on which the methods of the disclosure may be performed. A substrate can refer to an array, a cartridge, a chip, a device, and a slide, for example.

As used here, the term, "spatial label" can refer to a label which can be associated with a position in space.

As used herein, the term "stochastic barcode" can refer to a polynucleotide sequence comprising labels. A stochastic barcode can be a polynucleotide sequence that can be used for stochastic barcoding. Stochastic barcodes can be used to quantify targets within a sample. Stochastic barcodes can be used to control for errors which may occur after a label is associated with a target. For example, a stochastic barcode can be used to assess amplification or sequencing errors. A stochastic barcode associated with a target can be called a stochastic barcode-target or stochastic barcode-tag-target.

As used herein, the term "gene-specific stochastic barcode" can refer to a polynucleotide sequence comprising labels and a target-binding region that is gene-specific. A stochastic barcode can be a polynucleotide sequence that can be used for stochastic barcoding. Stochastic barcodes can be used to quantify targets within a sample. Stochastic barcodes can be used to control for errors which may occur after a label is associated with a target. For example, a stochastic barcode can be used to assess amplification or sequencing errors. A stochastic barcode associated with a target can be called a stochastic barcode-target or stochastic barcode-tag-target.

As used herein, the term "stochastic barcoding" can refer to the random labeling (e.g., barcoding) of nucleic acids. Stochastic barcoding can utilize a recursive Poisson strategy to associate and quantify labels associated with targets. As used herein, the term "stochastic barcoding" can be used interchangeably with "gene-specific stochastic barcoding."

As used here, the term "target" can refer to a composition which can be associated with a stochastic barcode. Exemplary suitable targets for analysis by the disclosed methods, devices, and systems include oligonucleotides, DNA, RNA, mRNA, microRNA, tRNA, and the like. Targets can be single or double stranded. In some embodiments targets can be proteins. In some embodiments targets are lipids.

The term "reverse transcriptases" can refer to a group of enzymes having reverse transcriptase activity (i.e., that catalyze synthesis of DNA from an RNA template). In general, such enzymes include, but are not limited to, retroviral reverse transcriptase, retrotransposon reverse transcriptase, retroplasmid reverse transcriptases, retron reverse transcriptases, bacterial reverse transcriptases, group II intron-derived reverse transcriptase, and mutants, variants or derivatives thereof. Non-retroviral reverse transcriptases include non-LTR retrotransposon reverse transcriptases, retroplasmid reverse transcriptases, retron reverse transciptases, and group II intron reverse transcriptases. Examples of group II intron reverse transcriptases include the Lactococc s lactis Ll.LtrB intron reverse transcriptase, the Thermosynechococcus elongatus TeI4c intron reverse transcriptase, or the Geobacillus stearothermophilus GsI-IIC intron reverse transcriptase. Other classes of reverse transcriptases can include many classes of non-retroviral reverse transcriptases (i.e., retrons, group II introns, and diversity- generating retroelements among others).

The term "template switching" can refer to the ability of a reverse transcriptase to switch from an initial nucleic acid sequence template to the 3' end of a new nucleic acid sequence template having little or no complementarity to the 3' end of the nucleic acid synthesized from the initial template. An example of template switching is the ability of a reverse transcriptase to switch from an initial nucleic acid sequence template/primer substrate to the 3' end of a new nucleic acid sequence template having little or no complementary to the 3' end of the nucleic acid primer strand. Nucleic acid copies of a target polynucleotide can be made using template switching. Template switching allows, e.g., a DNA copy to be prepared using a reverse transcriptase that switches from an initial nucleic acid sequence template to the 3' end of a new nucleic acid sequence template having little or no complementarity to the 3' end of the DNA synthesized from the initial template, thereby allowing the synthesis of a continuous product DNA that directly links an adaptor sequence to a target oligonucleotide sequence without ligation. Template switching can comprise ligation of adaptor, homopolymer tailing (e.g., polyadenylation), random primer, or an oligonucleotide that the polymerase can associate with.

The terms "universal adaptor primer," "universal primer adaptor" or "universal adaptor sequence" are used interchangeably to refer to a nucleotide sequence that can be used to hybridize stochastic barcodes to generate gene-specific stochastic barcodes. A universal adaptor sequence can, for example, be a known sequence that is universal across all stochastic barcodes used in methods of the disclosure. For example, when multiple targets are being labeled using the methods disclosed herein, each of the target-specific sequences may be linked to the same universal adaptor sequence. In some embodiments, more than one universal adaptor sequences may be used in the methods disclosed herein. For example, when multiple targets are being labeled using the methods disclosed herein, at least two of the target-specific sequences are linked to different universal adaptor sequences. A universal adaptor primer and its complement may be included in two oligonucleotides, one of which comprises a target-specific sequence and the other comprises a stochastic barcode. For example, a universal adaptor sequence may be part of an oligonucleotide comprising a target-specific sequence to generate a nucleotide sequence that is complementary to a target nucleic acid. A second oligonucleotide comprising a stochastic barcode and a complementary sequence of the universal adaptor sequence may hybridize with the nucleotide sequence and generate a target-specific stochastic barcode. In some embodiments, a universal adaptor primer has a sequence that is different from a universal PCR primer used in the methods of this disclosure.

As used herein, the term "specifically binds" refers to the binding specificity of a specific binding pair. Hybridization by a target-specific nucleic acid sequence of a particular target polynucleotide sequence in the presence of other potential targets is one characteristic of such binding. Specific binding involves two different nucleic acid molecules wherein one of the nucleic acid molecules specifically hybridizes with the second nucleic acid molecule through chemical or physical means. The two nucleic acid molecules are related in the sense that their binding with each other is such that they are capable of distinguishing their binding partner from other assay constituents having similar characteristics. The members of the binding component pair are referred to as ligand and receptor (anti-ligand), specific binding pair (SBP) member and SBP partner, and the like.

"Polynucleotide," or "nucleotide," as used interchangeably herein, refer to polymers of nucleotides of any length, and include DNA and RNA. A polynucleotide or nucleotide sequence could be either double-stranded or single-stranded. When a polynucleotide or nucleotide sequence is single stranded, it could refer to either of the two complementary strands. The nucleotides can be deoxyribonucleotides, ribonucleotides, modified nucleotides or bases, and/or their analogs, or any substrate that can be incorporated into a polymer by DNA or RNA polymerase. A polynucleotide may comprise modified nucleotides, such as methylated nucleotides and their analogs. If present, modification to the nucleotide structure may be imparted before or after assembly of the polymer. The sequence of nucleotides may be interrupted by non-nucleotide components. A polynucleotide may be further modified after polymerization, such as by conjugation with a labeling component. Other types of modifications include, for example, "caps", substitution of one or more of the naturally occurring nucleotides with an analog, internucleotide modifications such as, for example, those with uncharged linkages (e.g., methyl phosphonates, phosphotriesters, phosphoamidates, cabamates, etc.) and with charged linkages (e.g., phosphorothioates, phosphorodithioates, etc.), those containing pendant moieties, such as, for example, proteins (e.g., nucleases, toxins, antibodies, signal peptides, ply-L-lysine, etc.), those with intercalators (e.g., acridine, psoralen, etc.), those containing chelators (e.g., metals, radioactive metals, boron, oxidative metals, etc.), those containing alkylators, those with modified linkages (e.g., alpha anomeric nucleic acids, etc.), as well as unmodified forms of the polynucleotide(s). Further, any of the hydroxyl groups ordinarily present in the sugars may be replaced, for example, by phosphonate groups, phosphate groups, protected by standard protecting groups, or activated to prepare additional linkages to additional nucleotides, or may be conjugated to solid supports. The 5' and 3' terminal OH can be phosphorylated or substituted with amines or organic capping groups moieties of from 1 to 20 carbon atoms. Other hydroxyls may also be derivatized to standard protecting groups. Polynucleotides can also contain analogous forms of ribose or deoxyribose sugars that are generally known in the art, including, for example, 2'-O-methyl-2'-O- allyl, 2'-fluoro- or 2'-azido-ribose, carbocyclic sugar analogs, α-anomeric sugars, epimeric sugars such as arabinose, xyloses or lyxoses, pyranose sugars, furanose sugars, sedoheptuloses, acyclic analogs and abasic nucleoside analogs such as methyl riboside. One or more phosphodiester linkages may be replaced by alternative linking groups. These alternative linking groups include, but are not limited to, embodiments wherein phosphate is replaced by P(O)S("thioate"), P(S)S ("dithioate"), "(O)NR 2 ("amidate"), P(O)R, P(O)OR', CO or CH 2 ("formacetal"), in which each R or R' is independently H or substituted or unsubstituted alkyl (1-20 C) optionally containing an ether (-O--) linkage, aryl, alkenyl, cycloalkyl, cycloalkenyl or araldyl. Not all linkages in a polynucleotide need be identical. The preceding description applies to all polynucleotides referred to herein, including RNA and DNA.

"Oligonucleotide," as used herein, generally refers to short, generally single stranded, generally synthetic polynucleotides that are generally, but not necessarily, less than about 200 nucleotides in length. The terms "oligonucleotide" and "polynucleotide" are not mutually exclusive. The description above for polynucleotides is equally and fully applicable to oligonucleotides.

### Stochastic Barcodes

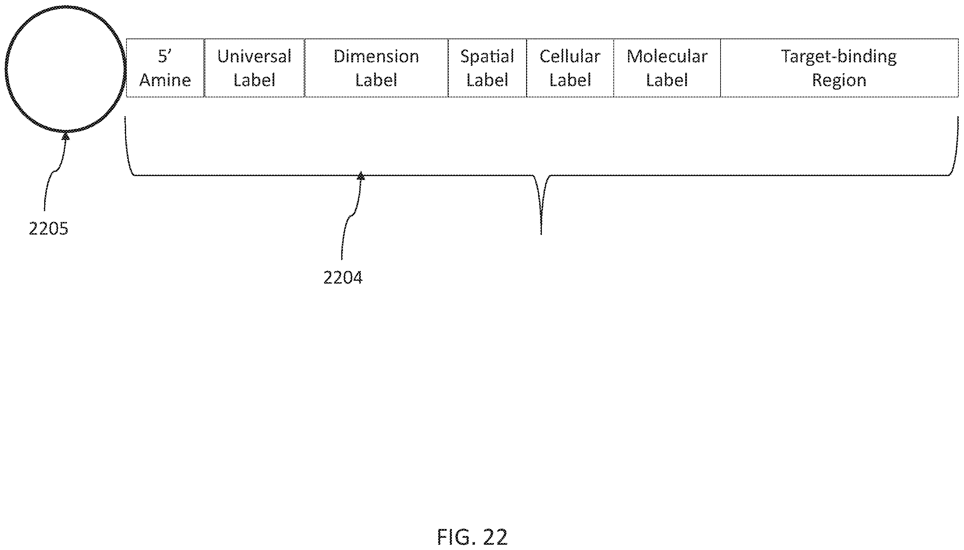

A stochastic barcode can refer to a polynucleotide sequence that can be used to stochastically label (e.g., barcode, tag) a target. A stochastic barcode can comprise one or more labels. Exemplary labels can include a universal label, a cellular label, a molecular label, a sample label, a plate label, a spatial label, and/or a pre-spatial label. **Figure 22** illustrates an exemplary stochastic barcode with a spatial label of the disclosure. For example, a stochastic barcode **2204** can comprise a 5'amine that may link the stochastic barcode to a solid support **2205.** The stochastic barcode can comprise a universal label, a dimension label, a spatial label, a cellular label, and/or a molecular label. The universal label may be 5'-most label. The molecular label may be the 3'-most label. The spatial label, dimension label, and the cellular label may be in any order. In some instances, the universal label, the spatial label, the dimension label, the cellular label, and the molecular label are in any order. The stochastic barcode can comprise a target-binding region. The target-binding region can interact with a target (e.g., target nucleic acid, RNA, mRNA, DNA) in a sample. For example, a target-binding region can comprise an oligo d(T)s sequence which can interact with poly-A tails of mRNAs. In some instances, any two of the labels of the stochastic barcode (e.g., universal label, dimension label, spatial label, cellular label, and molecular label) may be separated by, or by about, 1, 2, 3, 4, 5, 6, 7, 8, 9, 10, 11, 12, 13, 14, 15, 16, 17, 18, 19, or 20 or more nucleotides.

A stochastic barcode can comprise one or more universal labels. The one or more universal labels may be the same for all stochastic barcodes in the set of stochastic barcodes attached to a given solid support. In some embodiments, the one or more universal labels may be the same for all stochastic barcodes attached to a plurality of beads. In some embodiments, a universal label may comprise a nucleic acid sequence that is capable of hybridizing to a sequencing primer. Sequencing primers may be used for sequencing stochastic barcodes comprising a universal label. Sequencing primers (e.g., universal sequencing primers) may comprise sequencing primers associated with high-throughput sequencing platforms. In some embodiments, a universal label may comprise a nucleic acid sequence that is capable of hybridizing to a PCR primer. In some embodiments, the universal label may comprise a nucleic acid sequence that is capable of hybridizing to a sequencing primer and a PCR primer. The nucleic acid sequence of the universal label that is capable of hybridizing to a sequencing or PCR primer may be referred to as a primer binding site. A universal label may comprise a sequence that may be used to initiate transcription of the stochastic barcode. A universal label may comprise a sequence that may be used for extension of the stochastic barcode or a region within the stochastic barcode. A universal label may be at least, or at least about, 1, 2, 3, 4, 5, 10, 15, 20, 25, 30, 35, 40, 45, 50 or more nucleotides in length. A universal label may comprise at least about 10 nucleotides. A universal label may be at most, or at most about, 1, 2, 3, 4, 5, 10, 15, 20, 25, 30, 35, 40, 45, 50 or more nucleotides in length. In some embodiments, a cleavable linker or modified nucleotide may be part of the universal label sequence to enable the stochastic barcode to be cleaved off from the support. As used herein, a universal label can be used interchangeably with "universal PCR primer."

A stochastic barcode can comprise a dimension label. A dimension label can comprise a nucleic acid sequence that provides information about a dimension in which the stochastic labeling occurred. For example, a dimension label can provide information about the time at which a target was stochastically barcoded. A dimension label can be associated with a time of stochastic barcoding in a sample. A dimension label can activated at the time of stochastic labeling. Different dimension labels can be activated at different times. The dimension label provides information about the order in which targets, groups of targets, and/or samples were stochastically barcoded. For example, a population of cells can be stochastically barcoded at the GO phase of the cell cycle. The cells can be pulsed again with stochastic barcodes at the G1 phase of the cell cycle. The cells can be pulsed again with stochastic barcodes at the S phase of the cell cycle, and so on. Stochastic barcodes at each pulse (e.g., each phase of the cell cycle), can comprise different dimension labels. In this way, the dimension label provides information about which targets were labelled at which phase of the cell cycle. Dimension labels can interrogate many different biological times. Exemplary biological times can include, but are not limited to, the cell cycle, transcription (e.g., transcription initiation), and transcript degradation. In another example, a sample (e.g., a cell, a population of cells) can be stochastically labeled before and/or after treatment with a drug and/or therapy. The changes in the number of copies of distinct targets can be indicative of the sample's response to the drug and/or therapy.

A dimension label can be activatable. An activatable dimension label can be activated at a specific timepoint. The activatable may be constitutively activated (e.g., not turned off). The activatable dimension label can be reversibly activated (e.g., the activatable dimension label can be turned on and turned off). The dimension label can be reversibly activatable at least 1, 2, 3, 4, 5, 6, 7, 8, 9, or 10 or more times. The dimension label can be reversibly activatable at least 1, 2, 3, 4, 5, 6, 7, 8, 9, or 10 or more times. The dimension label can be activated with fluorescence, light, a chemical event (e.g., cleavage, ligation of another molecule, addition of modifications (e.g., pegylated, sumoylated, acetylated, methylated, deacetylated, demethylated), a photochemical event (e.g., photocaging), and introduction of a non-natural nucleotide.

The dimension label can be identical for all stochastic barcodes attached to a given solid support (e.g., bead), but different for different solid supports (e.g., beads). In some embodiments, at least 60%, 70%, 80%, 85%, 90%, 95%, 97%, 99% or 100% of stochastic barcodes on the same solid support may comprise the same dimension label. In some embodiments, at least 60% of stochastic barcodes on the same solid support may comprise the same dimension label. In some embodiments, at least 95% of stochastic barcodes on the same solid support may comprise the same dimension label.

There may be as many as 10⁶ or more unique dimension label sequences represented in a plurality of solid supports (e.g., beads). A dimension label may be at least about 1, 2, 3, 4, 5, 10, 15, 20, 25, 30, 35, 40, 45, 50 or more nucleotides in length. A dimension label may be at most about 300, 200, 100, 90, 80, 70, 60, 50, 40, 30, 20, 15, 12, 10, 9, 8, 7, 6, 5, 4 or fewer or more nucleotides in length. A dimension label may comprise between about 5 to about 200 nucleotides. A dimension label may comprise between about 10 to about 150 nucleotides. A dimension label may comprise between about 20 to about 125 nucleotides in length.

A stochastic barcode can comprise a spatial label. A spatial label can comprise a nucleic acid sequence that provides information about the spatial orientation of a target molecule which is associated with the stochastic barcode. A spatial label can be associated with a coordinate in a sample. The coordinate can be a fixed coordinate. For example a coordinate can be fixed in reference to a substrate. A spatial label can be in reference to a two or three-dimensional grid. A coordinate can be fixed in reference to a landmark. The landmark can be identifiable in space. A landmark can a structure which can be imaged. A landmark can be a biological structure, for example an anatomical landmark. A landmark can be a cellular landmark, for instance an organelle. A landmark can be a non-natural landmark such as a structure with an identifiable identifier such as a color code, bar code, magnetic property, fluorescents, radioactivity, or a unique size or shape. A spatial label can be associated with a physical partition (e.g. a well, a container, or a droplet). In some instances, multiple spatial labels are used together to encode one or more positions in space.

The spatial label can be identical for all stochastic barcodes attached to a given solid support (e.g., bead), but different for different solid supports (e.g., beads). In some embodiments, at least 60%, 70%, 80%, 85%, 90%, 95%, 97%, 99% or 100% of stochastic barcodes on the same solid support may comprise the same spatial label. In some embodiments, at least 60% of stochastic barcodes on the same solid support may comprise the same spatial label. In some embodiments, at least 95% of stochastic barcodes on the same solid support may comprise the same spatial label.

There may be as many as 10⁶ or more unique spatial label sequences represented in a plurality of solid supports (e.g., beads). A spatial label may be at least about 1, 2, 3, 4, 5, 10, 15, 20, 25, 30, 35, 40, 45, 50 or more nucleotides in length. A spatial label may be at most about 300, 200, 100, 90, 80, 70, 60, 50, 40, 30, 20, 15, 12, 10, 9, 8, 7, 6, 5, 4 or fewer or more nucleotides in length. A spatial label may comprise between about 5 to about 200 nucleotides. A spatial label may comprise between about 10 to about 150 nucleotides. A spatial label may comprise between about 20 to about 125 nucleotides in length.

Stochastic barcodes may comprise a cellular label. A cellular label may comprise a nucleic acid sequence that provides information for determining which target nucleic acid originated from which cell. In some embodiments, the cellular label is identical for all stochastic barcodes attached to a given solid support (e.g., bead), but different for different solid supports (e.g., beads). In some embodiments, at least 60%, 70%, 80%, 85%, 90%, 95%, 97%, 99% or 100% of stochastic barcodes on the same solid support may comprise the same cellular label. In some embodiments, at least 60% of stochastic barcodes on the same solid support may comprise the same cellular label. In some embodiment, at least 95% of stochastic barcodes on the same solid support may comprise the same cellular label.

There may be as many as 10⁶ or more unique cellular label sequences represented in a plurality of solid supports (e.g., beads). A cellular label may be at least about 1, 2, 3, 4, 5, 10, 15, 20, 25, 30, 35, 40, 45, 50 or more nucleotides in length. A cellular label may be at most about 300, 200, 100, 90, 80, 70, 60, 50, 40, 30, 20, 15, 12, 10, 9, 8, 7, 6, 5, 4 or fewer or more nucleotides in length. A cellular label may comprise between about 5 to about 200 nucleotides. A cellular label may comprise between about 10 to about 150 nucleotides. A cellular label may comprise between about 20 to about 125 nucleotides in length.

The cellular label may further comprise a unique set of nucleic acid sub-sequences of defined length, e.g. 7 nucleotides each (equivalent to the number of bits used in some Hamming error correction codes), which are designed to provide error correction capability. The set of error correction sub-sequences comprise 7 nucleotide sequences can be designed such that any pairwise combination of sequences in the set exhibits a defined "genetic distance" (or number of mismatched bases), for example, a set of error correction sub-sequences may be designed to exhibit a genetic distance of 3 nucleotides. In this case, review of the error correction sequences in the set of sequence data for labeled target nucleic acid molecules (described more fully below) may allow one to detect or correct amplification or sequencing errors. In some embodiments, the length of the nucleic acid sub-sequences used for creating error correction codes may vary, for example, they may be 3 nucleotides, 7 nucleotides, 15 nucleotides, or 31 nucleotides in length. In some embodiments, nucleic acid sub-sequences of other lengths may be used for creating error correction codes.

Stochastic barcodes may comprise a molecular label. A molecular label may comprise a nucleic acid sequence that provides identifying information for the specific type of target nucleic acid species hybridized to the stochastic barcode. A molecular label may comprise a nucleic acid sequence that provides a counter for the specific occurrence of the target nucleic acid species hybridized to the stochastic barcode (e.g., target-binding region). In some embodiments, a diverse set of molecular labels are attached to a given solid support (e.g., bead). In some embodiments, there may be as many as 10⁵ or more unique molecular label sequences attached to a given solid support (e.g., bead). In some embodiments, there may be as many as 10⁴ or more unique molecular label sequences attached to a given solid support (e.g., bead). In some embodiments, there may be as many as 10³ or more unique molecular label sequences attached to a given solid support (e.g., bead). In some embodiments, there may be as many as 10² or more unique molecular label sequences attached to a given solid support (e.g., bead). A molecular label may be at least about 1, 2, 3, 4, 5, 10, 15, 20, 25, 30, 35, 40, 45, 50 or more nucleotides in length. A molecular label may be at most about 300, 200, 100, 90, 80, 70, 60, 50, 40, 30, 20, 15, 12, 10, 9, 8, 7, 6, 5, 4 or fewer nucleotides in length.

Stochastic barcodes may comprise a target binding region. In some embodiments, the target binding regions may comprise a nucleic acid sequence that hybridizes specifically to a target (e.g., target nucleic acid, target molecule, e.g., a cellular nucleic acid to be analyzed), for example to a specific gene sequence. In some embodiments, a target binding region may comprise a nucleic acid sequence that may attach (e.g., hybridize) to a specific location of a specific target nucleic acid. In some embodiments, the target binding region may comprise a nucleic acid sequence that is capable of specific hybridization to a restriction site overhang (e.g. an EcoRI sticky-end overhang). The stochastic barcode may then ligate to any nucleic acid molecule comprising a sequence complementary to the restriction site overhang.

In some embodiments, a target binding region may comprise a non-specific target nucleic acid sequence. A non-specific target nucleic acid sequence may refer to a sequence that may bind to multiple target nucleic acids, independent of the specific sequence of the target nucleic acid. For example, target binding region may comprise a random multimer sequence, or an oligo-dT sequence that hybridizes to the poly-A tail on mRNA molecules. A random multimer sequence can be, for example, a random dimer, trimer, quatramer, pentamer, hexamer, septamer, octamer, nonamer, decamer, or higher multimer sequence of any length. In some embodiments, the target binding region is the same for all stochastic barcodes attached to a given bead. In some embodiments, the target binding regions for the plurality of stochastic barcodes attached to a given bead may comprise two or more different target binding sequences. A target binding region may be at least about 5, 10, 15, 20, 25, 30, 35, 40, 45, 50 or more nucleotides in length. A target binding region may be at most about 5, 10, 15, 20, 25, 30, 35, 40, 45, 50 or more nucleotides in length.

A stochastic barcode can comprise an orientation property which can be used to orient (e.g., align) the stochastic barcodes. A stochastic barcode can comprise a moiety for isoelectric focusing. Different stochastic barcodes can comprise different isoelectric focusing points. When these stochastic barcodes are introduced to a sample, the sample can undergo isoelectric focusing in order to orient the stochastic barcodes into a known way. In this way, the orientation property can be used to develop a known map of stochastic barcodes in a sample. Exemplary orientation properties can include, electrophoretic mobility (e.g., based on size of the stochastic barcode), isoelectric point, spin, conductivity, and/or self-assembly. For example, stochastic barcodes that with an orientation property of self-assembly, can self-assemble into a specific orientation (e.g., nucleic acid nanostructure) upon activation.

A stochastic barcode can comprise an affinity property. A spatial label can comprise an affinity property. An affinity property can include a chemical and/or biological moiety that can facilitate binding of the stochastic barcode to another entity (e.g., cell receptor). For example, an affinity property can comprise an antibody. An antibody can be specific for a specific moiety (e.g., receptor) on a sample. An antibody can guide the stochastic barcode to a specific cell type or molecule. Targets at and/or near the specific cell type or molecule can be stochastically labeled. An affinity property can also provide spatial information in addition to the nucleotide sequence of the spatial label because the antibody can guide the stochastic barcode to a specific location. An antibody can be a therapeutic antibody. An antibody can be a monoclonal antibody. An antibody can be a polyclonal antibody. An antibody can be humanized. An antibody can be chimeric. An antibody can be a naked antibody. An antibody can be a fusion antibody.

An antibody, can refer to a full-length (i.e., naturally occurring or formed by normal immunoglobulin gene fragment recombinatorial processes) immunoglobulin molecule (e.g., an IgG antibody) or an immunologically active (i.e., specifically binding) portion of an immunoglobulin molecule, like an antibody fragment.

An antibody can be an antibody fragment. An antibody fragment can be a portion of an antibody such as F(ab')2, Fab', Fab, Fv, sFv and the like. An antibody fragment can bind with the same antigen that is recognized by the full-length antibody. An antibody fragment can include isolated fragments consisting of the variable regions of antibodies, such as the "Fv" fragments consisting of the variable regions of the heavy and light chains and recombinant single chain polypeptide molecules in which light and heavy variable regions are connected by a peptide linker ("scFv proteins"). Exemplary antibodies can include, but are not limited to, antibodies for antibodies for cancer cells, antibodies for viruses, antibodies that bind to cell surface receptors (CD8, CD34, CD45), and therapeutic antibodies.

A stochastic barcode can comprise a target-binding region. A target-binding region can hybridize with a target of interest. For example, a target-binding region can comprise an oligo dT which can hybridize with mRNAs comprising poly-adenylated ends. A target-binding region can be gene-specific. For example, a target-binding region can be configured to hybridize to a specific region of a target. A target-binding region can be at least 1, 2, 3, 4, 5, 6, 7, 8, 9, 10, 11, 12, 13, 14, 15, 16, 17, 18, 19, 20, 21, 22, 23, 24, 25, 26 27, 28, 29, or 30 or more nucleotides in length. A target-binding region can be at most 1, 2, 3, 4, 5, 6, 7, 8, 9, 10, 11, 12, 13, 14, 15, 16, 17, 18, 19, 20, 21, 22, 23, 24, 25, 26 27, 28, 29, or 30 or more nucleotides in length. A target-binding region can be from 5-30 nucleotides in length. When a stochastic barcode comprises a gene-specific target-binding region, the stochastic barcode can be referred to as a gene-specific stochastic barcode.

A stochastic barcode can comprise a universal adaptor primer. A gene-specific stochastic barcode can comprise a universal adaptor primer. A universal adaptor primer can refer to a nucleotide sequence that is universal across all stochastic barcodes. A universal adaptor primer can be used for building gene-specific stochastic barcodes of the disclosure. A universal adaptor primer can be at least 1, 2, 3, 4, 5, 6, 7, 8, 9, 10, 11, 12, 13, 14, 15, 16, 17, 18, 19, 20, 21, 22, 23, 24, 25, 26 27, 28, 29, or 30 or more nucleotides in length. A universal adaptor primer can be at most 1, 2, 3, 4, 5, 6, 7, 8, 9, 10, 11, 12, 13, 14, 15, 16, 17, 18, 19, 20, 21, 22, 23, 24, 25, 26 27, 28, 29, or 30 or more nucleotides in length. A universal adaptor primer can be from 5-30 nucleotides in length.

### Method of making gene-specific stochastic barcodes

Some embodiments disclosed herein provide methods of labeling a nucleic acid in a sample, comprising: hybridizing a target with a first oligonucleotide comprising a universal adaptor sequence and a target-specific sequence; extending said first oligonucleotide to generate a first nucleotide sequence; hybridizing said first nucleotide sequence with a second oligonucleotide; extending said second oligonucleotide to generate a second nucleotide sequence; hybridizing said second nucleotide sequence with a third oligonucleotide comprising a binding site for a first amplification primer and the universal adaptor sequence, wherein one or both of the first oligonucleotide and the third oligonucleotide comprises part of a stochastic barcode; extending said third oligonucleotide to generate a third nucleotide sequence; and amplifying said third nucleotide sequence using a first amplification primer to generate a plurality of a fourth nucleotide sequences, wherein each of the plurality of the fourth nucleotide sequences comprises said target and the stochastic barcode. Some embodiments disclosed herein provide methods of labeling a target in a sample for quantification of said target, comprising: hybridizing said target with a first oligonucleotide comprising a first universal adaptor sequence and a target-specific sequence; extending said first oligonucleotide to generate a first nucleotide sequence; hybridizing said first nucleotide sequence with a second oligonucleotide comprising a second universal adaptor sequence and a second target-specific sequence; extending said second oligonucleotide to generate a second nucleotide sequence; hybridizing said second nucleotide sequence with a third oligonucleotide comprising a binding site for a first amplification primer and the first universal adaptor sequence; extending said third oligonucleotide to generate a third nucleotide sequence; hybridizing said third nucleotide sequence with a fourth oligonucleotide comprising a binding site for a second amplification primer and the second universal adaptor sequence, wherein one or both of the third oligonucleotide and the fourth oligonucleotide comprises part of a stochastic barcode; extending said fourth oligonucleotide to generate a fourth nucleotide sequence; and amplifying said fourth nucleotide sequence using a first amplification primer and a second amplification primer to generate a plurality of a fifth nucleotide sequences, wherein each of the plurality of the fifth nucleotide sequences comprises said target and the stochastic barcode. Some embodiments disclosed herein provide methods of labeling a target in a sample for quantification of said target, comprising: hybridizing said target with a first oligonucleotide comprising a target-specific sequence and a binding site for a first amplification primer; extending said first oligonucleotide to generate a first nucleotide sequence; hybridizing said first nucleotide sequence with a second oligonucleotide comprising a universal adaptor sequence and a second target-specific sequence; extending said second oligonucleotide to generate a second nucleotide sequence; hybridizing said second nucleotide sequence with a third oligonucleotide comprising a binding site for a second amplification primer and a sequence that specifically binds the universal adaptor sequence, wherein one or both of the second oligonucleotide and the third oligonucleotide comprises part of a stochastic barcode; extending said third oligonucleotide to generate a third nucleotide sequence; and amplifying said third nucleotide sequence using a first amplification primer and a second amplification primer to generate a plurality of a fourth nucleotide sequences, wherein each of the plurality of the fourth nucleotide sequences comprises said target and the stochastic barcode. Some embodiments disclosed herein provide methods of labeling a target in a sample for quantification of said target, comprising: hybridizing said target with a first oligonucleotide comprising a target-specific sequence and a first amplification primer sequence in the presence of a second oligonucleotide comprising a universal adaptor sequence; extending said first oligonucleotide to generate a first nucleotide sequence comprising a compliment of the universal adaptor sequence; hybridizing said first nucleotide sequence with a third oligonucleotide comprising the universal adaptor sequence, a stochastic barcode, and a binding site for a second amplification primer; extending said third oligonucleotide to generate a second nucleotide sequence; and amplifying said second nucleotide sequence using a first amplification primer and a second amplification primer to generate a plurality of a third nucleotide sequences, wherein each of the plurality of the third nucleotide sequences comprises said target and the stochastic barcode. Some embodiments disclosed herein provide methods of labeling a target in a sample for quantification of said target, comprising: hybridizing said target with a first oligonucleotide comprising a universal adaptor sequence and a target-specific sequence; extending said first oligonucleotide to generate a first nucleotide sequence; hybridizing said first nucleotide sequence with a second oligonucleotide comprising a second target-specific sequence and a third oligonucleotide comprising a stochastic barcode and a sequence that specifically binds the universal adaptor sequence; amplifying said first nucleotide sequence to generate a plurality of a second nucleotide sequences, wherein each of the plurality of the second nucleotide sequences comprises said target and said stochastic barcode. Some embodiments disclosed herein provide methods of characterizing a sample, comprising: hybridizing a first target in the sample with a first oligonucleotide comprising a first target-specific sequence and a first stochastic barcode, wherein the first target is an mRNA encoding a TCR alpha chain or beta chain; hybridizing a second target in the sample with a second oligonucleotide comprising a second target-specific sequence and a second stochastic barcode; extending the first oligonucleotide and the second oligonucleotide to incorporate the first stochastic barcode into the first target and the second stochastic barcode into the second target; and identifying the sequences of the first target and the second target. Some embodiments disclosed herein provide methods of labeling a target in a sample for quantification of said target, comprising: hybridizing said target with a first oligonucleotide comprising a universal adaptor sequence and a target-specific sequence; extending said first oligonucleotide to generate a first nucleotide sequence; hybridizing said first nucleotide sequence with a second oligonucleotide comprising a second target-specific sequence; extending said second oligonucleotide to generate a complementary strand of the first nucleotide sequence and a double-stranded nucleotide sequence; ligating a double-stranded adaptor oligonucleotide comprising a binding site for a first amplification primer to said double-stranded nucleotide sequence to form a ligated nucleotide sequence; amplifying said ligated nucleotide sequence using a first amplification primer and a second amplification primer comprising a stochastic barcode to generate a plurality of a second nucleotide sequence, wherein each of the plurality of the second nucleotide sequence comprises said target and said stochastic barcode. Some embodiments disclosed herein provide kits comprising: a first oligonucleotide comprising a universal adaptor sequence and a target-specific sequence; and a second oligonucleotide comprising a stochastic barcode and the universal adaptor sequence or the complement thereof.

In any of the above-mentioned embodiments, the sample may be a single cell, such as a B cell, a T cell, a cancer cell, etc., or a plurality of cells. In any of the above-mentioned embodiments, the target may be a DNA, such as genomic DNA, or an mRNA molecule. In any of the above-mentioned embodiments, the DNA or mRNA molecule may encode a T cell receptor (TCR) alpha chain or beta chain. In any of the above-mentioned embodiments, the target-specific sequence may specifically bind to the C region of a TCR alpha chain or beta chain. In any of the above-mentioned embodiments, the methods may comprise amplifying a nucleotide sequence using a pair of PCR primers, such as universal PCR primers. In any of the above-mentioned embodiments, a PCR amplification primer may specifically bind to a region selected from the group consisting of: a V region of a TCR alpha chain or beta chain, the junction of a V and D region of a TCR alpha chain or beta chain, or any combination thereof. In any of the above-mentioned embodiments, a target-specific sequence that specifically binds to a region selected from the group consisting of: a V region of a TCR alpha chain or beta chain, the junction of a V and D region of a TCR alpha chain or beta chain, or any combination thereof, may be used for one of the extension or amplification reactions. In any of the above-mentioned embodiments, part of or the entire of the stochastic barcode may be part of any of the oligonucleotides used for each of the extension steps. In any of the above-mentioned embodiments, the stochastic barcode may comprise a molecular label, a universal label, a dimension label, a cellular label, or a combination thereof. In any of the above-mentioned embodiments, any of the oligonucleotide may be attached to a solid support, such as a bead. In any of the above-mentioned embodiments, the sample may comprise a plurality of the target. In any of the above-mentioned embodiments, each of the plurality of the target may be labeled with a distinct stochastic barcode. In any of the above-mentioned embodiments, the methods may comprise counting the number of the plurality of the target by counting the distinct stochastic barcodes associated with the target. In any of the above-mentioned embodiments, template switching may be used to introduce a universal adaptor sequence or the complement thereof. In any of the above-mentioned embodiments, the methods may comprise labeling a second target in the sample with a second stochastic barcode. In any of the above-mentioned embodiments, the second target may be selected from the group consisting of the genes listed in Figure 39. In any of the above-mentioned embodiments, the second stochastic barcode comprises one or more of a molecular label, a universal label, a dimension label, and a cellular label.

The methods of the disclosure can use methods of reverse transcription, first strand cDNA synthesis, and second strand DNA synthesis and/or amplification methods.

Methods of first strand complementary DNA synthesis relevant to the present disclosure can rely upon the enzymatic synthesis of DNA from a nucleic acid template, e.g., messenger RNA. Enzymes capable of catalyzing the synthesis of DNA can be referred to as "RNA dependent DNA polymerases" where the nucleic acid template is RNA and "DNA dependent DNA polymerases" where the template is DNA (generally, however, RNA dependent DNA polymerases are also capable of functioning as DNA dependent polymerases). RNA dependent DNA polymerases such as AMV or MMLV reverse transcriptases can be relied upon for the enzymatic synthesis of the first strand of complementary DNA from a messenger RNA template. Both types of DNA polymerases may need, in addition to a template, a polynucleotide primer and deoxyribonucleotide triphosphates. The synthesis of first strand complementary DNA can be primed with an oligo-d(T), consisting of 12-18 nucleotides in length, that initiates synthesis by annealing to the poly-A tract at the 3' terminus of eukaryotic messenger RNA molecules. Other primers, including short random oligonucleotide primers, can be used to prime complementary DNA synthesis. In some instances, gene-specific primers can be used to prime cDNA synthesis. During the polymerase reaction the primer can be extended, stepwise, by the incorporation of deoxyribonucleotide triphosphates at the 3' end of the primer. DNA polymerases may comprise magnesium and other ions to be present in reaction buffers in well-defined concentrations. Following synthesis of the first strand of complementary DNA, several methods can be employed to replace the RNA-template with the second strand of DNA. One such method can involve removal of the messenger RNA with NaOH and self-priming by the first strand of complementary DNA for second strand synthesis. The 3' end of single stranded complementary DNA can be permitted to form a hairpin-like structure that primes synthesis of the second strand of complementary DNA by E. coli DNA polymerase I or reverse transcriptase. Another method can involve the replacement synthesis of second strand complementary DNA. During replacement synthesis the product of the first strand synthesis, a complementary DNA: messenger RNA hybrid, provides a template and a primer for a nick-translation reaction in which the enzyme RNase H produces nicks and gaps in the messenger RNA strand, resulting in a series of RNA primers for synthesis of the second strand of complementary DNA with the enzyme E. coli DNA polymerase I. After synthesis of a double stranded complementary DNA, the synthesized DNA can be introduced into a host cell, e.g., a bacterial strain, and replicated by means of a suitable vector, such as a bacteriophage lambda vector. Such methods can involve, for example, the addition of short synthetic polynucleotide linkers which are ligated to the ends of the complementary DNA after second strand synthesis. The linkers can contain sequences that can be recognized by an appropriate restriction endonuclease, e.g., EcoR I. After the linkers have been ligated to the ends of the complementary DNA they can be cut with the appropriate restriction endonuclease to generate identical cohesive DNA sequences on both ends of the complementary DNA, thereby facilitating ligation of the complementary DNA to a bacteriophage lambda vector. As complementary DNA clones of interest may contain restriction sites recognized by the restriction endonuclease, complementary DNA may be treated with a methylase, such as EcoR I methylase, prior to ligation of the synthetic linkers to protect the internal restriction sites from subsequent digestion.

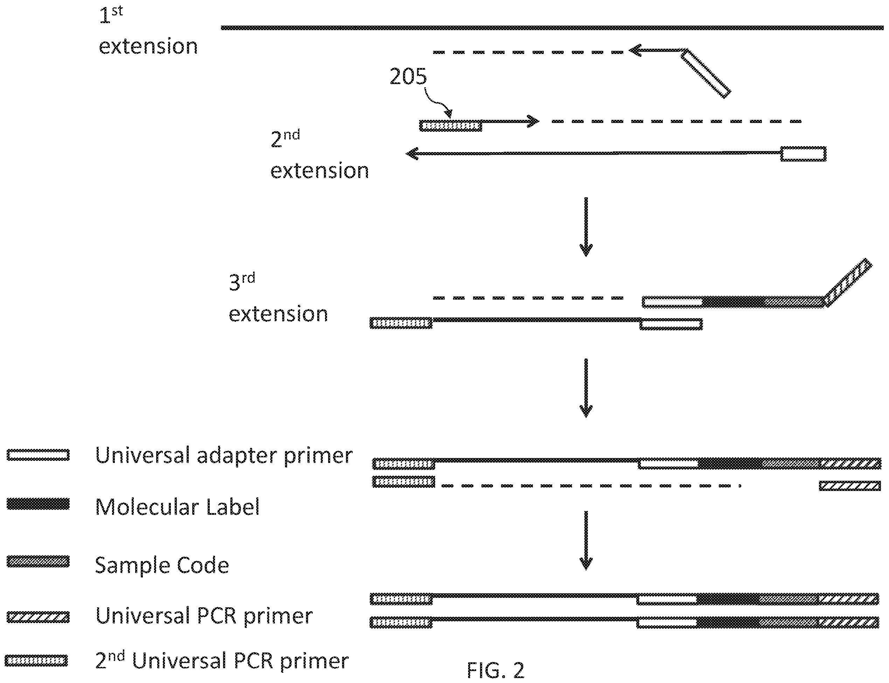

The methods of the disclosure can provide for a way to generating gene-specific stochastic barcodes and/or methods for gene-specific stochastic barcoding. For example, the method of gene-specific stochastic barcoding of the disclosure can be similar to as described and illustrated in **Figure 1****.** In some instances, as shown in **Figure 2**, the second extension can be performed with a reverse primer comprising a reverse gene-specific primer **122** linked to a second universal primer (e.g., a second universal PCR primer **205**). A second universal PCR primer can be similar to a universal label in that it can comprise a sequence that can be common among stochastic barcodes. A second universal PCR primer can be used for amplification and/or sequencing methods of the disclosure. A second universal PCR primer can be a universal label.

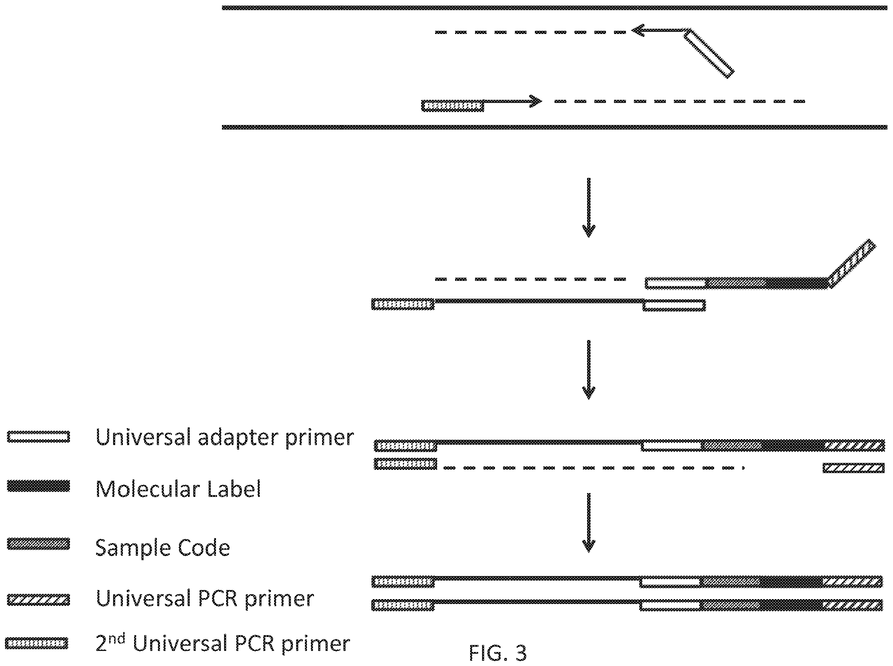

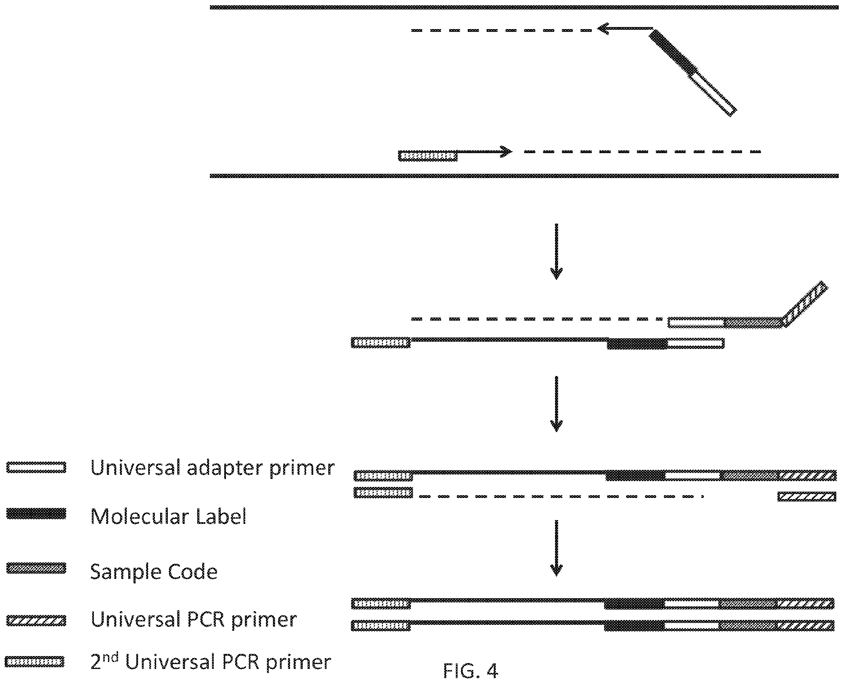

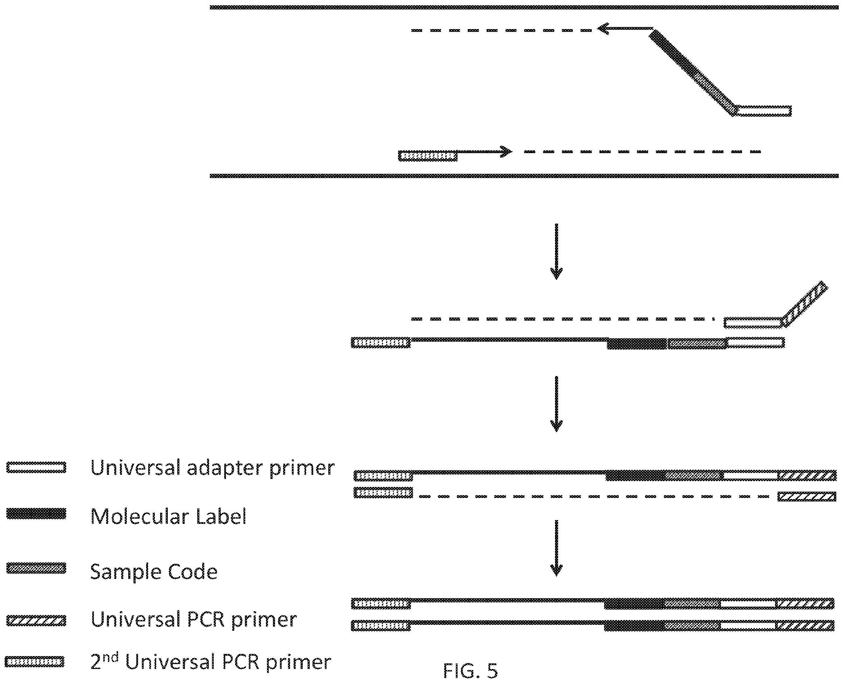

The labels of the stochastic barcode (e.g., sample label or sample code, which can be used herein is interchangeable with the term "sample label") can be in any order. **Figure** 3 illustrates an exemplary embodiment of the gene-specific stochastic barcode generating method of the disclosure such as illustrated in **Figure 2**, wherein the second extension is performed with a reverse gene-specific primer linked to a second universal primer and wherein the molecular label is 5' to the sample label. **Figure 2** illustrates the same method but with the sample label being 5' to the molecular label. All or part of the stochastic barcode may also be included in the oligonucleotide comprising the universal adaptor primer. For example, the labels of the stochastic barcode can be permutated such that the molecular barcode is 3' to the universal adaptor primer, as shown in **Figure 4****.** For example, both the sample label and molecular label can be 3' to the universal adaptor primer, as illustrated in **Figure 5**.

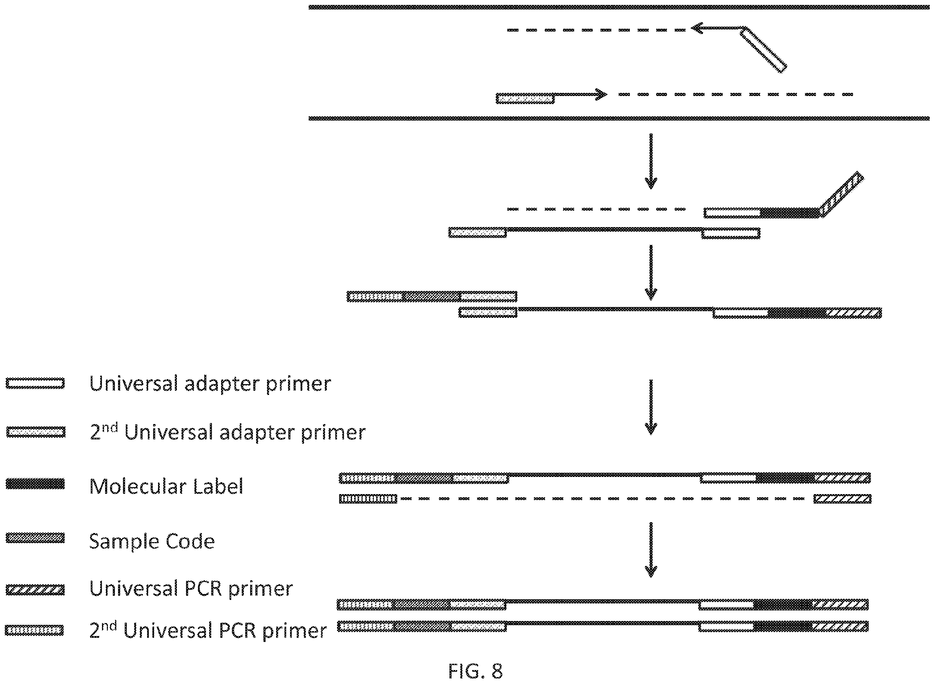

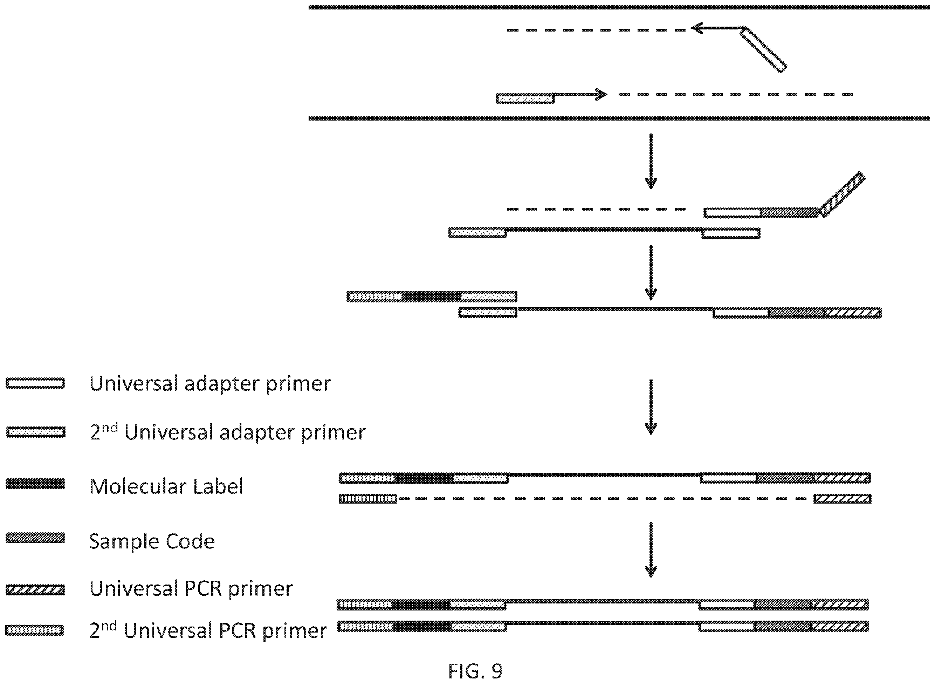

**Figure 8** discloses a method for generating a gene-specific stochastic barcode which utilizes two universal adaptor primer sequences. The target molecule can be contacted with a forward (e.g., first) and a reverse (e.g., second) universal adaptor primer sequences. The sequences can amplify the target. Stochastic barcodes can be associated with the universal adaptor primer sequences through their own sequences which can be complementary to the universal adaptor primer sequences. The stochastic barcodes can comprise any permutation of labels of the disclosure (e.g., sample labels, molecular labels). The target can be amplified using two universal labels (e.g., universal PCR primers). **Figure 9** depicts an alternative embodiment in which the sample label and the molecular label have changed position. **Figure 9** depicts an exemplary embodiment, that is not limiting, i.e., any label can be placed at any location in the configuration disclosed in **Figure 9****.**

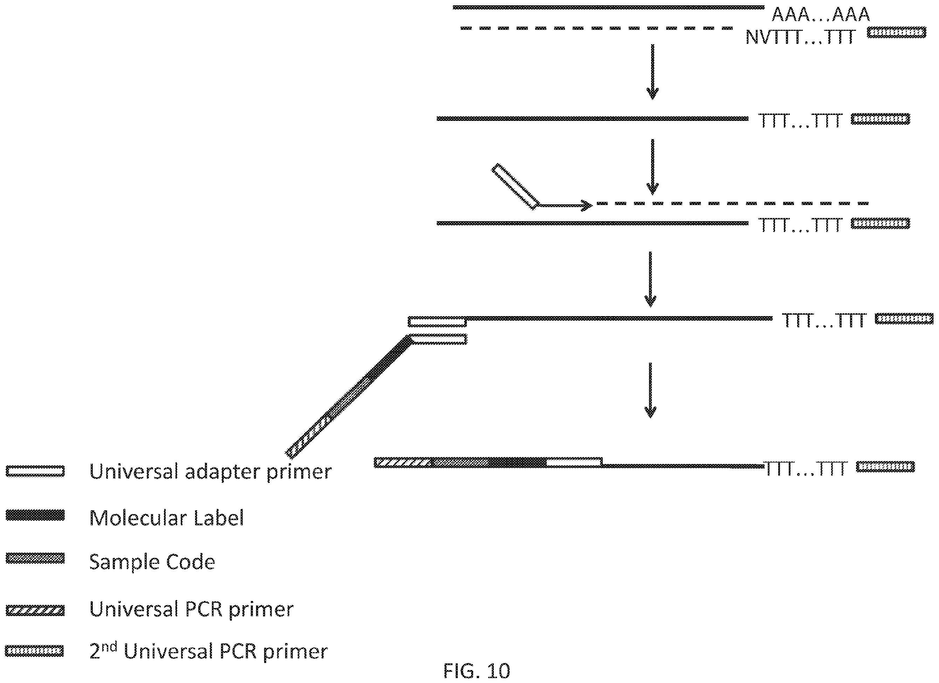

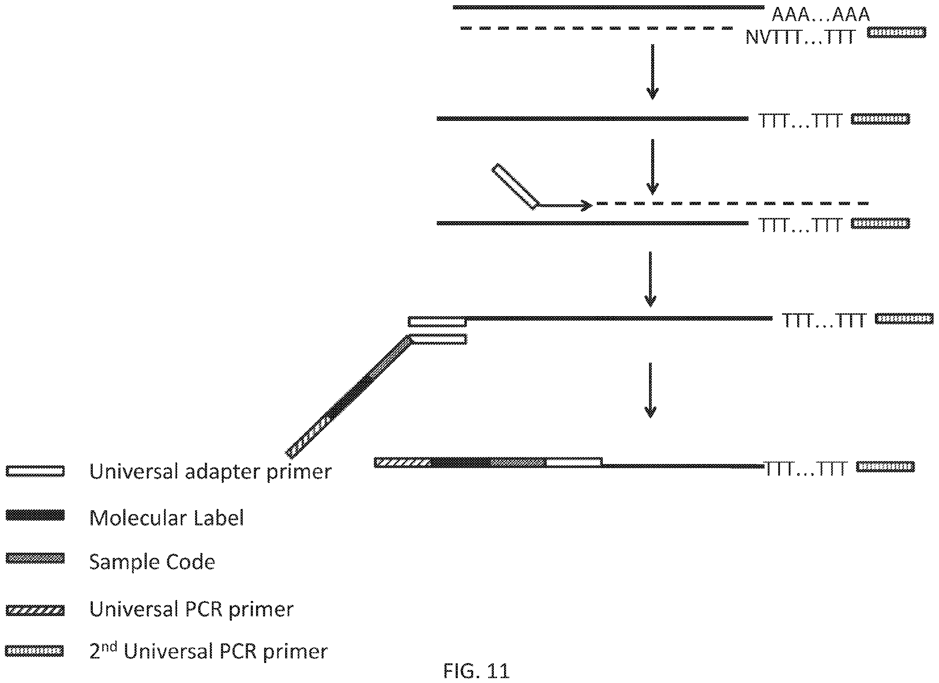

The methods of generating a gene-specific stochastic barcode can be performed at the 3' ends of molecules (e.g., at their polyadenylation sites). **Figure 10** illustrates an exemplary embodiment of using a gene-specific stochastic barcode to gene-specifically stochastically barcode a target. A target comprising a poly-A tail can hybridize to a polynucleotide comprising an oligo dT and a universal label (e.g., universal PCR primer). The oligo dT can be extended (e.g., reverse transcription) to incorporate the sequence of the target. A gene-specific primer attached to a universal adaptor primer can hybridize to the target sequence. Sequentially, or simultaneously, a stochastic barcode comprising a sequence complementary to the universal adaptor primer can hybridize to the universal adaptor primer and be extended (e.g., through primer extension), thereby generating a gene-specific stochastically barcoded target. The stochastically barcoded target can be amplified, sequenced, and/or digitally counted according to the methods of the disclosure. **Figure 10** is meant as an exemplary embodiment. The method disclosed in **Figure 10** can similarly be performed with a gene-specific primer sequence replacing the oligo dT sequence depicted. In the methods shown in **Figure 10**, the labels of the stochastic barcode can be in any order. For example, the sample label can be 5' of the molecular label. The molecular label can be 5' of the sample label, as shown in **Figure 11****.**

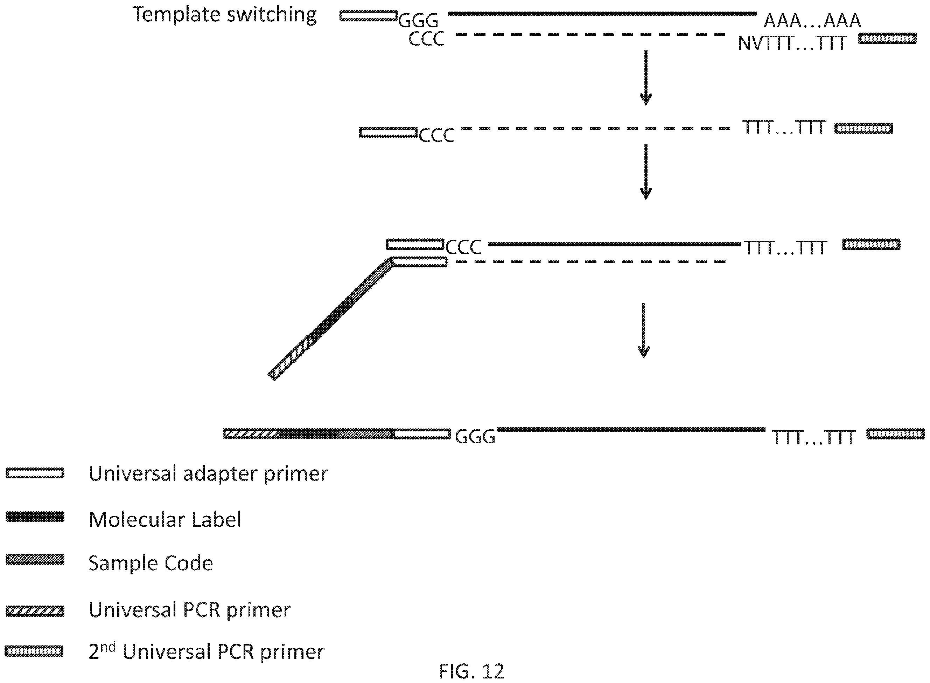

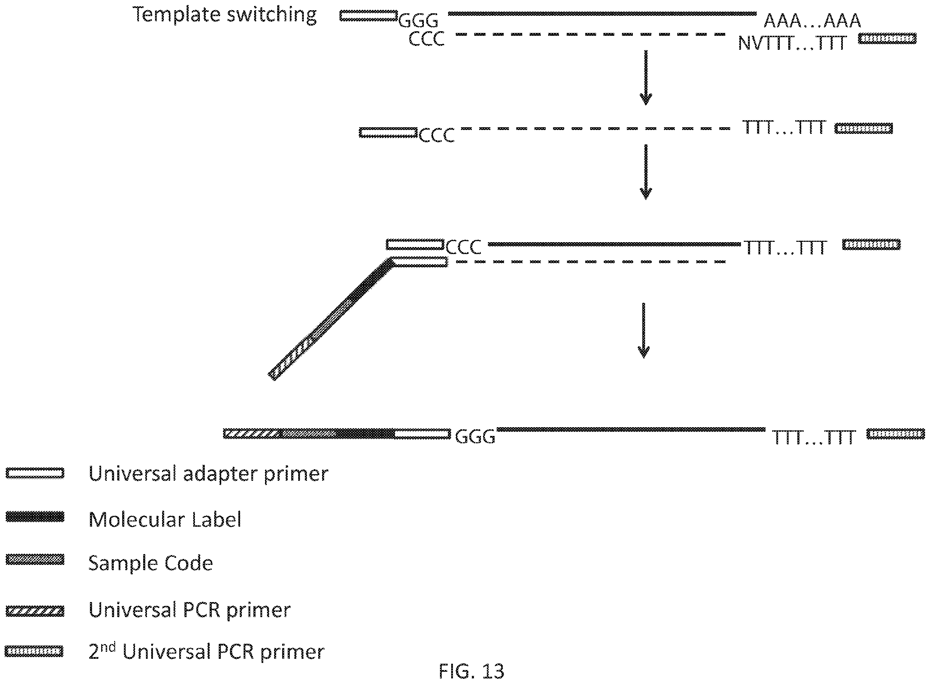

In some instances, template switching can be used to stochastically barcode a target using a gene-specific stochastic barcode. **Figure 12** depicts an exemplary embodiment of template switching. A target comprising, for example, a poly-A tail can be contacted to a polynucleotide comprising an oligo dT and a universal label (e.g., universal PCR primer. The oligo dT can be extended and tailed with an oligonucleotide sequence (e.g., CCC). Another oligonucleotide sequence encoding a universal adaptor primer and a sequence complementary to the tailed sequence (e.g., GGG) can be hybridized together, and incorporated into the transcript (e.g., template switching). A stochastic barcode comprising a sequence complementary to the universal adaptor primer sequence can hybridize to the universal adaptor primer and be extended (e.g., through primer extension), thereby generating a gene-specific stochastically labeled target. In the methods shown in **Figure 12**, the labels of the stochastic barcode can be in any order. For example, the molecular label can be 5' of the sample label. The sample label can be 5' of the molecular label, as shown in **Figure 13**.

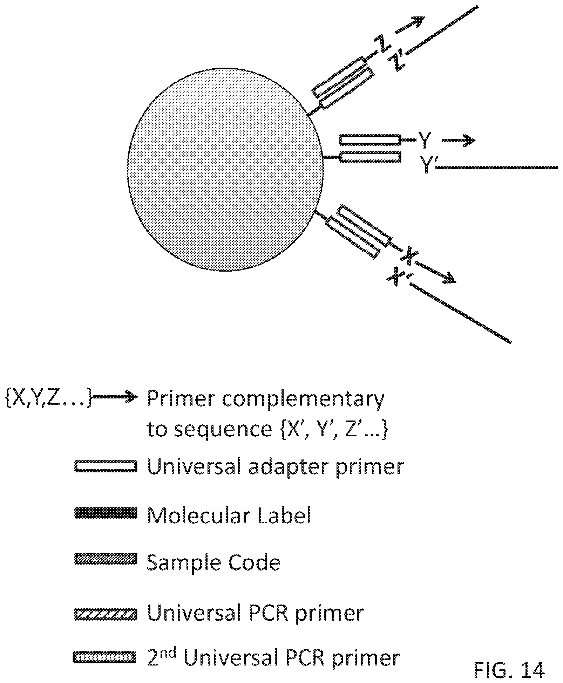

In some instances, a universal adaptor primer can be a support for generating a gene-specific stochastic barcode. For example, as shown in **Figure 14**, a universal adaptor primer can be attached to a solid support. The universal adaptor primer can be a support for another oligonucleotide that can comprise a sequence that can hybridize to the universal adaptor primer support and a gene-specific sequence (e.g., X, Y, Z). A complementary gene-specific sequence, (e.g., X', Y', Z') can be contacted to its cognate binding partner (e.g., X, Y, Z, respectively). X, Y, and Z, can be extended to incorporate the sequence of X', Y', and Z', thereby generating a gene-specific sequence attached to a universal adaptor primer.

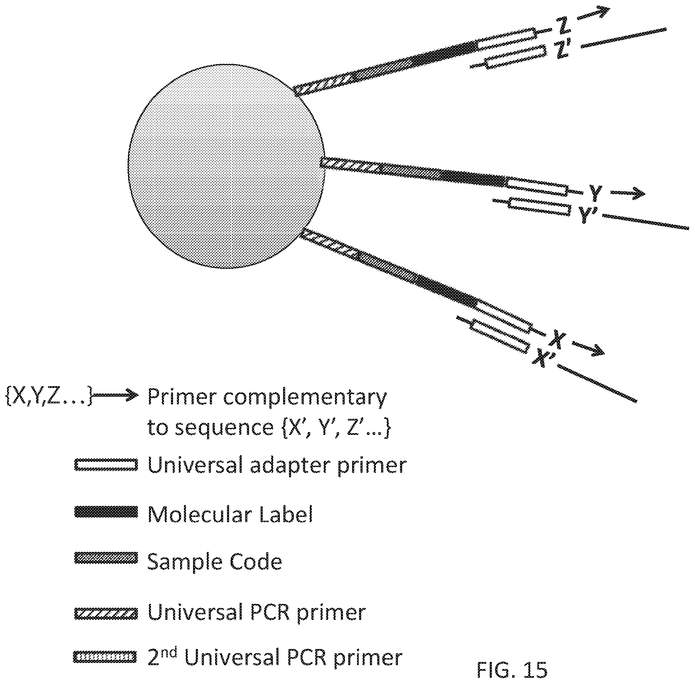

In some instances, a gene-specific stochastic barcode can be generated by hybridizing a stochastic barcode comprising a universal adaptor primer sequence with an oligonucleotide comprising a sequence complementary to the universal adaptor primer sequence (e.g., such that they can hybridize) and a sequence encoding a gene-specific primer. As shown in **Figure 15****,** an extension reaction can be performed to extend X, Y, and Z over the sequence of X', Y', and Z'. The sequences of X', Y', and Z' can be attached to a sequence that can hybridize to the universal adaptor primer of the stochastic barcode. The extension reaction can incorporate the sequence of X', Y', and Z' into the stochastic barcode, thereby generating a gene-specific stochastic barcode. The gene-specific stochastic barcode can be contacted to one or more targets in a sample according to the stochastic barcoding and counting methods of the disclosure. The targets associated with each gene-specific stochastic barcode can be amplified and/or counted to determine the number of distinct targets of each type in the sample. In some embodiments, the universal adaptor primer can be immobilized on a solid support, such as a bead, and used as a tether to immobilize a stochastic barcode through hybridization with a sequence complementary to the universal adaptor primer sequence linked to the stochastic barcode.

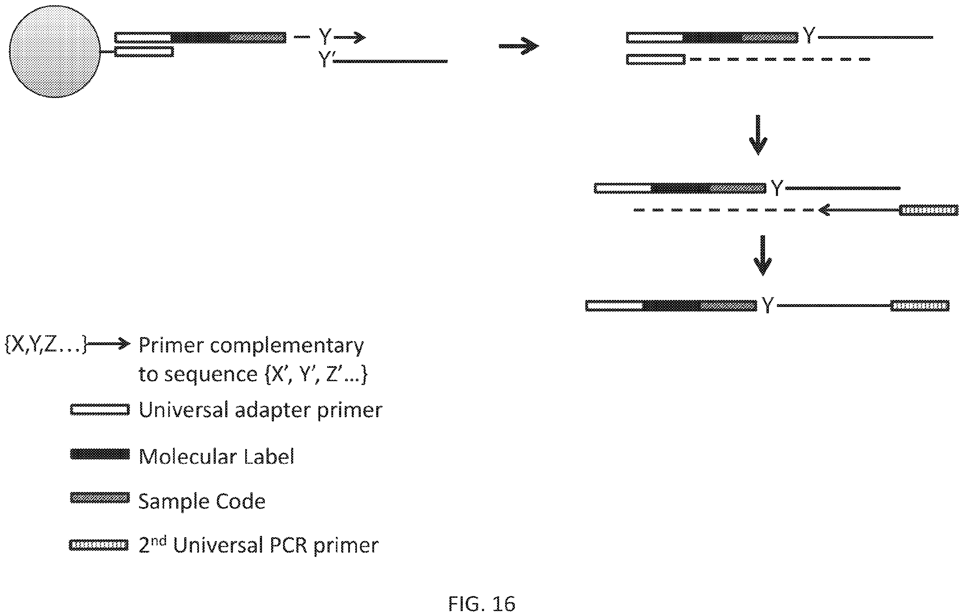

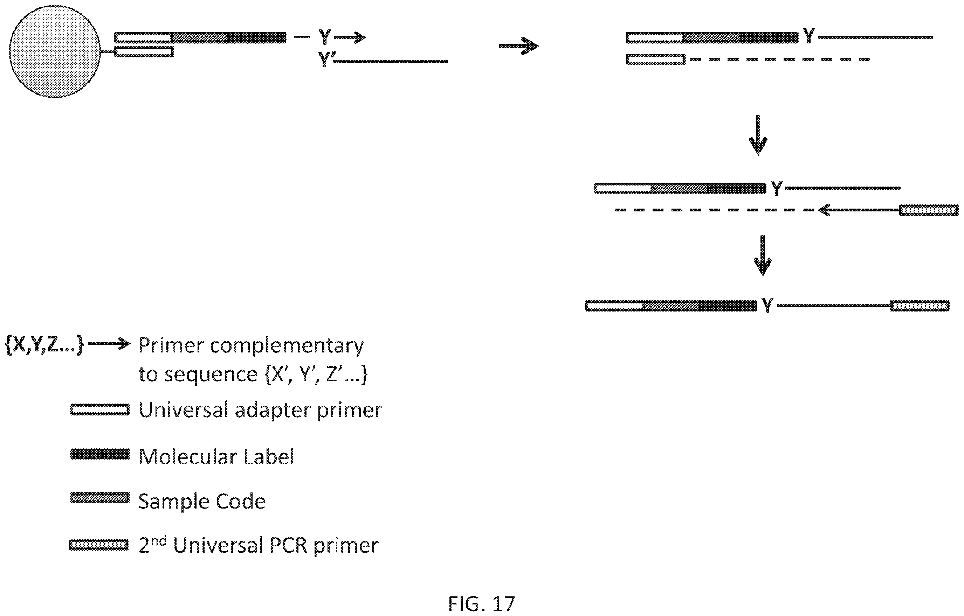

In some instances, the universal adaptor sequence can be 5' of the molecular label (and any other labels). **Figures 16** **and** **17** show an exemplary embodiment of how a solid support can comprise a sequence that can hybridize with a universal adaptor primer of the disclosure, which can be part of a stochastic barcode (or vice versa). The sequence that can hybridize to the universal adaptor primer can be attached to a solid support. The stochastic barcode can comprise a gene-specific binding region Y. Y', a sequence complementary to Y, can be hybridized to Y. Y can be extended to incorporate the sequence of Y', thereby generating a gene-specific stochastic barcode. A reverse primer, comprising a second universal PCR primer (e.g., a universal label) and a region complementary to Y can be used to generate a transcript comprising a stochastic barcode, a universal primer adaptor, a gene-specific binding region (Y), and a second universal PCR primer. The second universal sequence primer as depicted in **Figures 16** **and** **17** can be the universal sequence primer as described above, or provide the same function but with a different sequence. The universal sequencing primer (or second universal sequencing primer) can be used to sequence the transcript. The universal sequencing primer may not be associated in tandem (e.g., directly adjacent) to the labels of the disclosure (e.g., cellular label, molecular label, sample label). In the methods shown in **Figure 16****,** the labels of the stochastic barcode can be in any order. For example, the molecular label can be 5' of the sample label. The sample label can be 5' of the molecular label, as shown in **Figure 17****.**

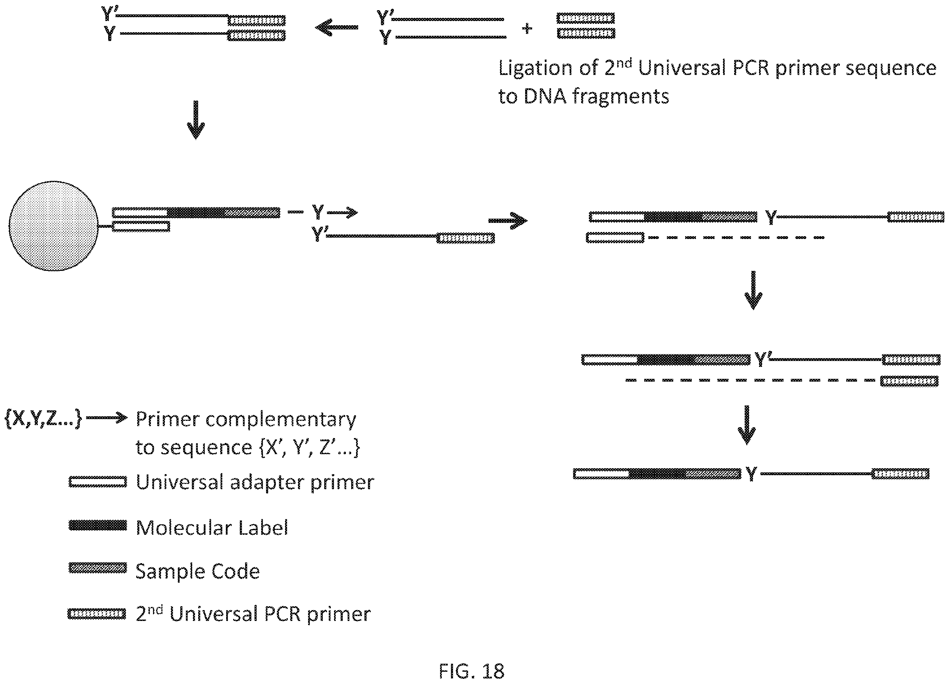

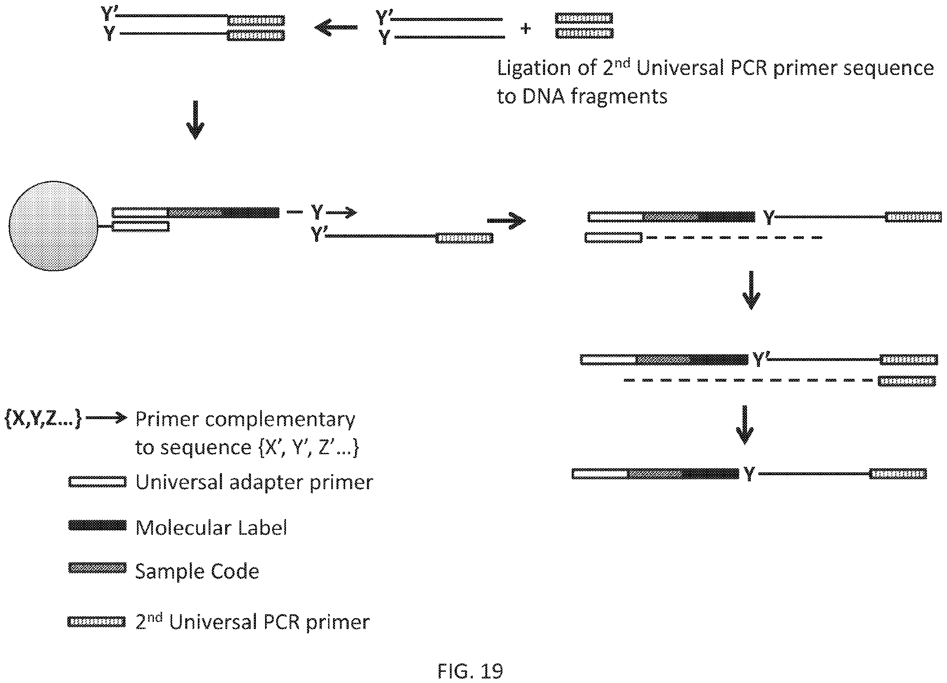

In some embodiments, the method disclosed in **Figures 16** **and** **17** can be performed with a sequence of Y' that comprises the second universal PCR primer (e.g., can also be the universal sequencing primer disclosed above), as shown in **Figures 18** **and** **19**. The oligonucleotide Y' comprising the second universal PCR primer can be contacted to a stochastic barcode comprising a gene-specific sequence Y, wherein Y can hybridize with Y'. The rest of the method can be performed as described for **Figures 16** **and** **17**. In the methods shown in **Figure 18****,** the labels of the stochastic barcode can be in any order. For example, the molecular label can be 5' of the sample label. The sample label can be 5' of the molecular label, as shown in **Figure 19****.**

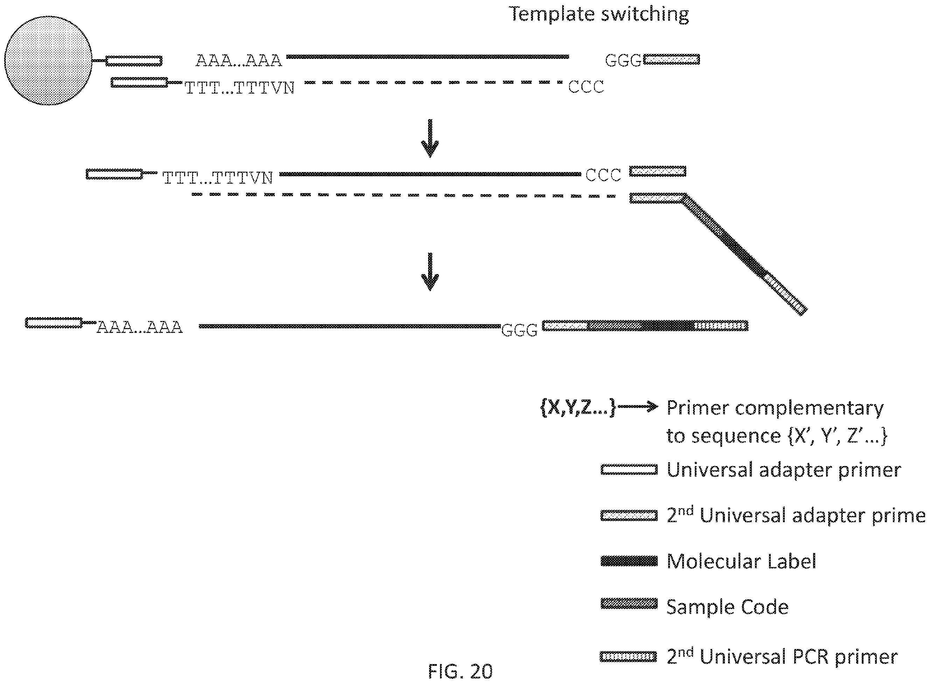

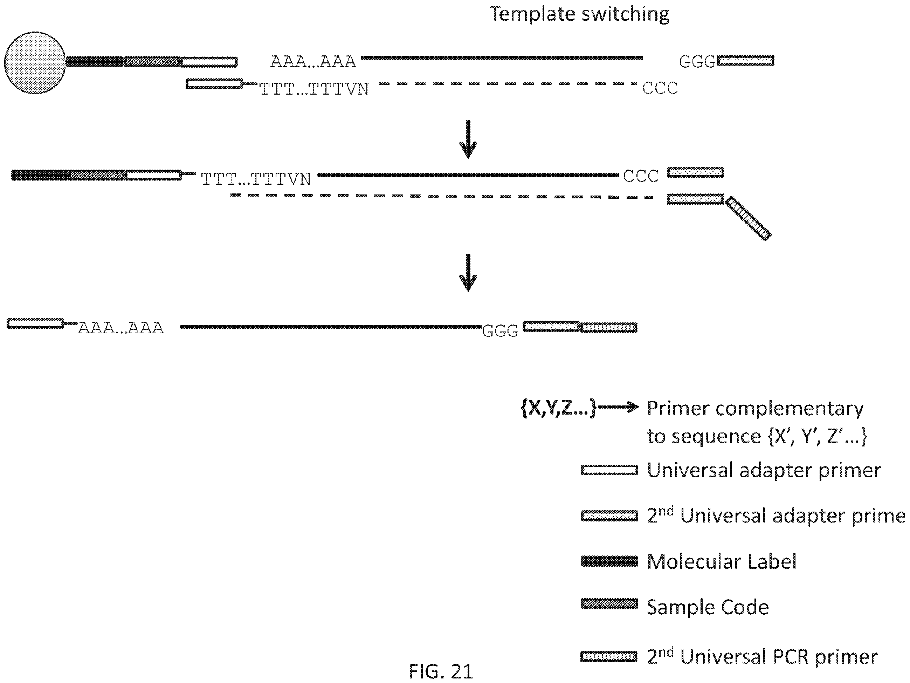

In some instances, template switching can be used to stochastically barcode a target using a gene-specific stochastic barcode can be performed substantially similar to the method disclosed in Figure 12. As shown in **Figure 20**, template switching can be performed using a universal adaptor primer attached to a solid support. A target comprising, for example, a poly-A tail can be contacted to a polynucleotide comprising an oligo dT and a universal label (e.g., universal PCR primer. The oligo dT can be extended and tailed with an oligonucleotide sequence (e.g., CCC). Another oligonucleotide sequence encoding a universal adaptor primer and a sequence complementary to the tailed sequence (e.g., GGG) can be hybridized together, and incorporated into the transcript (e.g., template switching). A stochastic barcode comprising a sequence complementary to the universal adaptor primer sequence can hybridize to the universal adaptor primer and be extended (e.g., through primer extension), thereby generating a gene-specific stochastically labeled target. In the methods shown in **Figure 20**, the labels of the stochastic barcode can be in any order. The stochastic barcode can hybridize to the second universal adaptor primer. In some instances, the stochastic barcode can be attached to the solid support as illustrated in **Figure 21****.** In some embodiments, the universal adaptor primer can be immobilized on a solid support, such as a bead, and used as a tether to immobilize a stochastic barcode through hybridization with a sequence complementary to the universal adaptor primer sequence linked to the stochastic barcode.

In all the methods described in **Figures 1-21**, the gene-specific stochastically barcoded target can be subjected to any other method of the disclosure, such as amplification, sequencing, and/or digitally counting to determine the number of unique copies of the target in a sample.

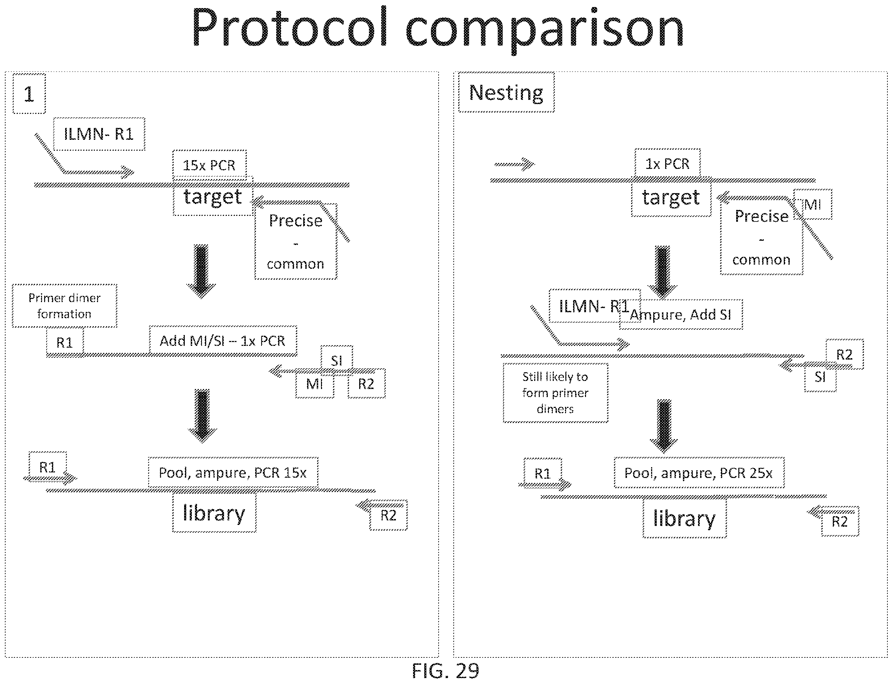

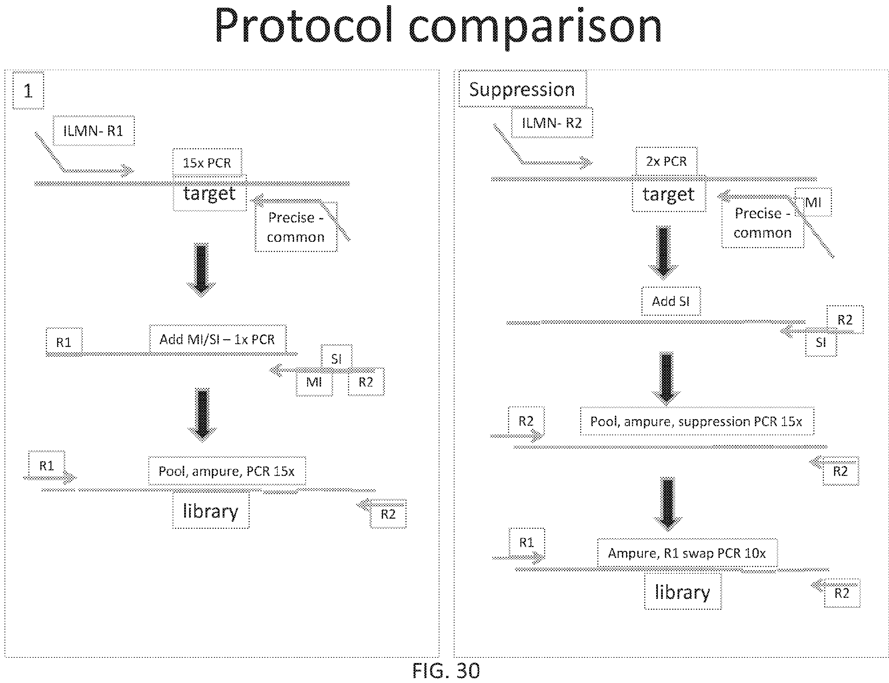

**Figure 29** illustrates an exemplary embodiment of a method to reduce primer dimers during amplification. On the left shows an exemplary method to stochastically barcode an amplified product (e.g., amplified in the first step). Primer dimers may form. On the right, is an exemplary embodiment for a method for reducing primer dimer formation. A molecular label can be introduced 3' of the universal adaptor primer. One PCR amplification reaction can be performed which can stochastically barcode the target, instead of barcoding amplified copies of the target. Primer dimers can be reduced by the introduction of a molecular label during the first amplification reaction. **Figure 30** shows a similar method to **Figure 29**, but the amplification primer comprises a universal sequence (e.g., compatible with Illumina sequencers). Excess molecular indexes can be removed prior to amplification, as shown in **Figure 30** on the right.

### Solid Supports

The stochastic barcodes disclosed herein may be attached to a solid support (e.g., bead, substrate). As used herein, the terms "tethered", "attached", and "immobilized" are used interchangeably, and may refer to covalent or non-covalent means for attaching stochastic barcodes to a solid support. Any of a variety of different solid supports may be used as solid supports for attaching pre-synthesized stochastic barcodes or for *in situ* solid-phase synthesis of stochastic barcode.

In some instances, a solid support is a bead. A bead may encompass any type of solid, porous, or hollow sphere, ball, bearing, cylinder, or other similar configuration composed of plastic, ceramic, metal, or polymeric material onto which a nucleic acid may be immobilized (e.g., covalently or non-covalently). A bead may comprise a discrete particle that may be spherical (e.g., microspheres) or have a non-spherical or irregular shape, such as cubic, cuboid, pyramidal, cylindrical, conical, oblong, or disc-shaped, and the like. A bead may be non-spherical in shape.

Beads may comprise a variety of materials including, but not limited to, paramagnetic materials (e.g. magnesium, molybdenum, lithium, and tantalum), superparamagnetic materials (e.g. ferrite (Fe₃O₄; magnetite) nanoparticles), ferromagnetic materials (e.g. iron, nickel, cobalt, some alloys thereof, and some rare earth metal compounds), ceramic, plastic, glass, polystyrene, silica, methylstyrene, acrylic polymers, titanium, latex, sepharose, agarose, hydrogel, polymer, cellulose, nylon, and any combination thereof.

The diameter of the beads may be at least about 5µm, 10µm, 20µm, 25µm, 30µm, 35µm, 40µm, 45µm or 50µm. The diameter of the beads may be at most about 5µm, 10µm, 20µm, 25µm, 30µm, 35µm, 40µm, 45µm or 50µm.

A bead may be attached to and/or embedded in a substrate. A bead may be attached to and/or embedded in a gel, hydrogel, polymer and/or matrix. The spatial position of a bead within a substrate (e.g., gel, matrix, scaffold, or polymer) may be identified using the spatial label present on the stochastic barcode on the bead which can serve as a location address.

Examples of beads can include, but are not limited to, streptavidin beads, agarose beads, magnetic beads, Dynabeads^{®}, MACS^{®} microbeads, antibody conjugated beads (e.g., anti-immunoglobulin microbead), protein A conjugated beads, protein G conjugated beads, protein A/G conjugated beads, protein L conjugated beads, oligodT conjugated beads, silica beads, silica-like beads, anti-biotin microbead, anti-fluorochrome microbead, and BcMag^{™} Carboxy-Terminated Magnetic Beads.

A bead may be associated with (e.g. impregnated with) quantum dots or fluorescent dyes to make it fluorescent in one fluorescence optical channel or multiple optical channels. A bead may be associated with iron oxide or chromium oxide to make it paramagnetic or ferromagnetic. Beads can be identifiable. For example, a bead can be imaged using a camera. A bead can have a detectable code associated with the bead. For example, a bead can comprise a stochastic barcode. A bead can change size, for example due to swelling in an organic or inorganic solution. A bead can be hydrophobic. A bead can be hydrophilic. A bead can be biocompatible.

A solid support (e.g., bead) can be visualized. The solid support can comprise a visualizing tag (e.g., fluorescent dye). A solid support (e.g., bead) can be etched with an identifier (e.g., a number). The identifier can be visualized through imaging the beads.

A solid support may refer to an insoluble, semi-soluble, or insoluble material. A solid support may be referred to as "functionalized" when it includes a linker, a scaffold, a building block, or other reactive moiety attached thereto, whereas a solid support may be "nonfunctionalized" when it lack such a reactive moiety attached thereto. The solid support may be employed free in solution, such as in a microtiter well format; in a flow-through format, such as in a column; or in a dipstick.

The solid support may comprise a membrane, paper, plastic, coated surface, flat surface, glass, slide, chip, or any combination thereof. A solid support may take the form of resins, gels, microspheres, or other geometric configurations. A solid support can comprise silica chips, microparticles, nanoparticles, plates, and arrays. Solid supports may include beads (e.g., silica gel, controlled pore glass, magnetic beads, Dynabeads, Wang resin; Merrifield resin, Sephadex/Sepharose beads, cellulose beads, polystyrene beads etc.), capillaries, flat supports such as glass fiber filters, glass surfaces, metal surfaces (steel, gold silver, aluminum, silicon and copper), glass supports, plastic supports, silicon supports, chips, filters, membranes, microwell plates, slides, or the like. plastic materials including multiwell plates or membranes (e.g., formed of polyethylene, polypropylene, polyamide, polyvinylidenedifluoride), wafers, combs, pins or needles (e.g., arrays of pins suitable for combinatorial synthesis or analysis) or beads in an array of pits or nanoliter wells of flat surfaces such as wafers (e.g., silicon wafers), wafers with pits with or without filter bottoms.

In some instances stochastic barcodes of the disclosure can be attached to a polymer matrix (e.g., gel, hydrogel). The polymer matrix may be able to permeate intracellular space (e.g., around organelles). The polymer matrix may able to be pumped throughout the circulatory system.

A solid support can be a biological molecule. For example a solid support can be a nucleic acid, a protein, an antibody, a histone, a cellular compartment, a lipid, a carbohydrate, and the like. Solid supports that are biological molecules can be amplified, translated, transcribed, degraded, and/or modified (e.g., pegylated, sumoylated). A solid support that is a biological molecule can provide spatial and time information in addition to the spatial label that is attached to the biological molecule. For example, a biological molecule can comprise a first confirmation when unmodified, but can change to a second confirmation when modified. The different conformations can expose stochastic barcodes of the disclosure to targets. For example, a biological molecule can comprise stochastic barcodes that are unaccessible due to folding of the biological molecule. Upon modification of the biological molecule (e.g., acetylation), the biological molecule can change conformation to expose the stochastic labels. The timing of the modification can provide another time dimension to the method of stochastic barcoding of the disclosure.

In another example, the biological molecule comprising stochastic barcodes of the disclosure can be located in the cytoplasm of a cell. Upon activation, the biological molecule can move to the nucleus, whereupon stochastic barcoding can take place. In this way, modification of the biological molecule can encode additional space-time information for the targets identified by the stochastic barcodes.

A dimension label can provide information about space-time of a biological event (e.g., cell division). For example, a dimension label can be added to a first cell, the first cell can divide generating a second daughter cell, the second daughter cell can comprise all, some or none of the dimension labels. The dimension labels can be activated in the original cell and the daughter cell. In this way, the dimension label can provide information about time of stochastic barcoded in distinct spaces.

### Substrates

A substrate can refer to a type of solid support. A substrate can refer to a solid support that can comprise stochastic barcodes of the disclosure. A substrate can comprise a plurality of microwells. A microwell can comprise a small reaction chamber of defined volume. A microwell can entrap one or more cells. A microwell can entrap only one cell. A microwell can entrap one or more solid supports. A microwell can entrap only one solid support. In some instances, a microwell entraps a single cell and a single solid support (e.g., bead).

The microwells of the array can be fabricated in a variety of shapes and sizes. Appropriate well geometries can include, but are not limited to, cylindrical, conical, hemispherical, rectangular, or polyhedral (e.g., three dimensional geometries comprised of several planar faces, for example, hexagonal columns, octagonal columns, inverted triangular pyramids, inverted square pyramids, inverted pentagonal pyramids, inverted hexagonal pyramids, or inverted truncated pyramids). The microwells may comprise a shape that combines two or more of these geometries. For example, a microwell may be partly cylindrical, with the remainder having the shape of an inverted cone. A microwell may include two side-by-side cylinders, one of larger diameter (e.g. that corresponds roughly to the diameter of the beads) than the other (e.g. that corresponds roughly to the diameter of the cells), that are connected by a vertical channel (that is, parallel to the cylinder axes) that extends the full length (depth) of the cylinders. The opening of the microwell may be at the upper surface of the substrate. The opening of the microwell may be at the lower surface of the substrate. The closed end (or bottom) of the microwell can be flat. The closed end (or bottom) of the microwell can have a curved surface (e.g., convex or concave). The shape and/or size of the microwell can be determined based on the types of cells or solid supports to be trapped within the microwells.

Microwell dimensions may be characterized in terms of the diameter and depth of the well. As used herein, the diameter of the microwell refers to the largest circle that can be inscribed within the planar cross-section of the microwell geometry. The diameter of the microwells may range from about 1-fold to about 10-fold the diameter of the cells or solid supports to be trapped within the microwells. The microwell diameter can be at least 1-fold, at least 1.5-fold, at least 2-fold, at least 3-fold, at least 4-fold, at least 5-fold, or at least 10-fold the diameter of the cells or solid supports to be trapped within the microwells. The microwell diameter can be at most 10-fold, at most 5-fold, at most 4-fold, at most 3-fold, at most 2-fold, at most 1.5-fold, or at most 1-fold the diameter of the cells or solid supports to be trapped within the microwells. The microwell diameter can be about 2.5-fold the diameter of the cells or solid supports to be trapped within the microwells.

The diameter of the microwells can be specified in terms of absolute dimensions. The diameter of the microwells may range from about 5 to about 50 micrometers. The microwell diameter can be at least 5 micrometers, at least 10 micrometers, at least 15 micrometers, at least 20 micrometers, at least 25 micrometers, at least 30 micrometers, at least 35 micrometers, at least 40 micrometers, at least 45 micrometers, or at least 50 micrometers. The microwell diameter can be at most 50 micrometers, at most 45 micrometers, at most 40 micrometers, at most 35 micrometers, at most 30 micrometers, at most 25 micrometers, at most 20 micrometers, at most 15 micrometers, at most 10 micrometers, or at most 5 micrometers. The microwell diameter can be about 30 micrometers.

The microwell depth may be chosen to provide efficient trapping of cells and solid supports. The microwell depth may be chosen to provide efficient exchange of assay buffers and other reagents contained within the wells. The ratio of diameter to height (i.e. aspect ratio) may be chosen such that once a cell and solid support settle inside a microwell, they will not be displaced by fluid motion above the microwell. The dimensions of the microwell may be chosen such that the microwell has sufficient space to accommodate a solid support and a cell of various sizes without being dislodged by fluid motion above the microwell. The depth of the microwells may range from about 1-fold to about 10-fold the diameter of the cells or solid supports to be trapped within the microwells. The microwell depth can be at least 1-fold, at least 1.5-fold, at least 2-fold, at least 3-fold, at least 4-fold, at least 5-fold, or at least 10-fold the diameter of the cells or solid supports to be trapped within the microwells. The microwell depth can be at most 10-fold, at most 5-fold, at most 4-fold, at most 3-fold, at most 2-fold, at most 1.5-fold, or at most 1-fold the diameter of the cells or solid supports to be trapped within the microwells. The microwell depth can be about 2.5-fold the diameter of the cells or solid supports to be trapped within the microwells.

The depth of the microwells can be specified in terms of absolute dimensions. The depth of the microwells may range from about 10 to about 60 micrometers. The microwell depth can be at least 10 micrometers, at least 20 micrometers, at least 25 micrometers, at least 30 micrometers, at least 35 micrometers, at least 40 micrometers, at least 50 micrometers, or at least 60 micrometers. The microwell depth can be at most 60 micrometers, at most 50 micrometers, at most 40 micrometers, at most 35 micrometers, at most 30 micrometers, at most 25 micrometers, at most 20 micrometers, or at most 10 micrometers. The microwell depth can be about 30 micrometers.

The volume of the microwells used in the methods, devices, and systems of the present disclosure may range from about 200 micrometers³ to about 120,000 micrometers³. The microwell volume can be at least 200 micrometers³, at least 500 micrometers³, at least 1,000 micrometers³, at least 10,000 micrometers³, at least 25,000 micrometers³, at least 50,000 micrometers³, at least 100,000 micrometers³, or at least 120,000 micrometers³. The microwell volume can be at most 120,000 micrometers³, at most 100,000 micrometers³, at most 50,000 micrometers³, at most 25,000 micrometers³, at most 10,000 micrometers³, at most 1,000 micrometers³, at most 500 micrometers³, or at most 200 micrometers³. The microwell volume can be about 25,000 micrometers³. The microwell volume may fall within any range bounded by any of these values (e.g. from about 18,000 micrometers³ to about 30,000 micrometers³).

The volumes of the microwells used in the methods, devices, and systems of the present disclosure may be further characterized in terms of the variation in volume from one microwell to another. The coefficient of variation (expressed as a percentage) for microwell volume may range from about 1% to about 10%. The coefficient of variation for microwell volume may be at least 1%, at least 2%, at least 3%, at least 4%, at least 5%, at least 6%, at least 7%, at least 8%, at least 9%, or at least 10%. The coefficient of variation for microwell volume may be at most 10%, at most 9%, at most 8%, at most 7%, at most 6%, at most 5%, at most 4%, at most 3%, at most 2%, or at most 1%. The coefficient of variation for microwell volume may have any value within a range encompassed by these values, for example between about 1.5% and about 6.5%. In some embodiments, the coefficient of variation of microwell volume may be about 2.5%.

The ratio of the volume of the microwells to the surface area of the beads (or to the surface area of a solid support to which stochastic barcode oligonucleotides may be attached) used in the methods, devices, and systems of the present disclosure may range from about 2.5 to about 1,520 micrometers. The ratio can be at least 2.5, at least 5, at least 10, at least 100, at least 500, at least 750, at least 1,000, or at least 1,520. The ratio can be at most 1,520, at most 1,000, at most 750, at most 500, at most 100, at most 10, at most 5, or at most 2.5. The ratio can be about 67.5. The ratio of microwell volume to the surface area of the bead (or solid support used for immobilization) may fall within any range bounded by any of these values (e.g. from about 30 to about 120).

The wells of the microwell array may be arranged in a one dimensional, two dimensional, or three-dimensional array. A three dimensional array may be achieved, for example, by stacking a series of two or more two dimensional arrays (that is, by stacking two or more substrates comprising microwell arrays).

The pattern and spacing between microwells can be chosen to optimize the efficiency of trapping a single cell and single solid support (e.g., bead) in each well, as well as to maximize the number of wells per unit area of the array. The microwells may be distributed according to a variety of random or non-random patterns. For example, they may be distributed entirely randomly across the surface of the array substrate, or they may be arranged in a square grid, rectangular grid, hexagonal grid, or the like. The center-to-center distance (or spacing) between wells may vary from about 15 micrometers to about 75 micrometers. In other embodiments, the spacing between wells is at least 15 micrometers, at least 20 micrometers, at least 25 micrometers, at least 30 micrometers, at least 35 micrometers, at least 40 micrometers, at least 45 micrometers, at least 50 micrometers, at least 55 micrometers, at least 60 micrometers, at least 65 micrometers, at least 70 micrometers, or at least 75 micrometers. The microwell spacing can be at most 75 micrometers, at most 70 micrometers, at most 65 micrometers, at most 60 micrometers, at most 55 micrometers, at most 50 micrometers, at most 45 micrometers, at most 40 micrometers, at most 35 micrometers, at most 30 micrometers, at most 25 micrometers, at most 20 micrometers, or at most 15 micrometers. The microwell spacing can be about 55 micrometers. The microwell spacing may fall within any range bounded by any of these values (e.g. from about 18 micrometers to about 72 micrometers).

The microwell array may comprise surface features between the microwells that are designed to help guide cells and solid supports into the wells and/or prevent them from settling on the surfaces between wells. Examples of suitable surface features can include, but are not limited to, domed, ridged, or peaked surface features that encircle the wells or straddle the surface between wells.

The total number of wells in the microwell array can be determined by the pattern and spacing of the wells and the overall dimensions of the array. The number of microwells in the array may range from about 96 to about 5,000,000 or more. The number of microwells in the array can be at least 96, at least 384, at least 1,536, at least 5,000, at least 10,000, at least 25,000, at least 50,000, at least 75,000, at least 100,000, at least 500,000, at least 1,000,000, or at least 5,000,000. The number of microwells in the array can be at most 5,000,000, at most 1,000,000, at most 75,000, at most 50,000, at most 25,000, at most 10,000, at most 5,000, at most 1,536, at most 384, or at most 96 wells. The number of microwells in the array can be about 96. The number of microwells can be about 150,000. The number of microwells in the array may fall within any range bounded by any of these values (e.g. from about 100 to 325,000).

Microwell arrays may be fabricated using any of a number of fabrication techniques. Examples of fabrication methods that may be used include, but are not limited to, bulk micromachining techniques such as photolithography and wet chemical etching, plasma etching, or deep reactive ion etching; micro-molding and micro-embossing; laser micromachining; 3D printing or other direct write fabrication processes using curable materials; and similar techniques.

Microwell arrays may be fabricated from any of a number of substrate materials. The choice of material can depend on the choice of fabrication technique, and vice versa. Examples of suitable materials can include, but are not limited to, silicon, fused-silica, glass, polymers (e.g. agarose, gelatin, hydrogels, polydimethylsiloxane (PDMS; elastomer), polymethylmethacrylate (PMMA), polycarbonate (PC), polypropylene (PP), polyethylene (PE), high density polyethylene (HDPE), polyimide, cyclic olefin polymers (COP), cyclic olefin copolymers (COC), polyethylene terephthalate (PET), epoxy resins, thiol-ene based resins, metals or metal films (e.g. aluminum, stainless steel, copper, nickel, chromium, and titanium), and the like. A hydrophilic material can be desirable for fabrication of the microwell arrays (e.g. to enhance wettability and minimize non-specific binding of cells and other biological material). Hydrophobic materials that can be treated or coated (e.g. by oxygen plasma treatment, or grafting of a polyethylene oxide surface layer) can also be used. The use of porous, hydrophilic materials for the fabrication of the microwell array may be desirable in order to facilitate capillary wicking/venting of entrapped air bubbles in the device. T the microwell array can be fabricated from a single material. The microwell array may comprise two or more different materials that have been bonded together or mechanically joined.

Microwell arrays may be fabricated using substrates of any of a variety of sizes and shapes. For example, the shape (or footprint) of the substrate within which microwells are fabricated may be square, rectangular, circular, or irregular in shape. The footprint of the microwell array substrate can be similar to that of a microtiter plate. The footprint of the microwell array substrate can be similar to that of standard microscope slides, e.g. about 75 mm long x 25 mm wide (about 3" long x 1" wide), or about 75 mm long x 50 mm wide (about 3" long x 2" wide). The thickness of the substrate within which the microwells are fabricated may range from about 0.1 mm thick to about 10 mm thick, or more. The thickness of the microwell array substrate may be at least 0.1 mm thick, at least 0.5 mm thick, at least 1 mm thick, at least 2 mm thick, at least 3 mm thick, at least 4 mm thick, at least 5 mm thick, at least 6 mm thick, at least 7 mm thick, at least 8 mm thick, at least 9 mm thick, or at least 10 mm thick. The thickness of the microwell array substrate may be at most 10 mm thick, at most 9 mm thick, at most 8 mm thick, at most 7 mm thick, at most 6 mm thick, at most 5 mm thick, at most 4 mm thick, at most 3 mm thick, at most 2 mm thick, at most 1 mm thick, at most 0.5 mm thick, or at most 0.1 mm thick. The thickness of the microwell array substrate can be about 1 mm thick. The thickness of the microwell array substrate may be any value within these ranges, for example, the thickness of the microwell array substrate may be between about 0.2 mm and about 9.5 mm.

A variety of surface treatments and surface modification techniques may be used to alter the properties of microwell array surfaces. Examples can include, but are not limited to, oxygen plasma treatments to render hydrophobic material surfaces more hydrophilic, the use of wet or dry etching techniques to smooth (or roughen) glass and silicon surfaces, adsorption or grafting of polyethylene oxide or other polymer layers (such as pluronic), or bovine serum albumin to substrate surfaces to render them more hydrophilic and less prone to non-specific adsorption of biomolecules and cells, the use of silane reactions to graft chemically-reactive functional groups to otherwise inert silicon and glass surfaces, etc. Photodeprotection techniques can be used to selectively activate chemically-reactive functional groups at specific locations in the array structure, for example, the selective addition or activation of chemically-reactive functional groups such as primary amines or carboxyl groups on the inner walls of the microwells may be used to covalently couple oligonucleotide probes, peptides, proteins, or other biomolecules to the walls of the microwells. The choice of surface treatment or surface modification utilized can depend both or either on the type of surface property that is desired and on the type of material from which the microwell array is made.

The openings of microwells can be sealed, for example, during cell lysis steps to prevent cross hybridization of target nucleic acid between adjacent microwells. A microwell (or array of microwells) may be sealed or capped using, for example, a flexible membrane or sheet of solid material (i.e. a plate or platten) that clamps against the surface of the microwell array substrate, or a suitable bead, where the diameter of the bead is larger than the diameter of the microwell.

A seal formed using a flexible membrane or sheet of solid material can comprise, for example, inorganic nanopore membranes (e.g., aluminum oxides), dialysis membranes, glass slides, coverslips, elastomeric films (e.g. PDMS), or hydrophilic polymer films (e.g., a polymer film coated with a thin film of agarose that has been hydrated with lysis buffer).

Solid supports (e.g., beads) used for capping the microwells may comprise any of the solid supports (e.g., beads) of the disclosure. In some instances, the solid supports are cross-linked dextran beads (e.g., Sephadex). Cross-linked dextran can range from about 10 micrometers to about 80 micrometers. The cross-linked dextran beads used for capping can be from 20 micrometers to about 50 micrometers. In some embodiments, the beads may be at least about 10, 20, 30, 40, 50, 60, 70, 80 or 90% larger than the diameter of the microwells. The beads used for capping may be at most about 10, 20, 30, 40, 50, 60, 70, 80 or 90% larger than the diameter of the microwells.

The seal or cap may allow buffer to pass into and out of the microwell, while preventing macromolecules (e.g., nucleic acids) from migrating out of the well. A macromolecule of at least about 1, 2, 3, 4, 5, 6, 7, 8, 9, 10, 12, 13, 14, 15, 16, 17, 18, 19, or 20 or more nucleotides may be blocked from migrating into or out of the microwell by the seal or cap. A macromolecule of at most about 1, 2, 3, 4, 5, 6, 7, 8, 9, 10, 12, 13, 14, 15, 16, 17, 18, 19, or 20 or more nucleotides may be blocked from migrating into or out of the microwell by the seal or cap.

Solid supports (e.g., beads) may be distributed among a substrate. Solid supports (e.g., beads) can be distributed among wells of the substrate, removed from the wells of the substrate, or otherwise transported through a device comprising one or more microwell arrays by means of centrifugation or other non-magnetic means. A microwell of a substrate can be pre-loaded with a solid support. A microwell of a substrate can hold at least 1, 2, 3, 4, or 5, or more solid supports. A microwell of a substrate can hold at most 1, 2, 3, 4, or 5 or more solid supports. In some instances, a microwell of a substrate can hold one solid support.

Individual cells and beads may be compartmentalized using alternatives to microwells, for example, a single solid support and single cell could be confined within a single droplet in an emulsion (e.g. in a droplet digital microfluidic system).

Cells could potentially be confined within porous beads that themselves comprise the plurality of tethered stochastic barcodes. Individual cells and solid supports may be compartmentalized in any type of container, microcontainer, reaction chamber, reaction vessel, or the like.

Single cell, stochastic barcoding or may be performed without the use of microwells. Single cell, stochastic barcoding assays may be performed without the use of any physical container. For example, stochastic barcoding without a physical container can be performed by embedding cells and beads in close proximity to each other within a polymer layer or gel layer to create a diffusional barrier between different cell/bead pairs. In another example, stochastic barcoding without a physical container can be performed in situ, in vivo, on an intact solid tissue, on an intact cell, and/or subcellularly.

Microwell arrays can be a consumable component of the assay system. Microwell arrays may be reusable. Microwell arrays can be configured for use as a stand-alone device for performing assays manually, or they may be configured to comprise a fixed or removable component of an instrument system that provides for full or partial automation of the assay procedure. In some embodiments of the disclosed methods, the bead-based libraries of stochastic barcodes can be deposited in the wells of the microwell array as part of the assay procedure. In some embodiments, the beads may be pre-loaded into the wells of the microwell array and provided to the user as part of, for example, a kit for performing stochastic barcoding and digital counting of nucleic acid targets.

In some embodiments, two mated microwell arrays may be provided, one pre-loaded with beads which are held in place by a first magnet, and the other for use by the user in loading individual cells. Following distribution of cells into the second microwell array, the two arrays may be placed face-to-face and the first magnet removed while a second magnet is used to draw the beads from the first array down into the corresponding microwells of the second array, thereby ensuring that the beads rest above the cells in the second microwell array and thus minimizing diffusional loss of target molecules following cell lysis, while maximizing efficient attachment of target molecules to the stochastic barcodes on the bead.

### Three-dimensional substrates

A three-dimensional array may be any shape. A three-dimensional substrate can be made of any material used in a substrate of the disclosure. In some instances, a three-dimensional substrate comprises a DNA origami. DNA origami structures incorporate DNA as a building material to make nanoscale shapes. The DNA origami process can involve the folding of one or more long, "scaffold" DNA strands into a particular shape using a plurality of rationally designed "staple DNA strands. The sequences of the staple strands can be designed such that they hybridize to particular portions of the scaffold strands and, in doing so, force the scaffold strands into a particular shape. The DNA origami may include a scaffold strand and a plurality of rationally designed staple strands. The scaffold strand can have any sufficiently nonrepetitive sequence.

The sequences of the staple strands can be selected such that the DNA origami has at least one shape to which stochastic labels can be attached. In some embodiments, the DNA origami can be of any shape that has at least one inner surface and at least one outer surface. An inner surface can be any surface area of the DNA origami that is sterically precluded from interacting with the surface of a sample, while an outer surface is any surface area of the DNA origami that is not sterically precluded from interacting with the surface of a sample. In some embodiments, the DNA origami has one or more openings (e.g., two openings), such that an inner surface of the DNA origami can be accessed by particles (e.g., solid supports). For example, in certain embodiments the DNA origami has one or more openings that allow particles smaller than 10 micrometers, 5 micrometers, 1 micrometer, 500 nm, 400 nm, 300 urn, 250 nm, 200 nm, 150 nm, 100 nm, 75 nm, 50 nm, 45 nm or 40 nm to contact an inner surface of the DNA origami.

The DNA origami can change shape (conformation) in response to one or more certain environmental stimuli. Thus an area of the DNA origami may be an inner surface when the DNA origami takes on some conformations, but may be an outer surface when the device takes on other conformations. In some embodiments, the DNA origami can respond to certain environmental stimuli by taking on a new conformation.

In some embodiments, the staple strands of the DNA origami can be selected such that the DNA origami is substantially barrel- or tube-shaped. The staples of the DNA origami can be selected such that the barrel shape is closed at both ends or is open at one or both ends, thereby permitting particles to enter the interior of the barrel and access its inner surface. In certain embodiments, the barrel shape of the DNA origami can be a hexagonal tube.

In some embodiments, the staple strands of the DNA origami can be selected such that the DNA origami has a first domain and a second domain, wherein the first end of the first domain is attached to the first end of the second domain by one or more single-stranded DNA hinges, and the second end of the first domain is attached to the second domain of the second domain by the one or more molecular latches. The plurality of staples can be selected such that the second end of the first domain becomes unattached to the second end of the second domain if all of the molecular latches are contacted by their respective external stimuli. Latches can be formed from two or more staple stands, including at least one staple strand having at least one stimulus-binding domain that is able to bind to an external stimulus, such as a nucleic acid, a lipid or a protein, and at least one other staple strand having at least one latch domain that binds to the stimulus binding domain. The binding of the stimulus -binding domain to the latch domain supports the stability of a first conformation of the DNA origami.

Spatial labels can be delivered to a sample in three dimensions. For example a sample can be associated with an array, wherein the array has spatial labels distributed or distributable in three dimensions. A three dimensional array can be a scaffolding, a porous substrate, a gel, a series of channels, or the like.

A three dimensional pattern of spatial labels can be associated with a sample by injecting the samples into known locations with the sample, for example using a robot. A single needle can be used to serially inject spatial labels at different depths into a sample. An array of needles can inject spatial labels at different depths to generate a three dimensional distribution of labels.

In some instances, a three dimensional solid support can be a device. For example, a needle array device (e.g., a biopsy needle array device) can be a substrate. Stochastic barcodes of the disclosure can be attached to the device. Placing the device in and/or on a sample can bring the stochastic barcodes of the disclosure into proximity with targets in and/or on the sample. Different parts of the device may have stochastic barcodes with different spatial labels. For example, on a needle array device, each needle of the device may be coated with stochastic barcodes with different spatial labels on each needle. In this way, spatial labels can provide information about the location of the targets (e.g., location in orientation to the needle array).

### Synthesis of stochastic barcodes on solid supports and substrates

A stochastic barcode can be synthesized on a solid support (e.g., bead). Pre-synthesized stochastic barcodes (e.g., comprising the 5'amine that can link to the solid support) may be attached to solid supports (e.g., beads) through any of a variety of immobilization techniques involving functional group pairs on the solid support and the stochastic barcode. The stochastic barcode can comprise a functional group. The solid support (e.g., bead) can comprise a functional group. The stochastic barcode functional group and the solid support functional group can comprise, for example, biotin, streptavidin, primary amine(s), carboxyl(s), hydroxyl(s), aldehyde(s), ketone(s), and any combination thereof. A stochastic barcode may be tethered to a solid support, for example, by coupling (e.g. using 1-Etbyl-3-(3-dimethylaminopropyl) carbodiimide) a 5' amino group on the stochastic barcode to the carboxyl group of the functionalized solid support. Residual non-coupled stochastic barcodes may be removed from the reaction mixture by performing multiple rinse steps. In some embodiments, the stochastic barcode and solid support are attached indirectly via linker molecules (e.g. short, functionalized hydrocarbon molecules or polyethylene oxide molecules) using similar attachment chemistries. The linkers may be cleavable linkers, e.g. acid-labile linkers or photocleavable linkers.

The stochastic barcodes can be synthesized on solid supports (e.g., beads) using any of a number of solid-phase oligonucleotide synthesis techniques, such as phosphodiester synthesis, phosphotriester synthesis, phosphite triester synthesis, and phosphoramidite synthesis. Single nucleotides may be coupled in step-wise fashion to the growing, tethered stochastic barcode. A short, pre-synthesized sequence (or block) of several oligonucleotides may be coupled to the growing, tethered stochastic barcode.

Stochastic barcodes may be synthesized by interspersing step-wise or block coupling reactions with one or more rounds of split-pool synthesis, in which the total pool of synthesis beads is divided into a number of individual smaller pools which are then each subjected to a different coupling reaction, followed by recombination and mixing of the individual pools to randomize the growing stochastic barcode sequence across the total pool of beads. Split-pool synthesis is an example of a combinatorial synthesis process in which a maximum number of chemical compounds are synthesized using a minimum number of chemical coupling steps. The potential diversity of the compound library thus created is determined by the number of unique building blocks (e.g. nucleotides) available for each coupling step, and the number of coupling steps used to create the library. For example, a split-pool synthesis comprising 10 rounds of coupling using 4 different nucleotides at each step will yield 4¹⁰ = 1,048,576 unique nucleotide sequences. In some embodiments, split-pool synthesis may be performed using enzymatic methods such as polymerase extension or ligation reactions rather than chemical coupling. For example, in each round of a split-pool polymerase extension reaction, the 3' ends of the stochastic barcodes tethered to beads in a given pool may be hybridized with the 5'ends of a set of semi-random primers, e.g. primers having a structure of 5'-(M)ₖ-(X)ᵢ-(N)ⱼ-3', where (X)i is a random sequence of nucleotides that is i nucleotides long (the set of primers comprising all possible combinations of (X)i), (N)j is a specific nucleotide (or series of j nucleotides), and (M)ₖ is a specific nucleotide (or series of k nucleotides), wherein a different deoxyribonucleotide triphosphate (dNTP) is added to each pool and incorporated into the tethered oligonucleotides by the polymerase.

The number of stochastic barcodes conjugated to or synthesized on a solid support may comprise at least 100, 1000, 10000, or 1000000 or more stochastic barcodes. The number of stochastic barcodes conjugated to or synthesized on a solid support may comprise at most 100, 1000, 10000, or 1000000 or more stochastic barcodes. The number of oligonucleotides conjugated to or synthesized on a solid support such as a bead may be at least 1, 2, 3, 4, 5, 6, 7, 8, 9, or 10-fold more than the number of target nucleic acids in a cell. The number of oligonucleotides conjugated to or synthesized on a solid support such as a bead may be at most 1, 2, 3, 4, 5, 6, 7, 8, 9, or 10-fold more than the number of target nucleic acids in a cell. At least 10, 20, 30, 40, 50, 60, 70, 80, 90 or 100% of the stochastic barcode can be bound by a target nucleic acid. At most 10, 20, 30, 40, 50, 60, 70, 80, 90 or 100% of the stochastic barcode can be bound by a target nucleic acid. At least 1, 2, 3, 4, 5, 6, 7, 8, 9, 10, 20, 30, 40, 50, 60, 70, 80, 90 or 100 or more different target nucleic acids can be captured by the stochastic barcode on the solid support. At most 1, 2, 3, 4, 5, 6, 7, 8, 9, 10, 20, 30, 40, 50, 60, 70, 80, 90 or 100 or more different target nucleic acids can be captured by the stochastic barcode on the solid support.

### Methods of stochastic barcoding

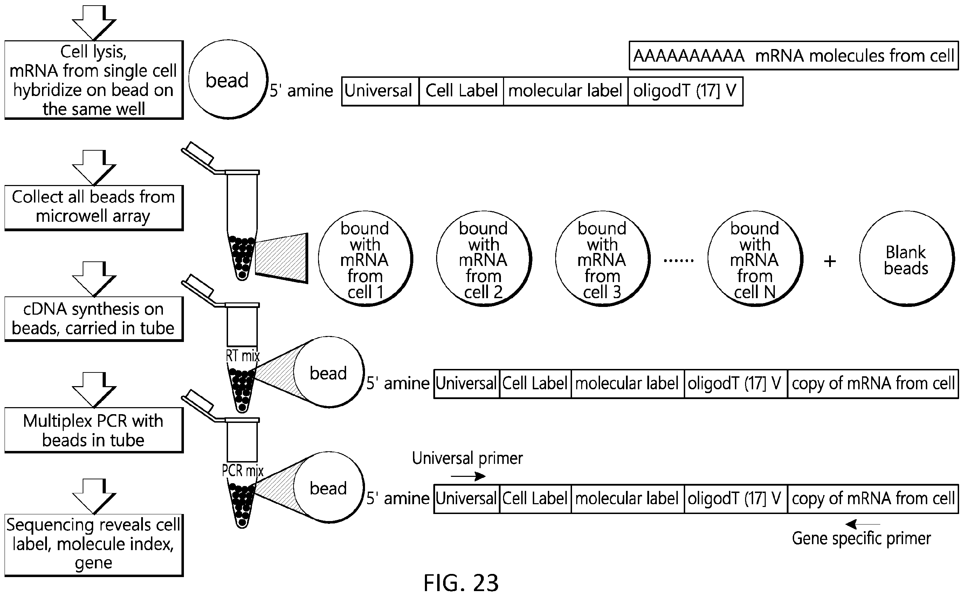

The disclosure provides for methods for estimating the number of distinct targets at distinct locations in a physical sample (e.g., tissue, organ, tumor, cell). The methods can comprise placing the stochastic barcodes in close proximity with the sample, lysing the sample, associating distinct targets with the stochastic barcodes, amplifying the targets and/or digitally counting the targets. The method can further comprise analyzing and/or visualizing the information obtained from the spatial labels on the stochastic barcodes. **Figure 23** illustrates an exemplary embodiment of the stochastic barcoding method of the disclosure. A sample (e.g., section of a sample, thin slice, and cell) can be contacted with a solid support comprising a stochastic barcode. Targets in the sample can be associated with the stochastic barcodes. The solid supports can be collected. cDNA synthesis can be performed on the solid support. cDNA synthesis can be performed off the solid support. cDNA synthesis can incorporate the label information from the labels in the stochastic barcode into the new cDNA target molecule being synthesized, thereby generating a target-barcode molecule. The target-barcode molecules can be amplified using PCT. The sequence of the targets and the labels of the stochastic barcode on the target-barcode molecule can be determined by sequencing methods.

### Contacting a sample and a stochastic barcode

A sample comprising, for example, a cell, organ, or tissue thin section, can be contacted to stochastic barcodes. The solid supports can be free floating. The solid supports can be embedded in a semi-solid or solid array. The stochastic barcodes may not be associated with solid supports. The stochastic barcodes may be individual nucleotides. The stochastic barcodes may be associated with a substrate. When stochastic barcodes are in close proximity to targets, the targets can hybridize to the stochastic barcode. The stochastic barcodes can be contacted at a non-depleatable ratio such that each distinct target can associate with a distinct stochastic barcode of the disclosure. To ensure efficient association between the target and the stochastic barcode, the targets can be crosslinked to the stochastic barcode.

### Cell Lysis

Following the distribution of cells and stochastic barcodes, the cells can be lysed to liberate the target molecules. Cell lysis may be accomplished by any of a variety of means, for example, by chemical or biochemical means, by osmotic shock, or by means of thermal lysis, mechanical lysis, or optical lysis. Cells may be lysed by addition of a cell lysis buffer comprising a detergent (e.g. SDS, Li dodecyl sulfate, Triton X-100, Tween-20, or NP-40), an organic solvent (e.g. methanol or acetone), or digestive enzymes (e.g. proteinase K, pepsin, or trypsin), or any combination thereof. To increase the association of a target and a stochastic barcode, the rate of the diffusion of the target molecules can be altered by for example, reducing the temperature and/or increasing the viscosity of the lysate.

### Attachment of Stochastic barcodes to Target Nucleic Acid Molecules

Following lysis of the cells and release of nucleic acid molecules therefrom, the nucleic acid molecules may randomly associate with the stochastic barcodes of the colocalized solid support. Association may comprise hybridization of a stochastic barcode's target recognition region to a complementary portion of the target nucleic acid molecule (e.g., oligo dT of the stochastic barcode can interact with a poly-A tail of a target). The assay conditions used for hybridization (e.g. buffer pH, ionic strength, temperature, etc.) can be chosen to promote formation of specific, stable hybrids.

Attachment may further comprise ligation of a stochastic barcode's target recognition region and a portion of the target nucleic acid molecule. For example, the target binding region may comprise a nucleic acid sequence that can be capable of specific hybridization to a restriction site overhang (e.g. an EcoRI sticky-end overhang). The assay procedure can further comprise treating the target nucleic acids with a restriction enzyme (e.g. EcoRI) to create a restriction site overhang. The stochastic barcode may then be ligated to any nucleic acid molecule comprising a sequence complementary to the restriction site overhang. A ligase (e.g., T4 DNA ligase) may be used to join the two fragments.

The labeled targets from a plurality of cells (or a plurality of samples) (e.g., target-barcode molecules) can be subsequently pooled, for example by retrieving the stochastic barcodes and/or the beads to which the target-barcode molecules are attached. The retrieval of solid support-based collections of attached target-barcode molecules may be implemented by use of magnetic beads and an externally-applied magnetic field. Once the target-barcode molecules have been pooled, all further processing may proceed in a single reaction vessel. Further processing can include, for example, reverse transcription reactions, amplification reactions, cleavage reactions, dissociation reactions, and/or nucleic acid extension reactions. Further processing reactions may be performed within the microwells, that is, without first pooling the labeled target nucleic acid molecules from a plurality of cells.

### Reverse Transcription

The disclosure provides for a method to create a stochastic target-barcode conjugate using reverse transcription. The stochastic target-barcode conjugate and comprise the stochastic barcode and a complementary sequence of all or a portion of the target nucleic acid (i.e. a stochastically barcoded cDNA molecule). Reverse transcription of the associated RNA molecule may occur by the addition of a reverse transcription primer along with the reverse transcriptase. The reverse transcription primer can be an oligo-dT primer, a random hexanucleotide primer, or a target-specific oligonucleotide primer. Oligo-dT primers can be 12― 18 nucleotides in length and bind to the endogenous poly-A tail at the 3' end of mammalian mRNA. Random hexanucleotide primers may bind to mRNA at a variety of complementary sites. Target-specific oligonucleotide primers typically selectively prime the mRNA of interest.

### Amplification

One or more nucleic acid amplification reactions may be performed to create multiple copies of the labeled target nucleic acid molecules. Amplification may be performed in a multiplexed manner, wherein multiple target nucleic acid sequences are amplified simultaneously. The amplification reaction may be used to add sequencing adaptors to the nucleic acid molecules. The amplification reactions may comprise amplifying at least a portion of a sample label, if present. The amplification reactions may comprise amplifying at least a portion of the cellular and/or molecular label. The amplification reactions may comprise amplifying at least a portion of a sample tag, a cellular label, a spatial label, a molecular label, a target nucleic acid, or a combination thereof. The amplification reactions may comprise amplifying at least 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 97%, or 100% of the plurality of nucleic acids. The method may further comprise conducting one or more cDNA synthesis reactions to produce one or more cDNA copies of target-barcode molecules comprising a sample label, a cellular label, a spatial label, and/or a molecular label.

In some embodiments, amplification may be performed using a polymerase chain reaction (PCR). As used herein, PCR may refer to a reaction for the in vitro amplification of specific DNA sequences by the simultaneous primer extension of complementary strands of DNA. As used herein, PCR may encompass derivative forms of the reaction, including but not limited to, RT-PCR, real-time PCR, nested PCR, quantitative PCR, multiplexed PCR, digital PCR, and assembly PCR.

Amplification of the labeled nucleic acids can comprise non-PCR based methods. Examples of non-PCR based methods include, but are not limited to, multiple displacement amplification (MDA), transcription-mediated amplification (TMA), nucleic acid sequence-based amplification (NASBA), strand displacement amplification (SDA), real-time SDA, rolling circle amplification, or circle-to-circle amplification. Other non-PCR-based amplification methods include multiple cycles of DNA-dependent RNA polymerase-driven RNA transcription amplification or RNA-directed DNA synthesis and transcription to amplify DNA or RNA targets, a ligase chain reaction (LCR), and a Qβ replicase (Qβ) method, use of palindromic probes, strand displacement amplification, oligonucleotide-driven amplification using a restriction endonuclease, an amplification method in which a primer is hybridized to a nucleic acid sequence and the resulting duplex is cleaved prior to the extension reaction and amplification, strand displacement amplification using a nucleic acid polymerase lacking 5' exonuclease activity, rolling circle amplification, and ramification extension amplification (RAM). In some instances, the amplification may not produce circularized transcripts.

In some instances, the methods disclosed herein further comprise conducting a polymerase chain reaction on the labeled nucleic acid (e.g., labeled-RNA, labeled-DNA, labeledcDNA) to produce a stochastically labeled-amplicon. The labeled-amplicon may be double-stranded molecule. The double-stranded molecule may comprise a double-stranded RNA molecule, a double-stranded DNA molecule, or a RNA molecule hybridized to a DNA molecule. One or both of the strands of the double-stranded molecule may comprise a sample label, a spatial label, a cellular label, and/or a molecular label. The stochastically labeled-amplicon can be a single-stranded molecule. The single-stranded molecule may comprise DNA, RNA, or a combination thereof. The nucleic acids of the disclosure may comprise synthetic or altered nucleic acids.

Amplification may comprise use of one or more non-natural nucleotides. Non-natural nucleotides may comprise photolabile or triggerable nucleotides. Examples of non-natural nucleotides can include, but are not limited to, peptide nucleic acid (PNA), morpholino and locked nucleic acid (LNA), as well as glycol nucleic acid (GNA) and threose nucleic acid (TNA). Non-natural nucleotides may be added to one or more cycles of an amplification reaction. The addition of the non-natural nucleotides may be used to identify products as specific cycles or time points in the amplification reaction.

Conducting the one or more amplification reactions may comprise the use of one or more primers. The one or more primers may comprise at least 1, 2, 3, 4, 5, 6, 7, 8, 9, 10, 11, 12, 13, 14, or 15 or more nucleotides. The one or more primers may comprise at least 1, 2, 3, 4, 5, 6, 7, 8, 9, 10, 11, 12, 13, 14, or 15 or more nucleotides. The one or more primers may comprise less than 12-15 nucleotides. The one or more primers may anneal to at least a portion of the plurality of stochastically labeled targets. The one or more primers may anneal to the 3' end or 5' end of the plurality of stochastically labeled targets. The one or more primers may anneal to an internal region of the plurality of stochastically labeled targets. The internal region may be at least about 50, 100, 150, 200, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 650, 700, 750, 800, 850, 900 or 1000 nucleotides from the 3' ends the plurality of stochastically labeled targets. The one or more primers may comprise a fixed panel of primers. The one or more primers may comprise at least one or more custom primers. The one or more primers may comprise at least one or more control primers. The one or more primers may comprise at least one or more gene-specific primers.

The one or more primers may comprise a universal primer. The universal primer may anneal to a universal primer binding site. The one or more custom primers may anneal to a first sample label, a second sample label, a spatial label, a cellular label, a molecular label, a target, or any combination thereof. The one or more primers may comprise a universal primer and a custom primer. The custom primer may be designed to amplify one or more targets. The targets may comprise a subset of the total nucleic acids in one or more samples. The targets may comprise a subset of the total stochastically labeled targets in one or more samples. The one or more primers may comprise at least 96 or more custom primers. The one or more primers may comprise at least 960 or more custom primers. The one or more primers may comprise at least 9600 or more custom primers. The one or more custom primers may anneal to two or more different labeled nucleic acids. The two or more different labeled nucleic acids may correspond to one or more genes.

Any amplification scheme can be used in the methods of the present disclosure. For example, in one scheme, the first round PCR can amplify molecules attached to the bead using a gene specific primer and a primer against the universal Illumina sequencing primer 1 sequence. The second round of PCR can amplify the first PCR products using a nested gene specific primer flanked by Illumina sequencing primer 2 sequence, and a primer against the universal Illumina sequencing primer 1 sequence. The third round of PCR adds P5 and P7 and sample index to turn PCR products into an Illumina sequencing library. Sequencing using 150bp x 2 sequencing can reveal the cell label and molecular index on read 1, the gene on read 2, and the sample index on index 1 read.

Amplification can be performed in one or more rounds. In some instances there are multiple rounds of amplification. For example the scheme depicted in Fig 15 can have two rounds of amplification. The first amplification can be an extension off X' to generate the gene specific region. The second amplification can occur when a sample nucleic hybridizes to the X strand.

In some embodiments hybridization does not need to occur at the end of a nucleic acid molecule. In some embodiments a target nucleic acid within an intact strand of a longer nucleic acid is hybridized and amplified. For example a target within a longer section of genomic DNA or mRNA. Target can be more than 50nt, more than 100nt, or more that 1000nt from an end of a polynucleotide.

### Sequencing

Determining the number of different stochastically labeled nucleic acids may comprise determining the sequence of the labeled target, the spatial label, the molecular label, the sample label, and the cellular label or any product thereof (e.g. labeled-amplicons, labeled-cDNA molecules). An amplified target may be subjected to sequencing. Determining the sequence of the stochastically labeled nucleic acid or any product thereof may comprise conducting a sequencing reaction to determine the sequence of at least a portion of a sample label, a spatial label, a cellular label, a molecular label, at least a portion of the stochastically labeled target, a complement thereof, a reverse complement thereof, or any combination thereof.

Determination of the sequence of a nucleic acid (e.g. amplified nucleic acid, labeled nucleic acid, cDNA copy of a labeled nucleic acid, etc.) may be performed using variety of sequencing methods including, but not limited to, sequencing by hybridization (SBH), sequencing by ligation (SBL), quantitative incremental fluorescent nucleotide addition sequencing (QIFNAS), stepwise ligation and cleavage, fluorescence resonance energy transfer (FRET), molecular beacons, TaqMan reporter probe digestion, pyrosequencing, fluorescent in situ sequencing (FISSEQ), FISSEQ beads, wobble sequencing, multiplex sequencing, polymerized colony (POLONY) sequencing; nanogrid rolling circle sequencing (ROLONY), allele-specific oligo ligation assays (e.g., oligo ligation assay (OLA), single template molecule OLA using a ligated linear probe and a rolling circle amplification (RCA) readout, ligated padlock probes, or single template molecule OLA using a ligated circular padlock probe and a rolling circle amplification (RCA) readout), and the like.

In some instances, determining the sequence of the labeled nucleic acid or any product thereof comprises paired-end sequencing, nanopore sequencing, high-throughput sequencing, shotgun sequencing, dye-terminator sequencing, multiple-primer DNA sequencing, primer walking, Sanger dideoxy sequencing, Maxim-Gilbert sequencing, pyrosequencing, true single molecule sequencing, or any combination thereof. Alternatively, the sequence of the labeled nucleic acid or any product thereof may be determined by electron microscopy or a chemical-sensitive field effect transistor (chemFET) array.

High-throughput sequencing methods, such as cyclic array sequencing using platforms such as Roche 454, Illumina Solexa, ABI-SOLiD, ION Torrent, Complete Genomics, Pacific Bioscience, Helicos, or the Polonator platform, may also be utilized. In some embodiment, sequencing may comprise MiSeq sequencing. In some embodiment, sequencing may comprise HiSeq sequencing.

The stochastically labeled targets can comprise nucleic acids representing from about 0.01% of the genes of an organism's genome to about 100% of the genes of an organism's genome. For example, about 0.01% of the genes of an organism's genome to about 100% of the genes of an organism's genome can be sequenced using a target complimentary region comprising a plurality of multimers by capturing the genes containing a complimentary sequence from the sample. In some embodiments, the labeled nucleic acids comprise nucleic acids representing from about 0.01% of the transcripts of an organism's transcriptome to about 100% of the transcripts of an organism's transcriptome. For example, about 0.501% of the transcripts of an organism's transcriptome to about 100% of the transcripts of an organism's transcriptome can be sequenced using a target complimentary region comprising a poly-T tail by capturing the mRNAs from the sample.

Sequencing may comprise sequencing at least about 10, 20, 30, 40, 50, 60, 70, 80, 90, 100 or more nucleotides or base pairs of the labeled nucleic acid. Sequencing can comprise sequencing at least about 200, 300, 400, 500, 600, 700, 800, 900, 1,000 or more nucleotides or base pairs of the labeled nucleic acid. Sequencing can comprise sequencing at least about 1,500; 2,000; 3,000; 4,000; 5,000; 6,000; 7,000; 8,000; 9,000; or 10,000 or more nucleotides or base pairs of the labeled nucleic acid.

Sequencing may comprise at least about 200, 300, 400, 500, 600, 700, 800, 900, 1,000 or more sequencing reads per run. In some instances, sequencing comprises sequencing at least about 1,500; 2,000; 3,000; 4,000; 5,000; 6,000; 7,000; 8,000; 9,000; or 10,000 or more sequencing reads per run. Sequencing may comprise less than or equal to about 1,600,000,000 sequencing reads per run. Sequencing may comprise less than or equal to about 200,000,000 reads per run.

### Samples

### Cells

A sample for use in the method of the disclosure can comprise one or more cells. A sample can refer to one or more cells. In some embodiments, the cells are cancer cells excised from a cancerous tissue, for example, breast cancer, lung cancer, colon cancer, prostate cancer, ovarian cancer, pancreatic cancer, brain cancer, melanoma and non-melanoma skin cancers, and the like. In some instances, the cells are derived from a cancer but collected from a bodily fluid (e.g. circulating tumor cells). Non-limiting examples of cancers may include, adenoma, adenocarcinoma, squamous cell carcinoma, basal cell carcinoma, small cell carcinoma, large cell undifferentiated carcinoma, chondrosarcoma, and fibrosarcoma.

In some embodiments, the cells are cells that have been infected with virus and contain viral oligonucleotides. In some embodiments, the viral infection may be caused by a virus selected from the group consisting of double-stranded DNA viruses (e.g. adenoviruses, herpes viruses, pox viruses), single-stranded (+ strand or "sense") DNA viruses (e.g. parvoviruses), double-stranded RNA viruses (e.g. reoviruses), single-stranded (+ strand or sense) RNA viruses (e.g. picornaviruses, togaviruses), single-stranded (- strand or antisense) RNA viruses (e.g. orthomyxoviruses, rhabdoviruses), single-stranded ((+ strand or sense) RNA viruses with a DNA intermediate in their life-cycle) RNA-RT viruses (e.g. retroviruses), and double-stranded DNA-RT viruses (e.g. hepadnaviruses). In some instances, the sample is a virus, or cells infected with a virus. The virus can be SIV or HIV.

In some embodiments, the cells are bacteria. These may include either gram-positive or gram-negative bacteria. Examples of bacteria that may be analyzed using the disclosed methods, devices, and systems include, but are not limited to, *Actinomedurae, Actinomyces israelii, Bacillus anthracis, Bacillus cereus, Clostridium botulinum, Clostridium difficile, Clostridium perfringens, Clostridium tetani, Corynebacterium, Enterococcus faecalis, Listeria monocytogenes, Nocardia, Propionibacterium acnes, Staphylococcus aureus, Staphylococcus epiderm, Streptococcus mutans, Streptococcus pneumoniae and the like. Gram negative bacteria include, but are not limited to, Afipia felis, Bacteriodes, Bartonella bacilliformis, Bortadella pertussis, Borrelia burgdorferi, Borrelia recurrentis, Brucella, Calymmatobacterium granulomatis, Campylobacter, Escherichia coli, Francisella tularensis, Gardnerella vaginalis, Haemophilius aegyptius, Haemophilius ducreyi, Haemophilius influenziae, Heliobacter pylori, Legionella pneumophila, Leptospira interrogans, Neisseria meningitidia, Porphyromonas gingivalis, Providencia sturti, Pseudomonas aeruginosa, Salmonella enteridis, Salmonella typhi, Serratia marcescens, Shigella boydii, Streptobacillus moniliformis, Streptococcus pyogenes, Treponema pallidum, Vibrio cholerae, Yersinia enterocolitica, Yersinia pestis and the like. Other bacteria may include Myobacterium avium, Myobacterium leprae, Myobacterium tuberculosis, Bartonella henseiae, Chlamydia psittaci, Chlamydia trachomatis, Coxiella burnetii, Mycoplasma pneumoniae, Rickettsia akari, Rickettsia prowazekii, Rickettsia rickettsii, Rickettsia tsutsugamushi, Rickettsia typhi, Ureaplasma urealyticum, Diplococcus pneumoniae, Ehrlichia chafensis, Enterococcus faecium, Meningococci* and the like.

In some embodiments, the cells are fungi. Non-limiting examples of fungi that may be analyzed using the disclosed methods, devices, and systems include, but are not limited to, *Aspergilli, Candidae, Candida albicans, Coccidioides immitis, Cryptococci,* and combinations thereof.

In some embodiments, the cells are protozoans or other parasites. Examples of parasites to be analyzed using the methods, devices, and systems of the present disclosure include, but are not limited to, *Balantidium coli, Cryptosporidium parvum, Cyclospora cayatanensis, Encephalitozoa, Entamoeba histolytica, Enterocytozoon bieneusi, Giardia lamblia, Leishmaniae, Plasmodii, Toxoplasma gondii, Trypanosomae, trapezoidal amoeba,* worms (e.g., helminthes), particularly parasitic worms including, but not limited to, Nematoda (roundworms, e.g., whipworms, hookworms, pinworms, ascarids, filarids and the like), Cestoda (e.g., tapeworms).

As used herein, the term "cell" can refer to one or more cells. In some embodiments, the cells are normal cells, for example, human cells in different stages of development, or human cells from different organs or tissue types (e.g. white blood cells, red blood cells, platelets, epithelial cells, endothelial cells, neurons, glial cells, fibroblasts, skeletal muscle cells, smooth muscle cells, gametes, or cells from the heart, lungs, brain, liver, kidney, spleen, pancreas, thymus, bladder, stomach, colon, small intestine). In some embodiments, the cells may be undifferentiated human stem cells, or human stem cells that have been induced to differentiate. In some embodiments, the cells may be fetal human cells. The fetal human cells may be obtained from a mother pregnant with the fetus. In some embodiments, the cells are rare cells. A rare cell may be, for example, a circulating tumor cell (CTC), circulating epithelial cell, circulating endothelial cell, circulating endometrial cell, circulating stem cell, stem cell, undifferentiated stem cell, cancer stem cell, bone marrow cell, progenitor cell, foam cell, mesenchymal cell, trophoblast, immune system cell (host or graft), cellular fragment, cellular organelle (e.g. mitochondria or nuclei), pathogen infected cell, and the like.

In some embodiments, the cells are non-human cells, for example, other types of mammalian cells (e.g. mouse, rat, pig, dog, cow, or horse). In some embodiments, the cells are other types of animal or plant cells. In other embodiments, the cells may be any prokaryotic or eukaryotic cells.

In some embodiments, a first cell sample is obtained from a person not having a disease or condition, and a second cell sample is obtained from a person having the disease or condition. In some embodiments, the persons are different. In some embodiments, the persons are the same but cell samples are taken at different time points. In some embodiments, the persons are patients, and the cell samples are patient samples. The disease or condition can be a cancer, a bacterial infection, a viral infection, an inflammatory disease, a neurodegenerative disease, a fungal disease, a parasitic disease, a genetic disorder, or any combination thereof.

In some embodiments, cells suitable for use in the presently disclosed methods may range in size from about 2 micrometers to about 100 micrometers in diameter. In some embodiments, the cells may have diameters of at least 2 micrometers, at least 5 micrometers, at least 10 micrometers, at least 15 micrometers, at least 20 micrometers, at least 30 micrometers, at least 40 micrometers, at least 50 micrometers, at least 60 micrometers, at least 70 micrometers, at least 80 micrometers, at least 90 micrometers, or at least 100 micrometers. In some embodiments, the cells may have diameters of at most 100 micrometers, at most 90 micrometers, at most 80 micrometers, at most 70 micrometers, at most 60 micrometers, at most 50 micrometers, at most 40 micrometers, at most 30 micrometers, at most 20 micrometers, at most 15 micrometers, at most 10 micrometers, at most 5 micrometers, or at most 2 micrometers. The cells may have a diameter of any value within a range, for example from about 5 micrometers to about 85 micrometers. In some embodiments, the cells have diameters of about 10 micrometers.

In some embodiments the cells are sorted prior to associating a cell with a bead. For example the cells can be sorted by fluorescence-activated cell sorting or magneticactivated cell sorting, or more generally by flow cytometry. The cells may be filtered by size. In some instances a retentate contains the cells to be associated with the bead. In some instances the flow through contains the cells to be associated with the bead.

In some embodiments, the sample comprises an immune cell. An immune cell can include, for example, T cell, B cell, lymphoid stem cell, myeloid progenitor cell, lymphocyte, granulocyte, B-cell progenitor, T cell progenitor, Natural Killer cell, Tc cell, Th cell, plasma cell, memory cell, neutrophil, eosinophil, basophil, mast cell, monocyte, dendritic cell and/or macrophage, or any combination thereof.

A T cell can be a T cell clone, which can refer to T cells derived from a single T cell or those having identical TCRs. A T cell can be part of a T cell line which can include T cell clones and mixed populations of T cells with different TCRs all of which may recognize the same target (e.g., antigen, tumor, virus). T cells can be obtained from a number of sources, including peripheral blood mononuclear cells, bone marrow, lymph node tissue, spleen tissue, and tumors. T cells can be obtained from a unit of blood collected from a subject, such as using the Ficoll separation. Cells from the circulating blood of an individual can be obtained by apheresis or leukapheresis. The apheresis product can comprise lymphocytes, including T cells, monocytes, granulocytes, B cells, other nucleated white blood cells, red blood cells, and platelets. The cells can be washed and resuspended in media to isolate the cell of interest.

T cells can be isolated from peripheral blood lymphocytes by lysing the red blood cells and depleting the monocytes, for example, by centrifugation through a PERCOLL^{™} gradient. A specific subpopulation of T cells, such as CD28+, CD4+, CDC, CD45RA+, and CD45RO+ T cells, can be further isolated by positive or negative selection techniques. For example, T cells can be isolated by incubation with anti-CD3/anti-CD28 (i.e., 3×28)-conjugated beads, such as DYNABEADS^{®} M-450 CD3/CD28 T, or XCYTE DYNABEADS^{™} for a time period sufficient for positive selection of the desired T cells. Immune cells (e.g., T cells and B cells) can be antigen specific (e.g., specific for a tumor.

In some embodiments, the cell can be an antigen-presenting cell (APC), such as a B cell, an activated B cell from a lymph node, a lymphoblastoid cell, a resting B-cell, or a neoplastic B cell, e.g. from a lymphoma. An APC can refer to a B-cell or a follicular dendritic cell expressing at least one of the BCRC proteins on its surface.

### Immunological methods

The methods of the disclosure can be used to trace the molecular phenotype of single T cells. Different subtypes of T cells can be distinguished by expression of different molecular markers. T cells express a unique T cell receptor (TCR) from a diverse repertoire of TCRs. In most T cells, the TCR can be composed of a heterodimer of a α and a β chain; each functional chain can be a product of somatic DNA recombination events during T cell development, allowing the expression of over a million different TCRs in a single individual. TCRs can be used to define the identity of individual T cells, allowing for lineage tracing for T cell clonal expansion during an immune response. The immunological methods of the disclosure can be used in a variety of ways, including but not limited to, identifying unique TCRα and TCRβ chain pairing in single T cells, quantifying TCR and marker expression at the single cell level, identifying TCR diversity in an individual, characterizing the TCR repertoire expressed in different T cell populations, determining functionality of the alpha and beta chain alleles of the TCR, and identifying clonal expansion of T cells during immune response.

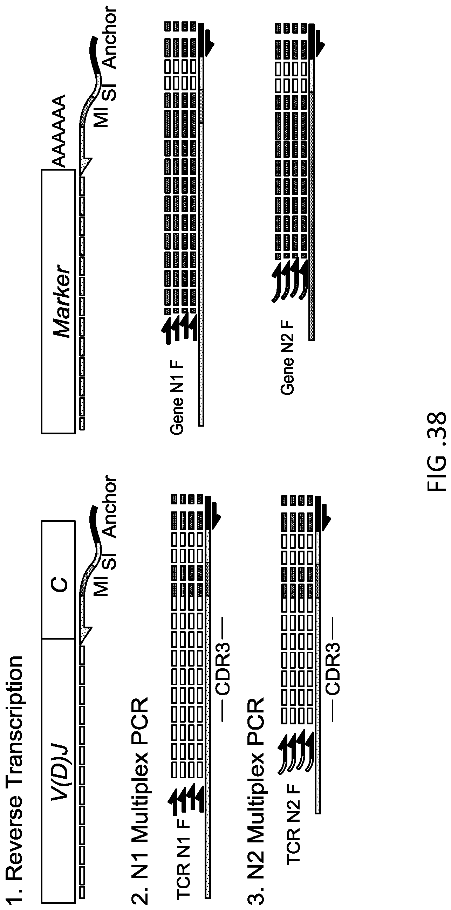

The disclosure provides methods for capturing the TCR repertoire as shown in **Figures 38-40****.** As shown in **Figure 38****,** a reverse transcription primer can comprise any labels of the disclosure (e.g., molecular label, and sample label) attached to an anchor (which can be a universal sequence). The primer can comprise a gene-specific region. The gene specific region can bind to a C region of one or more alpha chains of the TCR, one or more beta chains of the TCR, or one or more of both the alpha and beta chains of the TCR. Exemplary sequences for C-region binding for the primer are: TRAC: 5' CAGACAGACTTGT 3' (SEQ ID NO: 1); TRBC: 5' CCTTTTGGGTGTG 3' (SEQ ID NO: 2). In some instances, the reverse transcription primer can comprise an oligo dT that can bind to a polyA tail of a marker gene. A marker gene can be a T-cell specific gene. In some instances, a marker gene can refer to at least 1, 2, 3, 4, 5, 6, 7, 8, 9, or 10 or more genes. A marker gene can refer to at most 1, 2, 3, 4, 5, 6, 7, 8, 9, or 10 or more genes.

The forward primers for the N1 and N2 multiplex PCR steps (as shown in **Figure** 38) can be designed to target the V region of the VDJ combination, the D region of the VDJ combination, and/or the J region of the VDJ combination. The N1 and/or N2 forward primers can be designed to bind to the 5' end of the V region, the 3' end of the V region, anywhere in the V region, or at the junction between the V and D region. The primer can be designed such that the subsequent sequencing reads (e.g., output) can sequence a CDR of the VDJ region (e.g., CDR3). Exemplary genes that can be targeted in the N1 and/or N2 PCR reactions are shown in **Figure 39** and the forward primers used in the N1 and N2 PCR reactions are shown in **Tables 1** and **2,** respectively.

In one example, the methods of the disclosure (e.g., immunological methods of the disclosure) can be used to characterize tumor infiltrating lymphocytes in a tumor, monitoring clinical response of a patient with a tumor before and after treatment, and determining the minimal residual disease (e.g., left over cancer cells) after treatment.

**Table 1. N1 Forward primers**

| *Gene Name* | Primer Seq | SEQ ID NO |
|---|---|---|
| *RORC* | CCCAAACATTTCCATGGTGCTC | SEQ ID NO: 3 |
| *RUNX1* | GCAACGGGAAATGTGGTCCT | SEQ ID NO: 4 |
| *RUNX3* | CTGGTCCCAGGTCAGCGT | SEQ ID NO: 5 |
| *TBET* | CTGCCCTAACTACAGTCGTTTAC | SEQ ID NO: 6 |
| *GATA3* | CCGTTCACCAGTTGCCGTT | SEQ ID NO: 7 |
| *PDCD1* | ACATCCTACGGTCCCAAGGT | SEQ ID NO: 8 |
| *BCL2* | TGCAAGAGTGACAGTGGATTG | SEQ ID NO: 9 |
| *BCL6* | TGTCCTCACGGTGCCTTTT | SEQ ID NO: 10 |
| *BIRC5* | TGCCACGGCCTTTCCTTAAA | SEQ ID NO: 11 |
| *CCL14* | TTCCTCCTCATCACCATCGC | SEQ ID NO: 12 |
| *CCL17* | GAGTGCTGCCTGGAGTACTT | SEQ ID NO: 13 |
| *CCL18* | GAAGCTGAATGCCTGAGGGG | SEQ ID NO: 14 |
| *CCL20* | ACTTGCACATCATGGAGGGT | SEQ ID NO: 15 |
| *CCL3* | TGGACTGGTTGTTGCCAAAC | SEQ ID NO: 16 |
| *CCL4* | CCCAGCCAGCTGTGGTATTC | SEQ ID NO: 17 |
| *CCR4* | CCAAAGGGAAGAGTGCAGGG | SEQ ID NO: 18 |
| *CCR7* | GGGGAGAGTGTGGTGTTTCC | SEQ ID NO: 19 |
| *CD160* | GGAAGACAGCCAGATCCAGTG | SEQ ID NO: 20 |
| *CD244* | GGGCTGAGAATGAGGCAGTT | SEQ ID NO: 21 |
| *CD27* | TGCAGAGCCTTGTCGTTACA | SEQ ID NO: 22 |
| *CD28* | ACCATCACAGGCATGTTCCT | SEQ ID NO: 23 |
| *CD30* | TTTACTCATCGGGCAGCCAC | SEQ ID NO: 24 |
| *CD38* | AGGTCAATGCCAGAGACGGA | SEQ ID NO: 25 |
| *CD3D* | GAAAACGCATCCTGGACCCA | SEQ ID NO: 26 |
| *CD3E* | AAGTTGTCCCCCATCCCAAA | SEQ ID NO: 27 |
| *CD4* | CTGGGAGAGGGGGTAGCTAG | SEQ ID NO: 28 |
| *CD40LG* | CCTCCCCCAGTCTCTCTTCT | SEQ ID NO: 29 |
| *CD5* | ATCAATGGTCCAAGCCGCAT | SEQ ID NO: 30 |
| *CD69* | GCTGTAGACAGGTCCTTTTCG | SEQ ID NO: 31 |
| *CD8A* | ACTGCTGTCCCAAACATGCA | SEQ ID NO: 32 |
| *CD8B* | CCACCATCTTTGCAGGTTGC | SEQ ID NO: 33 |
| *CDCA7* | CCAGTCTAGTTTCTGGGCAGG | SEQ ID NO: 34 |
| *CSF2* | AGCCAGTCCAGGAGTGAGAC | SEQ ID NO: 35 |
| *CX3CR1* | CACCCGTCCAGACCTTGTT | SEQ ID NO: 36 |
| *CXCL12* | TGGGAGTTGATCGCCTTTCC | SEQ ID NO: 37 |
| *CXCL13* | AGGCAGATGGAACTTGAGCC | SEQ ID NO: 38 |
| *CXCR3* | GACCTCAGAGGCCTCCTACT | SEQ ID NO: 39 |
| *CXCR5* | CCTCCCCAGCCTTTGATCAG | SEQ ID NO: 40 |
| *EOMES* | ACTTAACAGCTGCAGGGGC | SEQ ID NO: 41 |
| *FAS* | ATTGCTGGTAGAGACCCCCA | SEQ ID NO: 42 |
| *FASLG* | CCTCAAGGGGGACTGTCTTTC | SEQ ID NO: 43 |
| *FOSL1* | CTCCTGACAGAAGGTGCCAC | SEQ ID NO: 44 |
| *FOXP3* | ACAGAAGCAGCGTCAGTACC | SEQ ID NO: 45 |
| *GAPDH* | GACTTCAACAGCGACACCCA | SEQ ID NO: 46 |
| *GZMA* | GGAACCATGTGCCAAGTTGC | SEQ ID NO: 47 |
| *GZMB* | AGGTGAAGATGACAGTGCAGG | SEQ ID NO: 48 |
| *GZMH* | AGTGTTGCTGACAGTGCAGA | SEQ ID NO: 49 |
| *GZMK* | TTGCCACAAAGCCTGGAATC | SEQ ID NO: 50 |
| *HLA-DRA* | GGGTCTGGTGGGCATCATTA | SEQ ID NO: 51 |
| *IFNG* | CTAGGCAGCCAACCTAAGCA | SEQ ID NO: 52 |
| *IL10* | CCCCAACCACTTCATTCTTG | SEQ ID NO: 53 |
| *IL12A* | GGTCCCTCCAAACCGTTGTC | SEQ ID NO: 54 |
| *IL12B* | GCTATGGTGAGCCGTGATTG | SEQ ID NO: 55 |
| *IL13* | GGAGCCAAGGGTTCAGAGAC | SEQ ID NO: 56 |
| *IL17A* | GCCTTCAAGACTGAACACCGA | SEQ ID NO: 57 |
| *IL17F* | TTGGAGAAGGTGCTGGTGAC | SEQ ID NO: 58 |
| *IL1A* | GGCATCCTCCACAATAGCAGA | SEQ ID NO: 59 |
| *IL1B* | CTTAAAGCCCGCCTGACAGA | SEQ ID NO: 60 |
| *IL2* | TCACTTAAGACCCAGGGACTT | SEQ ID NO: 61 |
| *IL21* | CCCAAGGTCAAGATCGCCAC | SEQ ID NO: 62 |
| *IL21R* | ATTTGAGGCTGCAGTGAGCT | SEQ ID NO: 63 |
| *IL22* | TGGGAAGCCAAACTCCATCAT | SEQ ID NO: 64 |
| *IL23R* | AGAATCATTAGGCCAGGCGTG | SEQ ID NO: 65 |
| *IL24* | CTCACCCCATCATCCCTTTCC | SEQ ID NO: 66 |
| *IL26* | TACTGACGGCATGTTAGGTG | SEQ ID NO: 67 |
| *IL3* | ACAGACGACTTTGAGCCTCG | SEQ ID NO: 68 |
| *IL31* | GGCCATCTCTTCCTTTCGGA | SEQ ID NO: 69 |
| *IL32* | CTTTCCAGTCCTACGGAGCC | SEQ ID NO: 70 |
| *IL33R* | TTCAGGACTCCCTCCAGCAT | SEQ ID NO: 71 |
| *IL4* | ACCATGAGAAGGACACTCGC | SEQ ID NO: 72 |
| *IL4R* | TGAACTTCAGGGAGGGTGGT | SEQ ID NO: 73 |
| *IL5* | GCAGTGAGAATGAGGGCCA | SEQ ID NO: 74 |
| *IL6* | CGGCAAATGTAGCATGGGC | SEQ ID NO: 75 |
| *IL6R* | CCAGCACCAGGGAGTTTCTA | SEQ ID NO: 76 |
| *IRF4* | CTCTTCAGCATCCCCCGTAC | SEQ ID NO: 77 |
| *LAG3* | AGCTGTACCAGGGGGAGAG | SEQ ID NO: 78 |
| *LIF* | TCCCCATCGTCCTCCTTGTC | SEQ ID NO: 79 |
| *LTA* | AGGCAGGGAGGGGACTATTT | SEQ ID NO: 80 |
| *MCL1* | GACTGGCTACGTAGTTCGGG | SEQ ID NO: 81 |
| *MKI67* | TACTTTTTCGCCTCCCAGGG | SEQ ID NO: 82 |
| *MYC* | AGCTACGGAACTCTTGTGCG | SEQ ID NO: 83 |
| *NKG2D* | CAACACCCAGGGGATCAGTG | SEQ ID NO: 84 |
| *PRDM1* | ACCAAAGCATCACGTTGACAT | SEQ ID NO: 85 |
| *PRF1* | GGAGTCCAGCGAATGACGTC | SEQ ID NO: 86 |
| *PTPRCv1* | GTTCCCATGTTAAATCCCATTCAT | SEQ ID NO: 87 |
| *PTPRCv2* | ACCCTCTCTCCCTCCCTTTC | SEQ ID NO: 88 |
| *SAP30* | ACCAACCAGACCAGGACTTA | SEQ ID NO: 89 |
| *SELL* | GCATCTCATGAGTGCCAAGC | SEQ ID NO: 90 |
| *TBX21* | TTATAACCATCAGCCCGCCA | SEQ ID NO: 91 |
| *TGFB1* | TATTCCTTTGCCCGGCATCA | SEQ ID NO: 92 |
| *TNF* | AGTGGACCTTAGGCCTTCCT | SEQ ID NO: 93 |
| *TNFRSF9* | TGGCATGTGAGTCATTGCTC | SEQ ID NO: 94 |
| *TYMS* | TCAGTCTTTAGGGGTTGGGC | SEQ ID NO: 95 |
| *UHRF1* | CCAGTTCTTCCTGACACCGG | SEQ ID NO: 96 |
| *ZBED2* | AATGTACCAGCCAGTCAGCG | SEQ ID NO: 97 |
| *ZNF683* | GGAGAGCGTCCATTCCAGTG | SEQ ID NO: 98 |
| *TRAV1-1* | TCTGGGTTTTATGGGCTGTCCTGGTA | SEQ ID NO: 99 |
| *TRAV1-2-01* | GGACAAAACATTGACCAGCCCAC | SEQ ID NO: 100 |
| *TRAV1-2-02* | ATCTGGGTTCAACGGGCTGTTCTGGTA | SEQ ID NO: 101 |
| *TRAV2* | AATCACTCTGTGTCCAATGCTTACA | SEQ ID NO: 102 |
| *TRAV3* | ATTCAGTCTCTGGAAACCCTTATC | SEQ ID NO: 103 |
| *TRAV4* | GTAGCCACAACAACATTGCTAC | SEQ ID NO: 104 |
| *TRAV5* | CAGCTCCTCCACCTACTTATACT | SEQ ID NO: 105 |
| *TRAV6* | CCAGCATACTTACAGTGGTA | SEQ ID NO: 106 |
| *TRAV7* | GCACGTACTCTGTCAGTCGTT | SEQ ID NO: 107 |
| *TRAV8-1* | GTGGAACTGTTAATCTCTTCTGGTA | SEQ ID NO: 108 |
| *TRAV8-2* | CTCTCTTCTGGTATGTGCAACACC | SEQ ID NO: 109 |
| *TRAV8-3* | GGCAACACCTTATCTCTTCTGGTA | SEQ ID NO: 110 |
| *TRA V8-4* | CTCTTCTGGTATGTGCAATA | SEQ ID NO: 111 |
| *TRAV8-5* | GCCATGGCAGAAGAATGTGGATT | SEQ ID NO: 112 |
| *TRAV8-6* | AGTGTATCTCTTCTGGTATGTGCAA | SEQ ID NO: 113 |
| *TRAV8-7* | GGAGTTCCTTCTCTCTTCTGGTA | SEQ ID NO: 114 |
| *TRAV9-1* | GAAACCACACAGTACCCTTCCCTT | SEQ ID NO: 115 |
| *TRAV9-2* | AGGATACCCTTCCCTTTTCTGGTA | SEQ ID NO: 116 |
| *TRAV10* | TGAGCCCCTTCAGCAACTTAAG | SEQ ID NO: 117 |
| *TRAV11* | CACTCTTCAATTTCCACTGGTTCCGG | SEQ ID NO: 118 |
| *TRAV12-1* | AGTGCTTCTCAGTCTTTCTTCTGG | SEQ ID NO: 119 |
| *TRAV12-2* | TTCCCAGTCCTTCTTCTGGTA | SEQ ID NO: 120 |
| *TRAV12-3* | CTCTCAACTGCACTTACAGCAAC | SEQ ID NO: 121 |
| *TRAV13-1* | GCCTCAAACTACTTCCCTTGGTA | SEQ ID NO: 122 |
| *TRAV13-2* | CAGCGCCTCAGACTACTTCATTT | SEQ ID NO: 123 |
| *TRAV14* | GGTCTATTCTGGTACAAGCAGC | SEQ ID NO: 124 |
| *TRAV15* | TAACTTCTACTGGTTCTGGCAGGGTC | SEQ ID NO: 125 |
| *TRAV16* | AACTATTCCTATTCTGGGAGTCCT | SEQ ID NO: 126 |
| *TRAV17* | GTGGTATAGACAAAATTCAGGTAGAGG | SEQ ID NO: 127 |
| *TRAV18* | CTGCACTTATCAGTCCAGCTATTCAAC | SEQ ID NO: 128 |
| *TRAV19* | TTCTGGTACAAGCAACCACC | SEQ ID NO: 129 |
| *TRAV20* | CACAGTCAGCGGTTTAAGAGG | SEQ ID NO: 130 |
| *TRAV21* | CTGATAGCGCTATTTACAACCTCC | SEQ ID NO: 131 |
| *TRAV22* | GCGGTGCAATTTTTCTGACTCTG | SEQ ID NO: 132 |
| *TRAV23* | CTGCGTTTGACTACTTTCCATGGTA | SEQ ID NO: 133 |
| *TRAV24* | CCCTTCCAGCAATTTTTATGCC | SEQ ID NO: 134 |
| *TRAV25* | CCACGTACTGCAATTCCTCAAC | SEQ ID NO: 135 |
| *TRAV26-1* | GTGTATTGGTATCGACAGATTCACTC | SEQ ID NO: 136 |
| *TRAV26-2* | TACATTGGTATCGACAGCTTCCCTCC | SEQ ID NO: 137 |
| *TRAV27* | ACTGTGTACTGCAACTCCTCAAG | SEQ ID NO: 138 |
| *TRAV28* | TGTTCTATTTACATGATCCGTGTGC | SEQ ID NO: 139 |
| *TRAV29* | TACCCTGCTGAAGGTCCTACAT | SEQ ID NO: 140 |
| *TRAV30* | GCAGTTCCTCCAAGGCTTTATA | SEQ ID NO: 141 |
| *TRAV31* | CGTGAAACTGGACTGTGCATACAA | SEQ ID NO: 142 |
| *TRAV32* | TGACAGTTATACAGATGGAGCTTTG | SEQ ID NO: 143 |
| *TRAV33* | CGTACACTTTATACTGGTACAAGCAAC | SEQ ID NO: 144 |
| *TRAV34* | CTCACCATAAACTGCACGTCATCAA | SEQ ID NO: 145 |
| *TRAV35* | GCATATTTAACACCTGGCTATGGTA | SEQ ID NO: 146 |
| *TRAV36* | GTGACTAACTTTCGAAGCCTACTA | SEQ ID NO: 147 |
| *TRAV37* | GCCCTTCAGATTTCTTCAGTGGTTC | SEQ ID NO: 148 |
| *TRAV38-1* | GTGCAGGAGGCAGAGACTGTGA | SEQ ID NO: 149 |
| *TRAV38-2* | TATTCTGGTACAAGCAGCCTCC | SEQ ID NO: 150 |
| *TRAV39* | CACTTCAGACAGACTGTATTGGT | SEQ ID NO: 151 |
| *TRAV40* | ACATACACATCCACGGGGT | SEQ ID NO: 152 |
| *TRAV41* | ACTCGGTAGGAATAAGTGCCTTAC | SEQ ID NO: 153 |
| *TRBV2* | CTTGGGGCAGAAAGTCGAGT | SEQ ID NO: 154 |
| *TRBV3-1* | GGTCACACAGATGGGAAACG | SEQ ID NO: 155 |
| *TRBV4-1* | TGGGGCACAGGGCTATGTA | SEQ ID NO: 156 |
| *TRBV4-2* | TACGCAGACACCAAGACACC | SEQ ID NO: 157 |
| *TRBV4-3* | ACGCAGACACCAAGACACC | SEQ ID NO: 158 |
| *TRBV5-1* | AGCAAGTGACACTGAGCTGC | SEQ ID NO: 159 |
| *TRBV5-3*/*5*/*6* | CCAAAGTCCCACACACCTGA | SEQ ID NO: 160 |
| *TRBV5-4* | TCACCCAAAGTCCCACACAC | SEQ ID NO: 161 |
| *TRBV5-7* | CAAAACGAGAGGACAGCACG | SEQ ID NO: 162 |
| *TRBV5-8* | GCGACTCTGAGATGCTCTCC | SEQ ID NO: 163 |
| *TRBV6-1*/*9* | GTGTGCCCAGGATATGAACCA | SEQ ID NO: 164 |
| *TRBV6-* | | SEQ ID NO: 165 |
| *2*/*3*/*5*/*6*/*8* | GTATCGACAAGACCCAGGCA | |
| *TRBV6-4* | TCACCCAGGCACCAACATC | SEQ ID NO: 166 |
| *TRBV6-7* | ATCGACAAGACCCAGGCAAG | SEQ ID NO: 167 |
| *TRBV7-1* | GTCCCTGAGACACAAGGTAGC | SEQ ID NO: 168 |
| *TRBV7-2* | TCTCCCAGTCCCCCAGTAAC | SEQ ID NO: 169 |
| *TRBV7-3* | TCTCCCAGACCCCCAGTAAC | SEQ ID NO: 170 |
| *TRBV7-4* | CCCCAAGGTACAAAGTCGCA | SEQ ID NO: 171 |
| *TRBV7-6*/*7* | TCCCAGTCTCCCAGGTACAAA | SEQ ID NO: 172 |
| *TRBV7-8* | CCCCTAGGTACAAAGTCGCA | SEQ ID NO: 173 |
| *TRBV7-9* | AACCGCCTTTATTGGTACCGA | SEQ ID NO: 174 |
| *TRBV9* | GATGCTCCCCTAGGTCTGGA | SEQ ID NO: 175 |
| *TRBV10-1* | ATCACCCAGAGCCCAAGACA | SEQ ID NO: 176 |
| *TRBV10-2* | CAGAGACAGGAAGGCAGGTG | SEQ ID NO: 177 |
| *TRBV10-3* | CTGGTATCGACAAGACCCGG | SEQ ID NO: 178 |
| *TRBV11-1* | ACCCTTTACTGGTACCGGCA | SEQ ID NO: 179 |
| *TRBV11-2* | TCTGGCCATGCTACCCTTTAC | SEQ ID NO: 180 |
| *TRBV11-3* | GTGGCTTTTTGGTGCAATCC | SEQ ID NO: 181 |
| *TRBV12-3* | ACAGACAGACCATGATGCGG | SEQ ID NO: 182 |
| *TRBV12-4* | CAGACAGACCATGATGCGGG | SEQ ID NO: 183 |
| *TRBV12-5* | CAGACACCAAGGCACAAGGT | SEQ ID NO: 184 |
| *TRBV13* | AGGGAAACAGCCACTCTGAA | SEQ ID NO: 185 |
| *TRBV14* | CCCAGCCACAGCGTAATAGA | SEQ ID NO: 186 |
| *TRBV15* | AAGCCAGTGACCCTGAGTTG | SEQ ID NO: 187 |
| *TRBV16* | CGCCCAGACTCCAAAACATC | SEQ ID NO: 188 |
| *TRBV17* | ACCGACAGAATCTGAGGCAAG | SEQ ID NO: 189 |
| *TRBV18* | GGAGGCAAGACTGAGATGCA | SEQ ID NO: 190 |
| *TRBV19* | GGCAAGGGCTGAGATTGATC | SEQ ID NO: 191 |
| *TRBV20-1* | GTGAAGATCGAGTGCCGTTC | SEQ ID NO: 192 |
| *TRBV23-1* | ACCCCCGAAAAAGGACATACT | SEQ ID NO: 193 |
| *TRBV24-1* | ATGCTGATGTTACCCAGACCC | SEQ ID NO: 194 |
| *TRBV25-1* | GTTCTCAAACCATGGGCCATG | SEQ ID NO: 195 |
| *TRBV27* | TGGTATCGACAAGACCCAGG | SEQ ID NO: 196 |
| *TRBV28* | GTGAAAGTAACCCAGAGCTCG | SEQ ID NO: 197 |
| *TRBV29-1* | TTCTGGTACCGTCAGCAACC | SEQ ID NO: 198 |
| *TRBV30* | GGGAACATCAAACCCCAACC | SEQ ID NO: 199 |

**Table 2. N2 forward primers.**

| *Gene Name* | **Primer Seq** | **SEQ ID NO** |
|---|---|---|
| *RORC* | CAGACGTGTGCTCTTCCGATCTACCCAAGAGAAGCAGAAGTCG | SEQ ID N O: 200 |
| *RUNX1* | CAGACGTGTGCTCTTCCGATCTGAAAATTCGTGCGTGGGCTT | SEQ ID NO: 201 |
| *RUNX3* | CAGACGTGTGCTCTTCCGATCTCACCTGCTGCACTCATCTGA | SEQ ID NO: 202 |
| *TBET* | CAGACGTGTGCTCTTCCGATCTGGTCAGGGAAAGGACTCACC | SEQ ID NO: 203 |
| *GATA3* | CAGACGTGTGCTCTTCCGATCTTAAGGTGGTTGTGCTCGGAG | SEQ ID NO: 204 |
| *PDCD1* | CAGACGTGTGCTCTTCCGATCTGCAGAAGTGCAGGCACCTA | SEQ ID NO: 205 |
| *BCL2* | | SEQ ID NO: 206 |
| *BCL6* | CAGACGTGTGCTCTTCCGATCTGTAGGCAGACACAGGGACTT | SEQ ID NO: 207 |
| *BIRC5* | CAGACGTGTGCTCTTCCGATCTTTGTCTAAGTGCAACCGCCT | SEQ ID NO: 208 |
| *CCL14* | CAGACGTGTGCTCTTCCGATCTCTTACCACCCCTCAGAGTGC | SEQ ID NO: 209 |
| *CCL17* | CAGACGTGTGCTCTTCCGATCTCTCACCCCAGACTCCTGACT | SEQ ID NO: 210 |
| *CCL18* | CAGACGTGTGCTCTTCCGATCTGTCCCATCTGCTATGCCCA | SEQ ID NO: 211 |
| *CCL20* | | SEQ ID NO: 212 |
| *CCL3* | CAGACGTGTGCTCTTCCGATCTCTCTGAGAGTTCCCCTGTCC | SEQ ID NO: 213 |
| *CCL4* | CAGACGTGTGCTCTTCCGATCTTGGAACTGAACTGAGCTGCT | SEQ ID NO: 214 |
| *CCR4* | | SEQ ID NO: 215 |
| *CCR7* | CAGACGTGTGCTCTTCCGATCTCTCTTGGCTCCACTGGGATG | SEQ ID NO: 216 |
| *CD160* | CAGACGTGTGCTCTTCCGATCTTTGTGCAGACCAAGAGCACC | SEQ ID NO: 217 |
| *CD244* | CAGACGTGTGCTCTTCCGATCTGGAAAGCGACAAGGGTGAAC | SEQ ID NO: 218 |
| *CD27* | CAGACGTGTGCTCTTCCGATCTCGTGACAGAGTGCCTTTTCG | SEQ ID NO: 219 |
| *CD28* | CAGACGTGTGCTCTTCCGATCTTGTAGATGACCTGGCTTGCC | SEQ ID NO: 220 |
| *CD30* | CAGACGTGTGCTCTTCCGATCTTGTTTGCCCAGTGTTTGTGC | SEQ ID NO: 221 |
| *CD38* | | SEQ ID NO: 222 |
| *CD3D* | CAGACGTGTGCTCTTCCGATCTTGATGTCATTGCCACTCTGCT | SEQ ID NO: 223 |
| *CD3E* | CAGACGTGTGCTCTTCCGATCTCTGGGGATGGACTGGGTAAAT | SEQ ID NO: 224 |
| *CD4* | CAGACGTGTGCTCTTCCGATCTACCACTTCCCTCAGTCCCAA | SEQ ID NO: 225 |
| *CD40LG* | CAGACGTGTGCTCTTCCGATCTGAGTCAGGCCGTTGCTAGTC | SEQ ID NO: 226 |
| *CD5* | CAGACGTGTGCTCTTCCGATCTAGGTCACAGATCTTCCCCCG | SEQ ID NO: 227 |
| *CD69* | | SEQ ID NO: 228 |
| *CD8A* | CAGACGTGTGCTCTTCCGATCTATGCCTGCCCATTGGAGAGAA | SEQ ID NO: 229 |
| *CD8B* | CAGACGTGTGCTCTTCCGATCTGCTGTCCAGTTCCCAGAAGG | SEQ ID NO: 230 |
| *CDCA7* | | SEQ ID NO: 231 |
| *CSF2* | CAGACGTGTGCTCTTCCGATCTGGCCACACTGACCCTGATAC | SEQ ID NO: 232 |
| *CX3CR1* | | SEQ ID NO: 233 |
| *CXCL12* | CAGACGTGTGCTCTTCCGATCTCTCATTCTGAAGGAGCCCCAT | SEQ ID NO: 234 |
| *CXCL13* | CAGACGTGTGCTCTTCCGATCTGCATTCGAAGATCCCCAGACTT | SEQ ID NO: 235 |
| *CXCR3* | CAGACGTGTGCTCTTCCGATCTCCAATATCCTCGCTCCCGG | SEQ ID NO: 236 |
| *CXCR5* | CAGACGTGTGCTCTTCCGATCTTCCTCGCAAGCTGGGTAATC | SEQ ID NO: 237 |
| *EOMES* | | SEQ ID NO: 238 |
| *FAS* | CAGACGTGTGCTCTTCCGATCTCCCCCATTTCCCCGATGT | SEQ ID NO: 239 |
| *FASLG* | CAGACGTGTGCTCTTCCGATCTGCATATCCTGAGCCATCGGT | SEQ ID NO: 240 |
| *FOSL1* | CAGACGTGTGCTCTTCCGATCTGGTGATTGGACCAGGCCATT | SEQ ID NO: 241 |
| *FOXP3* | CAGACGTGTGCTCTTCCGATCTGGGTCTCTTGAGTCCCGTG | SEQ ID NO: 242 |
| *GAPDH* | CAGACGTGTGCTCTTCCGATCTGCCCTCAACGACCACTTTGT | SEQ ID NO: 243 |
| *GZMA* | CAGACGTGTGCTCTTCCGATCTCCTTTGTTGTGCGAGGGTGT | SEQ ID NO: 244 |
| *GZMB* | CAGACGTGTGCTCTTCCGATCTAGGCCCTCTTGTGTGTAACA | SEQ ID NO: 245 |
| *GZMH* | CAGACGTGTGCTCTTCCGATCTCCAAAGAAGACACAGACCGGT | SEQ ID NO: 246 |
| *GZMK* | CAGACGTGTGCTCTTCCGATCTAAAGCAACCTTGTCCCGCCT | SEQ ID NO: 247 |
| *HLA-DRA* | CAGACGTGTGCTCTTCCGATCTGCCTCTTCGATTGCTCCGTA | SEQ ID NO: 248 |
| *IFNG* | CAGACGTGTGCTCTTCCGATCTCCTGCAATCTGAGCCAGTGC | SEQ ID NO: 249 |
| *IL10* | CAGACGTGTGCTCTTCCGATCTTTCAATTCCTCTGGGAATGTT | SEQ ID NO: 250 |
| *IL12A* | | SEQ ID NO: 251 |
| *IL12B* | CAGACGTGTGCTCTTCCGATCTTCCTCACCCCCACCTCTCTA | SEQ ID NO: 252 |
| *IL13* | CAGACGTGTGCTCTTCCGATCTTGCTACCTCACTGGGGTCCT | SEQ ID NO: 253 |
| *IL17A* | CAGACGTGTGCTCTTCCGATCTGCCCCTCAGAGATCAACAGAC | SEQ ID NO: 254 |
| *IL17F* | CAGACGTGTGCTCTTCCGATCTCTTACCCAGTGCTCTGCAAC | SEQ ID NO: 255 |
| *IL1A* | CAGACGTGTGCTCTTCCGATCTGCATTTTGGTCCAAGTTGTGC | SEQ ID NO: 256 |
| *IL1B* | CAGACGTGTGCTCTTCCGATCTACATTCTGATGAGCAACCGC | SEQ ID NO: 257 |
| *IL2* | | SEQ ID NO: 258 |
| *IL21* | CAGACGTGTGCTCTTCCGATCTCTGCCAGCTCCAGAAGATGT | SEQ ID NO: 259 |
| *IL21R* | CAGACGTGTGCTCTTCCGATCTAGACAAGAGCTGGCTCACCT | SEQ ID NO: 260 |
| *IL22* | CAGACGTGTGCTCTTCCGATCTGGAAACCAATGCCACTTTTGT | SEQ ID NO: 261 |
| *IL23R* | CAGACGTGTGCTCTTCCGATCTCTGGCCAATATGCTGAAACCC | SEQ ID NO: 262 |
| *IL24* | CAGACGTGTGCTCTTCCGATCTGCCCAGTGAGACTGTGTTGT | SEQ ID NO: 263 |
| *IL26* | CAGACGTGTGCTCTTCCGATCTTGTGTGTGGAGTGGGATGTG | SEQ ID NO: 264 |
| *IL3* | CAGACGTGTGCTCTTCCGATCTATTTCACCTTTTCCTGCGGC | SEQ ID NO: 265 |
| *IL31* | CAGACGTGTGCTCTTCCGATCTGTGTGGGAACTCTGCCGTG | SEQ ID NO: 266 |
| *IL32* | CAGACGTGTGCTCTTCCGATCTTGCTCTGAACCCCAATCCTC | SEQ ID NO: 267 |
| *IL33R* | CAGACGTGTGCTCTTCCGATCTAGGTACCAAATGCCTGTGCC | SEQ ID NO: 268 |
| *IL4* | CAGACGTGTGCTCTTCCGATCTCGGGCTTGAATTCCTGTCCT | SEQ ID NO: 269 |
| *IL4R* | CAGACGTGTGCTCTTCCGATCTTCCTCGTATGCATGGAACCC | SEQ ID NO: 270 |
| *IL5* | CAGACGTGTGCTCTTCCGATCTAGGCATACTGACACTTTGCC | SEQ ID NO: 271 |
| *IL6* | CAGACGTGTGCTCTTCCGATCTGGAAAGTGGCTATGCAGTTTG | SEQ ID NO: 272 |
| *IL6R* | CAGACGTGTGCTCTTCCGATCTACAGCATGTCACAAGGCTGT | SEQ ID NO: 273 |
| *IRF4* | CAGACGTGTGCTCTTCCGATCTGCCCCCAAATGAAAGCTTGA | SEQ ID NO: 274 |
| *LAG3* | CAGACGTGTGCTCTTCCGATCTCTTTGGAGAAGACAGTGGCGA | SEQ ID NO: 275 |
| *LIF* | CAGACGTGTGCTCTTCCGATCTTTGCCGGCTCTCCAGAGTA | SEQ ID NO: 276 |
| *LTA* | CAGACGTGTGCTCTTCCGATCTGGAGAAACAGAGACAGGCCC | SEQ ID NO: 277 |
| *MCL1* | CAGACGTGTGCTCTTCCGATCTTTTGCTTAGAAGGATGGCGC | SEQ ID NO: 278 |
| *MKI67* | CAGACGTGTGCTCTTCCGATCTTCCTGCCCCACCAAGATCAT | SEQ ID NO: 279 |
| *MYC* | CAGACGTGTGCTCTTCCGATCTCAACCTTGGCTGAGTCTTGA | SEQ ID NO: 280 |
| *NKG2D* | CAGACGTGTGCTCTTCCGATCTCCACCCTCCACAGGAAATTG | SEQ ID NO: 281 |
| *PRDM1* | CAGACGTGTGCTCTTCCGATCTACATGTGAATGTTGAGCCCA | SEQ ID NO: 282 |
| *PRF1* | CAGACGTGTGCTCTTCCGATCTCATGGCCACGTTGTCATTGT | SEQ ID NO: 283 |
| *PTPRCv1* | | SEQ ID NO: 284 |
| *PTPRCv2* | CAGACGTGTGCTCTTCCGATCTTAGTTGGCTATGCTGGCATG | SEQ ID NO: 285 |
| *SAP30* | CAGACGTGTGCTCTTCCGATCTTCACTAGGAGACGTGGAATTG | SEQ ID NO: 286 |
| *SELL* | CAGACGTGTGCTCTTCCGATCTCCTGCCCCCAGACCTTTTATC | SEQ ID NO: 287 |
| *TBX21* | CAGACGTGTGCTCTTCCGATCTAGAAAAGGGGCTGGAAAGGG | SEQ ID NO: 288 |
| *TGFB1* | CAGACGTGTGCTCTTCCGATCTACCTTGGGCACTGTTGAAGT | SEQ ID NO: 289 |
| *TNF* | CAGACGTGTGCTCTTCCGATCTGGCTCAGACATGTTTTCCGTG | SEQ ID NO: 290 |
| *TNFRSF9* | CAGACGTGTGCTCTTCCGATCTTTTTGATGTGAGGGGCGGAT | SEQ ID NO: 291 |
| *TYMS* | | SEQ ID NO: 292 |
| *UHRF1* | CAGACGTGTGCTCTTCCGATCTCCAAAGTTTGCAGCCTATACC | SEQ ID NO: 293 |
| *ZBED2* | CAGACGTGTGCTCTTCCGATCTGGTTTTGGTGGAGCTGACGA | SEQ ID NO: 294 |
| *ZNF683* | CAGACGTGTGCTCTTCCGATCTATCCACCTGAAGCTGCACC | SEQ ID NO: 295 |
| *TRAV1-1* | | SEQ ID NO: 296 |
| *TRAV1-2-01* | | SEQ ID NO: 297 |
| *TRAV1-2-02* | | SEQ ID NO: 298 |
| *TRAV2* | | SEQ ID NO: 299 |
| *TRAV3* | CAGACGTGTGCTCTTCCGATCTAACAAGAGCCAAACCTCCTTCC | SEQ ID NO: 300 |
| *TRAV4* | | SEQ ID NO: 301 |
| *TRAV5* | | SEQ ID NO: 302 |
| *TRAV6* | | SEQ ID NO: 303 |
| *TRAV7* | | SEQ ID NO: 304 |
| *TRAV8-1* | CAGACGTGTGCTCTTCCGATCTAGGAAACCCTCTGTGCAGTGG | SEQ ID NO: 305 |
| *TRAV8-2* | | SEQ ID NO: 306 |
| *TRAV8-3* | CAGACGTGTGCTCTTCCGATCTGGAAACCCTCTGTGCATTGG | SEQ ID NO: 307 |
| *TRAV8-4* | CAGACGTGTGCTCTTCCGATCTTATGAGCGACGCGGCTGAGTA | SEQ ID NO: 308 |
| *TRAV8-5* | | SEQ ID NO: 309 |
| *TRAV8-6* | | SEQ ID NO: 310 |
| *TRAV8-7* | | SEQ ID NO: 311 |
| *TRAV9-1* | | SEQ ID NO: 312 |
| *TRAV9-2* | | SEQ ID NO: 313 |
| *TRAV10* | CAGACGTGTGCTCTTCCGATCTAGCCTCCCAGCTCAGCGATTCA | SEQ ID NO: 314 |
| *TRAV11* | | SEQ ID NO: 315 |
| *TRAV12-1* | CAGACGTGTGCTCTTCCGATCTGAGACTCCAAGCTCAGTGATTC | SEQ ID NO: 316 |
| *TRAV12-2* | CAGACGTGTGCTCTTCCGATCTGACTCCCAGCCCAGTGATTC | SEQ ID NO: 317 |
| *TRAV12-3* | | SEQ ID NO: 318 |
| *TRAV13-1* | CAGACGTGTGCTCTTCCGATCTCAAACATTTCTCCCTGCACATC | SEQ ID NO: 319 |
| *TRAV13-2* | | SEQ ID NO: 320 |
| *TRAV14* | CAGACGTGTGCTCTTCCGATCTATCCGCCAACCTTGTCATCT | SEQ ID NO: 321 |
| *TRAV15* | CAGACGTGTGCTCTTCCGATCTGGTCTCTCATCCTGGAGATTCA | SEQ ID NO: 322 |
| *TRAV16* | | SEQ ID NO: 323 |
| *TRAV17* | | SEQ ID NO: 324 |
| *TRAV18* | | SEQ ID NO: 325 |
| *TRAV19* | | SEQ ID NO: 326 |
| *TRAV20* | CAGACGTGTGCTCTTCCGATCTTGCACATCACAGCCCCTAA | SEQ ID NO: 327 |
| *TRAV21* | | SEQ ID NO: 328 |
| *TRAV22* | CAGACGTGTGCTCTTCCGATCTCTCTTCCCAGACCACAGACTC | SEQ ID NO: 329 |
| *TRAV23* | | SEQ ID NO: 330 |
| *TRAV24* | | SEQ ID NO: 331 |
| *TRAV25* | | SEQ ID NO: 332 |
| *TRAV26-1* | | SEQ ID NO: 333 |
| *TRAV26-2* | CAGACGTGTGCTCTTCCGATCTTCTCTGGCAATCGCTGAAGAC | SEQ ID NO: 334 |
| *TRAV27* | | SEQ ID NO: 335 |
| *TRAV28* | | SEQ ID NO: 336 |
| *TRAV29* | | SEQ ID NO: 337 |
| *TRAV30* | CAGACGTGTGCTCTTCCGATCTCAGCAAAGCTCCCTGTACCT | SEQ ID NO: 338 |
| *TRAV31* | | SEQ ID NO: 339 |
| *TRAV32* | | SEQ ID NO: 340 |
| *TRAV33* | | SEQ ID NO: 341 |
| *TRAV34* | | SEQ ID NO: 342 |
| *TRAV35* | | SEQ ID NO: 343 |
| *TRAV36* | | SEQ ID NO: 344 |
| | | |
| *TRAV37* | CAGACGTGTGCTCTTCCGATCTAGATTCACAGCCAGGCTTAAAA | SEQ ID NO: 345 |
| *TRAV38-1* | CAGACGTGTGCTCTTCCGATCTTTCCAGAAAGCAGCCAAATCC | SEQ ID NO: 346 |
| *TRAV38-2* | CAGACGTGTGCTCTTCCGATCTCTTCCAGAAAGCAGCCAAATCC | SEQ ID NO: 347 |
| *TRAV39* | CAGACGTGTGCTCTTCCGATCTCTCAGCACCCTCCACATCAC | SEQ ID NO: 348 |
| *TRAV40* | | SEQ ID NO: 349 |
| *TRAV41* | | SEQ ID NO: 350 |
| *TRBV2?* | | SEQ ID NO: 351 |
| *TRBV3-1* | | SEQ ID NO: 352 |
| *TRBV4-1* | | SEQ ID NO: 353 |
| *TRBV(4-2, 4-3)*? | | SEQ ID NO: 354 |
| *TRBV5-1?* | | SEQ ID NO: 355 |
| *TRBV5-3?* | | SEQ ID NO: 356 |
| *TRBV(5-4, 5-5, 5-6, 5-7, 5-8)* | | SEQ ID NO: 357 |
| *TRBV6-1* | | SEQ ID NO: 358 |
| *TRBV(6-2, 6-3)* | CAGACGTGTGCTCTTCCGATCTGCTGGGGTTGGAGTCGGCTG | SEQ ID NO: 359 |
| *TRBV6-4?* | CAGACGTGTGCTCTTCCGATCTCCCTCACGTTGGCGTCTGCTG | SEQ ID NO: 360 |
| *TRBV6-5?* | CAGACGTGTGCTCTTCCGATCTGCTCAGGCTGCTGTCGGCTG | SEQ ID NO: 361 |
| *TRBV6-6?* | CAGACGTGTGCTCTTCCGATCTCGCTCAGGCTGGAGTTGGCTG | SEQ ID NO: 362 |
| *TRBV6-7?* | | SEQ ID NO: 363 |
| *TRBV6-8* | CAGACGTGTGCTCTTCCGATCTCACTCAGGCTGGTGTCGGCTG | SEQ ID NO: 364 |
| *?TRBV6-9?* | CAGACGTGTGCTCTTCCGATCTCGCTCAGGCTGGAGTCAGCTG | SEQ ID NO: 365 |
| *TRBV7-1?* | | SEQ ID NO: 366 |
| *TRBV7-2?* | | SEQ ID NO: 367 |
| *TRBV7-3?* | | SEQ ID NO: 368 |
| *TRBV7-4?* | | SEQ ID NO: 369 |
| *TRBV7-6?* | | SEQ ID NO: 370 |
| *TRBV7-7?* | | SEQ ID NO: 371 |
| *TRBV7-8?* | | SEQ ID NO: 372 |
| *TRBV7-9?* | | SEQ ID NO: 373 |
| *TRBV9?* | | SEQ ID NO: 374 |
| *TRBV10-1* | CAGACGTGTGCTCTTCCGATCTCCCCTCACTCTGGAGTCTGCTG | SEQ ID NO: 375 |
| *TRBV10-2?* | | SEQ ID NO: 376 |
| *TRBV10-3* | | SEQ ID NO: 377 |
| *TRBV(11-1, 11-3)* | | SEQ ID NO: 378 |
| *TRBV11-2* | | SEQ ID NO: 379 |
| *TRBV(12-3, 12-4, 12-5)* | | SEQ ID NO: 380 |
| *TRBV13* | | SEQ ID NO: 381 |
| *TRBV14* | | SEQ ID NO: 382 |
| *TRBV15* | | SEQ ID NO: 383 |
| *TRBV16* | | SEQ ID NO: 384 |
| *TRBV17* | | SEQ ID NO: 385 |
| *TRBV18* | | SEQ ID NO: 386 |
| *TRBV19* | CAGACGTGTGCTCTTCCGATCTCCTCTCACTGTGACATCGGCCC | SEQ ID NO: 387 |
| *TRBV20-1* | | SEQ ID NO: 388 |
| *TRBV23-1* | | SEQ ID NO: 389 |
| *TRBV24-1* | | SEQ ID NO: 390 |
| *TRBV25-1* | CAGACGTGTGCTCTTCCGATCTCCCTGACCCTGGAGTCTGCCA | SEQ ID NO: 391 |
| *TRBV27* | CAGACGTGTGCTCTTCCGATCTCCCTGATCCTGGAGTCGCCCA | SEQ ID NO: 392 |
| *TRBV28* | | SEQ ID NO: 393 |
| | | |
| *TRBV29-1* | | SEQ ID NO: 394 |
| *TRBV30* | | SEQ ID NO: 395 |

### Methods for Building and Decoding Substrates

### Methods for loading spatial labels on a substrate

Spatial labels can be pre-located on a substrate. A surface of substrate can be pre-imprinted with stochastic barcodes. In other words, the coordinates of each stochastic barcode on the surface of the substrate can be known. Stochastic barcodes can be pre-imprinted in any geometric manner. In some instances, a solid support comprising stochastic barcodes can be pre-located on a substrate.

In some instances, the coordinates of the stochastic barcodes on a substrate may be unknown. The location of the stochastic barcodes may be user-generated. When the location of the stochastic barcodes on a substrate is unknown, the location of the stochastic barcodes may be decoded.

The disclosure provides for methods for decoding substrates (e.g., arrays) comprising stochastic barcodes. Decoding may not rely solely on the use of optical signatures (although as described herein, the use of beads with optical signatures can allow the "reuse" of the decoding probes), but rather on the use of combinatorial decoding nucleic acids that are added during a decoding step. Decoding can be performed with sequential hybridizations. The decoding nucleic acids can hybridize either to a distinct identifier coding nucleic acid (identifier probe) that is placed on the beads, or to the bioactive agent itself, for example when the bioactive agent is a nucleic acid, at least some portion of which is single stranded to allow hybridization to a decoding probe. The decoding nucleic acids can be either directly or indirectly labeled. Decoding occurs by detecting the presence of the label.

The coding nucleic acids (also termed identifier probes (IP) or identifier nucleic acids) can comprise a primer sequence and an adjacent decoding sequence. Each decoder (or decoding) probe can comprise a priming sequence (sometimes referred to herein as an "invariant sequence"), that can hybridize to the primer sequence, and at least one decoding nucleotide, generally contained within a variable sequence. The decoder probes can be made as sets, with each set comprising at least four subsets that each have a different decoding nucleotide at the same position i.e. the detection position, (i.e. adenine, thymidine (or uracil, as desired), cytosine and guanine), with each nucleotide at the detection position (detection nucleotide) comprising a unique label, preferably a fluorophore. The decoder probes can be added under conditions that allow discrimination of perfect complementarity and imperfect complementarity. Thus, the decoding probe that comprises the correct base for base-pairing with the coding nucleotide being interrogated can hybridize the best. The other decoding probes can be washed away. The detection of the unique fluorophore associated with the detection nucleotide can allow for the identification of the coding nucleotide at that position. By repeating these steps with a new set of decoding probes that extends the position of the detection nucleotide by one base, the identity of next coding nucleotide can be elucidated. Decoding may use a large number of probes. Split and mix combinatorial synthesis can be used to prepare the decoding probes.

Parity analysis can be used during decoding to increase the robustness and accuracy of the system. Parity analysis can refer to a decoding step wherein the signal of a particular element can be analyzed across a plurality of decoding stages. That is, following at least one decoding step, the signal of an array element across the decoding stages can be analyzed. The signal from a particular bead can be evaluated across multiple stages. Although the analysis can include any parameter that can be obtained from the signals, such as evaluating the total signal obtained across the stages, the parity of the signals across the stages can be analyzed.

Parity can refer to the digital or modular readout of signals, i.e. odd or even, when binary signals are used. The digit sum of the signals across a plurality of stages can be translated into a parity determination. The parity determination can be useful in evaluating the decoding process. For example, codes can designed to have an odd number of a particular signal, for example a red signal, when viewed across all stages or decoding steps, or a pre-determined plurality of stages or decoding steps. The detection of an even number of red stages can provide an indication that an error has occurred at some point in decoding. When this result is obtained, the faulty code can either be discarded, or the analysis repeated.

The disclosure provides for introducing a "redundant stage" into the decoding system. A redundant stage can refer to a stage that serves as a parity check. That is, following the decoding stages, an additional stage can be included to analyze the parity. This analysis can provide an indication of the competence or validity of the decoding. When codes are designed with a pre-determined parity, the redundant stage can be used to detect the parity of the signals obtained from the decoding step. The redundant stage can detect errors in parity because if there has been an error in decoding, the parity detected following the redundant stage will be different from the parity designed into the codes.

In some instances, decoding can occur through the use of 8-mer oligonucleotides strung together to create a decoding oligonucleotide with a few 8-mers on it. The decoding oligonucleotide can comprise at least 1, 2, 3, 4, 5, 6, 7, 8, 9, or 10 or more 8-mers on it. The decoding oligonucleotide can comprise at most 1, 2, 3, 4, 5, 6, 7, 8, 9, or 10 or more 8-mers on it. The decoding oligonucleotide can hybridize to a few different stochastic barcodes (e.g., by hybridizing its different 8-mer regions to different 8-mer regions on stochastic barcodes). The decoding oligonucleotide can be fluorescently labeled, melted off, and sequenced. The decoding oligonucleotides can be fluorescently labeled in different colors. The decoding oligonucleotides can be fluorescently labeled with the same color but with various levels of fluorescent intensity, thereby generating a "gray-scale" map of a probe. Repeating this can provide a solvable map of where each 8-mer of a stochastic barcode is in relation to each other. The method can be repeated at least 1, 2, 3, 4, 5, 6, 7, 8, 9, or 10 or more times. The method can be repeated at most 1, 2, 3, 4, 5, 6, 7, 8, 9, or 10 or more times.

### Diffusion across a substrate

When a sample (e.g., one or more cells) is stochastically barcoded according to the methods of the disclosure, the cell may be lysed. Lysis of a cell can result in the diffusion of the contents of the lysis (e.g., cell contents) away from the initial location of lysis. In other words, the lysis contents can move into a larger surface area than the surface area taken up by the cell.

Diffusion of sample lysis mixture (e.g., comprising targets) can be modulated by various parameters including, but not limited to, viscosity of the lysis mixture, temperature of the lysis mixture, the size of the targets, the size of physical barriers in a substrate, the concentration of the lysis mixture, and the like. For example, the temperature of the lysis reaction can be performed at a temperature of at least 1, 2, 3, 4, 5, 10, 15, 20, 25, 30, 35, or 40C or more. The temperature of the lysis reaction can be performed at a temperature of at most 1, 2, 3, 4, 5, 10, 15, 20, 25, 30, 35, or 40C or more. The viscosity of the lysis mixture can be altered by, for example, adding thickening reagents (e.g., glycerol, beads) to slow the rate of diffusion. The viscosity of the lysis mixture can be altered by, for example, adding thinning reagents (e.g., water) to increase the rate of diffusion. A substrate can comprise physical barriers (e.g., wells, microwells, microhills) that can alter the rate of diffusion of targets from a sample. The concentration of the lysis mixture can be altered to increase or decrease the rate of diffusion of targets from a sample. The concentration of a lysis mixture can be increased or decreased by at least 1, 2, 3, 4, 5, 6, 7, 8, or 9 or more fold. The concentration of a lysis mixture can be increased or decreased by at most 1, 2, 3, 4, 5, 6, 7, 8, or 9 or more fold.

The rate of diffusion can be increased. The rate of diffusion can be decreased. The rate of diffusion of a lysis mixture can be increased or decreased by at least 1, 2, 3, 4, 5, 6, 7, 8, 9, or 10 or more fold compared to an un-altered lysis mixture. The rate of diffusion of a lysis mixture can be increased or decreased by at most 1, 2, 3, 4, 5, 6, 7, 8, 9, or 10 or more fold compared to an un-altered lysis mixture. The rate of diffusion of a lysis mixture can be increased or decreased by at least 10, 20, 30, 40, 50, 60, 70, 80, 90 or 100% compared to an un-altered lysis mixture. The rate of diffusion of a lysis mixture can be increased or decreased by at least 10, 20, 30, 40, 50, 60, 70, 80, 90 or 100% compared to an un-altered lysis mixture.

### Data Analysis and Display Software

### Data analysis and visualization of spatial resolution of targets

The disclosure provides for methods for estimating the number and position of targets with stochastic barcoding and digital counting using spatial labels. The data obtained from the methods of the disclosure can be visualized on a map. A map of the number and location of targets from a sample can be constructed using information generated using the methods described herein. The map can be used to locate a physical location of a target. The map can be used to identify the location of multiple targets. The multiple targets can be the same species of target, or the multiple targets can be multiple different targets. For example a map of a brain can be constructed to show the digital count and location of multiple targets.

The map can be generated from data from a single sample. The map can be constructed using data from multiple samples, thereby generating a combined map. The map can be constructed with data from tens, hundreds, and/or thousands of samples. A map constructed from multiple samples can show a distribution of digital counts of targets associated with regions common to the multiple samples. For example, replicated assays can be displayed on the same map. At least 1, 2, 3, 4, 5, 6, 7, 8, 9, or 10 or more replicates may be displayed (e.g., overlaid) on the same map. At most 1, 2, 3, 4, 5, 6, 7, 8, 9, or 10 or more replicates may be displayed (e.g., overlaid) on the same map. The spatial distribution and number of targets can be represented by a variety of statistics.

Combining data from multiple samples can increase the locational resolution of the combined map. The orientation of multiple samples can be registered by common landmarks, wherein the individual locational measurements across samples are at least in part non-contiguous. A particular example is sectioning a sample using a microtome on one axis and then sectioning a second sample along a different access. The combined dataset will give three dimensional spatial locations associated with digital counts of targets. Multiplexing the above approach will allow for high resolution three dimensional maps of digital counting statistics.

In some embodiments of the instrument system, the system will comprise computer-readable media that includes code for providing data analysis for the sequence datasets generated by performing single cell, stochastic barcoding assays. Examples of data analysis functionality that may be provided by the data analysis software include, but are not limited to, (i) algorithms for decoding/demultiplexing of the sample label, cell label, spatial label, and molecular label, and target sequence data provided by sequencing the stochastic barcode library created in running the assay, (ii) algorithms for determining the number of reads per gene per cell, and the number of unique transcript molecules per gene per cell, based on the data, and creating summary tables, (iii) statistical analysis of the sequence data, e.g. for clustering of cells by gene expression data, or for predicting confidence intervals for determinations of the number of transcript molecules per gene per cell, etc., (iv) algorithms for identifying sub-populations of rare cells, for example, using principal component analysis, hierarchical clustering, k-mean clustering, self-organizing maps, neural networks etc., , (v) sequence alignment capabilities for alignment of gene sequence data with known reference sequences and detection of mutation, polymorphic markers and splice variants, and (vi) automated clustering of molecular labels to compensate for amplification or sequencing errors. In some embodiments, commercially-available software may be used to perform all or a portion of the data analysis, for example, the Seven Bridges (https://www.sbgenomics.com/) software may be used to compile tables of the number of copies of one or more genes occurring in each cell for the entire collection of cells. In some embodiments, the data analysis software may include options for outputting the sequencing results in useful graphical formats, e.g. heatmaps that indicate the number of copies of one or more genes occurring in each cell of a collection of cells. In some embodiments, the data analysis software may further comprise algorithms for extracting biological meaning from the sequencing results, for example, by correlating the number of copies of one or more genes occurring in each cell of a collection of cells with a type of cell, a type of rare cell, or a cell derived from a subject having a specific disease or condition. In some embodiment, the data analysis software may further comprise algorithms for comparing populations of cells across different biological samples.

In some embodiments all of the data analysis functionality may be packaged within a single software package. In some embodiments, the complete set of data analysis capabilities may comprise a suite of software packages. In some embodiments, the data analysis software may be a standalone package that is made available to users independently of the assay instrument system. In some embodiments, the software may be web-based, and may allow users to share data.

### System Processors and Networks

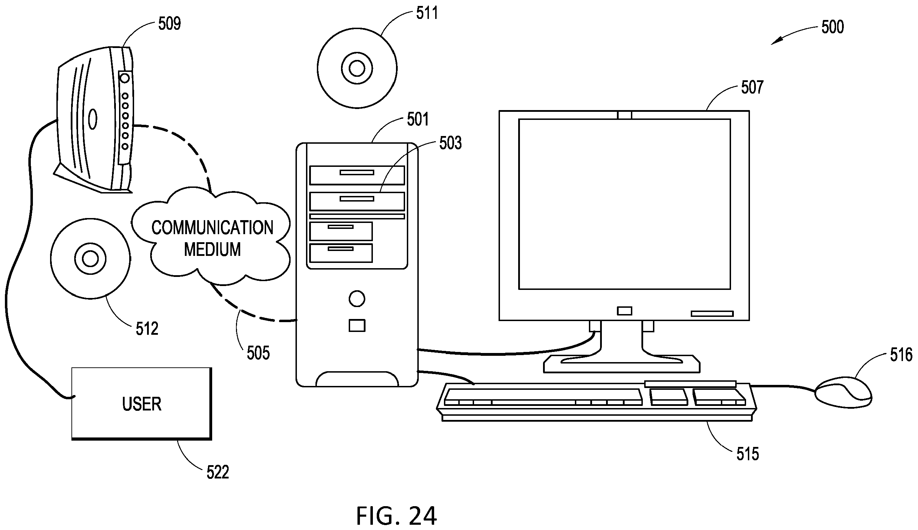

In general, the computer or processor included in the presently disclosed instrument systems, as illustrated in **Figure 24****,** may be further understood as a logical apparatus that can read instructions from media **511** or a network port **505,** which can optionally be connected to server **509** having fixed media **512.** The system **500,** such as shown in **Figure 24** can include a CPU **501,** disk drives **503,** optional input devices such as keyboard **515** or mouse **516** and optional monitor **507.** Data communication can be achieved through the indicated communication medium to a server at a local or a remote location. The communication medium can include any means of transmitting or receiving data. For example, the communication medium can be a network connection, a wireless connection or an internet connection. Such a connection can provide for communication over the World Wide Web. It is envisioned that data relating to the present disclosure can be transmitted over such networks or connections for reception or review by a party **522** as illustrated in **Figure 24****.**

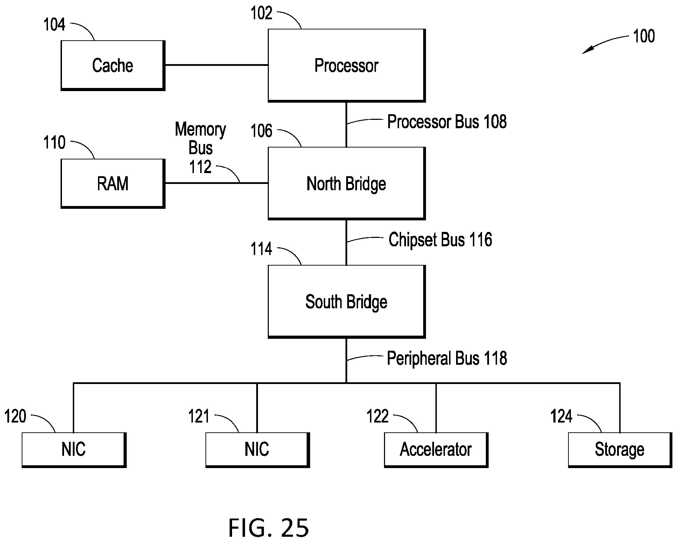

**Figure 25** illustrates an exemplary embodiment of a first example architecture of a computer system **100** that can be used in connection with example embodiments of the present disclosure. As depicted in **Figure 25****,** the example computer system can include a processor **102** for processing instructions. Non-limiting examples of processors include: Intel XeonTM processor, AMD OpteronTM processor, Samsung 32-bit RISC ARM 1176JZ(F)-S v1.0TM processor, ARM Cortex-A8 Samsung S5PC100TM processor, ARM Cortex-A8 Apple A4TM processor, Marvell PXA 930TM processor, or a functionally-equivalent processor. Multiple threads of execution can be used for parallel processing. In some embodiments, multiple processors or processors with multiple cores can also be used, whether in a single computer system, in a cluster, or distributed across systems over a network comprising a plurality of computers, cell phones, or personal data assistant devices.

As illustrated in **Figure 25****,** a high speed cache **104** can be connected to, or incorporated in, the processor **102** to provide a high speed memory for instructions or data that have been recently, or are frequently, used by processor **102.** The processor **102** is connected to a north bridge **106** by a processor bus **108.** The north bridge **106** is connected to random access memory (RAM) **110** by a memory bus **112** and manages access to the RAM **110** by the processor **102.** The north bridge **106** is also connected to a south bridge **114** by a chipset bus **116.** The south bridge **114** is, in turn, connected to a peripheral bus **118.** The peripheral bus can be, for example, PCI, PCI-X, PCI Express, or other peripheral bus. The north bridge and south bridge are often referred to as a processor chipset and manage data transfer between the processor, RAM, and peripheral components on the peripheral bus **118.** In some alternative architectures, the functionality of the north bridge can be incorporated into the processor instead of using a separate north bridge chip.

In some embodiments, system **100** can include an accelerator card **122** attached to the peripheral bus **118.** The accelerator can include field programmable gate arrays (FPGAs) or other hardware for accelerating certain processing. For example, an accelerator can be used for adaptive data restructuring or to evaluate algebraic expressions used in extended set processing.

Software and data are stored in external storage **124** and can be loaded into RAM **110** or cache **104** for use by the processor. The system **100** includes an operating system for managing system resources; non-limiting examples of operating systems include: Linux, WindowsTM, MACOSTM, BlackBerry OSTM, iOSTM, and other functionally-equivalent operating systems, as well as application software running on top of the operating system for managing data storage and optimization in accordance with example embodiments of the present disclosure.

In this example, system **100** also includes network interface cards (NICs) **120** and **121** connected to the peripheral bus for providing network interfaces to external storage, such as Network Attached Storage (NAS) and other computer systems that can be used for distributed parallel processing.

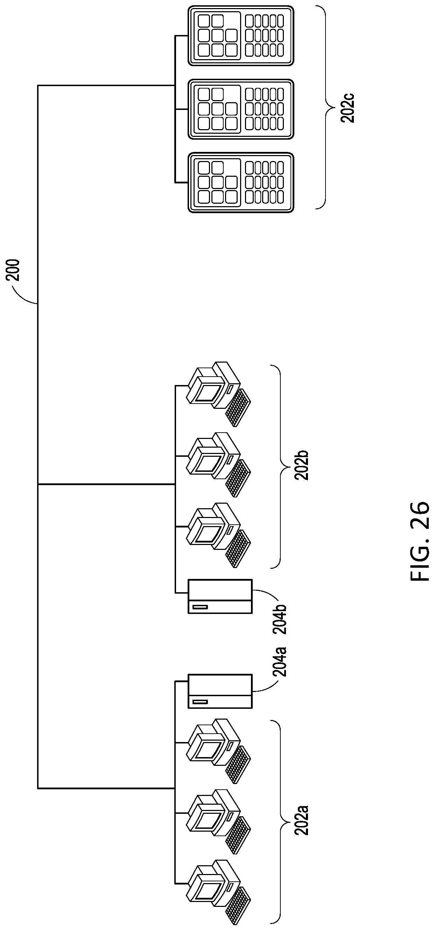

**Figure 26** illustrates an exemplary diagram showing a network **200** with a plurality of computer systems **202a,** and **202b,** a plurality of cell phones and personal data assistants **202c,** and Network Attached Storage (NAS) **204a,** and **204b.** In example embodiments, systems **212a, 212b,** and **212c** can manage data storage and optimize data access for data stored in Network Attached Storage (NAS) **214a** and **214b.** A mathematical model can be used for the data and be evaluated using distributed parallel processing across computer systems **212a,** and **212b,** and cell phone and personal data assistant systems **212c.** Computer systems **212a,** and **212b,** and cell phone and personal data assistant systems **212c** can also provide parallel processing for adaptive data restructuring of the data stored in Network Attached Storage (NAS) **214a** and **214b.** **Figure 26** illustrates an example only, and a wide variety of other computer architectures and systems can be used in conjunction with the various embodiments of the present invention. For example, a blade server can be used to provide parallel processing. Processor blades can be connected through a back plane to provide parallel processing. Storage can also be connected to the back plane or as Network Attached Storage (NAS) through a separate network interface.

In some example embodiments, processors can maintain separate memory spaces and transmit data through network interfaces, back plane or other connectors for parallel processing by other processors. In other embodiments, some or all of the processors can use a shared virtual address memory space.

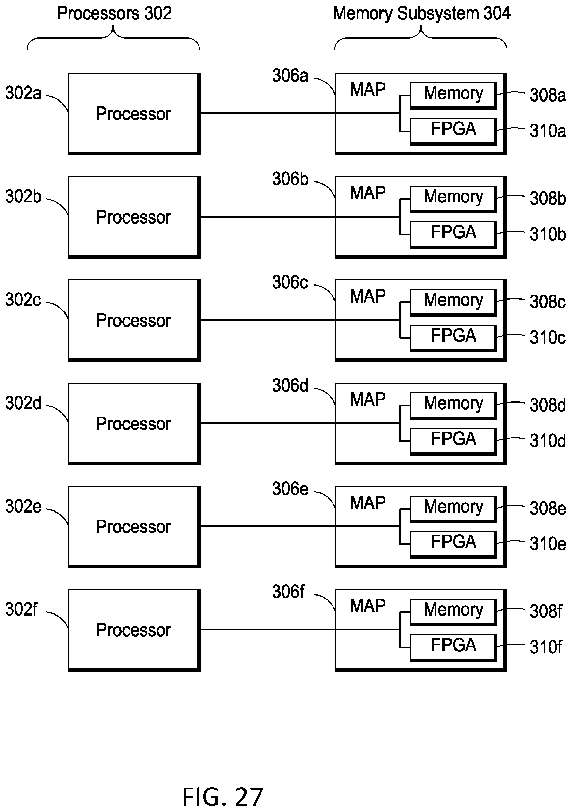

**Figure 27** illustrates an exemplary a block diagram of a multiprocessor computer system **300** using a shared virtual address memory space in accordance with an example embodiment. The system includes a plurality of processors **302a-f** that can access a shared memory subsystem **304.** The system incorporates a plurality of programmable hardware memory algorithm processors (MAPs) **306a-f** in the memory subsystem **304.** Each MAP **306a-f** can comprise a memory **308a-f** and one or more field programmable gate arrays (FPGAs) **310a-**f. The MAP provides a configurable functional unit and particular algorithms or portions of algorithms can be provided to the FPGAs **310a-f** for processing in close coordination with a respective processor. For example, the MAPs can be used to evaluate algebraic expressions regarding the data model and to perform adaptive data restructuring in example embodiments. In this example, each MAP is globally accessible by all of the processors for these purposes. In one configuration, each MAP can use Direct Memory Access (DMA) to access an associated memory **308a-f,** allowing it to execute tasks independently of, and asynchronously from, the respective microprocessor **302a-f.** In this configuration, a MAP can feed results directly to another MAP for pipelining and parallel execution of algorithms.

The above computer architectures and systems are examples only, and a wide variety of other computer, cell phone, and personal data assistant architectures and systems can be used in connection with example embodiments, including systems using any combination of general processors, co-processors, FPGAs and other programmable logic devices, system on chips (SOCs), application specific integrated circuits (ASICs), and other processing and logic elements. In some embodiments, all or part of the computer system can be implemented in software or hardware. Any variety of data storage media can be used in connection with example embodiments, including random access memory, hard drives, flash memory, tape drives, disk arrays, Network Attached Storage (NAS) and other local or distributed data storage devices and systems.

In example embodiments, the computer subsystem of the present disclosure can be implemented using software modules executing on any of the above or other computer architectures and systems. In other embodiments, the functions of the system can be implemented partially or completely in firmware, programmable logic devices such as field programmable gate arrays (FPGAs), system on chips (SOCs), application specific integrated circuits (ASICs), or other processing and logic elements. For example, the Set Processor and Optimizer can be implemented with hardware acceleration through the use of a hardware accelerator card, such as accelerator card.

### Kits

Disclosed herein are kits for performing single cell, stochastic barcoding assays. The kit may comprise one or more substrates (e.g., microwell array), either as a free-standing substrate (or chip) comprising one or more microwell arrays, or packaged within one or more flow-cells or cartridges, and one or more solid support suspensions, wherein the individual solid supports within a suspension comprise a plurality of attached stochastic barcodes of the disclosure. In some embodiments, the kit may further comprise a mechanical fixture for mounting a free-standing substrate in order to create reaction wells that facilitate the pipetting of samples and reagents into the substrate. The kit may further comprise reagents, e.g. lysis buffers, rinse buffers, or hybridization buffers, for performing the stochastic barcoding assay. The kit may further comprise reagents (e.g. enzymes, primers, dNTPs, NTPs, RNAse inhibitors, or buffers) for performing nucleic acid extension reactions, for example, reverse transcription reactions. The kit may further comprise reagents (e.g. enzymes, universal primers, sequencing primers, target-specific primers, or buffers) for performing amplification reactions to prepare sequencing libraries.

The kits can comprise a plate (e.g., 96 well plate) wherein each well of the plate can comprise one or more reverse transcription primer. The number of types of reverse transcription primers in each well can be at least 1, 2, 3, 4, 5, 6, 7, 8, 9 or 10 or more. The number of types of reverse transcription primers in each well can be at most 1, 2, 3, 4, 5, 6, 7, 8, 9 or 10 or more. The reverse transcription primers can be gene-specific, e.g., gene-specific for one or more alpha chains of a TCR, one or more beta chains of a TCR, or one or more of both alpha and beta chains of the TCR, and/or for a TCR marker.

The kit can comprise forward gene-specific primers. Forward gene-specific primers can be used in the N1 and/or N2 (e.g., nested). The gene-specific primers can be specific for any gene. In some instances, the gene specific forward primers are specific for T cells.

The kit can comprise one or more sequencing primers (e.g., sequencing flow cell primers) for use in a sequencing reaction.

The kit may comprise one or more molds, for example, molds comprising an array of micropillars, for casting substrates (e.g., microwell arrays), and one or more solid supports (e.g., bead), wherein the individual beads within a suspension comprise a plurality of attached stochastic barcodes of the disclosure. The kit may further comprise a material for use in casting substrates (e.g. agarose, a hydrogel, PDMS, and the like).

The kit may comprise one or more substrates that are pre-loaded with solid supports comprising a plurality of attached stochastic barcodes of the disclosure. In some instances, there may be on solid support per microwell of the substrate. In some embodiments, the plurality of stochastic barcodes may be attached directly to a surface of the substrate, rather than to a solid support. In any of these embodiments, the one or more microwell arrays may be provided in the form of free-standing substrates (or chips), or they may be packed in flow-cells or cartridges.

In some embodiments of the disclosed kits, the kit may comprise one or more cartridges that incorporate one or more substrates. In some embodiments, the one or more cartridges may further comprise one or more pre-loaded solid supports, wherein the individual solid supports within a suspension comprise a plurality of attached stochastic barcodes of the disclosure. In some embodiments, the beads may be pre-distributed into the one or more microwell arrays of the cartridge. In some embodiments, the beads, in the form of suspensions, may be pre-loaded and stored within reagent wells of the cartridge. In some embodiments, the one or more cartridges may further comprise other assay reagents that are pre-loaded and stored within reagent reservoirs of the cartridges.

The kit can include software (e.g., software of the disclosure). The software can comprise scripts that can be run locally on a computer or from the cloud. The software can be used for analyzing sequencing data (e.g., for counting the number of molecular indexes for a target, for adjusting the number of counted molecular indexes for a target, for providing sequences of the target that has been counted).

Kits can generally include instructions for carrying out one or more of the methods described herein. Instructions included in kits can be affixed to packaging material or can be included as a package insert. While the instructions are typically written or printed materials they are not limited to such. Any medium capable of storing such instructions and communicating them to an end user is contemplated by the disclosure. Such media can include, but are not limited to, electronic storage media (e.g., magnetic discs, tapes, cartridges, chips), optical media (e.g., CD ROM), RF tags, and the like. As used herein, the term "instructions" can include the address of an internet site that provides the instructions.

### Devices

### Flow Cells

The microwell array substrate can be packaged within a flow cell that provides for convenient interfacing with the rest of the fluid handling system and facilitates the exchange of fluids, e.g. cell and solid support suspensions, lysis buffers, rinse buffers, etc., that are delivered to the microwell array and/or emulsion droplet. Design features may include: (i) one or more inlet ports for introducing cell samples, solid support suspensions, or other assay reagents, (ii) one or more microwell array chambers designed to provide for uniform filling and efficient fluid-exchange while minimizing back eddies or dead zones, and (iii) one or more outlet ports for delivery of fluids to a sample collection point or a waste reservoir. The design of the flow cell may include a plurality of microarray chambers that interface with a plurality of microwell arrays such that one or more different cell samples may be processed in parallel. The design of the flow cell may further include features for creating uniform flow velocity profiles, i.e. "plug flow", across the width of the array chamber to provide for more uniform delivery of cells and beads to the microwells, for example, by using a porous barrier located near the chamber inlet and upstream of the microwell array as a "flow diffuser", or by dividing each array chamber into several subsections that collectively cover the same total array area, but through which the divided inlet fluid stream flows in parallel. In some embodiments, the flow cell may enclose or incorporate more than one microwell array substrate. In some embodiments, the integrated microwell array / flow cell assembly may constitute a fixed component of the system. In some embodiments, the microwell array / flow cell assembly may be removable from the instrument.

In general, the dimensions of fluid channels and the array chamber(s) in flow cell designs will be optimized to (i) provide uniform delivery of cells and beads to the microwell array, and (ii) to minimize sample and reagent consumption. In some embodiments, the width of fluid channels will be between 50 um and 20 mm. In other embodiments, the width of fluid channels may be at least 50 um, at least 100 um, at least 200 um, at least 300 um, at least 400 um, at least 500 um, at least 750 um, at least 1 mm, at least 2.5 mm, at least 5 mm, at least 10 mm, at least 20 mm, at least 50 mm, at least 100 mm, or at least 150 mm. In yet other embodiments, the width of fluid channels may be at most 150 mm, at most 100 mm, at most 50 mm, at most 20 mm, at most 10 mm, at most 5 mm, at most 2.5 mm, at most 1 mm, at most 750 um, at most 500 um, at most 400 um, at most 300 um, at most 200 um, at most 100 um, or at most 50 um. In one embodiment, the width of fluid channels is about 2 mm. The width of the fluid channels may fall within any range bounded by any of these values (e.g. from about 250 um to about 3 mm).

In some embodiments, the depth of the fluid channels will be between 50 um and 2 mm. In other embodiments, the depth of fluid channels may be at least 50 um, at least 100 um, at least 200 um, at least 300 um, at least 400 um, at least 500 um, at least 750 um, at least 1 mm, at least 1.25 mm, at least 1.5 mm, at least 1.75 mm, or at least 2 mm. In yet other embodiments, the depth of fluid channels may at most 2 mm, at most 1.75 mm, at most 1.5 mm, at most 1.25 mm, at most 1 mm, at most 750 um, at most 500 um, at most 400 um, at most 300 um, at most 200 um, at most 100 um, or at most 50 um. In one embodiment, the depth of the fluid channels is about 1 mm. The depth of the fluid channels may fall within any range bounded by any of these values (e.g. from about 800 um to about 1 mm).

Flow cells may be fabricated using a variety of techniques and materials known to those of skill in the art. In general, the flow cell will be fabricated as a separate part and subsequently either mechanically clamped or permanently bonded to the microwell array substrate. Examples of suitable fabrication techniques include conventional machining, CNC machining, injection molding, 3D printing, alignment and lamination of one or more layers of laser or die-cut polymer films, or any of a number of microfabrication techniques such as photolithography and wet chemical etching, dry etching, deep reactive ion etching, or laser micromachining. Once the flow cell part has been fabricated it may be attached to the microwell array substrate mechanically, e.g. by clamping it against the microwell array substrate (with or without the use of a gasket), or it may be bonded directly to the microwell array substrate using any of a variety of techniques (depending on the choice of materials used) known to those of skill in the art, for example, through the use of anodic bonding, thermal bonding, or any of a variety of adhesives or adhesive films, including epoxy-based, acrylic-based, silicone-based, UV curable, polyurethane-based, or cyanoacrylate-based adhesives.

Flow cells may be fabricated using a variety of materials known to those of skill in the art. In general, the choice of material used will depend on the choice of fabrication technique used, and vice versa. Examples of suitable materials include, but are not limited to, silicon, fused-silica, glass, any of a variety of polymers, e.g. polydimethylsiloxane (PDMS; elastomer), polymethylmethacrylate (PMMA), polycarbonate (PC), polypropylene (PP), polyethylene (PE), high density polyethylene (HDPE), polyimide, cyclic olefin polymers (COP), cyclic olefin copolymers (COC), polyethylene terephthalate (PET), epoxy resins, metals (e.g. aluminum, stainless steel, copper, nickel, chromium, and titanium), a non-stick material such as teflon (PTFE), or a combination of these materials.

### Cartridges

In some embodiments of the system, the microwell array, with or without an attached flow cell, can be packaged within a consumable cartridge that interfaces with the instrument system. Design features of cartridges may include (i) one or more inlet ports for creating fluid connections with the instrument or manually introducing cell samples, bead suspensions, or other assay reagents into the cartridge, (ii) one or more bypass channels, i.e. for self-metering of cell samples and bead suspensions, to avoid overfilling or back flow, (iii) one or more integrated microwell array / flow cell assemblies, or one or more chambers within which the microarray substrate(s) are positioned, (iv) integrated miniature pumps or other fluid actuation mechanisms for controlling fluid flow through the device, (v) integrated miniature valves (or other containment mechanisms) for compartmentalizing pre-loaded reagents (for example, bead suspensions)or controlling fluid flow through the device, (vi) one or more vents for providing an escape path for trapped air, (vii) one or more sample and reagent waste reservoirs, (viii) one or more outlet ports for creating fluid connections with the instrument or providing a processed sample collection point, (ix) mechanical interface features for reproducibly positioning the removable, consumable cartridge with respect to the instrument system, and for providing access so that external magnets can be brought into close proximity with the microwell array, (x) integrated temperature control components or a thermal interface for providing good thermal contact with the instrument system, and (xi) optical interface features, e.g. a transparent window, for use in optical interrogation of the microwell array.

The cartridge can be designed to process more than one sample in parallel. The cartridge may further comprise one or more removable sample collection chamber(s) that are suitable for interfacing with stand-alone PCR thermal cyclers or sequencing instruments. The cartridge itself can be suitable for interfacing with stand-alone PCR thermal cyclers or sequencing instruments. The term "cartridge" as used in this disclosure can be meant to include any assembly of parts which contains the sample and beads during performance of the assay.

The cartridge may further comprise components that are designed to create physical or chemical barriers that prevent diffusion of (or increase pathlengths and diffusion times for) large molecules in order to minimize cross-contamination between microwells. Examples of such barriers can include, but are not limited to, a pattern of serpentine channels used for delivery of cells and solid supports (e.g., beads) to the microwell array, a retractable platen or deformable membrane that is pressed into contact with the surface of the microwell array substrate during lysis or incubation steps, the use of larger beads, e.g. Sephadex beads as described previously, to block the openings of the microwells, or the release of an immiscible, hydrophobic fluid from a reservoir within the cartridge during lysis or incubation steps, to effectively separate and compartmentalize each microwell in the array.

The dimensions of fluid channels and the array chamber(s) in cartridge designs can be optimized to (i) provide uniform delivery of cells and beads to the microwell array, and (ii) to minimize sample and reagent consumption. The width of fluid channels can be between 50 micrometers and 20 mm. In other embodiments, the width of fluid channels may be at least 50 micrometers, at least 100 micrometers, at least 200 micrometers, at least 300 micrometers, at least 400 micrometers, at least 500 micrometers, at least 750 micrometers, at least 1 mm, at least 2.5 mm, at least 5 mm, at least 10 mm, or at least 20 mm. In yet other embodiments, the width of fluid channels may at most 20 mm, at most 10 mm, at most 5 mm, at most 2.5 mm, at most 1 mm, at most 750 micrometers, at most 500 micrometers, at most 400 micrometers, at most 300 micrometers, at most 200 micrometers, at most 100 micrometers, or at most 50 micrometers. The width of fluid channels can be about 2 mm. The width of the fluid channels may fall within any range bounded by any of these values (e.g. from about 250 um to about 3 mm).

The fluid channels in the cartridge can have a depth. The depth of the fluid channels in cartridge designs can be between 50 micrometers and 2 mm. The depth of fluid channels may be at least 50 micrometers, at least 100 micrometers, at least 200 micrometers, at least 300 micrometers, at least 400 micrometers, at least 500 micrometers, at least 750 micrometers, at least 1 mm, at least 1.25 mm, at least 1.5 mm, at least 1.75 mm, or at least 2 mm. The depth of fluid channels may at most 2 mm, at most 1.75 mm, at most 1.5 mm, at most 1.25 mm, at most 1 mm, at most 750 micrometers, at most 500 micrometers, at most 400 micrometers, at most 300 micrometers, at most 200 micrometers, at most 100 micrometers, or at most 50 micrometers. The depth of the fluid channels can be about 1 mm. The depth of the fluid channels may fall within any range bounded by any of these values (e.g. from about 800 micrometers to about 1 mm).

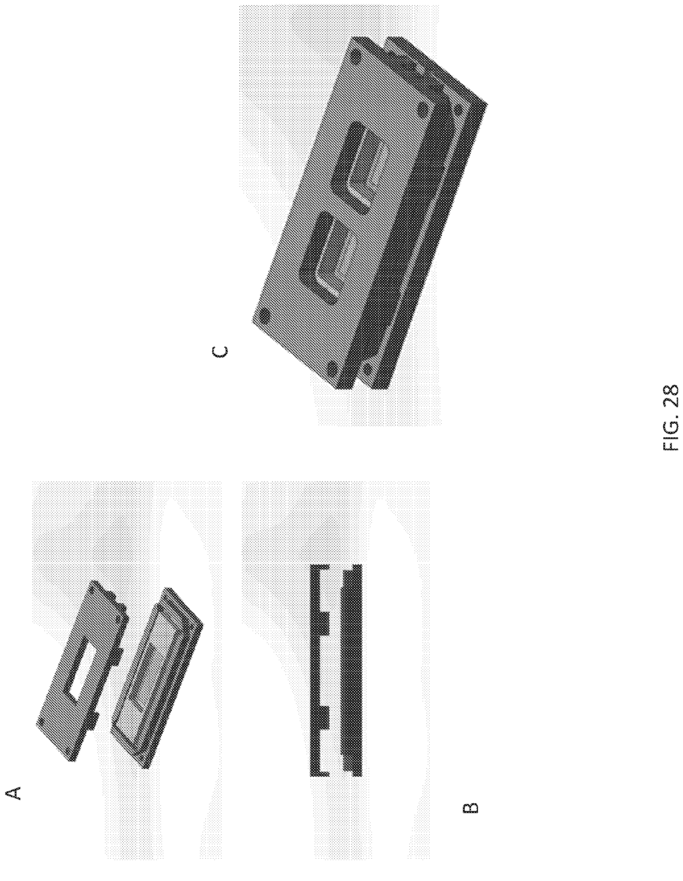

Cartridges may be fabricated using a variety of techniques and materials known to those of skill in the art. In general, the cartridges will be fabricated as a series of separate component parts **(****Figure 28A-C****)** and subsequently assembled using any of a number of mechanical assembly or bonding techniques. Examples of suitable fabrication techniques include, but are not limited to, conventional machining, CNC machining, injection molding, thermoforming, and 3D printing. Once the cartridge components have been fabricated they may be mechanically assembled using screws, clips, and the like, or permanently bonded using any of a variety of techniques (depending on the choice of materials used), for example, through the use of thermal bonding/welding or any of a variety of adhesives or adhesive films, including epoxy-based, acrylic-based, silicone-based, UV curable, polyurethane-based, or cyanoacrylate-based adhesives.

Cartridge components may be fabricated using any of a number of suitable materials, including but not limited to silicon, fused-silica, glass, any of a variety of polymers, e.g. polydimethylsiloxane (PDMS; elastomer), polymethylmethacrylate (PMMA), polycarbonate (PC), polypropylene (PP), polyethylene (PE), high density polyethylene (HDPE), polyimide, cyclic olefin polymers (COP), cyclic olefin copolymers (COC), polyethylene terephthalate (PET), epoxy resins, non-stick materials such as teflon (PTFE), metals (e.g. aluminum, stainless steel, copper, nickel, chromium, and titanium), or any combination thereof.

The inlet and outlet features of the cartridge may be designed to provide convenient and leak-proof fluid connections with the instrument, or may serve as open reservoirs for manual pipetting of samples and reagents into or out of the cartridge. Examples of convenient mechanical designs for the inlet and outlet port connectors can include, but are not limited to, threaded connectors, Luer lock connectors, Luer slip or "slip tip" connectors, press fit connectors, and the like. The inlet and outlet ports of the cartridge may further comprise caps, spring-loaded covers or closures, or polymer membranes that may be opened or punctured when the cartridge is positioned in the instrument, and which serve to prevent contamination of internal cartridge surfaces during storage or which prevent fluids from spilling when the cartridge is removed from the instrument. The one or more outlet ports of the cartridge may further comprise a removable sample collection chamber that is suitable for interfacing with stand-alone PCR thermal cyclers or sequencing instruments.

The cartridge may include integrated miniature pumps or other fluid actuation mechanisms for control of fluid flow through the device. Examples of suitable miniature pumps or fluid actuation mechanisms can include, but are not limited to, electromechanically- or pneumatically-actuated miniature syringe or plunger mechanisms, membrane diaphragm pumps actuated pneumatically or by an external piston, pneumatically-actuated reagent pouches or bladders, or electro-osmotic pumps.

The cartridge may include miniature valves for compartmentalizing pre-loaded reagents or controlling fluid flow through the device. Examples of suitable miniature valves can include, but are not limited to, one-shot "valves" fabricated using wax or polymer plugs that can be melted or dissolved, or polymer membranes that can be punctured; pinch valves constructed using a deformable membrane and pneumatic, magnetic, electromagnetic, or electromechanical (solenoid) actuation, one-way valves constructed using deformable membrane flaps, and miniature gate valves.

The cartridge may include vents for providing an escape path for trapped air. Vents may be constructed according to a variety of techniques, for example, using a porous plug of polydimethylsiloxane (PDMS) or other hydrophobic material that allows for capillary wicking of air but blocks penetration by water.

The mechanical interface features of the cartridge can provide for easily removable but highly precise and repeatable positioning of the cartridge relative to the instrument system. Suitable mechanical interface features can include, but are not limited to, alignment pins, alignment guides, mechanical stops, and the like. The mechanical design features can include relief features for bringing external apparatus, e.g. magnets or optical components, into close proximity with the microwell array chamber **(****Figure 28B****).**

The cartridge can also include temperature control components or thermal interface features for mating to external temperature control modules. Examples of suitable temperature control elements can include, but are not limited to, resistive heating elements, miniature infrared-emitting light sources, Peltier heating or cooling devices, heat sinks, thermistors, thermocouples, and the like. Thermal interface features can be fabricated from materials that are good thermal conductors (e.g. copper, gold, silver, etc.) and can comprise one or more flat surfaces capable of making good thermal contact with external heating blocks or cooling blocks.

The cartridge can include optical interface features for use in optical imaging or spectroscopic interrogation of the microwell array. The cartridge can include an optically transparent window, e.g. the microwell substrate itself or the side of the flow cell or microarray chamber that is opposite the microwell array, fabricated from a material that meets the spectral requirements for the imaging or spectroscopic technique used to probe the microwell array. Examples of suitable optical window materials can include, but are not limited to, glass, fused-silica, polymethylmethacrylate (PMMA), polycarbonate (PC), cyclic olefin polymers (COP), or cyclic olefin copolymers (COC).

It is intended that the following claims define the scope of the invention .

### EXAMPLES

Although the present disclosure has been illustrated by the following examples, it is not to be construed as being limited thereby; .

### Example 1. Adaptor ligation of sequencing primers

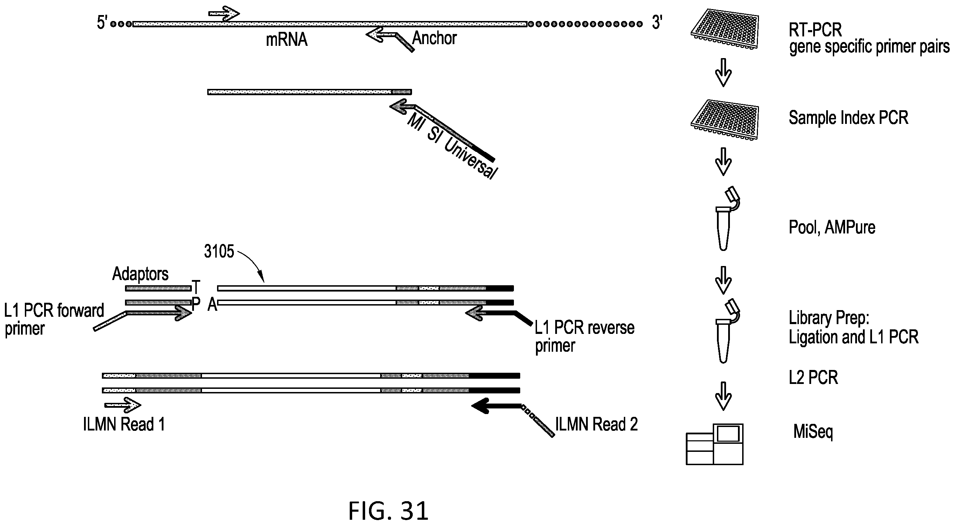

As shown in Figure 31, adaptors can be ligated to the gene-specific amplified product 3105. The gene-specific amplified product has an A nucleotide overhang deposited by the polymerase. The adaptors have a T overhang on one strand (e.g., sense strand) and a free phosphate on the other strand (e.g., antisense strand). The adaptors can be ligated to the gene-specific amplified product by ligation. The adaptor ligates to both the 5' and 3' ends of the gene-specific amplified product. The adaptor-ligated gene-specific product is amplified, for example, with sequencing (e.g., flow cell) primers. The subsequent PCR reaction originating from the forward sequencing primer (e.g., ILMN Read 1) extends into the read 2 sequence on the 3' end. The reverse sequencing primer (e.g., ILMN Read 2) may not extend into the Read 1 sequence (e.g., the 5' end) due to the lack of phosphorylation site on the sense strand of the adaptor. In this way, the reverse amplification reaction is linear while the forward amplification reaction is exponential.

### Example 2. High rates of demultiplexing and mapping of gene-specific product

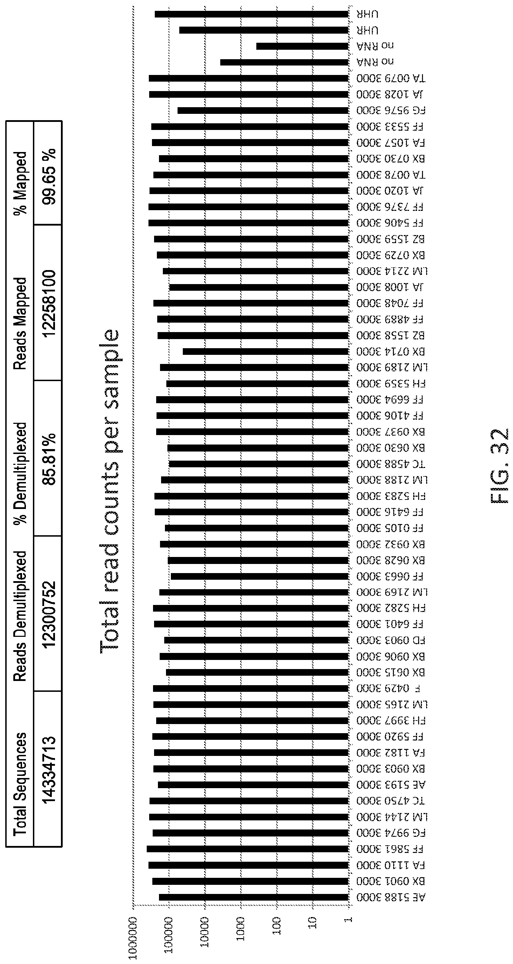

The adaptor-ligated amplified product shown in **Figure 31** can be subject to sequencing, demultiplexing, and mapping. **Figure 32** shows an exemplary embodiment of the demultiplexing and mapping rates of the gene-specific amplicons as well as the total read counts per sample.

### Example 3. Distribution of read counts across amplicons

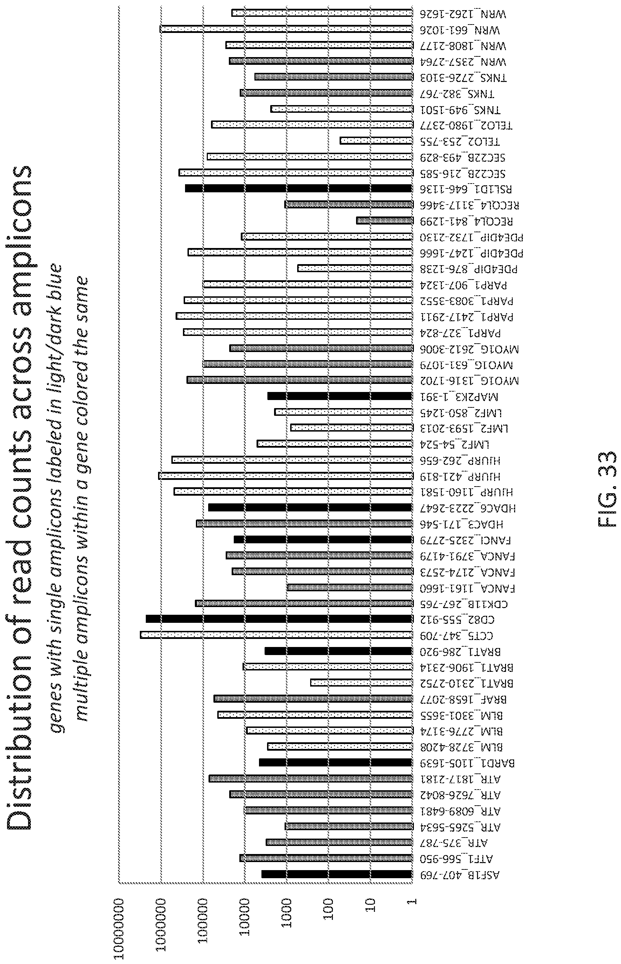

In the gene-specific methods of the disclosure, different regions (e.g., amplicons) of the same gene can be amplified, for example, for SNP identification. Figure 33 shows the read counts of amplicons within a gene. Genes with single amplicons are labeled in light or dark grey. Genes with multiple amplicons (e.g., multiple regions amplified from the same gene) are grouped together by color. In some instances, the read counts are similar for amplicons within the same gene (e.g., ATR gene). In some instances, the read counts are different for amplicons within the same gene (e.g., TELO2). Differences for read counts between amplicons in the same gene can be due to genetic variation in the gene (e.g., the gene has a variant that is not recognized by the designed primer). Amplicons in a gene should have similar read counts because they originate from the same gene with the same copy number.

### Example 4. SNP identification

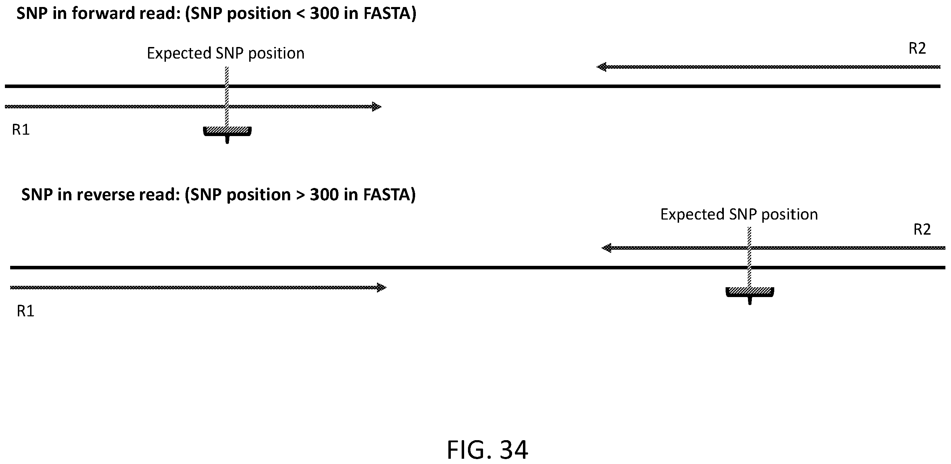

The methods of the disclosure can be used for identifying SNPs (either from multiple amplicons within the same gene, or across genes). Primers are designed to amplify select regions of interest (e.g., SNPs). The amplicons are sequenced. SNPs are identified using a software algorithm as shown in **Figure 34****.** The region around the expected SNP position is checked. If the correct flanking sequences are present, then the SNP is identified by the nucleotide. If the flanking sequences around the expected region of the SNP are incorrect, then the SNP is identified as an 'N' nucleotide. SNPs can be identified in the forward read, the reverse read, and/or both the forward and reverse read. When the SNP is identified in both read, the forward read or higher quality read is used for identification.

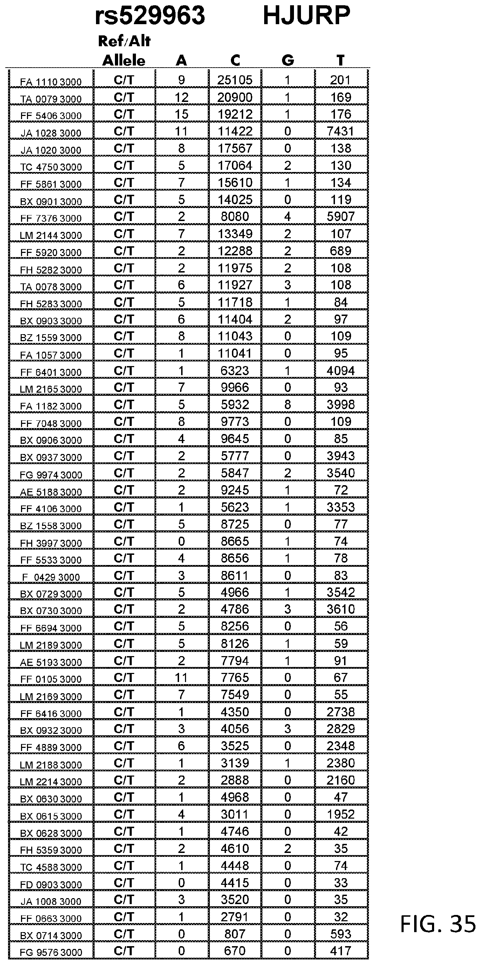

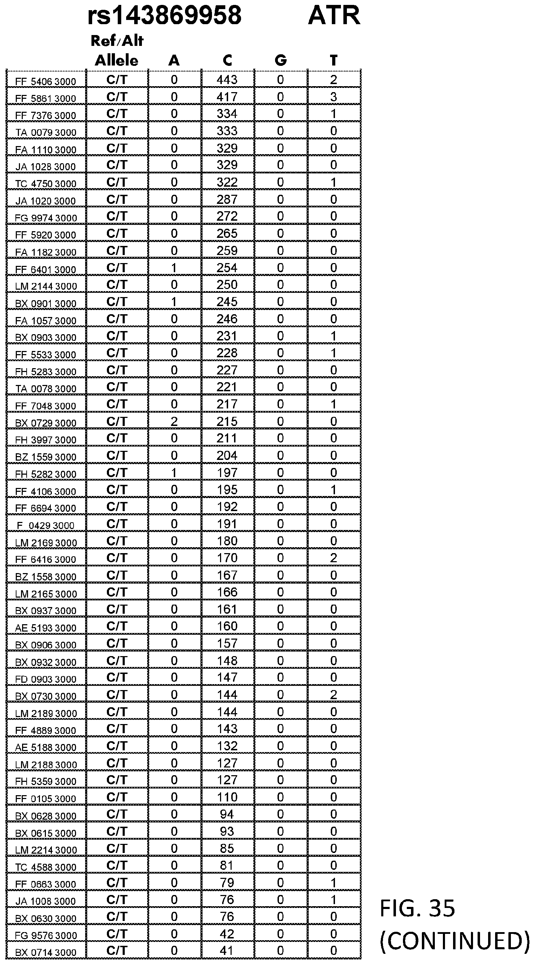

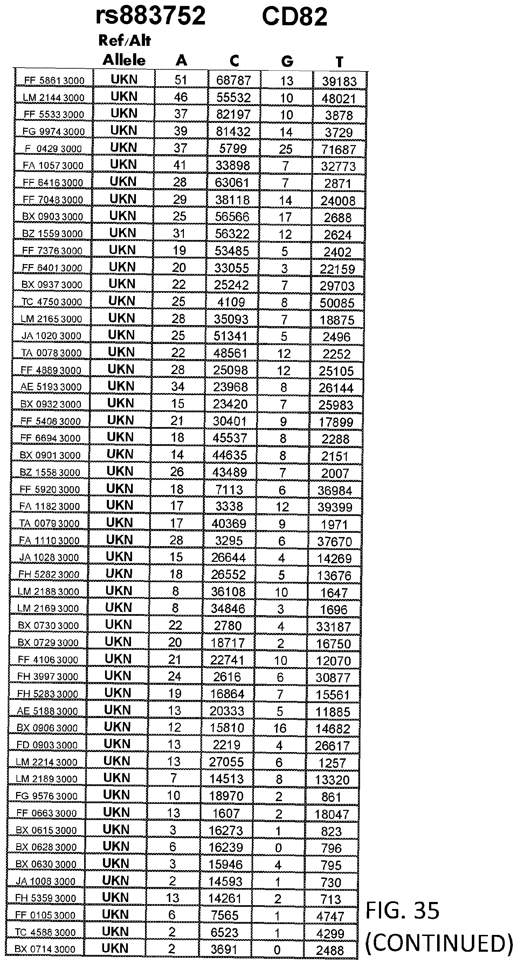

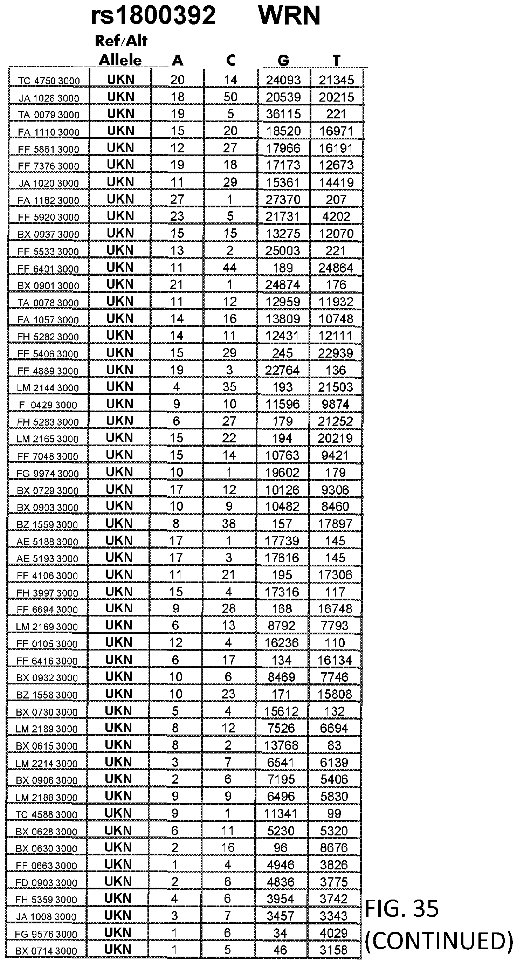

**Figure 35** illustrates allele specific expression for specific SNPs in the genes of HJURP, ATR, CD82, and WRN. The numbers in the table represent the number of reads containing the indicated base at the SNP position. Values highlighted in dark grey have very low counts while values highlighted in light grey have high counts. HJURP and ATR primarily contain the C SNP. WRN, for example, has a mixture of G and T nucleotides.

### Example 5. Detection of IgG mRNAwith targeted gene-specific primers

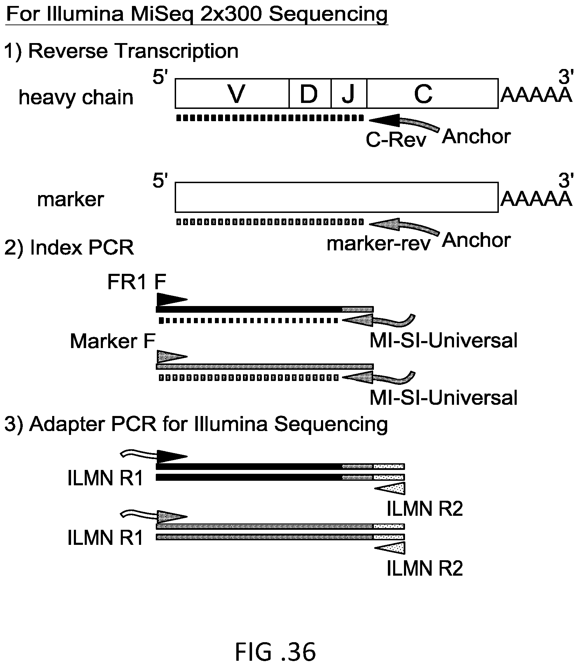

As depicted in **Figure 36****,** a sensitive method to detect IgG mRNAs can utilize a targeted approach. This method can be useful when the mRNA transcript level of IgG mRNAs are less abundant (<10% of mRNA pool per cell); the efficiency of a multiplex PCR reaction can enrich targeted regions for a higher number of sequencing reads. Given that antibody-encoding genes can mutate, primer design for this approach can be targeted towards regions with the least variation. Primers were designed that contain verified sequences in the constant (C) and FR1 region of heavy and light mRNAs (see **Table 3** for all targeted sequences) to minimize this problem.

In the workflow shown in **Figure 36****,** a reverse primer recognizing C-region with an anchor sequence is used for reverse transcription. The cDNA generated from this reaction serves as a template for a low-cycle PCR to attach molecular and sample indices into the 3'end, using FR1-specific forward and anchor-specific reverse primers. Illumina-compatible sequencing adapters are attached in a subsequent PCR reaction for elucidating the V(D)J sequence in IgG mRNAs.

### Example 6. Adaptor ligation for library preparation for detection of IgG mRNA

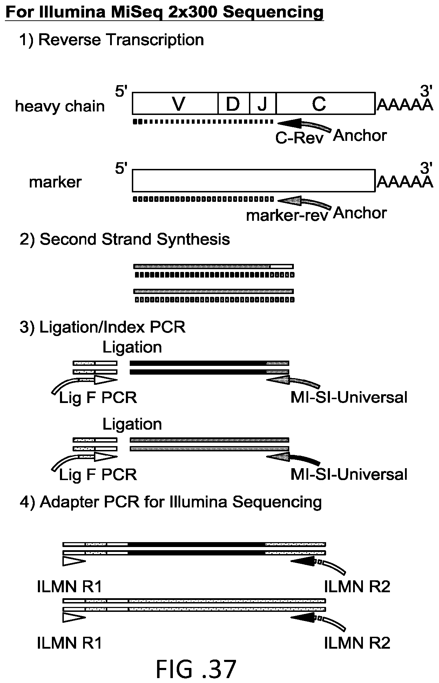

As shown in **Figure 37****,** this library preparation approach is an unbiased method to detect and sequence IgG mRNAs. This approach can be used when mRNAs of interest are abundant (>10% of mRNA pool per cell). The advantage of this approach is the unbiased enrichment of IgG mRNAs regardless of mutations that occur in the 5'ends.

In the workflow shown in **Figure 37****,** a reverse primer recognizing C-region with an anchor sequence is used for reverse transcription. The cDNA generated from this reaction serves as a template for second strand cDNA synthesis. This ds cDNA ligates to double stranded adapter oligo and is PCR amplified using a forward adapter primer and reverse anchor primer for molecular and sample indexing. Illumina-compatible sequencing adapters are attached in a subsequent PCR reaction for elucidating the V(D)J sequence in IgG mRNAs.

**Table 3. List of gene sequences used in examples 5 and 6. Sequences have been verified suitable for high throughput sequencing of IgG mRNAs in the Balb/c strain of mice.**

| **Chain** | **Gene Targeted Site** | **Sequence (5'→3')** |
|---|---|---|
| Heavy | IgG CH1 Reverse | ACCAGGCATCCCAGGGTCACCATGGAGT (SEQ ID NO: 396) |
| | 701/mIGHV5-6-5^{∗}01 | GAAGTGAAGCTGGTGGAGTCTGGGGGA (SEQ ID NO: 397) |
| | 702/mIGHV1-72^{∗}01 | CAGGTCCAACTGCAGCAGCCTGGGGCT (SEQ ID NO: 398) |
| | 703/mIGHV1-9^{∗}01 | CAGGTTCAGCTGCAGCAGTCTGGAGCT (SEQ ID NO: 399) |
| | 704/mIGHV1-14^{∗}01 | GAGTTCCAGCTGCAGCAGTCTGGACCT (SEQ ID NO: 400) |
| | 705/mIGHV14-3^{∗}02 | GAGGTTCAGCTGCAGCAGTCTGGGGCA (SEQ ID NO: 401) |
| | 706/mIGHV9-3-1^{∗}01 | CAGATCCAGTTGGTGCAGTCTGGACCT (SEQ ID NO: 402) |
| | 707/mIGHV2-9-1^{∗}01 | CAGGTGCAGCTGAAGGAGTCAGGACCT (SEQ ID NO: 403) |
| | 708/mIGHV3-6^{∗}02 | GATGTACAGCTTCAGGAGTCAGGACCT (SEQ |
| | | ID NO: 404) |
| | 772/IGHV8-12^{∗}01 | CAGGTTACTCTGAAAGAGTCTGGCCCT (SEQ ID NO: 405) |
| Light | IgG CK Reverse | GAGGCACCTCCAGATGTTAACTGCTCA (SEQ ID NO: 406) |
| | 728/mIGKV4-59^{∗}01 | CAAATTGTTCTCACCCAGTCTCCA (SEQ ID NO: 407) |
| | 729/mIGKV4-72^{∗}01 | CAAATTGTTCTCTCCCAGTCTCCA (SEQ ID NO: 408) |
| | 730/mIGKV3-2^{∗}01 | GACATTGTGCTGACCCAATCTCCA (SEQ ID NO: 409) |
| | 731/mIGKV6-23^{∗}01 | GACATTGTGATGACCCAGTCTCA (SEQ ID NO: 410) |
| | 732/mIGKV8-28^{∗}01 | GACATTGTGATGACCCAGTCTCCA (SEQ ID NO: 411) |
| | 733/mIGKV12-41^{∗}01 | GACATCCAGATGACTCAGTCTCCA (SEQ ID NO: 412) |
| | 734/mIGKV10-94^{∗}01 | GATATCCAGATGACACAGACTACA (SEQ ID NO: 413) |
| | 735/mIGKV14-111^{∗}01 | GACATCAAGATGACCCAGTCTCCA (SEQ ID NO: 414) |
| | 736/mIGKV1-110^{∗}01 | GATGTTGTGATGACCCAAACTCCA (SEQ ID NO: 415) |
| | 766/IGKV2-137^{∗}01 | GATATTGTGATGACTCAGGCTGCA (SEQ ID NO: 416) |
| | 767/IGKV5-43^{∗}01 | GaTaTTGTGCTaacTCaGTCTCCa (SEQ ID NO: 417) |
| | 768/1GKV11-125^{∗}01 | GATGTCCAGATGATTCAGTCTCCA (SEQ ID NO: 418) |
| | 769/IGKV13-84^{∗}01 | GACATCCAGATGACACAATCTTCA (SEQ ID NO: 419) |
| | 770/IGKV15-101^{∗}01 | AATACCCAGATGAACCAGACTCCA (SEQ ID NO: 420) |
| | 771/IGKV18-36^{∗}0 1 | ACTGGCGAAACAACACAGGCTCCA (SEQ ID NO: 421) |

### Example 7. Detection of TCR mRNAs and marker mRNAs using stochastic barcodes.

The experimental protocol shown in **Figure 38** was used to characterize the TCR alpha chain and beta chain as well as phenotype marker mRNAs expressed in T cells deposited in 96 well plate using 2 cell equivalent amount of T cell RNA (20 ng) per well. The phenotype markers used are listed in **Figure 39****.** The gene-specific primers that bind to the C-region of TCR alpha chain and beta chain are: TRAC: 5' CAGACAGACTTGT 3' (SEQ ID NO: 1); TRBC: 5' CCTTTTGGGTGTG 3' (SEQ ID NO: 2). Sequencing reads and molecular indexes that were de-multiplexed for each sample well of phenotype markers are shown in **Tables 4** and **5,** respectively. Sequencing reads and molecular indexes that were de-multiplexed for each sample well of TCR alpha chain are shown in **Tables 6** and 7. Sequencing reads and molecular indexes that were de-multiplexed for each sample well of TCR beta chain are shown in **Tables 8** and **9.**

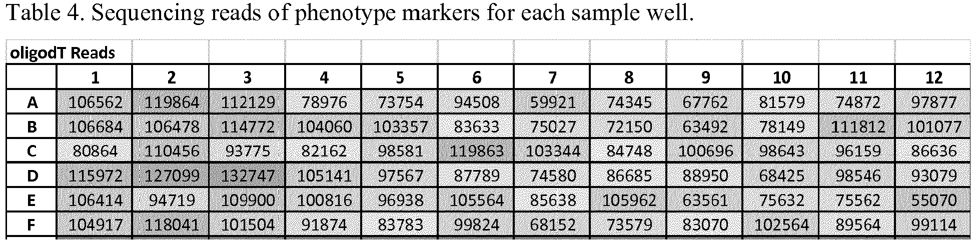

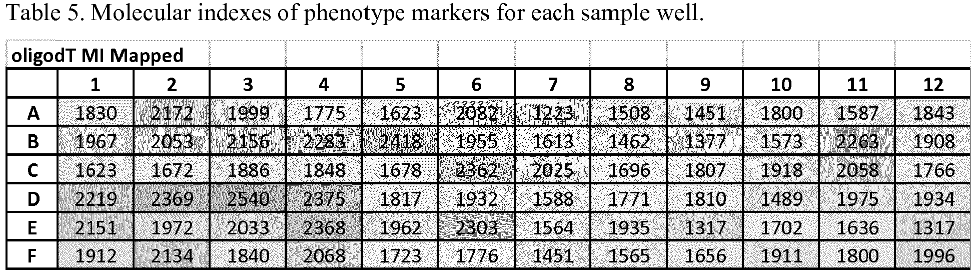

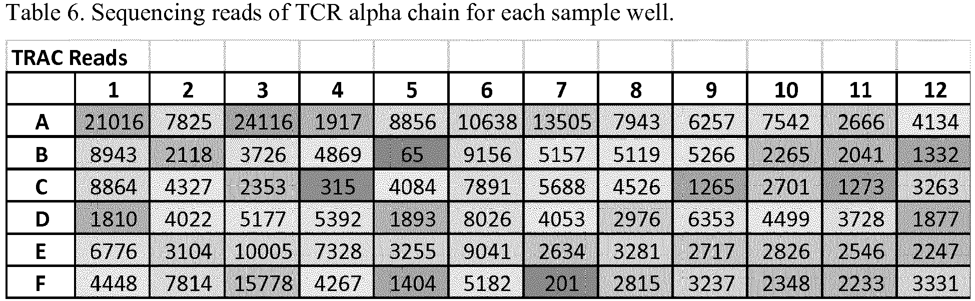

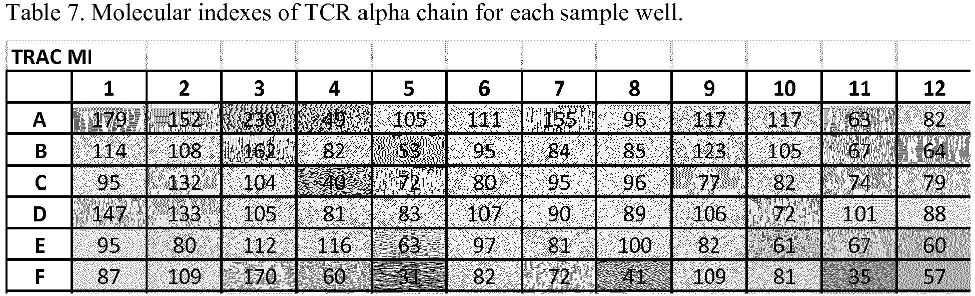

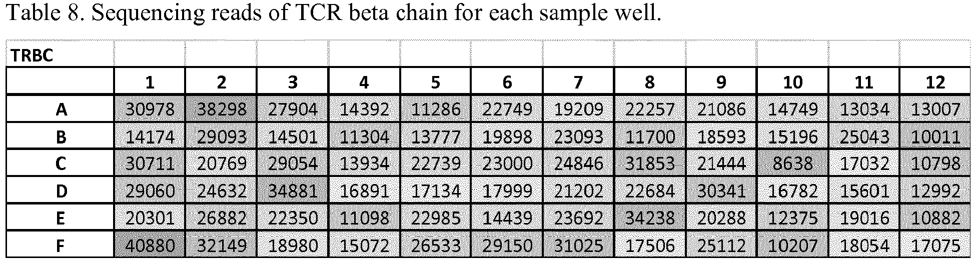

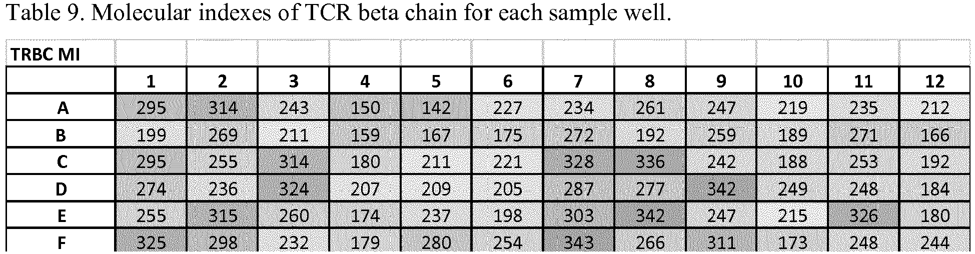

### Example 8. Detection of TCR repertoire in a sample using stochastic barcodes.

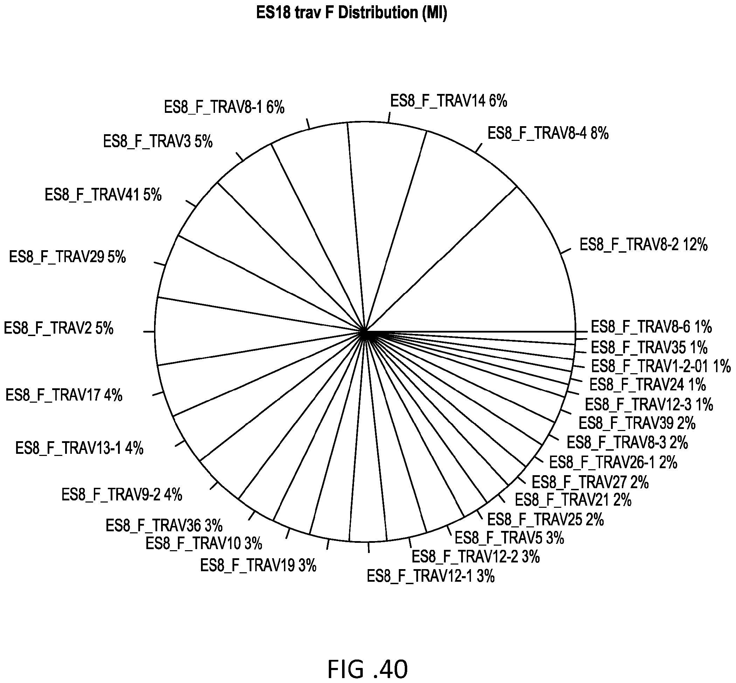

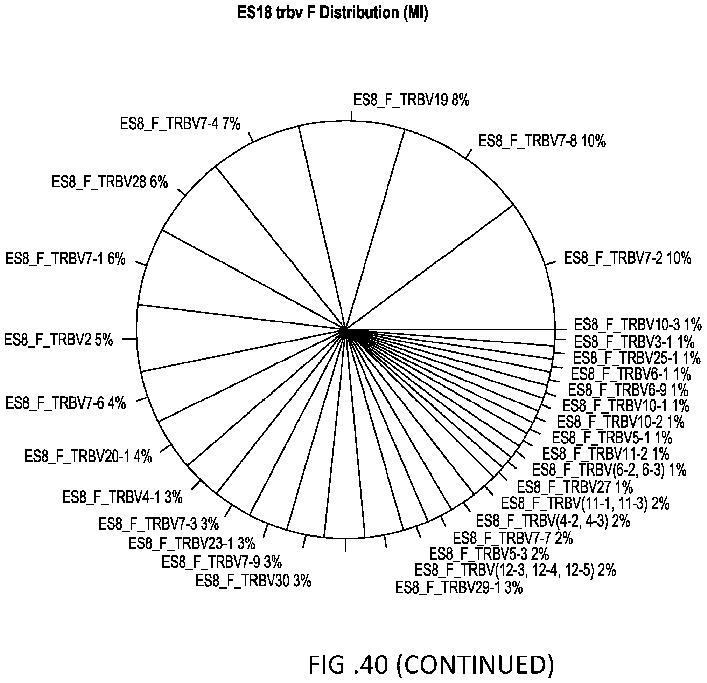

The experimental protocol shown in **Figure 38** was used to characterize the TCR repertoire in a sample of T cell RNA from a donor. The molecular indices for TCR alpha chain and beta chain mRNAs expressed are shown in **Figure 40****.** A wide range of TCRs expressed in the donor at the moment of sample collection was identified.

### Example 9. Phenotype analysis of single T cells.

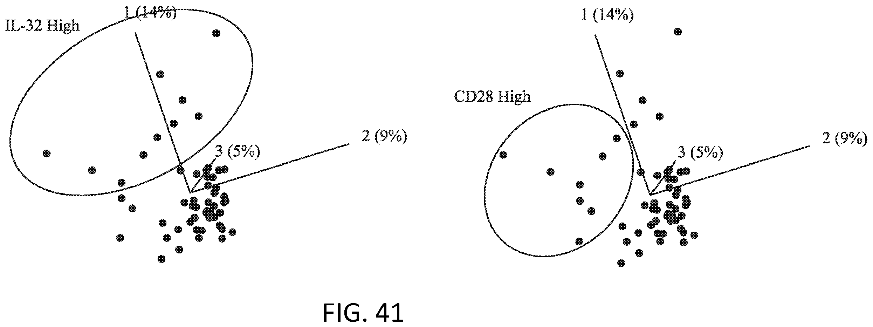

The experimental protocol shown in **Figure 38** was used to characterize the phenotype of single activated T cells in a sample. Principal Component Analysis (PCA) plot shown in **Figure 41** was used to visually distinguish different T cell subtypes. Each dot is a cell and cells that are high in IL-32 or CD28, which is an activated T cell marker, are highlighted in a circle. Activated T cells are distinguished from non-circled inactivated T cells.

The same sample was used for analyzing the CDR3 sequence of a single CD3⁺ T cell. The results are shown in **Table 10.**

**Table 10. Molecular indices of a single CD3⁺ T cell.**

| | **Molecular Indices** | **Reads** | **CDR3** | **V** | **D** | **J** | **C** |
|---|---|---|---|---|---|---|---|
| *TCRa* | 25 | 21383 | CAGGVVLRNSGNTPLVF (SEQ ID NO: 422) | TRAV27 | - | TRAJ29 | TRAC |
| *TCRb* | 59 | 37643 | CASSEGFQDTQYF (SEQ ID NO: 423) | TRBV6-1 | TRBD1 | TRBJ2-3 | TRBC2 |

### Example 10. Adding stochastic barcode using one step RT-PCR.

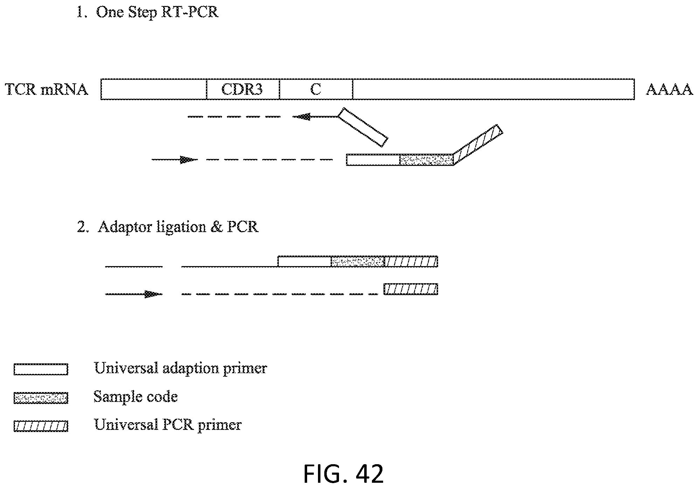

**Figure 42** shows an experimental protocol used to characterize the phenotype of single activated T cells in a sample. A one step RT-PCR was used to add stochastic barcodes to TCR mRNA using a first oligonucleotide comprising a target-specific sequence and a universal adaptor primer for reverse transcription, and a target-specific forward primer and a reverse primer comprising a binding partner to the universal adaptor primer and a stochastic barcode for the PCR. This protocol offered the advantages of higher sensitivity and simple workflow (data not shown).

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity.

It will be understood by those within the art that, in general, terms used herein, and especially in the appended claims (e.g., bodies of the appended claims) are generally intended as "open" terms *(e.g.,* the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" *(e.g.,* "a" and/or "an" should be interpreted to mean "at least one" or "one or more"); the same holds true for the use of definite articles used to introduce claim recitations. In addition, even if a specific number of an introduced claim recitation is explicitly recited, those skilled in the art will recognize that such recitation should be interpreted to mean at least the recited number (e.g., the bare recitation of "two recitations," without other modifiers, means at least two recitations, or two or more recitations).

## Claims

1. A method of labeling a nucleic acid in a sample, comprising:
hybridizing a target with a first oligonucleotide comprising a universal adaptor sequence and a target-specific sequence;
extending said first oligonucleotide to generate a first nucleotide sequence;
hybridizing said first nucleotide sequence with a second oligonucleotide;
extending said second oligonucleotide to generate a second nucleotide sequence;
hybridizing said second nucleotide sequence with a third oligonucleotide comprising a binding site for a first amplification primer and the universal adaptor sequence, wherein the third oligonucleotide comprises a stochastic barcode, wherein the stochastic barcode comprises a molecular label;
extending said third oligonucleotide to generate a third nucleotide sequence; and
amplifying said third nucleotide sequence using a first amplification primer to generate a plurality of a fourth nucleotide sequences,
wherein each of the plurality of the fourth nucleotide sequences comprises said target and the stochastic barcode,
wherein the target is an mRNA molecule; and
wherein the molecular label comprises a nucleic acid sequence that provides a counter for the specific occurrence of the target nucleic acid.

2. A method of labeling a target in a sample for quantification of said target, comprising:
hybridizing said target with a first oligonucleotide comprising a first universal adaptor sequence and a target-specific sequence;
extending said first oligonucleotide to generate a first nucleotide sequence;
hybridizing said first nucleotide sequence with a second oligonucleotide comprising a second universal adaptor sequence and a second target-specific sequence;
extending said second oligonucleotide to generate a second nucleotide sequence;
hybridizing said second nucleotide sequence with a third oligonucleotide comprising a binding site for a first amplification primer and the first universal adaptor sequence;
extending said third oligonucleotide to generate a third nucleotide sequence;
hybridizing said third nucleotide sequence with a fourth oligonucleotide comprising a binding site for a second amplification primer and the second universal adaptor sequence, wherein one or both of the third oligonucleotide and the fourth oligonucleotide comprises part of a stochastic barcode, wherein the stochastic barcode comprises a molecular label;
extending said fourth oligonucleotide to generate a fourth nucleotide sequence; and
amplifying said fourth nucleotide sequence using a first amplification primer and a second amplification primer to generate a plurality of a fifth nucleotide sequences,
wherein each of the plurality of the fifth nucleotide sequences comprises said target and the stochastic barcode,
wherein the target is an mRNA molecule; and
wherein the molecular label comprises a nucleic acid sequence that provides a counter for the specific occurrence of the target nucleic acid.

3. A method of labeling a target in a sample for quantification of said target, comprising:
hybridizing said target with a first oligonucleotide comprising a target-specific sequence and a binding site for a first amplification primer;
extending said first oligonucleotide to generate a first nucleotide sequence;
hybridizing said first nucleotide sequence with a second oligonucleotide comprising a universal adaptor sequence and a second target-specific sequence;
extending said second oligonucleotide to generate a second nucleotide sequence;
hybridizing said second nucleotide sequence with a third oligonucleotide comprising a binding site for a second amplification primer and a sequence that specifically binds the universal adaptor sequence, wherein the third oligonucleotide comprises a stochastic barcode, wherein the stochastic barcode comprises a molecular label;
extending said third oligonucleotide to generate a third nucleotide sequence; and
amplifying said third nucleotide sequence using a first amplification primer and a second amplification primer to generate a plurality of a fourth nucleotide sequences,
wherein each of the plurality of the fourth nucleotide sequences comprises said target and the stochastic barcode,
wherein the target is an mRNA molecule,
and wherein the molecular label comprises a nucleic acid sequence that provides a counter for the specific occurrence of the target nucleic acid.

4. The method of claim 1, 2 or 3, wherein the sample is a single cell and/or wherein the mRNA molecule encodes a T cell receptor (TCR) alpha chain or beta chain, optionally wherein the target-specific sequence specifically binds to the C region of the TCR alpha chain or beta chain.

5. The method of claim 1 or 4 when dependent on claim 1, comprising amplifying said third nucleotide sequence using a second amplification primer, optionally wherein the second amplification primer specifically binds to a region selected from the group consisting of: a V region of a TCR alpha chain or beta chain, the junction of a V and D region of a TCR alpha chain or beta chain, or any combination thereof.

6. The method of any one of claims 1-5, wherein the second oligonucleotide of claim 1 or the second target specific sequence of claim 2 or 3 specifically binds to a region selected from the group consisting of: a V region of a TCR alpha chain or beta chain, the junction of a V and D region of a TCR alpha chain or beta chain, or any combination thereof.

7. The method of any one of claims 1 and 4-6 when dependent on claim 1, wherein the second oligonucleotide comprises a binding site for a second amplification primer, optionally further comprising amplifying said third nucleotide sequence using the second amplification primer.

8. The method of any one of claims 1-7, wherein the stochastic barcode further comprises a universal label, a dimension label, a cellular label, or a combination thereof.

9. The method of any one of claims 1 and 4-8, wherein;
(a) the first oligonucleotide or the third oligonucleotide is attached to a solid support; and/or
(b) the sample comprises a plurality of the target, optionally wherein each of the plurality of the target is labeled with a distinct stochastic barcode, further optionally comprising counting the number of the plurality of the target by counting the distinct stochastic barcodes associated with the target.

10. A method of labeling a target in a sample for quantification of said target, comprising:
hybridizing said target with a first oligonucleotide comprising a target-specific sequence and a first amplification primer sequence in the presence of a second oligonucleotide comprising a universal adaptor sequence;
extending said first oligonucleotide to generate a first nucleotide sequence comprising a compliment of the universal adaptor sequence;
hybridizing said first nucleotide sequence with a third oligonucleotide comprising the universal adaptor sequence, a stochastic barcode, wherein the stochastic barcode comprises a molecular label, and a binding site for a second amplification primer;
extending said third oligonucleotide to generate a second nucleotide sequence; and
amplifying said second nucleotide sequence using a first amplification primer and a second amplification primer to generate a plurality of a third nucleotide sequences,
wherein each of the plurality of the third nucleotide sequences comprises said target and the stochastic barcode,
wherein the target is an mRNA molecule; and
wherein the molecular label comprises a nucleic acid sequence that provides a counter for the specific occurrence of the target nucleic acid.

11. The method of claim 10, wherein:
(a) the method comprises extending the first oligonucleotide to generate a first nucleotide sequence comprising a compliment of the universal adaptor sequence by template switching; and/or
(b) the stochastic barcode further comprises a universal label, a dimension label, a cellular label, or a combination thereof.

12. A method of labeling a target in a sample for quantification of said target, comprising:
hybridizing said target with a first oligonucleotide comprising a universal adaptor sequence and a target-specific sequence;
extending said first oligonucleotide to generate a first nucleotide sequence;
hybridizing said first nucleotide sequence with a second oligonucleotide comprising a second target-specific sequence and a third oligonucleotide comprising a stochastic barcode, wherein the stochastic barcode comprises a molecular label, and a sequence that specifically binds the universal adaptor sequence;
amplifying said first nucleotide sequence to generate a plurality of a second nucleotide sequences,
wherein each of the plurality of the second nucleotide sequences comprises said target and said stochastic barcode,
wherein the target is an mRNA molecule; and
wherein the molecular label comprises a nucleic acid sequence that provides a counter for the specific occurrence of the target nucleic acid.

## Patentansprüche

1. Verfahren zum Markieren einer Nukleinsäure in einer Probe, umfassend:
Hybridisieren eines Ziels mit einem ersten Oligonukleotid, das eine universelle Adaptersequenz und eine zielspezifische Sequenz umfasst;
Verlängern des ersten Oligonukleotids, um eine erste Nukleotidsequenz zu erzeugen;
Hybridisieren der ersten Nukleotidsequenz mit einem zweiten Oligonukleotid;
Verlängern des zweiten Oligonukleotids, um eine zweite Nukleotidsequenz zu erzeugen;
Hybridisieren der zweiten Nukleotidsequenz mit einem dritten Oligonukleotid, das eine Bindungsstelle für einen ersten Amplifikationsprimer und die universelle Adaptersequenz umfasst, wobei das dritte Oligonukleotid einen stochastischen Strichcode umfasst, wobei der stochastische Strichcode einen molekularen Marker umfasst;
Verlängern des dritten Oligonukleotids, um eine dritte Nukleotidsequenz zu erzeugen; und
Amplifizieren der dritten Nukleotidsequenz unter Verwendung eines ersten Amplifikationsprimers, um mehrere vierte Nukleotidsequenzen zu erzeugen,
wobei jede der mehreren vierten Nukleotidsequenzen das Ziel und den stochastischen Strichcode umfasst,
wobei das Ziel ein mRNA-Molekül ist; und
wobei der molekulare Marker eine Nukleinsäuresequenz umfasst, die einen Zähler für das spezifische Vorkommen der Zielnukleinsäure bereitstellt.

2. Verfahren zum Markieren eines Ziels in einer Probe zum Quantifizieren des Ziels, umfassend:
Hybridisieren des Ziels mit einem ersten Oligonukleotid, das eine erste universelle Adaptersequenz und eine zielspezifische Sequenz umfasst;
Verlängern des ersten Oligonukleotids, um eine erste Nukleotidsequenz zu erzeugen;
Hybridisieren der ersten Nukleotidsequenz mit einem zweiten Oligonukleotid, das eine zweite universelle Adaptersequenz und eine zweite zielspezifische Sequenz umfasst;
Verlängern des zweiten Oligonukleotids, um eine zweite Nukleotidsequenz zu erzeugen;
Hybridisieren der zweiten Nukleotidsequenz mit einem dritten Oligonukleotid, das eine Bindungsstelle für einen ersten Amplifikationsprimer und die erste universelle Adaptersequenz umfasst;
Verlängern des dritten Oligonukleotids, um eine dritte Nukleotidsequenz zu erzeugen;
Hybridisieren der dritten Nukleotidsequenz mit einem vierten Oligonukleotid, das eine Bindungsstelle für einen zweiten Amplifikationsprimer und die zweite universelle Adaptersequenz umfasst, wobei das dritte Oligonukleotid und/oder das vierte Oligonukleotid einen Teil eines stochastischen Strichcodes umfasst, wobei der stochastische Strichcode einen molekularen Marker umfasst;
Verlängern des vierten Oligonukleotids, um eine vierte Nukleotidsequenz zu erzeugen; und
Amplifizieren der vierten Nukleotidsequenz unter Verwendung eines ersten Amplifikationsprimers und eines zweiten Amplifikationsprimers, um mehrere fünfte Nukleotidsequenzen zu erzeugen,
wobei jede der mehreren fünften Nukleotidsequenzen das Ziel und den stochastischen Strichcode umfasst,
wobei das Ziel ein mRNA-Molekül ist; und
wobei der molekulare Marker eine Nukleinsäuresequenz umfasst, die einen Zähler für das spezifische Vorkommen der Zielnukleinsäure bereitstellt.

3. Verfahren zum Markieren eines Ziels in einer Probe zum Quantifizieren des Ziels, umfassend:
Hybridisieren des Ziels mit einem ersten Oligonukleotid, das eine zielspezifische Sequenz und eine Bindungsstelle für einen ersten Amplifikationsprimer umfasst;
Verlängern des ersten Oligonukleotids, um eine erste Nukleotidsequenz zu erzeugen;
Hybridisieren der ersten Nukleotidsequenz mit einem Oligonukleotid, das eine universelle Adaptersequenz und eine zweite zielspezifische Sequenz umfasst;
Verlängern des zweiten Oligonukleotids, um eine zweite Nukleotidsequenz zu erzeugen;
Hybridisieren der zweiten Nukleotidsequenz mit einem dritten Oligonukleotid, das eine Bindungsstelle für einen zweiten Amplifikationsprimer und eine Sequenz, die spezifisch die universelle Adaptersequenz bindet, umfasst, wobei das dritte Oligonukleotid einen stochastischen Strichcode umfasst, wobei der stochastische Strichcode einen molekularen Marker umfasst;
Verlängern des dritten Oligonukleotids, um eine dritte Nukleotidsequenz zu erzeugen; und
Amplifizieren der dritten Nukleotidsequenz unter Verwendung eines ersten Amplifikationsprimers und eines zweiten Amplifikationsprimers, um mehrere vierte Nukleotidsequenzen zu erzeugen,
wobei jede der mehreren vierten Nukleotidsequenzen das Ziel und den stochastischen Strichcode umfasst,
wobei das Ziel ein mRNA-Molekül ist
und wobei der molekulare Marker eine Nukleinsäuresequenz umfasst, die einen Zähler für das spezifische Vorkommen der Zielnukleinsäure bereitstellt.

4. Verfahren nach Anspruch 1, 2 oder 3, wobei die Probe eine einzelne Zelle ist und/oder wobei das mRNA-Molekül für eine T-Zell-Rezeptor(TCR)-Alpha-Kette oder -Beta-Kette kodiert, optional wobei die zielspezifische Sequenz spezifisch an die C-Region der TCR-Alpha-Kette oder der -Beta-Kette bindet.

5. Verfahren nach Anspruch 1 oder 4, falls von Anspruch 1 abhängig, welches das Amplifizieren der dritten Nukleotidsequenz unter Verwendung eines zweiten Amplifikationsprimers umfasst, optional wobei der zweite Amplifikationsprimer spezifisch an eine Region bindet, ausgewählt aus der Gruppe bestehend aus: einer V-Region einer TCR-Alpha-Kette oder -Beta-Kette, der Verbindung einer V- und einer D-Region einer TCR-Alpha-Kette oder - Beta-Kette oder einer beliebige Kombination daraus.

6. Verfahren nach einem der Ansprüche 1-5, wobei das zweite Oligonukleotid nach Anspruch 1 oder die zweite zielspezifische Sequenz nach Anspruch 2 oder 3 spezifisch an eine Region bindet, ausgewählt aus der Gruppe bestehend aus: einer V-Region einer TCR-Alpha-Kette oder -Beta-Kette, der Verbindung einer V- und einer D-Region einer TCR-Alpha-Kette oder -Beta-Kette oder einer beliebigen Kombination daraus.

7. Verfahren nach einem der Ansprüche 1 und 4-6, falls von Anspruch 1 abhängig, wobei das zweite Oligonukleotid eine Bindungsstelle für einen zweiten Amplifikationsprimer umfasst, optional ferner umfassend das Amplifizieren der dritten Nukleotidsequenz unter Verwendung des zweiten Amplifikationsprimers.

8. Verfahren nach einem der Ansprüche 1-7, wobei der stochastische Strichcode ferner einen universellen Marker, einen Dimensionsmarker, einen zellularen Marker oder eine Kombination daraus umfasst.

9. Verfahren nach einem der Ansprüche 1 und 4-8, wobei:
(a) das erste Oligonukleotid oder das dritte Oligonukleotid an einen festen Träger gebunden ist;
und/oder
(b) die Probe mehrere Ziele umfasst, optional wobei jedes der mehreren Ziele mit einem unterschiedlichen stochastischen Strichcode gekennzeichnet ist, ferner optional umfassend das Zählen der Anzahl der mehreren Ziele durch Zählen der unterschiedlichen stochastischen Strichcodes, die mit den Ziel verknüpft sind.

10. Verfahren zum Markieren eines Ziels in einer Probe zum Quantifizieren des Ziels, umfassend:
Hybridisieren des Ziels mit einem ersten Oligonukleotid, das eine zielspezifische Sequenz umfasst, und einer ersten Amplifikationsprimersequenz in Gegenwart eines zweiten Oligonukleotids, das eine universelle Adaptersequenz umfasst;
Verlängern des ersten Oligonukleotids, um eine erste Nukleotidsequenz, die ein Komplement der universellen Adaptersequenz umfasst, zu erzeugen;
Hybridisieren der ersten Nukleotidsequenz mit einem dritten Oligonukleotid, das die universelle Adaptersequenz, einen stochastischen Strichcode, wobei der stochastische Strichcode einen molekularen Marker umfasst, und eine Bindungsstelle für einen zweiten Amplifikationsprimer umfasst;
Verlängern des dritten Oligonukleotids, um eine zweite Nukleotidsequenz zu erzeugen; und
Amplifizieren der zweiten Nukleotidsequenz unter Verwendung eines ersten Amplifikationsprimers und eines zweiten Amplifikationsprimers, um mehrere dritte Nukleotidsequenzen zu erzeugen,
wobei jede der mehreren dritten Nukleotidsequenzen das Ziel und den stochastischen Strichcode umfasst,
wobei das Ziel ein mRNA-Molekül ist; und
wobei der molekulare Marker eine Nukleinsäuresequenz umfasst, die einen Zähler für das spezifische Vorkommen der Zielnukleinsäure bereitstellt.

11. Verfahren nach Anspruch 10, wobei:
(a) das Verfahren das Verlängern des ersten Oligonukleotids umfasst, um eine erste Nukleotidsequenz, die ein Komplement der universellen Adaptersequenz umfasst, durch Matrizenwechsel zu erzeugen; und/oder
(b) der stochastische Strichcode ferner einen universellen Marker, einen Dimensionsmarker, einen zellularen Marker oder eine Kombination daraus umfasst.

12. Verfahren zum Markieren eines Ziels in einer Probe zum Quantifizieren des Ziels, umfassend:
Hybridisieren des Ziels mit einem ersten Oligonukleotid, das eine universelle Adaptersequenz und eine zielspezifische Sequenz umfasst;
Verlängern des ersten Oligonukleotids, um eine erste Nukleotidsequenz zu erzeugen;
Hybridisieren der ersten Nukleotidsequenz mit einem zweiten Oligonukleotid, das eine zweite zielspezifische Sequenz umfasst, und mit einem dritten Oligonukleotid, das einen stochastischen Strichcode umfasst, wobei der stochastische Strichcode einen molekularen Marker und eine Sequenz, die spezifisch die universelle Adaptersequenz bindet, umfasst;
Amplifizieren der ersten Nukleotidsequenz, um mehrere zweite Nukleotidsequenzen zu erzeugen,
wobei jede der mehreren zweiten Nukleotidsequenzen das Ziel und den stochastischen Strichcode umfasst,
wobei das Ziel ein mRNA-Molekül ist; und
wobei der molekulare Marker eine Nukleinsäuresequenz umfasst, die einen Zähler für das spezifische Vorkommen der Zielnukleinsäure bereitstellt.

## Revendications

1. Procédé de marquage d'un acide nucléique dans un échantillon, comprenant les étapes consistant à :
hybrider une cible avec un premier oligonucléotide comprenant une séquence adaptatrice universelle et une séquence spécifique d'une cible ;
allonger ledit premier oligonucléotide pour générer une première séquence nucléotidique ;
hybrider ladite première séquence nucléotidique avec un deuxième oligonucléotide ;
allonger ledit deuxième oligonucléotide pour générer une deuxième séquence nucléotidique ;
hybrider ladite deuxième séquence nucléotidique avec un troisième oligonucléotide comprenant un site de liaison pour une première amorce d'amplification et la séquence adaptatrice universelle, dans lequel le troisième oligonucléotide comprend un code-barres stochastique, dans lequel le code-barres stochastique comprend un marqueur moléculaire ;
allonger ledit troisième oligonucléotide pour générer une troisième séquence nucléotidique ; et
amplifier ladite troisième séquence nucléotidique en utilisant une première amorce d'amplification pour générer une pluralité de quatrièmes séquences nucléotidiques,
dans lequel chacune des quatrièmes séquences nucléotidiques de la pluralité comprend ladite cible et le code-barres stochastique,
dans lequel la cible est une molécule d'ARNm ; et
dans lequel le marqueur moléculaire comprend une séquence d'acide nucléique qui fournit un compteur pour l'occurrence spécifique de l'acide nucléique cible.

2. Procédé de marquage d'une cible dans un échantillon pour la quantification de ladite cible, comprenant les étapes consistant à :
hybrider ladite cible avec un premier oligonucléotide comprenant une première séquence adaptatrice universelle et une séquence spécifique d'une cible ;
allonger ledit premier oligonucléotide pour générer une première séquence nucléotidique ;
hybrider ladite première séquence nucléotidique avec un deuxième oligonucléotide comprenant une deuxième séquence adaptatrice universelle et une deuxième séquence spécifique d'une cible ;
allonger ledit deuxième oligonucléotide pour générer une deuxième séquence nucléotidique ;
hybrider ladite deuxième séquence nucléotidique avec un troisième oligonucléotide comprenant un site de liaison pour une première amorce d'amplification et la première séquence adaptatrice universelle ;
allonger ledit troisième oligonucléotide pour générer une troisième séquence nucléotidique ;
hybrider ladite troisième séquence nucléotidique avec un quatrième oligonucléotide comprenant un site de liaison pour une deuxième amorce d'amplification et la deuxième séquence adaptatrice universelle, dans lequel un élément parmi le troisième oligonucléotide et le quatrième oligonucléotide, ou bien les deux, comprend/comprennent une partie d'un code-barres stochastique, dans lequel le code-barres stochastique comprend un marqueur moléculaire ;
allonger ledit quatrième oligonucléotide pour générer une quatrième séquence nucléotidique ; et
amplifier ladite quatrième séquence nucléotidique en utilisant une première amorce d'amplification et une deuxième amorce d'amplification pour générer une pluralité de cinquièmes séquences nucléotidiques,
dans lequel chacune des cinquièmes séquences nucléotidiques de la pluralité comprend ladite cible et le code-barres stochastique,
dans lequel la cible est une molécule d'ARNm ; et
dans lequel le marqueur moléculaire comprend une séquence d'acide nucléique qui fournit un compteur pour l'occurrence spécifique de l'acide nucléique cible.

3. Procédé de marquage d'une cible dans un échantillon pour la quantification de ladite cible, comprenant les étapes consistant à :
hybrider ladite cible avec un premier oligonucléotide comprenant une séquence spécifique d'une cible et un site de liaison pour une première amorce d'amplification ;
allonger ledit premier oligonucléotide pour générer une première séquence nucléotidique ;
hybrider ladite première séquence nucléotidique avec un deuxième oligonucléotide comprenant une séquence adaptatrice universelle et une deuxième séquence spécifique d'une cible ;
allonger ledit deuxième oligonucléotide pour générer une deuxième séquence nucléotidique ;
hybrider ladite deuxième séquence nucléotidique avec un troisième oligonucléotide comprenant un site de liaison pour une deuxième amorce d'amplification et une séquence qui se lie spécifiquement à la séquence adaptatrice universelle, dans lequel le troisième oligonucléotide comprend un code-barres stochastique, dans lequel le code-barres stochastique comprend un marqueur moléculaire ;
allonger ledit troisième oligonucléotide pour générer une troisième séquence nucléotidique ; et
amplifier ladite troisième séquence nucléotidique en utilisant une première amorce d'amplification et une deuxième amorce d'amplification pour générer une pluralité de quatrièmes séquences nucléotidiques,
dans lequel chacune des quatrièmes séquences nucléotidiques de la pluralité comprend ladite cible et le code-barres stochastique,
dans lequel la cible est une molécule d'ARNm,
et dans lequel le marqueur moléculaire comprend une séquence d'acide nucléique qui fournit un compteur pour l'occurrence spécifique de l'acide nucléique cible.

4. Procédé des revendications 1, 2 ou 3, dans lequel l'échantillon est une seule cellule et/ou dans lequel la molécule d'ARNm code pour une chaîne alpha ou une chaîne bêta d'un récepteur aux lymphocytes T (TCR), en option dans lequel la séquence spécifique d'une cible se lie spécifiquement à la région C de la chaîne alpha ou la chaîne bêta d'un TCR.

5. Procédé de la revendication 1 ou la 4 quand elle est dépendante de la revendication 1, comprenant une amplification de ladite troisième séquence nucléotidique en utilisant une deuxième amorce d'amplification, en option dans lequel la deuxième amorce d'amplification se lie spécifiquement à une région choisie dans le groupe constitué par : une région V d'une chaîne alpha ou d'une chaîne bêta d'un TCR, la jonction d'une région V et d'une D d'une chaîne alpha ou d'une chaîne bêta d'un TCR ou bien une quelconque combinaison de celles-ci.

6. Procédé de l'une quelconque des revendications 1-5, dans lequel le deuxième oligonucléotide de la revendication 1 ou la deuxième séquence spécifique d'une cible des revendications 2 ou 3 se lie spécifiquement à une région choisie dans le groupe constitué par : une région V d'une chaîne alpha ou d'une chaîne bêta d'un TCR, la jonction d'une région V et d'une D d'une chaîne alpha ou d'une chaîne bêta d'un TCR ou bien une quelconque combinaison de celles-ci.

7. Procédé de l'une quelconque des revendications 1 et 4-6 quand elle est dépendante de la revendication 1, dans lequel le deuxième oligonucléotide comprend un site de liaison pour une deuxième amorce d'amplification, en option comprenant en outre une amplification de ladite troisième séquence nucléotidique en utilisant la deuxième amorce d'amplification.

8. Procédé de l'une quelconque des revendications 1-7, dans lequel le code-barres stochastique comprend en outre un marqueur universel, un marqueur dimensionnel, un marqueur cellulaire ou une combinaison de ceux-ci.

9. Procédé de l'une quelconque des revendications 1 et 4-8, dans lequel ;
(a) le premier oligonucléotide ou le troisième oligonucléotide est attaché à un support solide ; et/ou
(b) l'échantillon comprend une pluralité de cibles, en option dans lequel chacune des cibles de la pluralité est marquée avec un code-barres stochastique distinct, en option comprenant en outre un dénombrement du nombre de la pluralité de cibles par un dénombrement des codes-barres stochastiques distincts associés à la cible.

10. Procédé de marquage d'une cible dans un échantillon pour la quantification de ladite cible, comprenant les étapes consistant à :
hybrider ladite cible avec un premier oligonucléotide comprenant une séquence spécifique d'une cible et une première séquence d'amorce d'amplification en présence d'un deuxième oligonucléotide comprenant une séquence adaptatrice universelle ;
allonger ledit premier oligonucléotide pour générer une première séquence nucléotidique comprenant un complément de la séquence adaptatrice universelle ;
hybrider ladite première séquence nucléotidique avec un troisième oligonucléotide comprenant la séquence adaptatrice universelle, un code-barres stochastique, dans lequel le code-barres stochastique comprend un marqueur moléculaire, et un site de liaison pour une deuxième amorce d'amplification ;
allonger ledit troisième oligonucléotide pour générer une deuxième séquence nucléotidique ; et
amplifier ladite deuxième séquence nucléotidique en utilisant une première amorce d'amplification et une deuxième amorce d'amplification pour générer une pluralité de troisièmes séquences nucléotidiques,
dans lequel chacune des troisièmes séquences nucléotidiques de la pluralité comprend ladite cible et le code-barres stochastique,
dans lequel la cible est une molécule d'ARNm ; et
dans lequel le marqueur moléculaire comprend une séquence d'acide nucléique qui fournit un compteur pour l'occurrence spécifique de l'acide nucléique cible.

11. Procédé de la revendication 10 :
(a) le procédé comprenant un allongement du premier oligonucléotide pour générer une première séquence nucléotidique comprenant un complément de la séquence adaptatrice universelle par un changement de matrice ; et/ou
(b) dans lequel le code-barres stochastique comprend en outre un marqueur universel, un marqueur dimensionnel, un marqueur cellulaire ou une combinaison de ceux-ci.

12. Procédé de marquage d'une cible dans un échantillon pour la quantification de ladite cible, comprenant les étapes consistant à :
hybrider ladite cible avec un premier oligonucléotide comprenant une séquence adaptatrice universelle et une séquence spécifique d'une cible ;
allonger ledit premier oligonucléotide pour générer une première séquence nucléotidique ;
hybrider ladite première séquence nucléotidique avec un deuxième oligonucléotide comprenant une deuxième séquence spécifique d'une cible et un troisième oligonucléotide comprenant un code-barres stochastique, dans lequel le code-barres stochastique comprend un marqueur moléculaire, et une séquence qui se lie spécifiquement à la séquence adaptatrice universelle ;
amplifier ladite première séquence nucléotidique pour générer une pluralité de deuxièmes séquences nucléotidiques,
dans lequel chacune des deuxièmes séquences nucléotidiques de la pluralité comprend ladite cible et ledit code-barres stochastique,
dans lequel la cible est une molécule d'ARNm ; et
dans lequel le marqueur moléculaire comprend une séquence d'acide nucléique qui fournit un compteur pour l'occurrence spécifique de l'acide nucléique cible.


### SEQUENCE LISTING

<110> Cellular Research, Inc. Fu, Glenn Wilhelmy, Julie Simbirsky, Maria Shum, Eleen
<120> METHODS AND COMPOSITIONS FOR LABELING
   TARGETS
<130> BDCRI.011WO
<150> 62/135,018
   <151> 2015-07-17
<150> 62/173,899
   <151> 2015-06-10
<150> 62/160,976
   <151> 2015-05-13
<150> 62/128,849
   <151> 2015-03-05
<150> 62/126,397
   <151> 2015-02-27
<160> 423
<170> FastSEQ for Windows Version 4.0
<210> 1
   <211> 13
   <212> DNA
   <213> Artificial Sequence
<220>
   <223> TCR alpha chain C-region binding primer
<400> 1
   cagacagact tgt 13
<210> 2
   <211> 13
   <212> DNA
   <213> Artificial Sequence
<220>
   <223> TCR beta chain C-region binding primer
<400> 2
   ccttttgggt gtg 13
<210> 3
   <211> 22
   <212> DNA
   <213> Artificial Sequence
<220>
   <223> RORC N1 Forward primer
<400> 3
   cccaaacatt tccatggtgc tc 22
<210> 4
   <211> 20
   <212> DNA
   <213> Artificial Sequence
<220>
   <223> RUNX1 N1 Forward primer
<400> 4
   gcaacgggaa atgtggtcct 20
<210> 5
   <211> 18
   <212> DNA
   <213> Artificial Sequence
<220>
   <223> RUNX3 N1 Forward primer
<400> 5
   ctggtcccag gtcagcgt 18
<210> 6
   <211> 23
   <212> DNA
   <213> Artificial Sequence
<220>
   <223> TBET N1 Forward primer
<400> 6
   ctgccctaac tacagtcgtt tac 23
<210> 7
   <211> 19
   <212> DNA
   <213> Artificial Sequence
<220>
   <223> GATA3 N1 Forward primer
<400> 7
   ccgttcacca gttgccgtt 19
<210> 8
   <211> 20
   <212> DNA
   <213> Artificial Sequence
<220>
   <223> PDCD1 N1 Forward primer
<400> 8
   acatcctacg gtcccaaggt 20
<210> 9
   <211> 21
   <212> DNA
   <213> Artificial Sequence
<220>
   <223> BCL2 N1 Forward primer
<400> 9
   tgcaagagtg acagtggatt g 21
<210> 10
   <211> 19
   <212> DNA
   <213> Artificial Sequence
<220>
   <223> BCL6 N1 Forward primer
<400> 10
   tgtcctcacg gtgcctttt 19
<210> 11
   <211> 20
   <212> DNA
   <213> Artificial Sequence
<220>
   <223> BIRC5 N1 Forward primer
<400> 11
   tgccacggcc tttccttaaa 20
<210> 12
   <211> 20
   <212> DNA
   <213> Artificial Sequence
<220>
   <223> CCL14 N1 Forward primer
<400> 12
   ttcctcctca tcaccatcgc 20
<210> 13
   <211> 20
   <212> DNA
   <213> Artificial Sequence
<220>
   <223> CCL17 N1 Forward primer
<400> 13
   gagtgctgcc tggagtactt 20
<210> 14
   <211> 20
   <212> DNA
   <213> Artificial Sequence
<220>
   <223> CCL18 N1 Forward primer
<400> 14
   gaagctgaat gcctgagggg 20
<210> 15
   <211> 20
   <212> DNA
   <213> Artificial Sequence
<220>
   <223> CCL20 N1 Forward primer
<400> 15
   acttgcacat catggagggt 20
<210> 16
   <211> 20
   <212> DNA
   <213> Artificial Sequence
<220>
   <223> CCL3 N1 Forward primer
<400> 16
   tggactggtt gttgccaaac 20
<210> 17
   <211> 20
   <212> DNA
   <213> Artificial Sequence
<220>
   <223> CCL4 N1 Forward primer
<400> 17
   cccagccagc tgtggtattc 20
<210> 18
   <211> 20
   <212> DNA
   <213> Artificial Sequence
<220>
   <223> CCR4 N1 Forward primer
<400> 18
   ccaaagggaa gagtgcaggg 20
<210> 19
   <211> 20
   <212> DNA
   <213> Artificial Sequence
<220>
   <223> CCR7 N1 Forward primer
<400> 19
   ggggagagtg tggtgtttcc 20
<210> 20
   <211> 21
   <212> DNA
   <213> Artificial Sequence
<220>
   <223> CD160 N1 Forward primer
<400> 20
   ggaagacagc cagatccagt g 21
<210> 21
   <211> 20
   <212> DNA
   <213> Artificial Sequence
<220>
   <223> CD244 N1 Forward primer
<400> 21
   gggctgagaa tgaggcagtt 20
<210> 22
   <211> 20
   <212> DNA
   <213> Artificial Sequence
<220>
   <223> CD27 N1 Forward primer
<400> 22
   tgcagagcct tgtcgttaca 20
<210> 23
   <211> 20
   <212> DNA
   <213> Artificial Sequence
<220>
   <223> CD28 N1 Forward primer
<400> 23
   accatcacag gcatgttcct 20
<210> 24
   <211> 20
   <212> DNA
   <213> Artificial Sequence
<220>
   <223> CD30 N1 Forward primer
<400> 24
   tttactcatc gggcagccac 20
<210> 25
   <211> 20
   <212> DNA
   <213> Artificial Sequence
<220>
   <223> CD38 N1 Forward primer
<400> 25
   aggtcaatgc cagagacgga 20
<210> 26
   <211> 20
   <212> DNA
   <213> Artificial Sequence
<220>
   <223> CD3D N1 Forward primer
<400> 26
   gaaaacgcat cctggaccca 20
<210> 27
   <211> 20
   <212> DNA
   <213> Artificial Sequence
<220>
   <223> CD3E N1 Forward primer
<400> 27
   aagttgtccc ccatcccaaa 20
<210> 28
   <211> 20
   <212> DNA
   <213> Artificial Sequence
<220>
   <223> CD4 N1 Forward primer
<400> 28
   ctgggagagg gggtagctag 20
<210> 29
   <211> 20
   <212> DNA
   <213> Artificial Sequence
<220>
   <223> CD40LG N1 Forward primer
<400> 29
   cctcccccag tctctcttct 20
<210> 30
   <211> 20
   <212> DNA
   <213> Artificial Sequence
<220>
   <223> CD5 N1 Forward primer
<400> 30
   atcaatggtc caagccgcat 20
<210> 31
   <211> 21
   <212> DNA
   <213> Artificial Sequence
<220>
   <223> CD69 N1 Forward primer
<400> 31
   gctgtagaca ggtccttttc g 21
<210> 32
   <211> 20
   <212> DNA
   <213> Artificial Sequence
<220>
   <223> CD8A N1 Forward primer
<400> 32
   actgctgtcc caaacatgca 20
<210> 33
   <211> 20
   <212> DNA
   <213> Artificial Sequence
<220>
   <223> CD8B N1 Forward primer
<400> 33
   ccaccatctt tgcaggttgc 20
<210> 34
   <211> 21
   <212> DNA
   <213> Artificial Sequence
<220>
   <223> CDCA7 N1 Forward primer
<400> 34
   ccagtctagt ttctgggcag g 21
<210> 35
   <211> 20
   <212> DNA
   <213> Artificial Sequence
<220>
   <223> CSF2 N1 Forward primer
<400> 35
   agccagtcca ggagtgagac 20
<210> 36
   <211> 19
   <212> DNA
   <213> Artificial Sequence
<220>
   <223> CX3CR1 N1 Forward primer
<400> 36
   cacccgtcca gaccttgtt 19
<210> 37
   <211> 20
   <212> DNA
   <213> Artificial Sequence
<220>
   <223> CXCL12 N1 Forward primer
<400> 37
   tgggagttga tcgcctttcc 20
<210> 38
   <211> 20
   <212> DNA
   <213> Artificial Sequence
<220>
   <223> CXCL13 N1 Forward primer
<400> 38
   aggcagatgg aacttgagcc 20
<210> 39
   <211> 20
   <212> DNA
   <213> Artificial Sequence
<220>
   <223> CXCR3 N1 Forward primer
<400> 39
   gacctcagag gcctcctact 20
<210> 40
   <211> 20
   <212> DNA
   <213> Artificial Sequence
<220>
   <223> CXCR5 N1 Forward primer
<400> 40
   cctccccagc ctttgatcag 20
<210> 41
   <211> 19
   <212> DNA
   <213> Artificial Sequence
<220>
   <223> EOMES N1 Forward primer
<400> 41
   acttaacagc tgcaggggc 19
<210> 42
   <211> 20
   <212> DNA
   <213> Artificial Sequence
<220>
   <223> FAS N1 Forward primer
<400> 42
   attgctggta gagaccccca 20
<210> 43
   <211> 21
   <212> DNA
   <213> Artificial Sequence
<220>
   <223> FASLG N1 Forward primer
<400> 43
   cctcaagggg gactgtcttt c 21
<210> 44
   <211> 20
   <212> DNA
   <213> Artificial Sequence
<220>
   <223> FOSL1 N1 Forward primer
<400> 44
   ctcctgacag aaggtgccac 20
<210> 45
   <211> 20
   <212> DNA
   <213> Artificial Sequence
<220>
   <223> FOXP3 N1 Forward primer
<400> 45
   acagaagcag cgtcagtacc 20
<210> 46
   <211> 20
   <212> DNA
   <213> Artificial Sequence
<220>
   <223> GAPDH N1 Forward primer
<400> 46
   gacttcaaca gcgacaccca 20
<210> 47
   <211> 20
   <212> DNA
   <213> Artificial Sequence
<220>
   <223> GZMA N1 Forward primer
<400> 47
   ggaaccatgt gccaagttgc 20
<210> 48
   <211> 21
   <212> DNA
   <213> Artificial Sequence
<220>
   <223> GZMB N1 Forward primer
<400> 48
   aggtgaagat gacagtgcag g 21
<210> 49
   <211> 20
   <212> DNA
   <213> Artificial Sequence
<220>
   <223> GZMH N1 Forward primer
<400> 49
   agtgttgctg acagtgcaga 20
<210> 50
   <211> 20
   <212> DNA
   <213> Artificial Sequence
<220>
   <223> GZMK N1 Forward primer
<400> 50
   ttgccacaaa gcctggaatc 20
<210> 51
   <211> 20
   <212> DNA
   <213> Artificial Sequence
<220>
   <223> HLA-DRA N1 Forward primer
<400> 51
   gggtctggtg ggcatcatta 20
<210> 52
   <211> 20
   <212> DNA
   <213> Artificial Sequence
<220>
   <223> IFNG N1 Forward primer
<400> 52
   ctaggcagcc aacctaagca 20
<210> 53
   <211> 20
   <212> DNA
   <213> Artificial Sequence
<220>
   <223> IL10 N1 Forward primer
<400> 53
   ccccaaccac ttcattcttg 20
<210> 54
   <211> 20
   <212> DNA
   <213> Artificial Sequence
<220>
   <223> IL12A N1 Forward primer
<400> 54
   ggtccctcca aaccgttgtc 20
<210> 55
   <211> 20
   <212> DNA
   <213> Artificial Sequence
<220>
   <223> IL12B N1 Forward primer
<400> 55
   gctatggtga gccgtgattg 20
<210> 56
   <211> 20
   <212> DNA
   <213> Artificial Sequence
<220>
   <223> IL13 N1 Forward primer
<400> 56
   ggagccaagg gttcagagac 20
<210> 57
   <211> 21
   <212> DNA
   <213> Artificial Sequence
<220>
   <223> IL17A N1 Forward primer
<400> 57
   gccttcaaga ctgaacaccg a 21
<210> 58
   <211> 20
   <212> DNA
   <213> Artificial Sequence
<220>
   <223> IL17F N1 Forward primer
<400> 58
   ttggagaagg tgctggtgac 20
<210> 59
   <211> 21
   <212> DNA
   <213> Artificial Sequence
<220>
   <223> IL1A N1 Forward primer
<400> 59
   ggcatcctcc acaatagcag a 21
<210> 60
   <211> 20
   <212> DNA
   <213> Artificial Sequence
<220>
   <223> IL1B N1 Forward primer
<400> 60
   cttaaagccc gcctgacaga 20
<210> 61
   <211> 21
   <212> DNA
   <213> Artificial Sequence
<220>
   <223> IL2 N1 Forward primer
<400> 61
   tcacttaaga cccagggact t 21
<210> 62
   <211> 20
   <212> DNA
   <213> Artificial Sequence
<220>
   <223> IL21 N1 Forward primer
<400> 62
   cccaaggtca agatcgccac 20
<210> 63
   <211> 20
   <212> DNA
   <213> Artificial Sequence
<220>
   <223> IL21R N1 Forward primer
<400> 63
   atttgaggct gcagtgagct 20
<210> 64
   <211> 21
   <212> DNA
   <213> Artificial Sequence
<220>
   <223> IL22 N1 Forward primer
<400> 64
   tgggaagcca aactccatca t 21
<210> 65
   <211> 21
   <212> DNA
   <213> Artificial Sequence
<220>
   <223> IL23R N1 Forward primer
<400> 65
   agaatcatta ggccaggcgt g 21
<210> 66
   <211> 21
   <212> DNA
   <213> Artificial Sequence
<220>
   <223> IL24 N1 Forward primer
<400> 66
   ctcaccccat catccctttc c 21
<210> 67
   <211> 20
   <212> DNA
   <213> Artificial Sequence
<220>
   <223> IL26 N1 Forward primer
<400> 67
   tactgacggc atgttaggtg 20
<210> 68
   <211> 20
   <212> DNA
   <213> Artificial Sequence
<220>
   <223> IL3 N1 Forward primer
<400> 68
   acagacgact ttgagcctcg 20
<210> 69
   <211> 20
   <212> DNA
   <213> Artificial Sequence
<220>
   <223> IL31 N1 Forward primer
<400> 69
   ggccatctct tcctttcgga 20
<210> 70
   <211> 20
   <212> DNA
   <213> Artificial Sequence
<220>
   <223> IL32 N1 Forward primer
<400> 70
   ctttccagtc ctacggagcc 20
<210> 71
   <211> 20
   <212> DNA
   <213> Artificial Sequence
<220>
   <223> IL33R N1 Forward primer
<400> 71
   ttcaggactc cctccagcat 20
<210> 72
   <211> 20
   <212> DNA
   <213> Artificial Sequence
<220>
   <223> IL4 N1 Forward primer
<400> 72
   accatgagaa ggacactcgc 20
<210> 73
   <211> 20
   <212> DNA
   <213> Artificial Sequence
<220>
   <223> IL4R N1 Forward primer
<400> 73
   tgaacttcag ggagggtggt 20
<210> 74
   <211> 19
   <212> DNA
   <213> Artificial Sequence
<220>
   <223> IL5 N1 Forward primer
<400> 74
   gcagtgagaa tgagggcca 19
<210> 75
   <211> 19
   <212> DNA
   <213> Artificial Sequence
<220>
   <223> IL6 N1 Forward primer
<400> 75
   cggcaaatgt agcatgggc 19
<210> 76
   <211> 20
   <212> DNA
   <213> Artificial Sequence
<220>
   <223> IL6R N1 Forward primer
<400> 76
   ccagcaccag ggagtttcta 20
<210> 77
   <211> 20
   <212> DNA
   <213> Artificial Sequence
<220>
   <223> IRF4 N1 Forward primer
<400> 77
   ctcttcagca tcccccgtac 20
<210> 78
   <211> 19
   <212> DNA
   <213> Artificial Sequence
<220>
   <223> LAG3 N1 Forward primer
<400> 78
   agctgtacca gggggagag 19
<210> 79
   <211> 20
   <212> DNA
   <213> Artificial Sequence
<220>
   <223> LIF N1 Forward primer
<400> 79
   tccccatcgt cctccttgtc 20
<210> 80
   <211> 20
   <212> DNA
   <213> Artificial Sequence
<220>
   <223> LTA N1 Forward primer
<400> 80
   aggcagggag gggactattt 20
<210> 81
   <211> 20
   <212> DNA
   <213> Artificial Sequence
<220>
   <223> MCL1 N1 Forward primer
<400> 81
   gactggctac gtagttcggg 20
<210> 82
   <211> 20
   <212> DNA
   <213> Artificial Sequence
<220>
   <223> MKI67 N1 Forward primer
<400> 82
   tactttttcg cctcccaggg 20
<210> 83
   <211> 20
   <212> DNA
   <213> Artificial Sequence
<220>
   <223> MYC N1 Forward primer
<400> 83
   agctacggaa ctcttgtgcg 20
<210> 84
   <211> 20
   <212> DNA
   <213> Artificial Sequence
<220>
   <223> NKG2D N1 Forward primer
<400> 84
   caacacccag gggatcagtg 20
<210> 85
   <211> 21
   <212> DNA
   <213> Artificial Sequence
<220>
   <223> PRDM1 N1 Forward primer
<400> 85
   accaaagcat cacgttgaca t 21
<210> 86
   <211> 20
   <212> DNA
   <213> Artificial Sequence
<220>
   <223> PRF1 N1 Forward primer
<400> 86
   ggagtccagc gaatgacgtc 20
<210> 87
   <211> 24
   <212> DNA
   <213> Artificial Sequence
<220>
   <223> PTPRCvl N1 Forward primer
<400> 87
   gttcccatgt taaatcccat tcat 24
<210> 88
   <211> 20
   <212> DNA
   <213> Artificial Sequence
<220>
   <223> PTPRCv2 N1 Forward primer
<400> 88
   accctctctc cctccctttc 20
<210> 89
   <211> 20
   <212> DNA
   <213> Artificial Sequence
<220>
   <223> SAP30 N1 Forward primer
<400> 89
   accaaccaga ccaggactta 20
<210> 90
   <211> 20
   <212> DNA
   <213> Artificial Sequence
<220>
   <223> SELL N1 Forward primer
<400> 90
   gcatctcatg agtgccaagc 20
<210> 91
   <211> 20
   <212> DNA
   <213> Artificial Sequence
<220>
   <223> TBX21 N1 Forward primer
<400> 91
   ttataaccat cagcccgcca 20
<210> 92
   <211> 20
   <212> DNA
   <213> Artificial Sequence
<220>
   <223> TGFB1 N1 Forward primer
<400> 92
   tattcctttg cccggcatca 20
<210> 93
   <211> 20
   <212> DNA
   <213> Artificial Sequence
<220>
   <223> TNF N1 Forward primer
<400> 93
   agtggacctt aggccttcct 20
<210> 94
   <211> 20
   <212> DNA
   <213> Artificial Sequence
<220>
   <223> TNFRSF9 N1 Forward primer
<400> 94
   tggcatgtga gtcattgctc 20
<210> 95
   <211> 20
   <212> DNA
   <213> Artificial Sequence
<220>
   <223> TYMS N1 Forward primer
<400> 95
   tcagtcttta ggggttgggc 20
<210> 96
   <211> 20
   <212> DNA
   <213> Artificial Sequence
<220>
   <223> UHRF1 N1 Forward primer
<400> 96
   ccagttcttc ctgacaccgg 20
<210> 97
   <211> 20
   <212> DNA
   <213> Artificial Sequence
<220>
   <223> ZBED2 N1 Forward primer
<400> 97
   aatgtaccag ccagtcagcg 20
<210> 98
   <211> 20
   <212> DNA
   <213> Artificial Sequence
<220>
   <223> ZNF683 N1 Forward primer
<400> 98
   ggagagcgtc cattccagtg 20
<210> 99
   <211> 26
   <212> DNA
   <213> Artificial Sequence
<220>
   <223> TRAV1-1 N1 Forward primer
<400> 99
   tctgggtttt atgggctgtc ctggta 26
<210> 100
   <211> 23
   <212> DNA
   <213> Artificial Sequence
<220>
   <223> TRAV1-2-01 N1 Forward primer
<400> 100
   ggacaaaaca ttgaccagcc cac 23
<210> 101
   <211> 27
   <212> DNA
   <213> Artificial Sequence
<220>
   <223> TRAV1-2-02 N1 Forward primer
<400> 101
   atctgggttc aacgggctgt tctggta 27
<210> 102
   <211> 25
   <212> DNA
   <213> Artificial Sequence
<220>
   <223> TRAV2 N1 Forward primer
<400> 102
   aatcactctg tgtccaatgc ttaca 25
<210> 103
   <211> 24
   <212> DNA
   <213> Artificial Sequence
<220>
   <223> TRAV3 N1 Forward primer
<400> 103
   attcagtctc tggaaaccct tatc 24
<210> 104
   <211> 22
   <212> DNA
   <213> Artificial Sequence
<220>
   <223> TRAV4 N1 Forward primer
<400> 104
   gtagccacaa caacattgct ac 22
<210> 105
   <211> 23
   <212> DNA
   <213> Artificial Sequence
<220>
   <223> TRAV5 N1 Forward primer
<400> 105
   cagctcctcc acctacttat act 23
<210> 106
   <211> 20
   <212> DNA
   <213> Artificial Sequence
<220>
   <223> TRAV6 N1 Forward primer
<400> 106
   ccagcatact tacagtggta 20
<210> 107
   <211> 21
   <212> DNA
   <213> Artificial Sequence
<220>
   <223> TRAV7 N1 Forward primer
<400> 107
   gcacgtactc tgtcagtcgt t 21
<210> 108
   <211> 25
   <212> DNA
   <213> Artificial Sequence
<220>
   <223> TRAV8-1 N1 Forward primer
<400> 108
   gtggaactgt taatctcttc tggta 25
<210> 109
   <211> 24
   <212> DNA
   <213> Artificial Sequence
<220>
   <223> TRAV8-2 N1 Forward primer
<400> 109
   ctctcttctg gtatgtgcaa cacc 24
<210> 110
   <211> 24
   <212> DNA
   <213> Artificial Sequence
<220>
   <223> TRAV8-3 N1 Forward primer
<400> 110
   ggcaacacct tatctcttct ggta 24
<210> 111
   <211> 20
   <212> DNA
   <213> Artificial Sequence
<220>
   <223> TRAV8-4 N1 Forward primer
<400> 111
   ctcttctggt atgtgcaata 20
<210> 112
   <211> 23
   <212> DNA
   <213> Artificial Sequence
<220>
   <223> TRAV8-5 N1 Forward primer
<400> 112
   gccatggcag aagaatgtgg att 23
<210> 113
   <211> 25
   <212> DNA
   <213> Artificial Sequence
<220>
   <223> TRAV8-6 N1 Forward primer
<400> 113
   agtgtatctc ttctggtatg tgcaa 25
<210> 114
   <211> 23
   <212> DNA
   <213> Artificial Sequence
<220>
   <223> TRAV8-7 N1 Forward primer
<400> 114
   ggagttcctt ctctcttctg gta 23
<210> 115
   <211> 24
   <212> DNA
   <213> Artificial Sequence
<220>
   <223> TRAV9-1 N1 Forward primer
<400> 115
   gaaaccacac agtacccttc cctt 24
<210> 116
   <211> 24
   <212> DNA
   <213> Artificial Sequence
<220>
   <223> TRAV9-2 N1 Forward primer
<400> 116
   aggataccct tcccttttct ggta 24
<210> 117
   <211> 22
   <212> DNA
   <213> Artificial Sequence
<220>
   <223> TRAV10 N1 Forward primer
<400> 117
   tgagcccctt cagcaactta ag 22
<210> 118
   <211> 26
   <212> DNA
   <213> Artificial Sequence
<220>
   <223> TRAV11 N1 Forward primer
<400> 118
   cactcttcaa tttccactgg ttccgg 26
<210> 119
   <211> 24
   <212> DNA
   <213> Artificial Sequence
<220>
   <223> TRAV12-1 N1 Forward primer
<400> 119
   agtgcttctc agtctttctt ctgg 24
<210> 120
   <211> 21
   <212> DNA
   <213> Artificial Sequence
<220>
   <223> TRAV12-2 N1 Forward primer
<400> 120
   ttcccagtcc ttcttctggt a 21
<210> 121
   <211> 23
   <212> DNA
   <213> Artificial Sequence
<220>
   <223> TRAV12-3 N1 Forward primer
<400> 121
   ctctcaactg cacttacagc aac 23
<210> 122
   <211> 23
   <212> DNA
   <213> Artificial Sequence
<220>
   <223> TRAV13-1 N1 Forward primer
<400> 122
   gcctcaaact acttcccttg gta 23
<210> 123
   <211> 23
   <212> DNA
   <213> Artificial Sequence
<220>
   <223> TRAV13-2 N1 Forward primer
<400> 123
   cagcgcctca gactacttca ttt 23
<210> 124
   <211> 22
   <212> DNA
   <213> Artificial Sequence
<220>
   <223> TRAV14 N1 Forward primer
<400> 124
   ggtctattct ggtacaagca gc 22
<210> 125
   <211> 26
   <212> DNA
   <213> Artificial Sequence
<220>
   <223> TRAV15 N1 Forward primer
<400> 125
   taacttctac tggttctggc agggtc 26
<210> 126
   <211> 24
   <212> DNA
   <213> Artificial Sequence
<220>
   <223> TRAV16 N1 Forward primer
<400> 126
   aactattcct attctgggag tcct 24
<210> 127
   <211> 27
   <212> DNA
   <213> Artificial Sequence
<220>
   <223> TRAV17 N1 Forward primer
<400> 127
   gtggtataga caaaattcag gtagagg 27
<210> 128
   <211> 27
   <212> DNA
   <213> Artificial Sequence
<220>
   <223> TRAV18 N1 Forward primer
<400> 128
   ctgcacttat cagtccagct attcaac 27
<210> 129
   <211> 20
   <212> DNA
   <213> Artificial Sequence
<220>
   <223> TRAV19 N1 Forward primer
<400> 129
   ttctggtaca agcaaccacc 20
<210> 130
   <211> 21
   <212> DNA
   <213> Artificial Sequence
<220>
   <223> TRAV20 N1 Forward primer
<400> 130
   cacagtcagc ggtttaagag g 21
<210> 131
   <211> 24
   <212> DNA
   <213> Artificial Sequence
<220>
   <223> TRAV21 N1 Forward primer
<400> 131
   ctgatagcgc tatttacaac ctcc 24
<210> 132
   <211> 23
   <212> DNA
   <213> Artificial Sequence
<220>
   <223> TRAV22 N1 Forward primer
<400> 132
   gcggtgcaat ttttctgact ctg 23
<210> 133
   <211> 25
   <212> DNA
   <213> Artificial Sequence
<220>
   <223> TRAV23 N1 Forward primer
<400> 133
   ctgcgtttga ctactttcca tggta 25
<210> 134
   <211> 22
   <212> DNA
   <213> Artificial Sequence
<220>
   <223> TRAV24 N1 Forward primer
<400> 134
   cccttccagc aatttttatg cc 22
<210> 135
   <211> 22
   <212> DNA
   <213> Artificial Sequence
<220>
   <223> TRAV25 N1 Forward primer
<400> 135
   ccacgtactg caattcctca ac 22
<210> 136
   <211> 26
   <212> DNA
   <213> Artificial Sequence
<220>
   <223> TRAV26-1 N1 Forward primer
<400> 136
   gtgtattggt atcgacagat tcactc 26
<210> 137
   <211> 26
   <212> DNA
   <213> Artificial Sequence
<220>
   <223> TRAV26-2 N1 Forward primer
<400> 137
   tacattggta tcgacagctt ccctcc 26
<210> 138
   <211> 23
   <212> DNA
   <213> Artificial Sequence
<220>
   <223> TRAV27 N1 Forward primer
<400> 138
   actgtgtact gcaactcctc aag 23
<210> 139
   <211> 25
   <212> DNA
   <213> Artificial Sequence
<220>
   <223> TRAV28 N1 Forward primer
<400> 139
   tgttctattt acatgatccg tgtgc 25
<210> 140
   <211> 22
   <212> DNA
   <213> Artificial Sequence
<220>
   <223> TRAV29 N1 Forward primer
<400> 140
   taccctgctg aaggtcctac at 22
<210> 141
   <211> 22
   <212> DNA
   <213> Artificial Sequence
<220>
   <223> TRAV30 N1 Forward primer
<400> 141
   gcagttcctc caaggcttta ta 22
<210> 142
   <211> 24
   <212> DNA
   <213> Artificial Sequence
<220>
   <223> TRAV31 N1 Forward primer
<400> 142
   cgtgaaactg gactgtgcat acaa 24
<210> 143
   <211> 25
   <212> DNA
   <213> Artificial Sequence
<220>
   <223> TRAV32 N1 Forward primer
<400> 143
   tgacagttat acagatggag ctttg 25
<210> 144
   <211> 27
   <212> DNA
   <213> Artificial Sequence
<220>
   <223> TRAV33 N1 Forward primer
<400> 144
   cgtacacttt atactggtac aagcaac 27
<210> 145
   <211> 25
   <212> DNA
   <213> Artificial Sequence
<220>
   <223> TRAV34 N1 Forward primer
<400> 145
   ctcaccataa actgcacgtc atcaa 25
<210> 146
   <211> 25
   <212> DNA
   <213> Artificial Sequence
<220>
   <223> TRAV35 N1 Forward primer
<400> 146
   gcatatttaa cacctggcta tggta 25
<210> 147
   <211> 24
   <212> DNA
   <213> Artificial Sequence
<220>
   <223> TRAV36 N1 Forward primer
<400> 147
   gtgactaact ttcgaagcct acta 24
<210> 148
   <211> 25
   <212> DNA
   <213> Artificial Sequence
<220>
   <223> TRAV37 N1 Forward primer
<400> 148
   gcccttcaga tttcttcagt ggttc 25
<210> 149
   <211> 22
   <212> DNA
   <213> Artificial Sequence
<220>
   <223> TRAV38-1 N1 Forward primer
<400> 149
   gtgcaggagg cagagactgt ga 22
<210> 150
   <211> 22
   <212> DNA
   <213> Artificial Sequence
<220>
   <223> TRAV38-2 N1 Forward primer
<400> 150
   tattctggta caagcagcct cc 22
<210> 151
   <211> 23
   <212> DNA
   <213> Artificial Sequence
<220>
   <223> TRAV39 N1 Forward primer
<400> 151
   cacttcagac agactgtatt ggt 23
<210> 152
   <211> 19
   <212> DNA
   <213> Artificial Sequence
<220>
   <223> TRAV40 N1 Forward primer
<400> 152
   acatacacat ccacggggt 19
<210> 153
   <211> 24
   <212> DNA
   <213> Artificial Sequence
<220>
   <223> TRAV41 N1 Forward primer
<400> 153
   actcggtagg aataagtgcc ttac 24
<210> 154
   <211> 20
   <212> DNA
   <213> Artificial Sequence
<220>
   <223> TRBV2 N1 Forward primer
<400> 154
   cttggggcag aaagtcgagt 20
<210> 155
   <211> 20
   <212> DNA
   <213> Artificial Sequence
<220>
   <223> TRBV3-1 N1 Forward primer
<400> 155
   ggtcacacag atgggaaacg 20
<210> 156
   <211> 19
   <212> DNA
   <213> Artificial Sequence
<220>
   <223> TRBV4-1 N1 Forward primer
<400> 156
   tggggcacag ggctatgta 19
<210> 157
   <211> 20
   <212> DNA
   <213> Artificial Sequence
<220>
   <223> TRBV4-2 N1 Forward primer
<400> 157
   tacgcagaca ccaagacacc 20
<210> 158
   <211> 19
   <212> DNA
   <213> Artificial Sequence
<220>
   <223> TRBV4-3 N1 Forward primer
<400> 158
   acgcagacac caagacacc 19
<210> 159
   <211> 20
   <212> DNA
   <213> Artificial Sequence
<220>
   <223> TRBV5-1 N1 Forward primer
<400> 159
   agcaagtgac actgagctgc 20
<210> 160
   <211> 20
   <212> DNA
   <213> Artificial Sequence
<220>
   <223> TRBV5-3/5/6 N1 Forward primer
<400> 160
   ccaaagtccc acacacctga 20
<210> 161
   <211> 20
   <212> DNA
   <213> Artificial Sequence
<220>
   <223> TRBV5-4 N1 Forward primer
<400> 161
   tcacccaaag tcccacacac 20
<210> 162
   <211> 20
   <212> DNA
   <213> Artificial Sequence
<220>
   <223> TRBV5-7 N1 Forward primer
<400> 162
   caaaacgaga ggacagcacg 20
<210> 163
   <211> 20
   <212> DNA
   <213> Artificial Sequence
<220>
   <223> TRBV5-8 N1 Forward primer
<400> 163
   gcgactctga gatgctctcc 20
<210> 164
   <211> 21
   <212> DNA
   <213> Artificial Sequence
<220>
   <223> TRBV6-1/9 N1 Forward primer
<400> 164
   gtgtgcccag gatatgaacc a 21
<210> 165
   <211> 20
   <212> DNA
   <213> Artificial Sequence
<220>
   <223> TRBV6-2/3/5/6/8 N1 Forward primer
<400> 165
   gtatcgacaa gacccaggca 20
<210> 166
   <211> 19
   <212> DNA
   <213> Artificial Sequence
<220>
   <223> TRBV6-4 N1 Forward primer
<400> 166
   tcacccaggc accaacatc 19
<210> 167
   <211> 20
   <212> DNA
   <213> Artificial Sequence
<220>
   <223> TRBV6-7 N1 Forward primer
<400> 167
   atcgacaaga cccaggcaag 20
<210> 168
   <211> 21
   <212> DNA
   <213> Artificial Sequence
<220>
   <223> TRBV7-1 N1 Forward primer
<400> 168
   gtccctgaga cacaaggtag c 21
<210> 169
   <211> 20
   <212> DNA
   <213> Artificial Sequence
<220>
   <223> TRBV7-2 N1 Forward primer
<400> 169
   tctcccagtc ccccagtaac 20
<210> 170
   <211> 20
   <212> DNA
   <213> Artificial Sequence
<220>
   <223> TRBV7-3 N1 Forward primer
<400> 170
   tctcccagac ccccagtaac 20
<210> 171
   <211> 20
   <212> DNA
   <213> Artificial Sequence
<220>
   <223> TRBV7-4 N1 Forward primer
<400> 171
   ccccaaggta caaagtcgca 20
<210> 172
   <211> 21
   <212> DNA
   <213> Artificial Sequence
<220>
   <223> TRBV7-6/7 N1 Forward primer
<400> 172
   tcccagtctc ccaggtacaa a 21
<210> 173
   <211> 20
   <212> DNA
   <213> Artificial Sequence
<220>
   <223> TRBV7-8 N1 Forward primer
<400> 173
   cccctaggta caaagtcgca 20
<210> 174
   <211> 21
   <212> DNA
   <213> Artificial Sequence
<220>
   <223> TRBV7-9 N1 Forward primer
<400> 174
   aaccgccttt attggtaccg a 21
<210> 175
   <211> 20
   <212> DNA
   <213> Artificial Sequence
<220>
   <223> TRBV9 N1 Forward primer
<400> 175
   gatgctcccc taggtctgga 20
<210> 176
   <211> 20
   <212> DNA
   <213> Artificial Sequence
<220>
   <223> TRBV10-1 N1 Forward primer
<400> 176
   atcacccaga gcccaagaca 20
<210> 177
   <211> 20
   <212> DNA
   <213> Artificial Sequence
<220>
   <223> TRBV10-2 N1 Forward primer
<400> 177
   cagagacagg aaggcaggtg 20
<210> 178
   <211> 20
   <212> DNA
   <213> Artificial Sequence
<220>
   <223> TRBV10-3 N1 Forward primer
<400> 178
   ctggtatcga caagacccgg 20
<210> 179
   <211> 20
   <212> DNA
   <213> Artificial Sequence
<220>
   <223> TRBV11-1 N1 Forward primer
<400> 179
   accctttact ggtaccggca 20
<210> 180
   <211> 21
   <212> DNA
   <213> Artificial Sequence
<220>
   <223> TRBV11-2 N1 Forward primer
<400> 180
   tctggccatg ctacccttta c 21
<210> 181
   <211> 20
   <212> DNA
   <213> Artificial Sequence
<220>
   <223> TRBV11-3 N1 Forward primer
<400> 181
   gtggcttttt ggtgcaatcc 20
<210> 182
   <211> 20
   <212> DNA
   <213> Artificial Sequence
<220>
   <223> TRBV12-3 N1 Forward primer
<400> 182
   acagacagac catgatgcgg 20
<210> 183
   <211> 20
   <212> DNA
   <213> Artificial Sequence
<220>
   <223> TRBV12-4 N1 Forward primer
<400> 183
   cagacagacc atgatgcggg 20
<210> 184
   <211> 20
   <212> DNA
   <213> Artificial Sequence
<220>
   <223> TRBV12-5 N1 Forward primer
<400> 184
   cagacaccaa ggcacaaggt 20
<210> 185
   <211> 20
   <212> DNA
   <213> Artificial Sequence
<220>
   <223> TRBV13 N1 Forward primer
<400> 185
   agggaaacag ccactctgaa 20
<210> 186
   <211> 20
   <212> DNA
   <213> Artificial Sequence
<220>
   <223> TRBV14 N1 Forward primer
<400> 186
   cccagccaca gcgtaataga 20
<210> 187
   <211> 20
   <212> DNA
   <213> Artificial Sequence
<220>
   <223> TRBV15 N1 Forward primer
<400> 187
   aagccagtga ccctgagttg 20
<210> 188
   <211> 20
   <212> DNA
   <213> Artificial Sequence
<220>
   <223> TRBV16 N1 Forward primer
<400> 188
   cgcccagact ccaaaacatc 20
<210> 189
   <211> 21
   <212> DNA
   <213> Artificial Sequence
<220>
   <223> TRBV17 N1 Forward primer
<400> 189
   accgacagaa tctgaggcaa g 21
<210> 190
   <211> 20
   <212> DNA
   <213> Artificial Sequence
<220>
   <223> TRBV18 N1 Forward primer
<400> 190
   ggaggcaaga ctgagatgca 20
<210> 191
   <211> 20
   <212> DNA
   <213> Artificial Sequence
<220>
   <223> TRBV19 N1 Forward primer
<400> 191
   ggcaagggct gagattgatc 20
<210> 192
   <211> 20
   <212> DNA
   <213> Artificial Sequence
<220>
   <223> TRBV20-1 N1 Forward primer
<400> 192
   gtgaagatcg agtgccgttc 20
<210> 193
   <211> 21
   <212> DNA
   <213> Artificial Sequence
<220>
   <223> TRBV23-1 N1 Forward primer
<400> 193
   acccccgaaa aaggacatac t 21
<210> 194
   <211> 21
   <212> DNA
   <213> Artificial Sequence
<220>
   <223> TRBV24-1 N1 Forward primer
<400> 194
   atgctgatgt tacccagacc c 21
<210> 195
   <211> 21
   <212> DNA
   <213> Artificial Sequence
<220>
   <223> TRBV25-1 N1 Forward primer
<400> 195
   gttctcaaac catgggccat g 21
<210> 196
   <211> 20
   <212> DNA
   <213> Artificial Sequence
<220>
   <223> TRBV27 N1 Forward primer
<400> 196
   tggtatcgac aagacccagg 20
<210> 197
   <211> 21
   <212> DNA
   <213> Artificial Sequence
<220>
   <223> TRBV28 N1 Forward primer
<400> 197
   gtgaaagtaa cccagagctc g 21
<210> 198
   <211> 20
   <212> DNA
   <213> Artificial Sequence
<220>
   <223> TRBV29-1 N1 Forward primer
<400> 198
   ttctggtacc gtcagcaacc 20
<210> 199
   <211> 20
   <212> DNA
   <213> Artificial Sequence
<220>
   <223> TRBV30 N1 Forward primer
<400> 199
   gggaacatca aaccccaacc 20
<210> 200
   <211> 43
   <212> DNA
   <213> Artificial Sequence
<220>
   <223> RORC N2 forward primer
<400> 200
   cagacgtgtg ctcttccgat ctacccaaga gaagcagaag tcg 43
<210> 201
   <211> 42
   <212> DNA
   <213> Artificial Sequence
<220>
   <223> RUNX1 N2 forward primer
<400> 201
   cagacgtgtg ctcttccgat ctgaaaattc gtgcgtgggc tt 42
<210> 202
   <211> 42
   <212> DNA
   <213> Artificial Sequence
<220>
   <223> RUNX3 N2 forward primer
<400> 202
   cagacgtgtg ctcttccgat ctcacctgct gcactcatct ga 42
<210> 203
   <211> 42
   <212> DNA
   <213> Artificial Sequence
<220>
   <223> TBET N2 forward primer
<400> 203
   cagacgtgtg ctcttccgat ctggtcaggg aaaggactca cc 42
<210> 204
   <211> 42
   <212> DNA
   <213> Artificial Sequence
<220>
   <223> GATA3 N2 forward primer
<400> 204
   cagacgtgtg ctcttccgat cttaaggtgg ttgtgctcgg ag 42
<210> 205
   <211> 41
   <212> DNA
   <213> Artificial Sequence
<220>
   <223> PDCD1 N2 forward primer
<400> 205
   cagacgtgtg ctcttccgat ctgcagaagt gcaggcacct a 41
<210> 206
   <211> 46
   <212> DNA
   <213> Artificial Sequence
<220>
   <223> BCL2 N2 forward primer
<400> 206
   cagacgtgtg ctcttccgat ctgctgatat tctgcaacac tgtaca 46
<210> 207
   <211> 42
   <212> DNA
   <213> Artificial Sequence
<220>
   <223> BCL6 N2 forward primer
<400> 207
   cagacgtgtg ctcttccgat ctgtaggcag acacagggac tt 42
<210> 208
   <211> 42
   <212> DNA
   <213> Artificial Sequence
<220>
   <223> BIRC5 N2 forward primer
<400> 208
   cagacgtgtg ctcttccgat ctttgtctaa gtgcaaccgc ct 42
<210> 209
   <211> 42
   <212> DNA
   <213> Artificial Sequence
<220>
   <223> CCL14 N2 forward primer
<400> 209
   cagacgtgtg ctcttccgat ctcttaccac ccctcagagt gc 42
<210> 210
   <211> 42
   <212> DNA
   <213> Artificial Sequence
<220>
   <223> CCL17 N2 forward primer
<400> 210
   cagacgtgtg ctcttccgat ctctcacccc agactcctga ct 42
<210> 211
   <211> 41
   <212> DNA
   <213> Artificial Sequence
<220>
   <223> CCL18 N2 forward primer
<400> 211
   cagacgtgtg ctcttccgat ctgtcccatc tgctatgccc a 41
<210> 212
   <211> 47
   <212> DNA
   <213> Artificial Sequence
<220>
   <223> CCL20 N2 forward primer
<400> 212
   cagacgtgtg ctcttccgat cttccataag ctattttggt ttagtgc 47
<210> 213
   <211> 42
   <212> DNA
   <213> Artificial Sequence
<220>
   <223> CCL3 N2 forward primer
<400> 213
   cagacgtgtg ctcttccgat ctctctgaga gttcccctgt cc 42
<210> 214
   <211> 42
   <212> DNA
   <213> Artificial Sequence
<220>
   <223> CCL4 N2 forward primer
<400> 214
   cagacgtgtg ctcttccgat cttggaactg aactgagctg ct 42
<210> 215
   <211> 49
   <212> DNA
   <213> Artificial Sequence
<220>
   <223> CCR4 N2 forward primer
<400> 215
   cagacgtgtg ctcttccgat ctattctgta taacactcat atctttgcc 49
<210> 216
   <211> 42
   <212> DNA
   <213> Artificial Sequence
<220>
   <223> CCR7 N2 forward primer
<400> 216
   cagacgtgtg ctcttccgat ctctcttggc tccactggga tg 42
<210> 217
   <211> 42
   <212> DNA
   <213> Artificial Sequence
<220>
   <223> CD160 N2 forward primer
<400> 217
   cagacgtgtg ctcttccgat ctttgtgcag accaagagca cc 42
<210> 218
   <211> 42
   <212> DNA
   <213> Artificial Sequence
<220>
   <223> CD244 N2 forward primer
<400> 218
   cagacgtgtg ctcttccgat ctggaaagcg acaagggtga ac 42
<210> 219
   <211> 42
   <212> DNA
   <213> Artificial Sequence
<220>
   <223> CD27 N2 forward primer
<400> 219
   cagacgtgtg ctcttccgat ctcgtgacag agtgcctttt cg 42
<210> 220
   <211> 42
   <212> DNA
   <213> Artificial Sequence
<220>
   <223> CD28 N2 forward primer
<400> 220
   cagacgtgtg ctcttccgat cttgtagatg acctggcttg cc 42
<210> 221
   <211> 42
   <212> DNA
   <213> Artificial Sequence
<220>
   <223> CD30 N2 forward primer
<400> 221
   cagacgtgtg ctcttccgat cttgtttgcc cagtgtttgt gc 42
<210> 222
   <211> 49
   <212> DNA
   <213> Artificial Sequence
<220>
   <223> CD38 N2 forward primer
<400> 222
   cagacgtgtg ctcttccgat ctatcagcat acctttattg tgatctatc 49
<210> 223
   <211> 43
   <212> DNA
   <213> Artificial Sequence
<220>
   <223> CD3D N2 forward primer
<400> 223
   cagacgtgtg ctcttccgat cttgatgtca ttgccactct get 43
<210> 224
   <211> 43
   <212> DNA
   <213> Artificial Sequence
<220>
   <223> CD3E N2 forward primer
<400> 224
   cagacgtgtg ctcttccgat ctctggggat ggactgggta aat 43
<210> 225
   <211> 42
   <212> DNA
   <213> Artificial Sequence
<220>
   <223> CD4 N2 forward primer
<400> 225
   cagacgtgtg ctcttccgat ctaccacttc cctcagtccc aa 42
<210> 226
   <211> 42
   <212> DNA
   <213> Artificial Sequence
<220>
   <223> CD40LG N2 forward primer
<400> 226
   cagacgtgtg ctcttccgat ctgagtcagg ccgttgctag tc 42
<210> 227
   <211> 42
   <212> DNA
   <213> Artificial Sequence
<220>
   <223> CD5 N2 forward primer
<400> 227
   cagacgtgtg ctcttccgat ctaggtcaca gatcttcccc cg 42
<210> 228
   <211> 47
   <212> DNA
   <213> Artificial Sequence
<220>
   <223> CD69 N2 forward primer
<400> 228
   cagacgtgtg ctcttccgat ctagtgttgg aaaatgtgca atatgtg 47
<210> 229
   <211> 43
   <212> DNA
   <213> Artificial Sequence
<220>
   <223> CD8A N2 forward primer
<400> 229
   cagacgtgtg ctcttccgat ctatgcctgc ccattggaga gaa 43
<210> 230
   <211> 42
   <212> DNA
   <213> Artificial Sequence
<220>
   <223> CD8B N2 forward primer
<400> 230
   cagacgtgtg ctcttccgat ctgctgtcca gttcccagaa gg 42
<210> 231
   <211> 45
   <212> DNA
   <213> Artificial Sequence
<220>
   <223> CDCA7 N2 forward primer
<400> 231
   cagacgtgtg ctcttccgat ctatgtaaac cattgctgtg ccatt 45
<210> 232
   <211> 42
   <212> DNA
   <213> Artificial Sequence
<220>
   <223> CSF2 N2 forward primer
<400> 232
   cagacgtgtg ctcttccgat ctggccacac tgaccctgat ac 42
<210> 233
   <211> 47
   <212> DNA
   <213> Artificial Sequence
<220>
   <223> CX3CR1 N2 forward primer
<400> 233
   cagacgtgtg ctcttccgat cttgttttcc tcttaacgtt agaccac 47
<210> 234
   <211> 43
   <212> DNA
   <213> Artificial Sequence
<220>
   <223> CXCL12 N2 forward primer
<400> 234
   cagacgtgtg ctcttccgat ctctcattct gaaggagccc cat 43
<210> 235
   <211> 44
   <212> DNA
   <213> Artificial Sequence
<220>
   <223> CXCL13 N2 forward primer
<400> 235
   cagacgtgtg ctcttccgat ctgcattcga agatccccag actt 44
<210> 236
   <211> 41
   <212> DNA
   <213> Artificial Sequence
<220>
   <223> CXCR3 N2 forward primer
<400> 236
   cagacgtgtg ctcttccgat ctccaatatc ctcgctcccg g 41
<210> 237
   <211> 42
   <212> DNA
   <213> Artificial Sequence
<220>
   <223> CXCR5 N2 forward primer
<400> 237
   cagacgtgtg ctcttccgat cttcctcgca agctgggtaa tc 42
<210> 238
   <211> 45
   <212> DNA
   <213> Artificial Sequence
<220>
   <223> EOMES N2 forward primer
<400> 238
   cagacgtgtg ctcttccgat ctactaactt gaaccgtgtt taagg 45
<210> 239
   <211> 40
   <212> DNA
   <213> Artificial Sequence
<220>
   <223> FAS N2 forward primer
<400> 239
   cagacgtgtg ctcttccgat ctcccccatt tccccgatgt 40
<210> 240
   <211> 42
   <212> DNA
   <213> Artificial Sequence
<220>
   <223> FASLG N2 forward primer
<400> 240
   cagacgtgtg ctcttccgat ctgcatatcc tgagccatcg gt 42
<210> 241
   <211> 42
   <212> DNA
   <213> Artificial Sequence
<220>
   <223> FOSL1 N2 forward primer
<400> 241
   cagacgtgtg ctcttccgat ctggtgattg gaccaggcca tt 42
<210> 242
   <211> 41
   <212> DNA
   <213> Artificial Sequence
<220>
   <223> FOXP3 N2 forward primer
<400> 242
   cagacgtgtg ctcttccgat ctgggtctct tgagtcccgt g 41
<210> 243
   <211> 42
   <212> DNA
   <213> Artificial Sequence
<220>
   <223> GAPDH N2 forward primer
<400> 243
   cagacgtgtg ctcttccgat ctgccctcaa cgaccacttt gt 42
<210> 244
   <211> 42
   <212> DNA
   <213> Artificial Sequence
<220>
   <223> GZMA N2 forward primer
<400> 244
   cagacgtgtg ctcttccgat ctcctttgtt gtgcgagggt gt 42
<210> 245
   <211> 42
   <212> DNA
   <213> Artificial Sequence
<220>
   <223> GZMB N2 forward primer
<400> 245
   cagacgtgtg ctcttccgat ctaggccctc ttgtgtgtaa ca 42
<210> 246
   <211> 43
   <212> DNA
   <213> Artificial Sequence
<220>
   <223> GZMH N2 forward primer
<400> 246
   cagacgtgtg ctcttccgat ctccaaagaa gacacagacc ggt 43
<210> 247
   <211> 42
   <212> DNA
   <213> Artificial Sequence
<220>
   <223> GZMK N2 forward primer
<400> 247
   cagacgtgtg ctcttccgat ctaaagcaac cttgtcccgc ct 42
<210> 248
   <211> 42
   <212> DNA
   <213> Artificial Sequence
<220>
   <223> HLA-DRA N2 forward primer
<400> 248
   cagacgtgtg ctcttccgat ctgcctcttc gattgctccg ta 42
<210> 249
   <211> 42
   <212> DNA
   <213> Artificial Sequence
<220>
   <223> IFNG N2 forward primer
<400> 249
   cagacgtgtg ctcttccgat ctcctgcaat ctgagccagt gc 42
<210> 250
   <211> 43
   <212> DNA
   <213> Artificial Sequence
<220>
   <223> IL10 N2 forward primer
<400> 250
   cagacgtgtg ctcttccgat ctttcaattc ctctgggaat gtt 43
<210> 251
   <211> 44
   <212> DNA
   <213> Artificial Sequence
<220>
   <223> IL12A N2 forward primer
<400> 251
   cagacgtgtg ctcttccgat ctgaactagg gagggggaaa gaag 44
<210> 252
   <211> 42
   <212> DNA
   <213> Artificial Sequence
<220>
   <223> IL12B N2 forward primer
<400> 252
   cagacgtgtg ctcttccgat cttcctcacc cccacctctc ta 42
<210> 253
   <211> 42
   <212> DNA
   <213> Artificial Sequence
<220>
   <223> IL13 N2 forward primer
<400> 253
   cagacgtgtg ctcttccgat cttgctacct cactggggtc ct 42
<210> 254
   <211> 43
   <212> DNA
   <213> Artificial Sequence
<220>
   <223> IL17A N2 forward primer
<400> 254
   cagacgtgtg ctcttccgat ctgcccctca gagatcaaca gac 43
<210> 255
   <211> 42
   <212> DNA
   <213> Artificial Sequence
<220>
   <223> IL17F N2 forward primer
<400> 255
   cagacgtgtg ctcttccgat ctcttaccca gtgctctgca ac 42
<210> 256
   <211> 43
   <212> DNA
   <213> Artificial Sequence
<220>
   <223> IL1A N2 forward primer
<400> 256
   cagacgtgtg ctcttccgat ctgcattttg gtccaagttg tgc 43
<210> 257
   <211> 42
   <212> DNA
   <213> Artificial Sequence
<220>
   <223> IL1B N2 forward primer
<400> 257
   cagacgtgtg ctcttccgat ctacattctg atgagcaacc gc 42
<210> 258
   <211> 45
   <212> DNA
   <213> Artificial Sequence
<220>
   <223> IL2 N2 forward primer
<400> 258
   cagacgtgtg ctcttccgat ctaagcatca tctcaacact gactt 45
<210> 259
   <211> 42
   <212> DNA
   <213> Artificial Sequence
<220>
   <223> IL21 N2 forward primer
<400> 259
   cagacgtgtg ctcttccgat ctctgccagc tccagaagat gt 42
<210> 260
   <211> 42
   <212> DNA
   <213> Artificial Sequence
<220>
   <223> IL21R N2 forward primer
<400> 260
   cagacgtgtg ctcttccgat ctagacaaga gctggctcac ct 42
<210> 261
   <211> 43
   <212> DNA
   <213> Artificial Sequence
<220>
   <223> IL22 N2 forward primer
<400> 261
   cagacgtgtg ctcttccgat ctggaaacca atgccacttt tgt 43
<210> 262
   <211> 43
   <212> DNA
   <213> Artificial Sequence
<220>
   <223> IL23R N2 forward primer
<400> 262
   cagacgtgtg ctcttccgat ctctggccaa tatgctgaaa ccc 43
<210> 263
   <211> 42
   <212> DNA
   <213> Artificial Sequence
<220>
   <223> IL24 N2 forward primer
<400> 263
   cagacgtgtg ctcttccgat ctgcccagtg agactgtgtt gt 42
<210> 264
   <211> 42
   <212> DNA
   <213> Artificial Sequence
<220>
   <223> IL26 N2 forward primer
<400> 264
   cagacgtgtg ctcttccgat cttgtgtgtg gagtgggatg tg 42
<210> 265
   <211> 42
   <212> DNA
   <213> Artificial Sequence
<220>
   <223> IL3 N2 forward primer
<400> 265
   cagacgtgtg ctcttccgat ctatttcacc ttttcctgcg gc 42
<210> 266
   <211> 41
   <212> DNA
   <213> Artificial Sequence
<220>
   <223> IL31 N2 forward primer
<400> 266
   cagacgtgtg ctcttccgat ctgtgtggga actctgccgt g 41
<210> 267
   <211> 42
   <212> DNA
   <213> Artificial Sequence
<220>
   <223> IL32 N2 forward primer
<400> 267
   cagacgtgtg ctcttccgat cttgctctga accccaatcc tc 42
<210> 268
   <211> 42
   <212> DNA
   <213> Artificial Sequence
<220>
   <223> IL33R N2 forward primer
<400> 268
   cagacgtgtg ctcttccgat ctaggtacca aatgcctgtg cc 42
<210> 269
   <211> 42
   <212> DNA
   <213> Artificial Sequence
<220>
   <223> IL4 N2 forward primer
<400> 269
   cagacgtgtg ctcttccgat ctcgggcttg aattcctgtc ct 42
<210> 270
   <211> 42
   <212> DNA
   <213> Artificial Sequence
<220>
   <223> IL4R N2 forward primer
<400> 270
   cagacgtgtg ctcttccgat cttcctcgta tgcatggaac cc 42
<210> 271
   <211> 42
   <212> DNA
   <213> Artificial Sequence
<220>
   <223> IL5 N2 forward primer
<400> 271
   cagacgtgtg ctcttccgat ctaggcatac tgacactttg cc 42
<210> 272
   <211> 43
   <212> DNA
   <213> Artificial Sequence
<220>
   <223> IL6 N2 forward primer
<400> 272
   cagacgtgtg ctcttccgat ctggaaagtg gctatgcagt ttg 43
<210> 273
   <211> 42
   <212> DNA
   <213> Artificial Sequence
<220>
   <223> IL6R N2 forward primer
<400> 273
   cagacgtgtg ctcttccgat ctacagcatg tcacaaggct gt 42
<210> 274
   <211> 42
   <212> DNA
   <213> Artificial Sequence
<220>
   <223> IRF4 N2 forward primer
<400> 274
   cagacgtgtg ctcttccgat ctgcccccaa atgaaagctt ga 42
<210> 275
   <211> 43
   <212> DNA
   <213> Artificial Sequence
<220>
   <223> LAG3 N2 forward primer
<400> 275
   cagacgtgtg ctcttccgat ctctttggag aagacagtgg cga 43
<210> 276
   <211> 41
   <212> DNA
   <213> Artificial Sequence
<220>
   <223> LIF N2 forward primer
<400> 276
   cagacgtgtg ctcttccgat ctttgccggc tctccagagt a 41
<210> 277
   <211> 42
   <212> DNA
   <213> Artificial Sequence
<220>
   <223> LTA N2 forward primer
<400> 277
   cagacgtgtg ctcttccgat ctggagaaac agagacaggc cc 42
<210> 278
   <211> 42
   <212> DNA
   <213> Artificial Sequence
<220>
   <223> MCL1 N2 forward primer
<400> 278
   cagacgtgtg ctcttccgat cttttgctta gaaggatggc gc 42
<210> 279
   <211> 42
   <212> DNA
   <213> Artificial Sequence
<220>
   <223> MKI67 N2 forward primer
<400> 279
   cagacgtgtg ctcttccgat cttcctgccc caccaagatc at 42
<210> 280
   <211> 42
   <212> DNA
   <213> Artificial Sequence
<220>
   <223> MYC N2 forward primer
<400> 280
   cagacgtgtg ctcttccgat ctcaaccttg gctgagtctt ga 42
<210> 281
   <211> 42
   <212> DNA
   <213> Artificial Sequence
<220>
   <223> NKG2D N2 forward primer
<400> 281
   cagacgtgtg ctcttccgat ctccaccctc cacaggaaat tg 42
<210> 282
   <211> 42
   <212> DNA
   <213> Artificial Sequence
<220>
   <223> PRDM1 N2 forward primer
<400> 282
   cagacgtgtg ctcttccgat ctacatgtga atgttgagcc ca 42
<210> 283
   <211> 42
   <212> DNA
   <213> Artificial Sequence
<220>
   <223> PRF1 N2 forward primer
<400> 283
   cagacgtgtg ctcttccgat ctcatggcca cgttgtcatt gt 42
<210> 284
   <211> 47
   <212> DNA
   <213> Artificial Sequence
<220>
   <223> PTPRCv1 N2 forward primer
<400> 284
   cagacgtgtg ctcttccgat cttaccagga atggatgtcg ctaatca 47
<210> 285
   <211> 42
   <212> DNA
   <213> Artificial Sequence
<220>
   <223> PTPRCv2 N2 forward primer
<400> 285
   cagacgtgtg ctcttccgat cttagttggc tatgctggca tg 42
<210> 286
   <211> 43
   <212> DNA
   <213> Artificial Sequence
<220>
   <223> SAP30 N2 forward primer
<400> 286
   cagacgtgtg ctcttccgat cttcactagg agacgtggaa ttg 43
<210> 287
   <211> 43
   <212> DNA
   <213> Artificial Sequence
<220>
   <223> SELL N2 forward primer
<400> 287
   cagacgtgtg ctcttccgat ctcctgcccc cagacctttt atc 43
<210> 288
   <211> 42
   <212> DNA
   <213> Artificial Sequence
<220>
   <223> TBX21 N2 forward primer
<400> 288
   cagacgtgtg ctcttccgat ctagaaaagg ggctggaaag gg 42
<210> 289
   <211> 42
   <212> DNA
   <213> Artificial Sequence
<220>
   <223> TGFB1 N2 forward primer
<400> 289
   cagacgtgtg ctcttccgat ctaccttggg cactgttgaa gt 42
<210> 290
   <211> 43
   <212> DNA
   <213> Artificial Sequence
<220>
   <223> TNF N2 forward primer
<400> 290
   cagacgtgtg ctcttccgat ctggctcaga catgttttcc gtg 43
<210> 291
   <211> 42
   <212> DNA
   <213> Artificial Sequence
<220>
   <223> TNFRSF9 N2 forward primer
<400> 291
   cagacgtgtg ctcttccgat ctttttgatg tgaggggcgg at 42
<210> 292
   <211> 45
   <212> DNA
   <213> Artificial Sequence
<220>
   <223> TYMS N2 forward primer
<400> 292
   cagacgtgtg ctcttccgat ctatgtgcat ttcaatccca cgtac 45
<210> 293
   <211> 43
   <212> DNA
   <213> Artificial Sequence
<220>
   <223> UHRF1 N2 forward primer
<400> 293
   cagacgtgtg ctcttccgat ctccaaagtt tgcagcctat acc 43
<210> 294
   <211> 42
   <212> DNA
   <213> Artificial Sequence
<220>
   <223> ZBED2 N2 forward primer
<400> 294
   cagacgtgtg ctcttccgat ctggttttgg tggagctgac ga 42
<210> 295
   <211> 41
   <212> DNA
   <213> Artificial Sequence
<220>
   <223> ZNF683 N2 forward primer
<400> 295
   cagacgtgtg ctcttccgat ctatccacct gaagctgcac c 41
<210> 296
   <211> 46
   <212> DNA
   <213> Artificial Sequence
<220>
   <223> TRAV1-1 N2 forward primer
<400> 296
   cagacgtgtg ctcttccgat ctccttagtc gctctgatag ttatgg 46
<210> 297
   <211> 46
   <212> DNA
   <213> Artificial Sequence
<220>
   <223> TRAV1-2-01 N2 forward primer
<400> 297
   cagacgtgtg ctcttccgat ctcgaagcac ccacatttct gtctta 46
<210> 298
   <211> 46
   <212> DNA
   <213> Artificial Sequence
<220>
   <223> TRAV1-2-02 N2 forward primer
<400> 298
   cagacgtgtg ctcttccgat ctgtcggtct aaagggtaca gttacc 46
<210> 299
   <211> 46
   <212> DNA
   <213> Artificial Sequence
<220>
   <223> TRAV2 N2 forward primer
<400> 299
   cagacgtgtg ctcttccgat ctcagggacg atacaacatg acctat 46
<210> 300
   <211> 44
   <212> DNA
   <213> Artificial Sequence
<220>
   <223> TRAV3 N2 forward primer
<400> 300
   cagacgtgtg ctcttccgat ctaacaagag ccaaacctcc ttcc 44
<210> 301
   <211> 45
   <212> DNA
   <213> Artificial Sequence
<220>
   <223> TRAV4 N2 forward primer
<400> 301
   cagacgtgtg ctcttccgat ctagaaagtc cagcactctg agcct 45
<210> 302
   <211> 44
   <212> DNA
   <213> Artificial Sequence
<220>
   <223> TRAV5 N2 forward primer
<400> 302
   cagacgtgtg ctcttccgat ctcgcattgc agacacccag actg 44
<210> 303
   <211> 45
   <212> DNA
   <213> Artificial Sequence
<220>
   <223> TRAV6 N2 forward primer
<400> 303
   cagacgtgtg ctcttccgat ctggtcacct ttgataccac cctta 45
<210> 304
   <211> 46
   <212> DNA
   <213> Artificial Sequence
<220>
   <223> TRAV7 N2 forward primer
<400> 304
   cagacgtgtg ctcttccgat ctacagccgt gcagcctgaa gattca 46
<210> 305
   <211> 43
   <212> DNA
   <213> Artificial Sequence
<220>
   <223> TRAV8-1 N2 forward primer
<400> 305
   cagacgtgtg ctcttccgat ctaggaaacc ctctgtgcag tgg 43
<210> 306
   <211> 46
   <212> DNA
   <213> Artificial Sequence
<220>
   <223> TRAV8-2 N2 forward primer
<400> 306
   cagacgtgtg ctcttccgat ctcatatgag cgacgcggct gagtac 46
<210> 307
   <211> 42
   <212> DNA
   <213> Artificial Sequence
<220>
   <223> TRAV8-3 N2 forward primer
<400> 307
   cagacgtgtg ctcttccgat ctggaaaccc tctgtgcatt gg 42
<210> 308
   <211> 43
   <212> DNA
   <213> Artificial Sequence
<220>
   <223> TRAV8-4 N2 forward primer
<400> 308
   cagacgtgtg ctcttccgat cttatgagcg acgcggctga gta 43
<210> 309
   <211> 46
   <212> DNA
   <213> Artificial Sequence
<220>
   <223> TRAV8-5 N2 forward primer
<400> 309
   cagacgtgtg ctcttccgat ctgacactta tcacttcccc aatcaa 46
<210> 310
   <211> 46
   <212> DNA
   <213> Artificial Sequence
<220>
   <223> TRAV8-6 N2 forward primer
<400> 310
   cagacgtgtg ctcttccgat ctaccctcag tccatataag cgacac 46
<210> 311
   <211> 45
   <212> DNA
   <213> Artificial Sequence
<220>
   <223> TRAV8-7 N2 forward primer
<400> 311
   cagacgtgtg ctcttccgat ctaagaagag cgaaacctcc ttcta 45
<210> 312
   <211> 45
   <212> DNA
   <213> Artificial Sequence
<220>
   <223> TRAV9-1 N2 forward primer
<400> 312
   cagacgtgtg ctcttccgat ctgaagccat gtaccgtaaa gaaac 45
<210> 313
   <211> 44
   <212> DNA
   <213> Artificial Sequence
<220>
   <223> TRAV9-2 N2 forward primer
<400> 313
   cagacgtgtg ctcttccgat ctttggagaa aggctcagtt caag 44
<210> 314
   <211> 44
   <212> DNA
   <213> Artificial Sequence
<220>
   <223> TRAV10 N2 forward primer
<400> 314
   cagacgtgtg ctcttccgat ctagcctccc agctcagcga ttca 44
<210> 315
   <211> 45
   <212> DNA
   <213> Artificial Sequence
<220>
   <223> TRAV11 N2 forward primer
<400> 315
   cagacgtgtg ctcttccgat ctgtttggaa tatcgcagcc tctca 45
<210> 316
   <211> 44
   <212> DNA
   <213> Artificial Sequence
<220>
   <223> TRAV12-1 N2 forward primer
<400> 316
   cagacgtgtg ctcttccgat ctgagactcc aagctcagtg attc 44
<210> 317
   <211> 42
   <212> DNA
   <213> Artificial Sequence
<220>
   <223> TRAV12-2 N2 forward primer
<400> 317
   cagacgtgtg ctcttccgat ctgactccca gcccagtgat tc 42
<210> 318
   <211> 45
   <212> DNA
   <213> Artificial Sequence
<220>
   <223> TRAV12-3 N2 forward primer
<400> 318
   cagacgtgtg ctcttccgat ctaggtttac agcacaggtc gataa 45
<210> 319
   <211> 44
   <212> DNA
   <213> Artificial Sequence
<220>
   <223> TRAV13-1 N2 forward primer
<400> 319
   cagacgtgtg ctcttccgat ctcaaacatt tctccctgca catc 44
<210> 320
   <211> 47
   <212> DNA
   <213> Artificial Sequence
<220>
   <223> TRAV13-2 N2 forward primer
<400> 320
   cagacgtgtg ctcttccgat ctcagtgaaa catctctctc tgcaaat 47
<210> 321
   <211> 42
   <212> DNA
   <213> Artificial Sequence
<220>
   <223> TRAV14 N2 forward primer
<400> 321
   cagacgtgtg ctcttccgat ctatccgcca accttgtcat ct 42
<210> 322
   <211> 44
   <212> DNA
   <213> Artificial Sequence
<220>
   <223> TRAV15 N2 forward primer
<400> 322
   cagacgtgtg ctcttccgat ctggtctctc atcctggaga ttca 44
<210> 323
   <211> 45
   <212> DNA
   <213> Artificial Sequence
<220>
   <223> TRAV16 N2 forward primer
<400> 323
   cagacgtgtg ctcttccgat ctgagacatc tttccacctg aagaa 45
<210> 324
   <211> 46
   <212> DNA
   <213> Artificial Sequence
<220>
   <223> TRAV17 N2 forward primer
<400> 324
   cagacgtgtg ctcttccgat cttccaagaa aagcagttcc ttgttg 46
<210> 325
   <211> 45
   <212> DNA
   <213> Artificial Sequence
<220>
   <223> TRAV18 N2 forward primer
<400> 325
   cagacgtgtg ctcttccgat ctagaggttt tcaggccagt cctat 45
<210> 326
   <211> 46
   <212> DNA
   <213> Artificial Sequence
<220>
   <223> TRAV19 N2 forward primer
<400> 326
   cagacgtgtg ctcttccgat ctacaagtcg tggactcagc agtata 46
<210> 327
   <211> 41
   <212> DNA
   <213> Artificial Sequence
<220>
   <223> TRAV20 N2 forward primer
<400> 327
   cagacgtgtg ctcttccgat cttgcacatc acagccccta a 41
<210> 328
   <211> 46
   <212> DNA
   <213> Artificial Sequence
<220>
   <223> TRAV21 N2 forward primer
<400> 328
   cagacgtgtg ctcttccgat ctatgcctcg ctggataaat catcag 46
<210> 329
   <211> 43
   <212> DNA
   <213> Artificial Sequence
<220>
   <223> TRAV22 N2 forward primer
<400> 329
   cagacgtgtg ctcttccgat ctctcttccc agaccacaga ctc 43
<210> 330
   <211> 46
   <212> DNA
   <213> Artificial Sequence
<220>
   <223> TRAV23 N2 forward primer
<400> 330
   cagacgtgtg ctcttccgat ctgccaagca gttctcattg catatc 46
<210> 331
   <211> 46
   <212> DNA
   <213> Artificial Sequence
<220>
   <223> TRAV24 N2 forward primer
<400> 331
   cagacgtgtg ctcttccgat ctaccaagga gggttacagc tatttg 46
<210> 332
   <211> 45
   <212> DNA
   <213> Artificial Sequence
<220>
   <223> TRAV25 N2 forward primer
<400> 332
   cagacgtgtg ctcttccgat ctcacatcac agccacccag actac 45
<210> 333
   <211> 46
   <212> DNA
   <213> Artificial Sequence
<220>
   <223> TRAV26-1 N2 forward primer
<400> 333
   cagacgtgtg ctcttccgat ctgacagaaa gtccagcacc ttgatc 46
<210> 334
   <211> 43
   <212> DNA
   <213> Artificial Sequence
<220>
   <223> TRAV26-2 N2 forward primer
<400> 334
   cagacgtgtg ctcttccgat cttctctggc aatcgctgaa gac 43
<210> 335
   <211> 45
   <212> DNA
   <213> Artificial Sequence
<220>
   <223> TRAV27 N2 forward primer
<400> 335
   cagacgtgtg ctcttccgat ctaaggacag ttctctccac atcac 45
<210> 336
   <211> 46
   <212> DNA
   <213> Artificial Sequence
<220>
   <223> TRAV28 N2 forward primer
<400> 336
   cagacgtgtg ctcttccgat ctactttatg gccacctata catcag 46
<210> 337
   <211> 47
   <212> DNA
   <213> Artificial Sequence
<220>
   <223> TRAV29 N2 forward primer
<400> 337
   cagacgtgtg ctcttccgat ctcagcctgg agactctgca gtgtact 47
<210> 338
   <211> 42
   <212> DNA
   <213> Artificial Sequence
<220>
   <223> TRAV30 N2 forward primer
<400> 338
   cagacgtgtg ctcttccgat ctcagcaaag ctccctgtac ct 42
<210> 339
   <211> 45
   <212> DNA
   <213> Artificial Sequence
<220>
   <223> TRAV31 N2 forward primer
<400> 339
   cagacgtgtg ctcttccgat ctcacagcca gaagacctgc aacat 45
<210> 340
   <211> 46
   <212> DNA
   <213> Artificial Sequence
<220>
   <223> TRAV32 N2 forward primer
<400> 340
   cagacgtgtg ctcttccgat ctaacatgtc tccctgcata ttacag 46
<210> 341
   <211> 46
   <212> DNA
   <213> Artificial Sequence
<220>
   <223> TRAV33 N2 forward primer
<400> 341
   cagacgtgtg ctcttccgat ctttcatcaa cctcaccatc aattcc 46
<210> 342
   <211> 45
   <212> DNA
   <213> Artificial Sequence
<220>
   <223> TRAV34 N2 forward primer
<400> 342
   cagacgtgtg ctcttccgat ctcagcaaag ttccctgcat atcac 45
<210> 343
   <211> 44
   <212> DNA
   <213> Artificial Sequence
<220>
   <223> TRAV35 N2 forward primer
<400> 343
   cagacgtgtg ctcttccgat ctataaccag aaaggacagc ttcc 44
<210> 344
   <211> 47
   <212> DNA
   <213> Artificial Sequence
<220>
   <223> TRAV36 N2 forward primer
<400> 344
   cagacgtgtg ctcttccgat ctatcacagc cacccagacc ggagact 47
<210> 345
   <211> 44
   <212> DNA
   <213> Artificial Sequence
<220>
   <223> TRAV37 N2 forward primer
<400> 345
   cagacgtgtg ctcttccgat ctagattcac agccaggctt aaaa 44
<210> 346
   <211> 43
   <212> DNA
   <213> Artificial Sequence
<220>
   <223> TRAV38-1 N2 forward primer
<400> 346
   cagacgtgtg ctcttccgat ctttccagaa agcagccaaa tcc 43
<210> 347
   <211> 44
   <212> DNA
   <213> Artificial Sequence
<220>
   <223> TRAV38-2 N2 forward primer
<400> 347
   cagacgtgtg ctcttccgat ctcttccaga aagcagccaa atcc 44
<210> 348
   <211> 42
   <212> DNA
   <213> Artificial Sequence
<220>
   <223> TRAV39 N2 forward primer
<400> 348
   cagacgtgtg ctcttccgat ctctcagcac cctccacatc ac 42
<210> 349
   <211> 46
   <212> DNA
   <213> Artificial Sequence
<220>
   <223> TRAV40 N2 forward primer
<400> 349
   cagacgtgtg ctcttccgat ctcaaaaact tcggaggcgg aaatat 46
<210> 350
   <211> 46
   <212> DNA
   <213> Artificial Sequence
<220>
   <223> TRAV41 N2 forward primer
<400> 350
   cagacgtgtg ctcttccgat ctaaagcaca gctccctgca catcac 46
<210> 351
   <211> 53
   <212> DNA
   <213> Artificial Sequence
<220>
   <223> TRBV2? N2 forward primer
<400> 351
   cagacgtgtg ctcttccgat cttcaaattt cactctgaag atccggtcca caa 53
<210> 352
   <211> 53
   <212> DNA
   <213> Artificial Sequence
<220>
   <223> TRBV3-1 N2 forward primer
<400> 352
   cagacgtgtg ctcttccgat ctgctcactt aaatcttcac atcaattccc tgg 53
<210> 353
   <211> 48
   <212> DNA
   <213> Artificial Sequence
<220>
   <223> TRBV4-1 N2 forward primer
<400> 353
   cagacgtgtg ctcttccgat ctcttaaacc ttcacctaca cgccctgc 48
<210> 354
   <211> 48
   <212> DNA
   <213> Artificial Sequence
<220>
   <223> TRBV(4-2, 4-3)? N2 forward primer
<400> 354
   cagacgtgtg ctcttccgat ctcttattcc ttcacctaca caccctgc 48
<210> 355
   <211> 48
   <212> DNA
   <213> Artificial Sequence
<220>
   <223> TRBV5-1? N2 forward primer
<400> 355
   cagacgtgtg ctcttccgat ctgctctgag atgaatgtga gcaccttg 48
<210> 356
   <211> 48
   <212> DNA
   <213> Artificial Sequence
<220>
   <223> TRBV5-3? N2 forward primer
<400> 356
   cagacgtgtg ctcttccgat ctgctctgag atgaatgtga gtgccttg 48
<210> 357
   <211> 48
   <212> DNA
   <213> Artificial Sequence
<220>
   <223> TRBV(5-4, 5-5, 5-6, 5-7, 5-8) N2 forward primer
<400> 357
   cagacgtgtg ctcttccgat ctgctctgag ctgaatgtga acgccttg 48
<210> 358
   <211> 44
   <212> DNA
   <213> Artificial Sequence
<220>
   <223> TRBV6-1 N2 forward primer
<400> 358
   cagacgtgtg ctcttccgat cttcgctcag gctggagtcg gctg 44
<210> 359
   <211> 42
   <212> DNA
   <213> Artificial Sequence
<220>
   <223> TRBV(6-2, 6-3) N2 forward primer
<400> 359
   cagacgtgtg ctcttccgat ctgctggggt tggagtcggc tg 42
<210> 360
   <211> 43
   <212> DNA
   <213> Artificial Sequence
<220>
   <223> TRBV6-4? N2 forward primer
<400> 360
   cagacgtgtg ctcttccgat ctccctcacg ttggcgtctg ctg 43
<210> 361
   <211> 42
   <212> DNA
   <213> Artificial Sequence
<220>
   <223> TRBV6-5? N2 forward primer
<400> 361
   cagacgtgtg ctcttccgat ctgctcaggc tgctgtcggc tg 42
<210> 362
   <211> 43
   <212> DNA
   <213> Artificial Sequence
<220>
   <223> TRBV6-6? N2 forward primer
<400> 362
   cagacgtgtg ctcttccgat ctcgctcagg ctggagttgg ctg 43
<210> 363
   <211> 44
   <212> DNA
   <213> Artificial Sequence
<220>
   <223> TRBV6-7? N2 forward primer
<400> 363
   cagacgtgtg ctcttccgat ctcccctcaa gctggagtca gctg 44
<210> 364
   <211> 43
   <212> DNA
   <213> Artificial Sequence
<220>
   <223> TRBV6-8 N2 forward primer
<400> 364
   cagacgtgtg ctcttccgat ctcactcagg ctggtgtcgg ctg 43
<210> 365
   <211> 43
   <212> DNA
   <213> Artificial Sequence
<220>
   <223> ?TRBV6-9? N2 forward primer
<400> 365
   cagacgtgtg ctcttccgat ctcgctcagg ctggagtcag ctg 43
<210> 366
   <211> 46
   <212> DNA
   <213> Artificial Sequence
<220>
   <223> TRBV7-1? N2 forward primer
<400> 366
   cagacgtgtg ctcttccgat ctccactctg aagttccagc gcacac 46
<210> 367
   <211> 45
   <212> DNA
   <213> Artificial Sequence
<220>
   <223> TRBV7-2? N2 forward primer
<400> 367
   cagacgtgtg ctcttccgat ctcactctga cgatccagcg cacac 45
<210> 368
   <211> 48
   <212> DNA
   <213> Artificial Sequence
<220>
   <223> TRBV7-3? N2 forward primer
<400> 368
   cagacgtgtg ctcttccgat ctctctactc tgaagatcca gcgcacag 48
<210> 369
   <211> 46
   <212> DNA
   <213> Artificial Sequence
<220>
   <223> TRBV7-4? N2 forward primer
<400> 369
   cagacgtgtg ctcttccgat ctccactctg aagatccagc gcacag 46
<210> 370
   <211> 45
   <212> DNA
   <213> Artificial Sequence
<220>
   <223> TRBV7-6? N2 forward primer
<400> 370
   cagacgtgtg ctcttccgat ctcactctga cgatccagcg cacag 45
<210> 371
   <211> 46
   <212> DNA
   <213> Artificial Sequence
<220>
   <223> TRBV7-7? N2 forward primer
<400> 371
   cagacgtgtg ctcttccgat ctccactctg acgattcagc gcacag 46
<210> 372
   <211> 46
   <212> DNA
   <213> Artificial Sequence
<220>
   <223> TRBV7-8? N2 forward primer
<400> 372
   cagacgtgtg ctcttccgat ctccactctg aagatccagc gcacac 46
<210> 373
   <211> 45
   <212> DNA
   <213> Artificial Sequence
<220>
   <223> TRBV7-9? N2 forward primer
<400> 373
   cagacgtgtg ctcttccgat ctcaccttgg agatccagcg cacag 45
<210> 374
   <211> 50
   <212> DNA
   <213> Artificial Sequence
<220>
   <223> TRBV9? N2 forward primer
<400> 374
   cagacgtgtg ctcttccgat ctgcactctg aactaaacct gagctctctg 50
<210> 375
   <211> 44
   <212> DNA
   <213> Artificial Sequence
<220>
   <223> TRBV10-1 N2 forward primer
<400> 375
   cagacgtgtg ctcttccgat ctcccctcac tctggagtct gctg 44
<210> 376
   <211> 45
   <212> DNA
   <213> Artificial Sequence
<220>
   <223> TRBV10-2? N2 forward primer
<400> 376
   cagacgtgtg ctcttccgat ctccccctca ctctggagtc agcta 45
<210> 377
   <211> 45
   <212> DNA
   <213> Artificial Sequence
<220>
   <223> TRBV10-3 N2 forward primer
<400> 377
   cagacgtgtg ctcttccgat ctcctcctca ctctggagtc cgcta 45
<210> 378
   <211> 46
   <212> DNA
   <213> Artificial Sequence
<220>
   <223> TRBV(11-1, 11-3) N2 forward primer
<400> 378
   cagacgtgtg ctcttccgat ctccactctc aagatccagc ctgcag 46
<210> 379
   <211> 48
   <212> DNA
   <213> Artificial Sequence
<220>
   <223> TRBV11-2 N2 forward primer
<400> 379
   cagacgtgtg ctcttccgat ctctccactc tcaagatcca gcctgcaa 48
<210> 380
   <211> 46
   <212> DNA
   <213> Artificial Sequence
<220>
   <223> TRBV(12-3, 12-4, 12-5) N2 forward primer
<400> 380
   cagacgtgtg ctcttccgat ctccactctg aagatccagc cctcag 46
<210> 381
   <211> 50
   <212> DNA
   <213> Artificial Sequence
<220>
   <223> TRBV13 N2 forward primer
<400> 381
   cagacgtgtg ctcttccgat ctcattctga actgaacatg agctccttgg 50
<210> 382
   <211> 46
   <212> DNA
   <213> Artificial Sequence
<220>
   <223> TRBV14 N2 forward primer
<400> 382
   cagacgtgtg ctcttccgat ctctactctg aaggtgcagc ctgcag 46
<210> 383
   <211> 50
   <212> DNA
   <213> Artificial Sequence
<220>
   <223> TRBV15 N2 forward primer
<400> 383
   cagacgtgtg ctcttccgat ctgataactt ccaatccagg aggccgaaca 50
<210> 384
   <211> 48
   <212> DNA
   <213> Artificial Sequence
<220>
   <223> TRBV16 N2 forward primer
<400> 384
   cagacgtgtg ctcttccgat ctctgtagcc ttgagatcca ggctacga 48
<210> 385
   <211> 46
   <212> DNA
   <213> Artificial Sequence
<220>
   <223> TRBV17 N2 forward primer
<400> 385
   cagacgtgtg ctcttccgat ctcttccacg ctgaagatcc atcccg 46
<210> 386
   <211> 46
   <212> DNA
   <213> Artificial Sequence
<220>
   <223> TRBV18 N2 forward primer
<400> 386
   cagacgtgtg ctcttccgat ctgcatcctg aggatccagc aggtag 46
<210> 387
   <211> 44
   <212> DNA
   <213> Artificial Sequence
<220>
   <223> TRBV19 N2 forward primer
<400> 387
   cagacgtgtg ctcttccgat ctcctctcac tgtgacatcg gccc 44
<210> 388
   <211> 48
   <212> DNA
   <213> Artificial Sequence
<220>
   <223> TRBV20-1 N2 forward primer
<400> 388
   cagacgtgtg ctcttccgat ctcttgtcca ctctgacagt gaccagtg 48
<210> 389
   <211> 45
   <212> DNA
   <213> Artificial Sequence
<220>
   <223> TRBV23-1 N2 forward primer
<400> 389
   cagacgtgtg ctcttccgat ctcagcctgg caatcctgtc ctcag 45
<210> 390
   <211> 46
   <212> DNA
   <213> Artificial Sequence
<220>
   <223> TRBV24-1 N2 forward primer
<400> 390
   cagacgtgtg ctcttccgat ctctccctgt ccctagagtc tgccat 46
<210> 391
   <211> 43
   <212> DNA
   <213> Artificial Sequence
<220>
   <223> TRBV25-1 N2 forward primer
<400> 391
   cagacgtgtg ctcttccgat ctccctgacc ctggagtctg cca 43
<210> 392
   <211> 43
   <212> DNA
   <213> Artificial Sequence
<220>
   <223> TRBV27 N2 forward primer
<400> 392
   cagacgtgtg ctcttccgat ctccctgatc ctggagtcgc cca 43
<210> 393
   <211> 45
   <212> DNA
   <213> Artificial Sequence
<220>
   <223> TRBV28 N2 forward primer
<400> 393
   cagacgtgtg ctcttccgat ctctccctga ttctggagtc cgcca 45
<210> 394
   <211> 53
   <212> DNA
   <213> Artificial Sequence
<220>
   <223> TRBV29-1 N2 forward primer
<400> 394
   cagacgtgtg ctcttccgat ctctaacatt ctcaactctg actgtgagca aca 53
<210> 395
   <211> 50
   <212> DNA
   <213> Artificial Sequence
<220>
   <223> TRBV30 N2 forward primer
<400> 395
   cagacgtgtg ctcttccgat ctcggcagtt catcctgagt tctaagaagc 50
<210> 396
   <211> 28
   <212> DNA
   <213> Artificial Sequence
<220>
   <223> IgG CH1 Reverse Heavy Chain
<400> 396
   accaggcatc ccagggtcac catggagt 28
<210> 397
   <211> 27
   <212> DNA
   <213> Artificial Sequence
<220>
   <223> 701/mIGHV5-6-5*01 Heavy Chain
<400> 397
   gaagtgaagc tggtggagtc tggggga 27
<210> 398
   <211> 27
   <212> DNA
   <213> Artificial Sequence
<220>
   <223> 702/mIGHV1-72*01 Heavy Chain
<400> 398
   caggtccaac tgcagcagcc tggggct 27
<210> 399
   <211> 27
   <212> DNA
   <213> Artificial Sequence
<220>
   <223> 703/mIGHV1-9*01 Heavy Chain
<400> 399
   caggttcagc tgcagcagtc tggagct 27
<210> 400
   <211> 27
   <212> DNA
   <213> Artificial Sequence
<220>
   <223> 704/mIGHV1-14*01 Heavy Chain
<400> 400
   gagttccagc tgcagcagtc tggacct 27
<210> 401
   <211> 27
   <212> DNA
   <213> Artificial Sequence
<220>
   <223> 705/mIGHV14-3*02 Heavy Chain
<400> 401
   gaggttcagc tgcagcagtc tggggca 27
<210> 402
   <211> 27
   <212> DNA
   <213> Artificial Sequence
<220>
   <223> 706/mIGHV9-3-1*01 Heavy Chain
<400> 402
   cagatccagt tggtgcagtc tggacct 27
<210> 403
   <211> 27
   <212> DNA
   <213> Artificial Sequence
<220>
   <223> 707/mIGHV2-9-1*01 Heavy Chain
<400> 403
   caggtgcagc tgaaggagtc aggacct 27
<210> 404
   <211> 27
   <212> DNA
   <213> Artificial Sequence
<220>
   <223> 708/mIGHV3-6*02 Heavy Chain
<400> 404
   gatgtacagc ttcaggagtc aggacct 27
<210> 405
   <211> 27
   <212> DNA
   <213> Artificial Sequence
<220>
   <223> 772/IGHV8-12*01 Heavy Chain
<400> 405
   caggttactc tgaaagagtc tggccct 27
<210> 406
   <211> 27
   <212> DNA
   <213> Artificial Sequence
<220>
   <223> IgG CK Reverse Light Chain
<400> 406
   gaggcacctc cagatgttaa ctgctca 27
<210> 407
   <211> 24
   <212> DNA
   <213> Artificial Sequence
<220>
   <223> 728/mIGKV4-59*01 Light Chain
<400> 407
   caaattgttc tcacccagtc tcca 24
<210> 408
   <211> 24
   <212> DNA
   <213> Artificial Sequence
<220>
   <223> 729/mIGKV4-72*01 Light Chain
<400> 408
   caaattgttc tctcccagtc tcca 24
<210> 409
   <211> 24
   <212> DNA
   <213> Artificial Sequence
<220>
   <223> 730/mIGKV3-2*01 Light Chain
<400> 409
   gacattgtgc tgacccaatc tcca 24
<210> 410
   <211> 23
   <212> DNA
   <213> Artificial Sequence
<220>
   <223> 731/mIGKV6-23*01 Light Chain
<400> 410
   gacattgtga tgacccagtc tca 23
<210> 411
   <211> 24
   <212> DNA
   <213> Artificial Sequence
<220>
   <223> 732/mIGKV8-28*01 Light Chain
<400> 411
   gacattgtga tgacccagtc tcca 24
<210> 412
   <211> 24
   <212> DNA
   <213> Artificial Sequence
<220>
   <223> 733/mIGKV12-41*01 Light Chain
<400> 412
   gacatccaga tgactcagtc tcca 24
<210> 413
   <211> 24
   <212> DNA
   <213> Artificial Sequence
<220>
   <223> 734/mIGKV10-94*01 Light Chain
<400> 413
   gatatccaga tgacacagac taca 24
<210> 414
   <211> 24
   <212> DNA
   <213> Artificial Sequence
<220>
   <223> 735/mIGKV14-111*01 Light Chain
<400> 414
   gacatcaaga tgacccagtc tcca 24
<210> 415
   <211> 24
   <212> DNA
   <213> Artificial Sequence
<220>
   <223> 736/mIGKV1-110*01 Light Chain
<400> 415
   gatgttgtga tgacccaaac tcca 24
<210> 416
   <211> 24
   <212> DNA
   <213> Artificial Sequence
<220>
   <223> 766/IGKV2-137*01 Light Chain
<400> 416
   gatattgtga tgactcaggc tgca 24
<210> 417
   <211> 24
   <212> DNA
   <213> Artificial Sequence
<220>
   <223> 767/IGKV5-43*01 Light Chain
<400> 417
   gatattgtgc taactcagtc tcca 24
<210> 418
   <211> 24
   <212> DNA
   <213> Artificial Sequence
<220>
   <223> 768/IGKV11-125*01 Light Chain
<400> 418
   gatgtccaga tgattcagtc tcca 24
<210> 419
   <211> 24
   <212> DNA
   <213> Artificial Sequence
<220>
   <223> 769/IGKV13-84*01 Light Chain
<400> 419
   gacatccaga tgacacaatc ttca 24
<210> 420
   <211> 24
   <212> DNA
   <213> Artificial Sequence
<220>
   <223> 770/IGKV15-101*01 Light Chain
<400> 420
   aatacccaga tgaaccagac tcca 24
<210> 421
   <211> 24
   <212> DNA
   <213> Artificial Sequence
<220>
   <223> 771/IGKV18-36*01 Light Chain
<400> 421
   actggcgaaa caacacaggc tcca 24
<210> 422
   <211> 17
   <212> PRT
   <213> Artificial Sequence
<220>
   <223> TCRa CDR3
<400> 422
   Cys Ala Gly Gly Val Val Leu Arg Asn Ser Gly Asn Thr Pro Leu Val
    1               5                   10                  15
   Phe
<210> 423
   <211> 13
   <212> PRT
   <213> Artificial Sequence
<220>
   <223> TCRb CDR3
<400> 423
   Cys Ala Ser Ser Glu Gly Phe Gln Asp Thr Gln Tyr Phe
   1               5                   10